# EUROPÄISCHE PATENTSCHRIFT

(11) **EP 1 098 891 B1**
(45) Veröffentlichungstag und Bekanntmachung des Hinweises auf die Patenterteilung: **10.08.2005**
(21) Anmeldenummer: 99934568.9
(22) Anmeldetag: 03.07.1999
(51) Int. Cl.: C07D 277/60, C07D 513/04, A61K 31/428, A61K 31/429, C07D 417/04

(54) **POLYCYCLISCHE THIAZOLIDIN-2-YLIDEN AMINE, VERFAHREN ZU IHRER HERSTELLUNG UND IHRE VERWENDUNG ALS ARZNEIMITTEL**
POLYCYCLIC THIAZOLIDIN-2-YLIDENE AMINES, METHOD FOR THE PRODUCTION AND USE THEREOF AS MEDICAMENTS
AMINES THIAZOLIDIN-2-YLIDENES POLYCYCLIQUES, LEUR PROCEDE DE PRODUCTION ET LEUR UTILISATION COMME MEDICAMENTS

(30) Priorität: 17.07.1998 DE 19831878
(43) Veröffentlichungstag der Anmeldung: 16.05.2001
(73) Patentinhaber: Aventis Pharma Deutschland GmbH, 65929 Frankfurt am Main (DE)
(72) Erfinder: JÄHNE, Gerhard, D-65929 Frankfurt am Main (DE); GEISEN, Karl, D-60318 Frankfurt am Main (DE); LANG, Hans, Jochen, D-65719 Hofheim (DE)
(86) Internationale Anmeldenummer: PCT/EP1999/004644
(87) Internationale Veröffentlichungsnummer: WO 2000/004006

(56) Entgegenhaltungen:
- DE-A- 2 640 358
- US-A- 3 507 868

## Beschreibung

Die Erfindung betrifft polycyclische Thiazolidin-2-yliden Amine sowie deren physiologisch verträgliche Salze und physiologisch funktionelle Derivate.

Es sind bereits Thiazolidinderivate mit anorektischer Wirkung im Stand der Technik beschrieben (Österreichisches Patent Nr. 365181).

Der Erfindung lag die Aufgabe zugrunde, weitere Verbindungen zur Verfügung zu stellen, die eine therapeutisch verwertbare anorektische Wirkung entfalten. In diesem Zusammenhang bestand die Aufgabe insbesondere auch darin, Verbindungen zu finden, bei denen die anorektische Wirkung gegenüber den Verbindungen aus AT 365181 erhöht ist.

Die Erfindung betrifft daher polycyclische Thiazolidin-2-yliden Amine der Formel 1, 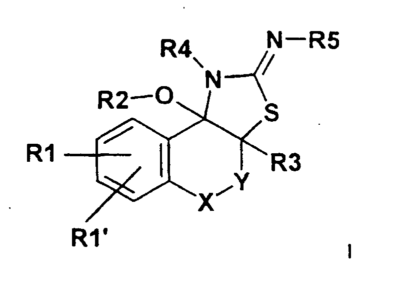 worin bedeuten
A)
   - Y: eine direkte Bindung, -CH₂-, -CH₂-CH₂-;
   - X: CH₂, CH(CH₃), CH(C₂H₅), CH(C₃H₇);
   - R1: CN, COOH, COO(C₁-C₆)Alkyl, CONH₂, CONH(C₁-C₆)Alkyl, CON[(C₁-C₆)Alkyl]₂, (C₂-C₆)-Alkyl; (C₂-C₆)-Alkenyl; (C₂-C₆)-Alkinyl; O-CH₂-CF₃, O-CH₂-CF₂-CF₃, O-(C₄-C₆)-Alkyl, wobei in den Alkylresten ein, mehrere, oder alle Wasserstoff(e) durch Fluor ersetzt sein können, oder ein Wasserstoff durch OH, OC(O)CH₃, O-CH₂-Ph, NH₂ oder N(COOCH₂Ph)₂ ersetzt sein kann;
   S-(C₁-C₆)-Alkyl, S-(CH₂)ₙ-Phenyl, SO-(C₁-C₆)-Alkyl, SO-(CH₂)ₙ-Phenyl, SO₂-(C₁-C₆)-Alkyl, SO₂-(CH₂)ₙ-Phenyl, wobei n = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl, NH₂ substituiert sein kann; NH₂, NH-(C₁-C₆)-Alkyl, N((C₁-C₆)-Alkyl)₂, NH(C₁-C₇)-Acyl, Phenyl, Biphenyl, O-(CH₂)ₙ-Phenyl, wobei n = 0 - 6 sein kann, 1- oder 2-Naphthyl, 2-, 3- oder 4-Pyridyl, 2- oder 3-Furanyl oder 2- oder 3-Thienyl, wobei die Phenyl-, Biphenyl-, Naphthyl-, Pyridyl-, Furanyl- oder Thienylringe jeweils ein bis 3-fach substituiert sein können mit F, Cl, Br, J, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂; 1,2,3-Triazol-5-yl, wobei der Triazolring in 1-, 2- oder 3-Stellung mit Methyl oder Benzyl substituiert sein kann;
   Tetrazol-5-yl, wobei der Tetrazolring in 1- oder 2-Stellung mit Methyl oder Benzyl substituiert sein kann;
   - R1': H, F, Cl, Br, J, CH₃, CF₃, O-(C₁-C₃)-Alkyl, NO₂, SO₂-NH₂, SO₂NH(C₁-C₆)-Alkyl, SO₂N[(C₁-C₆)-Alkyl]₂ oder R1;
   - R2: H, (C₁-C₆)-Alkyl, (C₃-C₆)-Cycloalkyl,(CH₂)ₙ-Phenyl, (CH₂)ₙ-Thienyl, (CH₂)ₙ-Pyridyl, (CH₂)ₙ-Furyl, C(O)-(CH₂)ₙ-Phenyl, C(O)-(CH₂)ₙ-Thienyl, C(O)-(CH₂)ₙ-Pyridyl, C(O)-(CH₂)ₙ-Furyl, wobei n = 0 - 5 sein kann und worin Phenyl, Thienyl, Pyridyl, Furyl jeweils bis zu zweimal mit Cl, F, CN, CF₃, (C₁-C₃)-Alkyl, OH, O-(C1-C₆)-Alkyl substituiert sein kann, C(O)-(C₁-C₆)-Alkyl, C(O)-(C₃-C₆)-Cycloalkyl;
   - R3: H, (C₁-C₆)-Alkyl, F, CN, N₃, O-(C₁-C₆)-Alkyl, CH₂-COO(C₁-C₆ Alkyl), CH₂-COO(C₃-C₈ Cycloalkyl), CH₂-COOH, CH₂-CONH₂, CH₂-CONHCH₃, CH₂-CON(CH₃)₂, (CH₂)ₙ-Phenyl, (CH₂)ₙ-Thienyl, (CH₂)ₙ-Pyridyl, (CH₂)ₙ-Furyl, wobei n = 0 - 5 sein kann und worin Phenyl, Thienyl, Pyridyl, Furyl jeweils bis zu zweimal mit Cl, F, CN, CF₃, (C₁-C₃)-Alkyl, OH, O-(C1-C₆)-Alkyl substituiert sein kann, (C₂-C₆)-Alkinyl, (C₂-C₆)-Alkenyl, C(O)OCH₃, C(O)OCH₂CH₃, C(O)OH, C(O)NH₂, C(O)NHCH₃, C(O)N(CH₃)₂, OC(O)CH₃;
   - R4: (C₁-C₆)-Alkyl, (C₃-C₆)-Cycloalkyl, (CH₂)ₙ-Phenyl, (CH₂)ₙ-Thienyl, (CH₂)ₙ-Pyridyl, (CH₂)ₙ-Furyl, wobei n = 0 - 5 sein kann und worin Phenyl, Thienyl, Pyridyl, Furyl jeweils bis zu zweimal mit Cl, F, CN, CF₃, (C₁-C₃)-Alkyl, OH, O-(C₁-C₆)-Alkyl substituiert sein kann;
   - R5: (C₁-C₆)-Alkyl, (C₃-C₆)-Cycloalkyl, (CH₂)ₙ-Phenyl, (CH₂)ₙ-Thienyl, (CH₂)ₙ-Pyridyl, (CH₂)ₙ-Furyl, wobei n = 0 - 5 sein kann und worin Phenyl, Thienyl, Pyridyl, Furyl jeweils bis zu zweimal mit Cl, F, CN, CF₃, (C₁-C₃)-Alkyl, OH, O-(C₁-C₆)-Alkyl substituiert sein kann;
   oder
   - R4 und R5: bilden gemeinsam eine -CH₂-CH₂-, -CH₂-C(CH₃)₂-, -CH₂-CH₂-CH₂-, oder -CH₂-CH₂-CH₂-CH₂- Gruppe;
   oder
B)
   - Y: eine direkte Bindung, -CH₂- oder -CH₂-CH₂-;
   - X: CH₂, CH(CH₃), CH(C₂H₅), CH(C₃H₇);
   - R1 und R1': unabhängig voneinander H, F, Cl, Br, J, NO₂, CN, COOH, COO(C₁-C₆)Alkyl, CONH₂, CONH(C₁-C₆)Alkyl, CON[(C₁-C₆)Alkyl]₂, (C₁-C₆)-Alkyl, (C₂-C₆)-Alkenyl, (C₂-C₆)-Alkinyl, O-(C₁-C₆)-Alkyl, O-CH₂-CF₃, O-CH₂-CF₂-CF₃, O-(C₄-C₆)-Alkyl, wobei in den Alkylresten ein, mehrere, oder alle Wasserstoff(e) durch Fluor ersetzt sein können oder ein Wasserstoff durch OH, OC(O)CH₃, O-CH₂-Ph, NH₂ oder N(COOCH₂Ph)₂ ersetzt sein kann;
   S-(C₁-C₆)-Alkyl, S-(CH₂)n-Phenyl, SO-(C₁-C₆)-Alkyl, SO-(CH₂)ₙ-Phenyl, SO₂-(C₁-C₆)-Alkyl, SO₂-(CH₂)ₙ-Phenyl, wobei n = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl, NH₂ substituiert sein kann; SO₂-NH₂, SO₂NH(C₁-C₆)-Alkyl, SO₂N[(C₁-C₆)-Alkyl]₂, NH₂, NH-(C₁-C₆)-Alkyl, N((C₁-C₆)-Alkyl)₂, NH(C₁-C₇)-Acyl, Phenyl, Biphenyl, O-(CH₂)ₙ-Phenyl, wobei n = 0 - 6 sein kann, 1- oder 2-Naphthyl, 2-, 3- oder 4-Pyridyl, 2- oder 3-Furanyl oder 2- oder 3-Thienyl, wobei die Phenyl-, Biphenyl-, Naphthyl-, Pyridyl-, Furanyl- oder Thienylringe jeweils ein bis 3-fach substituiert sein können mit F, Cl, Br, J, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)_{2,} SO₂-CH₃, COOH, COO-(C₁-C₆)-Alkyl, CONH₂; 1,2,3-Triazol-5-yl, wobei der Triazolring in 1-, 2- oder 3-Stellung mit Methyl oder Benzyl substituiert sein kann;
   Tetrazol-5-yl, wobei der Tetrazolring in 1- oder 2-Stellung mit Methyl oder Benzyl substituiert sein kann;
   - R2: H, (C₁-C₆)-Alkyl, (C₃-C₆)-Cycloalkyl, (CH₂)ₙ-Phenyl, (CH₂)ₙ-Thienyl, (CH₂)ₙ-Pyridyl, (CH₂)ₙ-Furyl, C(O)-(CH₂)ₙ-Phenyl, C(O)-(CH₂)ₙ-Thienyl, C(O)-(CH₂)ₙ-Pyridyl, C(O)-(CH₂)ₙ-Furyl, wobei n = 0 - 5 sein kann und worin Phenyl, Thienyl, Pyridyl, Furyl jeweils bis zu zweimal mit Cl, F, CN, CF₃, (C₁-C₃)-Alkyl, OH, O-(C1-C₆)-Alkyl substituiert sein kann, C(O)-(C₁-C₆)-Alkyl, C(O)-(C₃-C₆)-Cycloalkyl;
   - R3: (C₄-C₆)-Alkyl, F, CN, N₃, O-(C₁-C₆)-Alkyl, CH₂-COO(C₁-C₆ Alkyl), CH₂-COO(C₃-C₈ Cycloalkyl), CH₂-COOH, CH₂-CONH₂, CH₂-CONHCH₃, CH₂-CON(CH₃)₂, (CH₂)ₙ-Phenyl, (CH₂)ₙ-Thienyl, (CH₂)ₙ-Pyridyl, (CH₂)ₙ-Furyl, wobei n = 0 - 5 sein kann und worin Phenyl, Thienyl, Pyridyl, Furyl jeweils bis zu zweimal mit Cl, F, CN, CF₃, (C₁-C₃)-Alkyl, OH, O-(C1-C₆)-Alkyl substituiert sein kann, (C₂-C₆)-Alkinyl, (C₂-C₆)-Alkenyl, C(O)OCH₃, C(O)OCH₂CH₃, C(O)OH, C(O)NH₂, C(O)NHCH₃, C(O)N(CH₃)₂, OC(O)CH_{3:}
   - R4: (C₁-C₆)-Alkyl, (C₃-C₆)-Cycloalkyl, (CH₂)ₙ-Phenyl, (CH₂)ₙ-Thienyl, (CH₂)ₙ-Pyridyl, (CH₂)ₙ-Furyl, wobei n = 0 - 5 sein kann und worin Phenyl, Thienyl, Pyridyl, Furyl jeweils bis zu zweimal mit Cl, F, CN, CF₃, (C₁-C₃)-Alkyl, OH, O-(C1-C₆)-Alkyl substituiert sein kann;
   - R5: (C₁-C₆)-Alkyl, (C₃-C₆)-Cycloalkyl, (CH₂)ₙ-Phenyl, (CH₂)ₙ-Thienyl, (CH₂)ₙ-Pyridyl, (CH₂)ₙ-Furyl, wobei n = 0 - 5 sein kann und worin Phenyl, Thienyl, Pyridyl, Furyl jeweils bis zu zweimal mit Cl, F, CN, CF₃, (C₁-C₃)-Alkyl, OH, O-(C₁-C₆)-Alkyl substituiert sein kann;
   oder
   - R4 und R5: bilden gemeinsam eine -CH₂-CH₂-, -CH₂-C(CH₃)₂-, -CH₂-CH₂-CH₂-, oder -CH₂-CH₂-CH₂-CH₂- Gruppe;
   oder
C)
   - Y: eine direkte Bindung, -CH₂- oder -CH₂-CH₂-;
   - X: CH₂, CH(CH₃), CH(C₂H₅), CH(C₃H₇);
   - R1 und R1': unabhängig voneinander
   H, F, Cl, Br, J, NO₂, CN, COOH, COO(C₁-C₆)Alkyl, CONH₂, CONH(C₁-C₆)Alkyl, CON[(C₁-C₆)Alkyl]₂, (C₁-C₆)-Alkyl, (C₂-C₆)-Alkenyl, (C₂-C₆)-Alkinyl, O-(C₁-C₆)-Alkyl, O-CH₂-CF₃, O-CH₂-CF₂-CF₃, O-(C₄-C₆)-Alkyl, wobei in den Alkylresten ein, mehrere, oder alle Wasserstoff(e) durch Fluor ersetzt sein können oder ein Wasserstoff durch OH, OC(O)CH₃, O-CH₂-Ph, NH₂ oder N(COOCH₂Ph)₂ ersetzt sein kann;
   S-(C₁-C₆)-Alkyl, S-(CH₂)n-Phenyl, SO-(C₁-C₆)-Alkyl, SO-(CH₂)ₙ-Phenyl, SO₂-(C₁-C₆)-Alkyl, SO₂-(CH₂)n-Phenyl, wobei n = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl, NH₂ substituiert sein kann; SO₂-NH₂, SO₂NH(C₁-C₆)-Alkyl, SO₂N[(C₁-C₆)-Alkyl]₂, NH₂, NH-(C₁-C₆)-Alkyl, N((C₁-C₆)-Alkyl)₂, NH(C₁-C₇)-Acyl, Phenyl, Biphenyl, O-(CH₂)ₙ-Phenyl, wobei n = 0 - 6 sein kann, 1- oder 2-Naphthyl, 2-, 3- oder 4-Pyridyl, 2- oder 3-Furanyl oder 2- oder 3-Thienyl, wobei die Phenyl-, Biphenyl-, Naphthyl-, Pyridyl-, Furanyl- oder Thienylringe jeweils ein bis 3-fach substituiert sein können mit F, Cl, Br, J, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)_{2.} SO₂-CH₃, COOH, COO-(C₁-C₆)-Alkyl, CONH₂;
   1,2,3-Triazol-5-yl, wobei der Triazolring in 1-, 2- oder 3-Stellung mit Methyl oder Benzyl substituiert sein kann;
   Tetrazol-5-yl, wobei der Tetrazolring in 1- oder 2-Stellung mit Methyl oder Benzyl substituiert sein kann;
   - R2: (C₁-C₆)-Alkyl, (C₃-C₆)-Cycloalkyl, C₂-C₈ Alkenyl, C₂-C₈ Alkinyl, (CH₂)ₙ-Phenyl, (CH₂)ₙ-Thienyl, (CH₂)ₙ-Pyridyl, (CH₂)ₙ-Furyl, C(O)-(CH₂)ₙ-Phenyl, C(O)-(CH₂)ₙ-Thienyl, C(O)-(CH₂)ₙ-Pyridyl, C(O)-(CH₂)ₙ-Furyl, wobei n = 0 - 5 sein kann und worin Phenyl, Thienyl, Pyridyl, Furyl jeweils bis zu zweimal mit Cl, F, CN, CF₃, (C₁-C₃)-Alkyl, OH, O-(C1-C₆)-Alkyl substituiert sein kann, C(O)-(C₁-C₆)-Alkyl, C(O)-(C₃-C₆)-Cycloalkyl;
   - R3: H, (C₁-C₆)-Alkyl, F, CN, N₃, O-(C₁-C₆)-Alkyl, CH₂-COO(C₁-C₆ Alkyl), CH₂-COO(C₃-C₈ Cycloalkyl), CH₂-COOH, CH₂-CONH₂, CH₂-CONHCH₃, CH₂-CON(CH₃)₂, (CH₂)ₙ-Phenyl, (CH₂)ₙ-Thienyl, (CH₂)ₙ-Pyridyl, (CH₂)ₙFuryl, wobei n = 0 - 5 sein kann und worin Phenyl, Thienyl, Pyridyl, Furyl jeweils bis zu zweimal mit Cl. F, CN, CF₃, (C₁-C₃)-Alkyl, OH, O-(C1-C₆)-Alkyl substituiert sein kann, (C₂-C₆)-Alkinyl, (C₂-C₅)-Alkenyl, C(O)OCH₃, C(O)OCH₂CH₃, C(O)OH, C(O)NH₂, C(O)NHCH₃, C(O)N(CH₃)₂, OC(O)CH_{3;}
   - R4: (C₁-C₆)-Alkyl, (C₃-C₆)-Cycloalkyl, (CH₂)ₙ-Phenyl, (CH₂)ₙ-Thienyl, (CH₂)ₙ-Pyridyl, (CH₂)ₙ-Furyl, wobei n = 0 - 5 sein kann und worin Phenyl, Thienyl, Pyridyl, Furyl jeweils bis zu zweimal mit Cl, F, CN, CF₃, (C₁-C₃)-Alkyl, OH, O-(C1-C₆)-Alkyl substituiert sein kann;
   - R5: (C₁-C₆)-Alkyl, (C₃-C₆)-Cycloalkyl, (CH₂)ₙ-Phenyl, (CH₂)ₙ-Thienyl, (CH₂)ₙ-Pyridyl, (CH₂)ₙ-Furyl, wobei n = 0 - 5 sein kann und worin Phenyl, Thienyl, Pyridyl, Furyl jeweils bis zu zweimal mit Cl, F, CN, CF₃, (C₁-C₃)-Alkyl, OH, O-(C1-C₆)-Alkyl substituiert sein kann;
   oder
   - R4 und R5: bilden gemeinsam eine -CH₂-CH₂-, -CH₂-C(CH₃)₂-, -CH₂-CH₂-CH₂-, oder -CH₂-CH₂-CH₂-CH₂- Gruppe;
   oder
D)
   - Y: eine direkte Bindung, -CH₂- oder -CH₂-CH₂-;
   - X: CH(Phenyl), wobei der Phenylrest mit F, Cl, Br oder J substituiert sein kann,
   O, S, SO, SO₂ oder N-R6;
   - R1 und R1': unabhängig voneinander
   H, F, Cl, Br, J, NO₂, CN, COOH, COO(C₁-C₆)Alkyl, CONH₂, CONH(C₁-C₆)Alkyl, CON[(C₁-C₆)Alkyl]₂, (C₁-C₆)-Alkyl, (C₂-C₆)-Alkenyl, (C₂-C₆)-Alkinyl, O-(C₁-C₆)-Alkyl, O-CH₂-CF₃, O-CH₂-CF₂-CF₃, O-(C₄-C₆)-Alkyl, wobei in den Alkylresten ein, mehrere, oder alle Wasserstoff(e) durch Fluor ersetzt sein können oder ein Wasserstoff durch OH, OC(O)CH₃, O-CH₂-Ph, NH₂ oder N(COOCH₂Ph)₂ ersetzt sein kann;
   S-(C₁-C₅)-Alkyl, S-(CH₂)n-Phenyl, SO-(C₁-C₆)-Alkyl, SO-(CH₂)ₙ-Phenyl, SO₂-(C₁-C₆)-Alkyl, SO₂-(CH₂)n-Phenyl, wobei n = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl, NH₂ substituiert sein kann; SO₂-NH₂, SO₂NH(C₁-C₆)-Alkyl, SO₂N[(C₁-C₆)-Alkyl]₂, NH₂, NH-(C₁-C₆)-Alkyl, N((C₁-C₆)-Alkyl)₂, NH(C₁-C₇)-Acyl, Phenyl, Biphenyl, O-(CH₂)ₙ-Phenyl, wobei n = 0 - 6 sein kann, 1- oder 2-Naphthyl, 2-, 3- oder 4-Pyridyl, 2- oder 3-Furanyl oder 2- oder 3-Thienyl, wobei die Phenyl-, Biphenyl-, Naphthyl-, Pyridyl-, Furanyl- oder Thienylringe jeweils ein bis 3-fach substituiert sein können mit F, Cl, Br, J, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)_{2,} COOH, COO-(C₁-C₆)-Alkyl, CONH₂;
   1,2,3-Triazol-5-yl, wobei der Triazolring in 1-, 2- oder 3-Stellung mit Methyl oder Benzyl substituiert sein kann;
   Tetrazol-5-yl, wobei der Tetrazolring in 1- oder 2-Stellung mit Methyl oder Benzyl substituiert sein kann;
   - R2: H, (C₁-C₆)-Alkyl, (C₃-C₆)-Cycloalkyl, (CH₂)ₙ-Phenyl, (CH₂)ₙ-Thienyl, (CH₂)ₙ-Pyridyl, (CH₂)ₙ-Furyl, C(O)-(CH₂)ₙ-Phenyl, C(O)-(CH₂)ₙ-Thienyl, C(O)-(CH₂)ₙ-Pyridyl, C(O)-(CH₂)ₙ-Furyl, wobei n = 0 - 5 sein kann und worin Phenyl, Thienyl, Pyridyl, Furyl jeweils bis zu zweimal mit Cl, F, CN, CF₃, (C₁-C₃)-Alkyl, OH, O-(C1-C₆)-Alkyl substituiert sein kann, C(O)-(C₁-C₆)-Alkyl, C(O)-(C₃-C₆)-Cycloalkyl;
   - R3: H, (C₁-C₆)-Alkyl, F, CN, N₃, O-(C₁-C₆)-Alkyl, CH₂-COO(C₁-C₆ Alkyl), CH₂-COO(C₃-C₈ Cycloalkyl), CH₂-COOH, CH₂-CONH₂, CH₂-CONHCH₃, CH₂-CON(CH₃)₂, (CH₂)ₙ-Phenyl, (CH₂)ₙ-Thienyl, (CH₂)ₙ-Pyridyl, (CH₂)ₙ-Furyl, wobei n = 0 - 5 sein kann und worin Phenyl, Thienyl, Pyridyl, Furyl jeweils bis zu zweimal mit Cl, F, CN, CF₃, (C₁-C₃)-Alkyl, OH, O-(C1-C₆)-Alkyl substituiert sein kann, (C₂-C₆)-Alkinyl, (C₂-C₆)-Alkenyl, C(O)OCH₃, C(O)OCH₂CH₃, C(O)OH, C(O)NH₂, C(O)NHCH₃, C(O)N(CH₃)₂, OC(O)CH₃;
   - R4: (C₁-C₆)-Alkyl, (C₃-C₆)-Cycloalkyl, (CH₂)ₙ-Phenyl, (CH₂)ₙ-Thienyl, (CH₂)ₙ-Pyridyl, (CH₂)ₙ-Furyl, wobei n = 0 - 5 sein kann und worin Phenyl, Thienyl, Pyridyl, Furyl jeweils bis zu zweimal mit Cl, F, CN, CF₃, (C₁-C₃)-Alkyl, OH, O-(C1-C₆)-Alkyl substituiert sein kann;
   - R5: (C₁-C₆)-Alkyl, (C₃-C₆)-Cycloalkyl, (CH₂)ₙ-Phenyl, (CH₂)ₙ-Thienyl, (CH₂)ₙ-Pyridyl, (CH₂)ₙ-Furyl, wobei n = 0 - 5 sein kann und worin Phenyl, Thienyl, Pyridyl, Furyl jeweils bis zu zweimal mit Cl, F, CN, CF₃, (C₁-C₃)-Alkyl, OH, O-(C1-C₆)-Alkyl substituiert sein kann;
   oder
   - R4 und R5: bilden gemeinsam eine -CH₂-CH₂-, -CH₂-C(CH₃)₂-, -CH₂-CH₂-CH₂-, oder -CH₂-CH₂-CH₂-CH₂- Gruppe;
   - R6: SO₂-(C₆H₄-4-CH₃)
   oder
E)
   - Y: eine direkte Bindung, -CH₂- oder -CH₂-CH₂-;
   - X: CH₂, CH(CH₃), CH(C₂H₅), CH(C₃H₇);
   - R1: H, F, Cl, Br, J, CH₃, CF₃, O-(C₁-C₃)-Alkyl;
   - R1': H, F, Cl, Br, J, NO₂;
   - R2: H;
   - R3: H, (C₁-C₃)-Alkyl;
   - R4: Phenyl, wobei der Phenylrest bis zu zweifach substituiert sein kann mit F, Cl, Br, J, (C₁-C₆)-Alkyl, (C₃-C₆)-Cycloalkyl, O-(C₁-C₃)-Alkyl, CF₃, OCF₃, O-CH₂-Phenyl, COOH, COO(C₁-C₆)Alkyl, COO(C₃-C₆)-Cycloalkyl, CONH₂;
   - R5: Phenyl, wobei der Phenylrest bis zu zweifach substituiert sein kann mit F, Cl, Br, J, (C₁-C₆)-Alkyl, (C₃-C₆)-Cycloalkyl, O-(C₁-C₃)-Alkyl, CF₃, OCF₃, O-CH₂-Phenyl, COOH, COO(C₁-C₆)Alkyl, COO(C₃-C₆)-Cycloalkyl, CONH₂;
sowie deren physiologisch verträgliche Salze.

Bevorzugt sind Verbindungen der Formel I, worin bedeuten
A)
   - Y: eine direkte Bindung, -CH₂;
   - X: CH₂, CH(CH₃), CH(C₂H₅), CH(C₃H₇);
   - R1: CN, COOH, COO(C₁-C₆)Alkyl, CONH₂, CONH(C₁-C₆)Alkyl, CON[(C₁-C₆)Alkyl]_{2,} (C₂-C₆)-Alkyl; (C₂-C₆)-Alkenyl; (C₂-C₆)-Alkinyl; O-CH₂-CF₃, O-CH₂-CF₂-CF₃, O-(C₄-C₆)-Alkyl, wobei in den Alkylresten ein, mehrere, oder alle Wasserstoff(e) durch Fluor ersetzt sein können, oder ein Wasserstoff durch OH, OC(O)CH₃, O-CH₂-Ph, NH₂ oder N(COOCH₂Ph)₂ ersetzt sein kann;
   S-(C₁-C₆)-Alkyl, S-(CH₂)ₙ-Phenyl, SO-(C₁-C₆)-Alkyl, SO-(CH₂)ₙ-Phenyl, wobei der Phenylrest bis zu zweifach mit F, Cl, Br, OH, CF₃, NO₂, CN, OCF₃, O-(C₁-C₆)-Alkyl, (C₁-C₆)-Alkyl substituiert sein kann: NH-(C₁-C₆)-Alkyl, N((C₁-C₆)-Alkyl)₂, NH(C₁-C₇)-Acyl, Phenyl, O-(CH₂)ₙ-Phenyl, wobei n = 0 - 6 sein kann, 1- oder 2-Naphthyl, 2-. 3- oder 4-Pyridyl, 2- oder 3-Furanyl oder 2- oder 3-Thienyl, wobei die Phenyl-, Naphthyl-, Pyridyl-, Furanyl- oder Thienylringe jeweils ein bis 3-fach substituiert sein können mit F, Cl, J, OH, CF₃, CN, OCF₃, O-(C₁-C₆)-Alkyl, (C₁-C₆)-Alkyl, NH₂, NH(C₁-C₆)-Alkyl, N((C₁-C₆)-Alkyl)₂, COOH, COO-(C₁-C₆)-Alkyl, CONH₂;
   1,2,3-Triazol-5-yl, wobei der Triazolring in 1-, 2- oder 3-Stellung mit Methyl oder Benzyl substituiert sein kann;
   Tetrazol-5-yl, wobei der Tetrazolring in 1- oder 2-Stellung mit Methyl oder Benzyl substituiert sein kann;
   - R1': H, F, Cl, Br, J, CH₃, CF₃, O-(C₁-C₃)-Alkyl, SO₂-NH₂, SO₂NH(C₁-C₆)-Alkyl, SO₂N[(C₁-C₆)-Alkyl]₂ oder R1;
   - R2: H, (C₁-C₆)-Alkyl, (CH₂)ₙ-Phenyl, (CH₂)ₙ-Thienyl, (CH₂)ₙ-Pyridyl, C(O)-(CH₂)ₙ-Phenyl, C(O)-(CH₂)ₙ-Thienyl, C(O)-(CH₂)ₙ-Pyridyl, wobei n = 0 - 3 sein kann und worin Phenyl, Thienyl, Pyridyl, Furyl jeweils bis zu zweimal mit Cl, F, CN, CF₃, (C₁-C₃)-Alkyl, OH, O-(C₁-C₆)-Alkyl substituiert sein kann;
   - R3: H, (C₁-C₆)-Alkyl, F, CN, O-(C₁-C₆)-Alkyl, CH₂-COO(C₁-C₆ Alkyl), CH₂-COO(C₃-C₈ Cycloalkyl), CH₂-COOH, CH₂-CONH₂, CH₂-CONHCH₃, CH₂-CON(CH₃)₂, (CH₂)ₙ-Phenyl, (CH₂)ₙ-Thienyl, (CH₂)ₙ-Pyridyl, wobei n = 0 - 3 sein kann und worin Phenyl, Thienyl, Pyridyl jeweils bis zu zweimal mit Cl, F, CN, CF₃, (C₁-C₃)-Alkyl, OH, O-(C1-C₆)-Alkyl substituiert sein kann, (C₂-C₆)-Alkinyl, (C₂-C₆)-Alkenyl, C(O)OCH₃, C(O)OCH₂CH₃, C(O)OH, C(O)NH₂, C(O)N(CH₃)₂, OC(O)CH₃;
   - R4: (C₁-C₆)-Alkyl, (C₃-C₆)-Cycloalkyl, (CH₂)ₙ-Phenyl, (CH₂)ₙ-Thienyl, (CH₂)ₙ-Pyridyl, wobei n = 0 - 3 sein kann und worin Phenyl, Thienyl, Pyridyl jeweils bis zu zweimal mit Cl, F, CN. CF₃, (C₁-C₃)-Alkyl, OH, O-(C1-C₆)-Alkyl substituiert sein kann;
   - R5: (C₁-C₆)-Alkyl, (C₃-C₆)-Cycloalkyl, (CH₂)ₙ-Phenyl, (CH₂)ₙ-Thienyl, (CH₂)ₙ-Pyridyl, wobei n = 0 - 3 sein kann und worin Phenyl, Thienyl, Pyridyl jeweils bis zu zweimal mit Cl, F, CN, CF₃, (C₁-C₃)-Alkyl, OH, O-(C1-C₆)-Alkyl substituiert sein kann;
   oder
   - R4 und R5: bilden gemeinsam eine -CH₂-CH₂-, -CH₂-C(CH₃)₂- oder -CH₂-CH₂-CH₂- Gruppe;
   oder
B)
   - Y: eine direkte Bindung, -CH₂-;
   - X: CH₂, CH(CH₃), CH(C₂H₅), CH(C₃H₇);
   - R1 und R1': unabhängig voneinander
   H, F, Cl, Br, J, CN, COOH, COO(C₁-C₆)Alkyl, CONH₂, CONH(C₁-C₆)Alkyl, CON[(C₁-C₆)Alkyl]₂, (C₁-C₆)-Alkyl, (C₂-C₆)-Alkenyl, (C₂-C₆)-Alkinyl, O-(C₁-C₆)-Alkyl, O-CH₂-CF₃, O-CH₂-CF₂-CF₃, O-(C₄-C₆)-Alkyl, wobei in den Alkylresten ein, mehrere, oder alle Wasserstoff(e) durch Fluor ersetzt sein können oder ein Wasserstoff durch OH, OC(O)CH₃, O-CH₂-Ph; NH₂ oder N(COOCH₂Ph)₂ ersetzt sein kann;
   S-(C₁-C₆)-Alkyl, S-(CH₂)n-Phenyl, SO-(C₁-C₆)-Alkyl, SO-(CH₂)ₙ-Phenyl, wobei der Phenylrest bis zu zweifach mit F, Cl, Br, OH, CF₃, NO₂, CN, OCF₃, O-(C₁-C₆)-Alkyl, (C₁-C₆)-Alkyl substituiert sein kann;
   SO₂-NH₂, SO₂NH(C₁-C₆)-Alkyl, SO₂N[(C₁-C₆)-Alkyl]₂, NH-(C₁-C₆)-Alkyl, N((C₁-C₆)-Alkyl)₂, NH(C₁-C₇)-Acyl, Phenyl, O-(CH₂)ₙ-Phenyl, wobei n = 0 - 6 sein kann, 1- oder 2-Naphthyl, 2-, 3- oder 4-Pyridyl, 2- oder 3-Furanyl oder 2- oder 3-Thienyl, wobei die Phenyl-, Naphthyl-, Pyridyl-, Furanyl- oder Thienylringe jeweils ein bis 3-fach substituiert sein können mit F, Cl, J, OH, CF₃, CN, OCF₃, O-(C₁-C₅)-Alkyl, (C₁-C₆)-Alkyl, NH₂, NH(C₁-C₆)-Alkyl, N((C₁-C₆)-Alkyl)_{2.} COOH, COO-(C₁-C₆)-Alkyl, CONH₂; 1,2,3-Triazol-5-yl, wobei der Triazolring in 1-, 2- oder 3-Stellung mit Methyl oder Benzyl substituiert sein kann; Tetrazol-5-yl, wobei der Tetrazolring in 1- oder 2-Stellung mit Methyl oder Benzyl substituiert sein kann;
   - R2: H, (C₁-C₆)-Alkyl, (CH₂)ₙ-Phenyl, (CH₂)ₙ-Thienyl, (CH₂)ₙ-Pyridyl, C(O)-(CH₂)ₙ-Phenyl, C(O)-(CH₂)ₙ-Thienyl, C(O)-(CH₂)ₙ-Pyridyl, wobei n = 0 - 3 sein kann und worin Phenyl, Thienyl, Pyridyl, Furyl jeweils bis zu zweimal mit Cl, F, CN, CF₃, (C₁-C₃)-Alkyl, OH, O-(C₁-C₆)-Alkyl substituiert sein kann;
   - R3: (C₄-C₆)-Alkyl, F, CN, N₃, O-(C₁-C₆)-Alkyl, CH₂-COO(C₁-C₆ Alkyl), CH₂-COO(C₃-C₈ Cycloalkyl), CH₂-COOH, CH₂-CONH₂, CH₂-CONHCH₃, CH₂-CON(CH₃)₂, (CH₂)ₙ-Phenyl, (CH₂)ₙ-Thienyl, (CH₂)ₙ-Pyridyl, wobei n = 0 - 3 sein kann und worin Phenyl, Thienyl, Pyridyl jeweils bis zu zweimal mit Cl, F, CN, CF₃, (C₁-C₃)-Alkyl, OH, O-(C1-C₆)-Alkyl substituiert sein kann, (C₂-C₆)-Alkinyl, (C₂-C₆)-Alkenyl, C(O)OCH₃, C(O)OCH₂CH₃, C(O)OH, C(O)NH₂, C(O)NHCH₃, C(O)N(CH₃)₂, OC(O)CH₃;
   - R4: (C₁-C₆)-Alkyl, (C₃-C₆)-Cycloalkyl, (CH₂)ₙ-Phenyl, (CH₂)ₙ-Thienyl, (CH₂)ₙ-Pyridyl, wobei n = 0 - 3 sein kann und worin Phenyl, Thienyl, Pyridyl jeweils bis zu zweimal mit Cl, F, CN, CF₃, (C₁-C₃)-Alkyl, OH, O-(C1-C₆)-Alkyl substituiert sein kann;
   - R5: (C₁-C₆)-Alkyl, (C₃-C₆)-Cycloalkyl, (CH₂)ₙ-Phenyl, (CH₂)ₙ-Thienyl, (CH₂)ₙ Pyridyl, wobei n = 0 - 3 sein kann und worin Phenyl, Thienyl, Pyridyl jeweils bis zu zweimal mit Cl, F, CN, CF₃, (C₁-C₃)-Alkyl, OH, O-(C1-C₆)-Alkyl substituiert sein kann;
   oder
   - R4 und R5: bilden gemeinsam eine -CH₂-CH₂-, -CH₂-C(CH₃)₂- oder -CH₂-CH₂-CH₂- Gruppe;
   oder
C)
   - Y: eine direkte Bindung oder -CH₂-;
   - X: CH₂, CH(CH₃), CH(C₂H₅), CH(C₃H₇);
   - R1 und R1': unabhängig voneinander
   H, F, Cl, Br, J, CN, COOH, COO(C₁-C₆)Alkyl, CONH₂, CONH(C₁-C₆)Alkyl, CON[(C₁-C₆)Alkyl]₂, (C₁-C₆)-Alkyl, (C₂-C₆)-Alkenyl, (C₂-C₆)-Alkinyl, O-(C₁-C₆)-Alkyl, O-CH₂-CF₃, O-CH₂-CF₂-CF₃, O-(C₄-C₆)-Alkyl, wobei in den Alkylresten ein, mehrere, oder alle Wasserstoff(e) durch Fluor ersetzt sein können oder ein Wasserstoff durch OH, OC(O)CH₃, O-CH₂-Ph, NH₂ oder N(COOCH₂Ph)₂ ersetzt sein kann;
   S-(C₁-C₆)-Alkyl, S-(CH₂)ₙ-Phenyl, SO₂-(C₁-C₆)-Alkyl, SO₂-(CH₂)n-Phenyl, wobei n = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl substituiert sein kann; SO₂-NH₂, SO₂NH(C₁-C₆)-Alkyl, SO₂N[(C₁-C₆)-Alkyl]₂, NH-(C₁-C₆)-Alkyl, N((C₁-C₆)-Alkyl)₂, NH(C₁-C₇)-Acyl, Phenyl, O-(CH₂)ₙ-Phenyl, wobei n = 0 - 4 sein kann, 1- oder 2-Naphthyl, 2-, 3- oder 4-Pyridyl, 2- oder 3-Furanyl oder 2- oder 3-Thienyl, wobei die Phenyl-, Naphthyl-, Pyridyl-, Furanyl- oder Thienylringe jeweils ein bis 3-fach substituiert sein können mit F, Cl, J, OH, CF₃, CN, OCF₃, O-(C₁-C₆)-Alkyl, (C₁-C₆)-Alkyl, NH₂, NH(C₁-C₆)-Alkyl, N((C₁-C₆)-Alkyl)₂ COOH, COO-(C₁-C₆)-Alkyl, CONH₂;
   1,2,3-Triazol-5-yl, wobei der Triazolring in 1-, 2- oder 3-Stellung mit Methyl oder Benzyl substituiert sein kann;
   Tetrazol-5-yl, wobei der Tetrazolring in 1- oder 2-Stellung mit Methyl oder Benzyl substituiert sein kann;
   - R2: (C₁-C₆)-Alkyl, C₂-C₈ Alkenyl, C₂-C₈ Alkinyl, (CH₂)ₙ-Phenyl, (CH₂)ₙ-Thienyl, (CH₂)ₙ-Pyridyl, C(O)-(CH₂)ₙ-Phenyl, C(O)-(CH₂)ₙ-Thienyl, C(O)-(CH₂)ₙ-Pyridyl, wobei n = 0 - 3 sein kann und worin Phenyl, Thienyl, Pyridyl, Furyl jeweils bis zu zweimal mit Cl, F, CN, CF₃, (C₁-C₃)-Alkyl, OH, O-(C₁-C₆)-Alkyl substituiert sein kann;
   - R3: H, (C₁-C₆)-Alkyl, F, CN, O-(C₁-C₆)-Alkyl, CH₂-COO(C₁-C₆ Alkyl), CH₂-COO(C₃-C₈ Cycloalkyl), CH₂-COOH, CH₂-CONH₂, CH₂-CONHCH₃, CH₂-CON(CH₃)₂, (CH₂)ₙ-Phenyl, (CH₂)ₙ-Thienyl, (CH₂)ₙ-Pyridyl, wobei n = 0 - 3 sein kann und worin Phenyl, Thienyl, Pyridyl jeweils bis zu zweimal mit Cl, F, CN, CF₃, (C₁-C₃)-Alkyl, OH, O-(C1-C₆)-Alkyl substituiert sein kann, (C₂-C₆)-Alkinyl, (C₂-C₆)-Alkenyl, C(O)OCH₃, C(O)OCH₂CH₃, C(O)OH, C(O)NH₂, C(O)N(CH₃)₂, OC(O)CH₃;
   - R4: (C₁-C₆)-Alkyl, (C₃-C₆)-Cycloalkyl, (CH₂)ₙ-Phenyl, (CH₂)ₙ-Thienyl, (CH₂)ₙ-Pyridyl, wobei n = 0 - 3 sein kann und worin Phenyl, Thienyl, Pyridyl jeweils bis zu zweimal mit Cl, F, CN, CF₃, (C₁-C₃)-Alkyl, OH, O-(C1-C₆)-Alkyl substituiert sein kann;
   - R5: (C₁-C₆)-Alkyl, (C₃-C₆)-Cycloalkyl, (CH₂)ₙ-Phenyl, (CH₂)ₙ-Thienyl, (CH₂)ₙ-Pyridyl, wobei n = 0 - 3 sein kann und worin Phenyl, Thienyl, Pyridyl jeweils bis zu zweimal mit Cl, F, CN, CF₃, (C₁-C₃)-Alkyl, OH, O-(C1-C₆)-Alkyl substituiert sein kann;
   oder
   - R4 und R5: bilden gemeinsam eine -CH₂-CH₂-, -CH₂-C(CH₃)₂- oder -CH₂-CH₂-CH₂- Gruppe;
   oder
D)
   - Y: eine direkte Bindung oder -CH₂-;
   - X: CH(Phenyl), wobei der Phenylrest mit F, Cl oder Br substituiert sein kann,
   O, S, SO, SO₂ oder N-R6;
   - R1 und R1': unabhängig voneinander
   H, F, Cl, Br, J, CN, COOH, COO(C₁-C₆)Alkyl, CONH₂, CONH(C₁-C₆)Alkyl, CON[(C₁-C₆)Alkyl]₂, (C₁-C₆)-Alkyl, (C₂-C₆)-Alkenyl, (C₂-C₆)-Alkinyl, O-(C₁-C₆)-Alkyl, O-CH₂-CF₃, O-CH₂-CF₂-CF₃, O-(C₄-C₆)-Alkyl, wobei in den Alkylresten ein, mehrere, oder alle Wasserstoff(e) durch Fluor ersetzt sein können oder ein Wasserstoff durch OH, OC(O)CH₃, O-CH₂-Ph, NH₂ oder N(COOCH₂Ph)₂ ersetzt sein kann;
   S-(C₁-C₆)-Alkyl, S-(CH₂)n-Phenyl, SO₂-(C₁-C₆)-Alkyl, SO₂-(CH₂)n-Phenyl, wobei n = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl substituiert sein kann; SO₂-NH₂, SO₂NH(C₁-C₆)-Alkyl, SO₂N[(C₁-C₆)-Alkyl]₂, NH₂, NH-(C₁-C₆)-Alkyl, N((C₁-C₆)-Alkyl)₂, NH(C₁-C₇)-Acyl, Phenyl, O-(CH₂)ₙ-Phenyl, wobei n = 0 - 6 sein kann, 1- oder 2-Naphthyl, 2-, 3- oder 4-Pyridyl, 2- oder 3-Furanyl oder 2- oder 3-Thienyl, wobei die Phenyl-, Naphthyl-, Pyridyl-, Furanyl- oder Thienylringe jeweils ein bis 3-fach substituiert sein können mit F, Cl, J, OH, CF₃, CN, OCF₃, O-(C₁-C₆)-Alkyl, (C₁-C₆)-Alkyl, NH₂, NH(C₁-C₆)-Alkyl, N((C₁-C₆)-Alkyl)_{2.} COOH, COO-(C₁-C₆)-Alkyl, CONH₂; 1,2,3-Triazol-5-yl, wobei der Triazolring in 1-, 2- oder 3-Stellung mit Methyl oder Benzyl substituiert sein kann;
   Tetrazol-5-yl, wobei der Tetrazolring in 1- oder 2-Stellung mit Methyl oder Benzyl substituiert sein kann;
   - R2: H, (C₁-C₆)-Alkyl, (CH₂)ₙ-Phenyl, (CH₂)ₙ-Thienyl, (CH₂)ₙ-Pyridyl, C(O)-(CH₂)ₙ-Phenyl, C(O)-(CH₂)ₙ-Thienyl, C(O)-(CH₂)ₙ-Pyridyl, wobei n = 0 - 3 sein kann und worin Phenyl, Thienyl, Pyridyl, Furyl jeweils bis zu zweimal mit Cl, F, CN, CF₃, (C₁-C₃)-Alkyl, OH, O-(C₁-C₆)-Alkyl substituiert sein kann;
   - R3: H, (C₁-C₆)-Alkyl, F, CN, O-(C₁-C₆)-Alkyl, CH₂-COO(C₁-C₆ Alkyl), CH₂-COO(C₃-C₈ Cycloalkyl), CH₂-COOH, CH₂-CONH₂, CH₂-CONHCH₃, CH₂-CON(CH₃)₂, (CH₂)ₙ-Phenyl, (CH₂)ₙ-Thienyl, (CH₂)ₙ-Pyridyl, wobei n = 0 - 3 sein kann und worin Phenyl, Thienyl, Pyridyl jeweils bis zu zweimal mit Cl, F, CN, CF₃, (C₁-C₃)-Alkyl, OH, O-(C1-C₆)-Alkyl substituiert sein kann, (C₂-C₆)-Alkinyl, (C₂-C₆)-Alkenyl, C(O)OCH₃, C(O)OCH₂CH₃, C(O)OH, C(O)NH₂, C(O)NHCH₃, C(O)N(CH₃)₂, OC(O)CH₃;
   - R4: (C₁-C₆)-Alkyl, (C₃-C₆)-Cycloalkyl, (CH₂)ₙ-Phenyl, (CH₂)ₙ-Thienyl, (CH₂)ₙ-Pyridyl, wobei n = 0 - 3 sein kann und worin Phenyl, Thienyl, Pyridyl jeweils bis zu zweimal mit Cl, F, CN, CF₃, (C₁-C₃)-Alkyl, OH, O-(C1-C₆)-Alkyl substituiert sein kann;
   - R5: (C₁-C₆)-Alkyl, (C₃-C₆)-Cycloalkyl, (CH₂)ₙ-Phenyl, (CH₂)ₙ-Thienyl, (CH₂)ₙ-Pyridyl, wobei n = 0 - 3 sein kann und worin Phenyl, Thienyl, Pyridyl jeweils bis zu zweimal mit Cl, F, CN, CF₃, (C₁-C₃)-Alkyl, OH, O-(C1-C₆)-Alkyl substituiert sein kann;
   oder
   - R4 und R5: bilden gemeinsam eine -CH₂-CH₂-, -CH₂-C(CH₃)₂- oder -CH₂-CH₂-CH₂- Gruppe;
   - R6: SO₂-(C₆H₄-4-CH₃)
   oder
E)
   - Y: eine direkte Bindung oder -CH₂-;
   - X: CH₂, CH(CH₃), CH(C₂H₅), CH(C₃H₇);
   - R1: H, F, Cl, Br, J, CH₃, CF₃, O-(C₁-C₃)-Alkyl;
   - R1': H, F, Cl, Br, J;
   - R2: H;
   - R3: H, (C₁-C₃)-Alkyl;
   - R4: Phenyl, wobei der Phenylrest bis zu zweifach substituiert sein kann mit F, Cl, Br, J, (C₁-C₆)-Alkyl, (C₃-C₆)-Cycloalkyl, O-(C₁-C₃)-Alkyl, CF₃, OCF₃, O-CH₂-Phenyl, COOH, COO(C₁-C₆)Alkyl, CONH₂;
   - R5: Phenyl, wobei der Phenylrest bis zu zweifach substituiert sein kann mit F, Cl, Br, J, (C₁-C₆)-Alkyl, (C₃-C₆)-Cycloalkyl, O-(C₁-C₃)-Alkyl, CF₃, OCF₃, O-CH₂-Phenyl, COOH, COO(C₁-C₆)Alkyl, CONH₂;
   sowie deren physiologisch verträgliche Salze.

Besonders bevorzugt sind Verbindungen der Formell, worin bedeuten
A)
   - Y: eine direkte Bindung;
   - X: CH₂, CH(CH₃), CH(C₂H₅), CH(C₃H₇);
   - R1: CN, COOH, COO(C₁-C₆)Alkyl, CONH₂, CON[(C₁-C₆)Alkyl]₂, (C₂-C₆)-Alkyl; (C₂-C₆)-Alkenyl; (C₂-C₆)-Alkinyl; O-CH₂-CF₃, O-CH₂-CF₂-CF₃, O-(C₄-C₆)-Alkyl, wobei in den Alkylresten ein, mehrere, oder alle Wasserstoff(e) durch Fluor ersetzt sein können, oder ein Wasserstoff durch OH, OC(O)CH₃, O-CH₂-Ph, NH₂ oder N(COOCH₂Ph)₂ ersetzt sein kann;
   S-(C₁-C₆)-Alkyl, S-(CH₂)ₙ-Phenyl, SO-(C₁-C₆)-Alkyl, SO-(CH₂)ₙ-Phenyl, wobei der Phenylrest bis zu zweifach mit F, Cl, Br, OH, CF₃, NO₂, CN, OCF₃, O-(C₁-C₆)-Alkyl, (C₁-C₆)-Alkyl substituiert sein kann; NH-(C₁-C₆)-Alkyl, N((C₁-C₆)-Alkyl)₂, NH(C₁-C₇)-Acyl, Phenyl, O-(CH₂)ₙ-Phenyl, wobei n = 0 - 6 sein kann, 1- oder 2-Naphthyl, 2-, 3- oder 4-Pyridyl, 2- oder 3-Furanyl oder 2- oder 3-Thienyl, wobei die Phenyl-, Naphthyl-, Pyridyl-, Furanyl- oder Thienylringe jeweils ein bis 2-fach substituiert sein können mit F, Cl, OH, CF₃, CN, OCF₃, O-(C₁-C₆)-Alkyl, (C₁-C₆)-Alkyl, COOH, COO-(C1-C₆)-Alkyl, CONH₂;
   1,2,3-Triazol-5-yl, wobei der Triazofring in 1-, 2- oder 3-Stellung mit Methyl oder Benzyl substituiert sein kann;
   Tetrazol-5-yl, wobei der Tetrazolring in 1- oder 2-Stellung mit Methyl oder Benzyl substituiert sein kann;
   - R1': H, F, Cl, CH₃, CF₃, O-(C₁-C₃)-Alkyl, SO₂-NH₂, SO₂NH(C₁-C₆)-Alkyl, SO₂N[(C₁-C₆)-Alkyl]₂ oder R1;
   - R2: H, (C₁-C₆)-Alkyl, (CH₂)ₙ-Phenyl, (CH₂)ₙ-Pyridyl, C(O)-(CH₂)ₙ-Phenyl, C(O)-(CH₂)ₙ-Pyridyl, wobei n = 0 - 3 sein kann und worin Phenyl, Pyridyl jeweils bis zu zweimal mit Cl, F, CN, CF₃, (C₁-C₃)-Alkyl, O-(C₁-C₆)-Alkyl substituiert sein kann, C(O)-(C₁-C₆)-Alkyl;
   - R3: H, (C₁-C₆)-Alkyl, F, CN, O-(C₁-C₆)-Alkyl, CH₂-COO(C₁-C₆ Alkyl), CH₂-COO(C₃-C₈ Cycloalkyl), CH₂-COOH, CH₂-CONH₂, CH₂-CONHCH₃, CH₂-CON(CH₃)₂, (CH₂)ₙ-Phenyl, (CH₂)ₙ-Pyridyl, wobei n = 0 - 3 sein kann und worin Phenyl, Pyridyl jeweils bis zu zweimal mit Cl, F, CN, CF₃, (C₁-C₃)-Alkyl, O-(C₁-C₅)-Alkyl substituiert sein kann. (C₂-C₆)-Alkinyl, C(O)OCH₃, C(O)OCH₂CH₃, C(O)OH, C(O)NH₂, C(O)N(CH₃)₂, OC(O)CH₃;
   - R4: (C₁-C₆)-Alkyl, (C₃-C₆)-Cycloalkyl, (CH₂)ₙ-Phenyl, (CH₂)ₙ-Pyridyl, wobei n = 0 - 3 sein kann und worin Phenyl, Pyridyl jeweils bis zu zweimal mit Cl, F, CN, CF₃, (C₁-C₃)-Alkyl, O-(C₁-C₆)-Alkyl substituiert sein kann;
   - R5: (C₁-C₆)-Alkyl, (C₃-C₆)-Cycloalkyl, (CH₂)ₙ-Phenyl, (CH₂)ₙ-Pyridyl, wobei n = 0 - 3 sein kann und worin Phenyl, Pyridyl jeweils bis zu zweimal mit Cl, F, CN, CF₃, (C₁-C₃)-Alkyl, O-(C₁-C₆)-Alkyl substituiert sein kann;
   oder
   - R4 und R5: bilden gemeinsam eine -CH₂-CH₂-, -CH₂-C(CH₃)₂- oder -CH₂-CH₂-CH₂- Gruppe;
   oder
B)
   - Y: eine direkte Bindung, -CH₂-;
   - X: CH₂, CH(CH₃), CH(C₂H₅), CH(C₃H₇);
   - R1 und R1': unabhängig voneinander
   H, F, Cl, Br, J, CN, COOH, COO(C₁-C₆)Alkyl, CONH₂, CON[(C₁-C₆)Alkyl]₂, (C₁-C₆)-Alkyl, (C₂-C₆)-Alkenyl, (C₂-C₆)-Alkinyl, O-(C₁-C₆)-Alkyl, O-CH₂-CF₃, O-CH₂-CF₂-CF₃, O-(C₄-C₆)-Alkyl, wobei in den Alkylresten ein, mehrere, oder alle Wasserstoff(e) durch Fluor ersetzt sein können oder ein Wasserstoff durch OH, OC(O)CH₃, O-CH₂-Ph, NH₂ oder N(COOCH₂Ph)₂ ersetzt sein kann;
   S-(C₁-C₆)-Alkyl, S-(CH₂)n-Phenyl, SO-(C₁-C₆)-Alkyl, SO-(CH₂)ₙ-Phenyl, wobei der Phenylrest bis zu zweifach mit F, Cl, OH, CF₃, OCF₃, O-(C₁-C₆)-Alkyl, (C₁-C₆)-Alkyl substituiert sein kann; SO₂-NH₂, SO₂NH(C₁-C₆)-Alkyl, SO₂N[(C₁-C₆)-Alkyl]₂, N((C₁-C₆)-Alkyl)₂, NH(C₁-C₇)-Acyl, Phenyl, O-(CH₂)ₙ-Phenyl, wobei n = 0 - 6 sein kann, 1 - oder 2-Naphthyl, 2-, 3- oder 4-Pyridyl, 2- oder 3-Furanyl oder 2- oder 3-Thienyl, wobei die Phenyl-, Naphthyl-, Pyridyl-, Furanyl- oder Thienylringe jeweils ein bis 3-fach substituiert sein können mit F, Cl, J, OH. CF₃, CN, OCF₃, O-(C₁-C₆)-Alkyl, (C₁-C₆)-Alkyl, NH₂, N((C₁-C₆)-Alkyl)₂. COOH, COO-(C₁-C₆)-Alkyl, CONH₂;
   1,2,3-Triazol-5-yl, wobei der Triazolring in 1-, 2- oder 3-Stellung mit Methyl oder Benzyl substituiert sein kann;
   Tetrazol-5-yl, wobei der Tetrazolring in 1- oder 2-Stellung mit Methyl oder Benzyl substituiert sein kann;
   - R2: H, (C₁-C₆)-Alkyl, (CH₂)ₙ-Phenyl, (CH₂)ₙ-Pyridyl, C(O)-(CH₂)ₙ-Phenyl, C(O)-(CH₂)ₙ-Pyridyl, wobei n = 0 - 3 sein kann und worin Phenyl, Pyridyl jeweils bis zu zweimal mit Cl, F, CN, CF₃, (C₁-C₃)-Alkyl, OH, O-(C₁-C₆)-Alkyl substituiert sein kann, C(O)-(C₁-C₆)-Alkyl;
   - R3: (C₄-C₆)-Alkyl, F, CN, N₃, O-(C₁-C₆)-Alkyl, CH₂-COO(C₁-C₆ Alkyl), CH₂-COO(C₃-C₈ Cycloalkyl), CH₂-COOH, CH₂-CONH₂, CH₂-CONHCH₃, CH₂-CON(CH₃)₂, (CH₂)ₙ-Phenyl, (CH₂)ₙ-Pyridyl, wobei n = 0 - 3 sein kann und worin Phenyl, Pyridyl jeweils bis zu zweimal mit Cl, F, CN, CF₃, (C₁-C₃)-Alkyl, O-(C1-C₆)-Alkyl substituiert sein kann, (C₂-C₆)-Alkinyl, C(O)OCH₃, C(O)OCH₂CH₃, C(O)OH, C(O)NH₂, C(O)NHCH₃, C(O)N(CH₃)₂, OC(O)CH₃;
   - R4: (C₁-C₆)-Alkyl, (C₃-C₆)-Cycloalkyl, (CH₂)ₙ-Phenyl, (CH₂)ₙ-Pyridyl, wobei n = 0 - 3 sein kann und worin Phenyl, Pyridyl jeweils bis zu zweimal mit Cl, F, CN, CF₃, (C₁-C₃)-Alkyl, O-(C1-C₆)-Alkyl substituiert sein kann;
   - R5: (C₁-C₆)-Alkyl, (C₃-C₆)-Cycloalkyl, (CH₂)ₙ-Phenyl, (CH₂)ₙ-Pyridyl, wobei n = 0 - 3 sein kann und worin Phenyl, Pyridyl jeweils bis zu zweimal mit Cl, F, CN, CF₃, (C₁-C₃)-Alkyl, O-(C1-C₆)-Alkyl substituiert sein kann;
   oder
   - R4 und R5: bilden gemeinsam eine -CH₂-CH₂-, -CH₂-C(CH₃)₂- oder -CH₂-CH₂-CH₂-, Gruppe;
   oder
C)
   - Y: eine direkte Bindung oder -CH₂-;
   - X: CH₂, CH(CH₃), CH(C₂H₅), CH(C₃H₇);
   - R1 und R1': unabhängig voneinander
   H, F, Cl, Br, J, CN, COOH, COO(C₁-C₆)Alkyl, CONH₂, CON[(C₁-C₆)Alkyl]₂, (C₁-C₆)-Alkyl, (C₂-C₆)-Alkenyl, (C₂-C₆)-Alkinyl, O-(C₁-C₆)-Alkyl, O-CH₂-CF₃, O-CH₂-CF₂-CF₃, O-(C₄-C₆)-Alkyl, wobei in den Alkylresten ein, mehrere, oder alle Wasserstoff(e) durch Fluor ersetzt sein können oder ein Wasserstoff durch OH, OC(O)CH₃, O-CH₂-Ph, NH₂ oder N(COOCH₂Ph)₂ ersetzt sein kann;
   S-(C₁-C₆)-Alkyl, S-(CH₂)ₙ-Phenyl, SO₂-(C₁-C₆)-Alkyl, SO₂-(CH₂)n-Phenyl, wobei n = 0 - 6 sein kann und der Phenylrest bis zu zweifach mit F, Cl, Br, OH, CF₃, NO₂, OCF₃, O-(C₁-C₆)-Alkyl, (C₁-C₆)-Alkyl substituiert sein kann; SO₂-NH₂, SO₂NH(C₁-C₆)-Alkyl, SO₂N[(C₁-C₆)-Alkyl]₂, N((C₁-C₆)-Alkyl)₂, NH(C₁-C₇)-Acyl, Phenyl, O-(CH₂)ₙ-Phenyl, wobei n = 0 - 4 sein kann, 1- oder 2-Naphthyl, 2-, 3- oder 4-Pyridyl, 2- oder 3-Furanyl oder 2- oder 3-Thienyl, wobei die Phenyl-, Naphthyl-, Pyridyl-, Furanyl- oder Thienylringe jeweils ein bis 3-fach substituiert sein können mit F, Cl, J, OH, CF₃, CN, OCF₃, O-(C₁-C₆)-Alkyl, (C₁-C₆)-Alkyl, NH₂, NH(C₁-C₆)-Alkyl, N((C₁-C₆)-Alkyl)₂ COOH, COO-(C₁-C₆)-Alkyl, CONH₂;
   1,2,3-Triazol-5-yl, wobei der Triazolring in 1-, 2- oder 3-Stellung mit Methyl oder Benzyl substituiert sein kann;
   Tetrazol-5-yl, wobei der Tetrazolring in 1- oder 2-Stellung mit Methyl oder Benzyl substituiert sein kann;
   - R2: (C₁-C₆)-Alkyl, C₂-C₈ Alkenyl, C₂-C₈ Alkinyl, (CH₂)ₙ-Phenyl, (CH₂)ₙ-Thienyl, (CH₂)ₙ-Pyridyl, C(O)-(CH₂)ₙ-Phenyl, C(O)-(CH₂)ₙ-Thienyl, C(O)-(CH₂)ₙ-Pyridyl, wobei n = 0 - 3 sein kann und worin Phenyl, Thienyl, Pyridyl, Furyl jeweils bis zu zweimal mit Cl, F, CN, CF₃, (C₁-C₃)-Alkyl, OH, O-(C₁-C₆)-Alkyl substituiert sein kann, C(O)-(C₁-C₆)-Alkyl;
   - R3: H, (C₁-C₆)-Alkyl, F, CN, O-(C₁-C₆)-Alkyl, CH₂-COO(C₁-C₆ Alkyl), CH₂-COO(C₃-C₈ Cycloalkyl), CH₂-COOH, CH₂-CONH₂, CH₂-CONHCH₃, CH₂-CON(CH₃)₂, (CH₂)ₙ-Phenyl, (CH₂)ₙ-Pyridyl, wobei n = 0 - 3 sein kann und worin Phenyl, Pyridyl jeweils bis zu zweimal mit Cl, F, CN, CF₃, (C₁-C₃)-Alkyl, O-(C1-C₆)-Alkyl substituiert sein kann, (C₂-C₆)-Alkinyl, C(O)OCH₃, C(O)OCH₂CH₃, C(O)OH, C(O)NH₂, C(O)N(CH₃)₂, OC(O)CH₃;
   - R4: (C₁-C₆)-Alkyl, (C₃-C₆)-Cycloalkyl, (CH₂)ₙ-Phenyl, (CH₂)ₙ-Pyridyl, wobei n = 0 - 3 sein kann und worin Phenyl, Pyridyl jeweils bis zu zweimal mit Cl, F, CN, CF₃, (C₁-C₃)-Alkyl, O-(C1-C₆)-Alkyl substituiert sein kann;
   - R5: (C₁-C₆)-Alkyl, (C₃-C₆)-Cycloalkyl, (CH₂)ₙ-Phenyl, (CH₂)ₙ-Pyridyl, wobei n = 0 - 3 sein kann und worin Phenyl, Pyridyl jeweils bis zu zweimal mit Cl, F, CN, CF₃, (C₁-C₃)-Alkyl, O-(C1-C₆)-Alkyl substituiert sein kann;
   oder
   - R4 und R5: bilden gemeinsam eine -CH₂-CH₂-, -CH₂-C(CH₃)₂- oder -CH₂-CH₂-CH₂- Gruppe;
   oder
D)
   - Y: eine direkte Bindung oder -CH₂-;
   - X: CH(Phenyl), wobei der Phenylrest mit F oder Cl substituiert sein kann,
   O, S, SO₂ oder N-R6;
   - R1 und R1: unabhängig voneinander
   H, F, Cl, Br, J, CN, COOH, COO(C₁-C₆)Alkyl, CONH₂, CON[(C₁-C₆)Alkyl]₂, (C₁-C₆)-Alkyl, (C₂-C₆)-Alkenyl, (C₂-C₆)-Alkinyl, O-(C₁-C₆)-Alkyl, O-CH₂-CF₃, O-CH₂-CF₂-CF₃, O-(C₄-C₆)-Alkyl, wobei in den Alkylresten ein, mehrere, oder alle Wasserstoff(e) durch Fluor ersetzt sein können oder ein Wasserstoff durch OH, OC(O)CH₃, O-CH₂-Ph, NH₂ oder N(COOCH₂Ph)₂ ersetzt sein kann;
   S-(C₁-C₆)-Alkyl, S-(CH₂)n-Phenyl, SO₂-(C₁-C₆)-Alkyl, SO₂-(CH₂)n-Phenyl, wobei n = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl substituiert sein kann; SO₂-NH₂, SO₂NH(C₁-C₆)-Alkyl, SO₂N[(C₁-C₆)-Alkyl]₂, NH₂, N((C₁-C₆)-Alkyl)₂, NH(C₁-C₇)-Acyl, Phenyl, O-(CH₂)ₙ-Phenyl, wobei n = 0 - 6 sein kann, 1- oder 2-Naphthyl, 2-, 3- oder 4-Pyridyl, 2- oder 3-Furanyl oder 2- oder 3-Thienyl, wobei die Phenyl-, Naphthyl-, Pyridyl-, Furanyl- oder Thienylringe jeweils ein bis 3-fach substituiert sein können mit F, Cl, J, OH, CF₃, CN, OCF₃, O-(C₁-C₆)-Alkyl, (C₁-C₆)-Alkyl, NH₂, N((C₁-C₆)-Alkyl)₂ COOH, COO-(C₁-C₆)-Alkyl, CONH₂;
   1,2,3-Triazol-5-yl, wobei der Triazolring in 1-, 2- oder 3-Stellung mit Methyl oder Benzyl substituiert sein kann;
   Tetrazol-5-yl, wobei der Tetrazolring in 1- oder 2-Stellung mit Methyl oder Benzyl substituiert sein kann;
   - R2: H, (C₁-C₆)-Alkyl, (CH₂)ₙ-Phenyl, (CH₂)ₙ-Thienyl, (CH₂)ₙ-Pyridyl, C(O)-(CH₂)ₙ-Phenyl, C(O)-(CH₂)ₙ-Thienyl, C(O)-(CH₂)ₙ-Pyridyl, wobei n = 0 - 3 sein kann und worin Phenyl, Thienyl, Pyridyl, Furyl jeweils bis zu zweimal mit Cl, F, CN, CF₃, (C₁-C₃)-Alkyl, OH, O-(C₁-C₆)-Alkyl substituiert sein kann, C(O)-(C₁-C₆)-Alkyl;
   - R3: H, (C₁-C₆)-Alkyl, F, CN, O-(C₁-C₆)-Alkyl, CH₂-COO(C₁-C₆ Alkyl), CH₂-COO(C₃-C₈ Cycloalkyl), CH₂-COOH, CH₂-CONH₂, CH₂-CONHCH₃, CH₂-CON(CH₃)₂, (CH₂)ₙ-Phenyl, (CH₂)ₙ-Pyridyl, wobei n = 0 - 3 sein kann und worin Phenyl, Pyridyl jeweils bis zu zweimal mit Cl, F, CN, CF₃, (C₁-C₃)-Alkyl, O-(C₁-C₆)-Alkyl substituiert sein kann, (C₂-C₆)-Alkinyl, C(O)OCH₃, C(O)OCH₂CH₃, C(O)OH, C(O)NH₂, C(O)NHCH₃, C(O)N(CH₃)₂, OC(O)CH₃;
   - R4: (C₁-C₆)-Alkyl, (C₃-C₆)-Cycloalkyl, (CH₂)ₙ-Phenyl, (CH₂)ₙ-Pyridyl, wobei n = 0 - 3 sein kann und worin Phenyl, Pyridyl jeweils bis zu zweimal mit CI, F, CN, CF₃, (C₁-C₃)-Alkyl, O-(C₁-C₆)-Alkyl substituiert sein kann;
   - R5: (C₁-C₆)-Alkyl, (C₃-C₆)-Cycloalkyl, (CH₂)ₙ-Phenyl, (CH₂)ₙ-Pyridyl, wobei n = 0 - 3 sein kann und worin Phenyl, Pyridyl jeweils bis zu zweimal mit CI, F, CN, CF₃, (C₁-C₃)-Alkyl, O-(C₁-C₆)-Alkyl substituiert sein kann;
   oder
   - R4 und R5: bilden gemeinsam eine -CH₂-CH₂-, -CH₂-C(CH₃)₂ oder -CH₂-CH₂-CH₂- Gruppe;
   - R6: SO₂-(C₆H₄-4-CH₃)
   oder
E)
   - Y: eine direkte Bindung oder -CH₂-;
   - X: CH₂, CH(CH₃), CH(C₂H₅), CH(C₃H₇);
   - R1: H, F, Cl, CH₃, CF₃, O-(C₁-C₃)-Alkyl;
   - R1': H, F, Cl;
   - R2: H;
   - R3: H, (C₁-C₃)-Alkyl;
   - R4: Phenyl, wobei der Phenylrest bis zu zweifach substituiert sein kann mit F, Cl, (C₁-C₆)-Alkyl, (C₃-C₆)-Cycloalkyl, O-(C₁-C₃)-Alkyl, CF₃, O-CH₂-Phenyl, COOH, COO(C₁-C₆)Alkyl, CONH₂;
   - R5: Phenyl, wobei der Phenylrest bis zu zweifach substituiert sein kann mit F, Cl, (C₁-C₆)-Alkyl, (C₃-C₆)-Cycloalkyl, O-(C₁-C₃)-Alkyl, CF₃, O-CH₂-Phenyl, COOH, COO(C₁-C₆)Alkyl, CONH₂;
sowie deren physiologisch verträgliche Salze.

Ganz besonders bevorzugt sind Verbindungen der Formel I, worin bedeuten
- Y: eine direkte Bindung;
- X: CH₂
- R1 und R1': unabhängig voneinander H, F, Cl, CN, COOH, CONH₂, COO(C₁-C₃)-Alkyl, (C₁-C₆)-Alkyl, (C₂-C₆)-Alkenyl, (C₂-C₆)-Alkinyl, wobei in den Alkyl-, Alkenyl- und Alkinresten ein Wasserstoff durch OH, OC(O)CH₃, O-CH₂-Ph, NH₂ oder N(COOCH2Ph)2 ersetzt sein kann; OCF₃, OCH₂CF₃ ; O-(C1-C4)-Alkyl, wobei in den Alkylresten ein, mehrere, oder alle Wasserstoff(e) durch Fluor ersetzt sein können oder ein Wasserstoff durch OH, OC(O)CH₃, O-CH₂-Ph, NH₂ oder N(COOCH₂Ph)₂ ersetzt sein kann;
SO₂-(C₁-C₆)-Alkyl, SO₂-(CH₂)ₙ-Phenyl, wobei n = 0 - 3 sein kann und der Phenylrest mit F, Cl, OH, CF₃, O-(C₁-C₄)-Alkyl substituiert sein kann;
NH-(CO)-(C₁-C₃)-Alkyl; (CH₂)ₙ-Phenyl, S-(CH₂)ₙ-Phenyl, O-(CH₂)ₙ-Phenyl, wobei n = 0 - 3 sein kann, 1- oder 2-Naphthyl, 2-, 3- oder 4-Pyridyl, 2- oder 3- Furanyl oder 2- oder 3-Thienyl, wobei die Phenyl-, Naphthyl-, Pyridyl-, Furanyl- oder Thienylringe jeweils substituiert sein können mit F, Cl, CF₃, (C₁-C₆)-Alkyl, O-(C₁-C₆)-Alkyl und wobei in den Alkylresten ein Wasserstoff durch OH, OC(O)CH₃, O-CH₂-Ph, NH₂ oder N(COOCH₂Ph)₂ ersetzt sein kann;
1,2,3-Triazol-5-yl, wobei der Triazolring in 1-, 2- oder 3-Stellung mit Methyl oder Benzyl substituiert sein kann;
Tetrazol-5-yl, wobei der Tetrazolring in 1- oder 2-Stellung mit Methyl oder Benzyl substituiert sein kann;
- R2: H, (C₁-C₄)-Alkyl, (C₅-C₆)-Cycloalkyl; (CH₂)ₙ-Phenyl, wobei n = 0 - 3 sein kann, C(O)-(C₁-C₄)-Alkyl oder C(O)-Phenyl;
- R3: F, (C₄-C₆)-Alkyl, CH₂-Phenyl, wobei Phenyl bis zu zweimal mit F, Cl, CF₃, O-(C₁-C₃)-Alkyl, (C₁-C₃)-Alkyl, COOH, CO-O-(C₁-C₃)-Alkyl oder CONH₂ substituiert sein kann;
- R4: (C₁-C₆)-Alkyl, (C₃-C₆)-Cycloalkyl, (CH₂)ₙ-Phenyl, wobei n = 0 - 3 sein kann und der Phenylrest bis zu zweimal mit F, Cl, O-(C₁-C₄)-Alkyl oder OH substituiert sein kann;
- R5: (C₁-C₅)-Alkyl, (C₃-C₆)-Cycloalkyl, (CH₂)ₙ-Phenyl, wobei n = 0 - 3 sein kann und der Phenylrest bis zu zweimal mit F, Cl, O-(C₁-C₄)-Alkyl oder OH substituiert sein kann;
sowie deren physiologisch verträgliche Salze.

Ganz besonders bevorzugt sind Verbindungen der Formel l mit folgender Struktur:

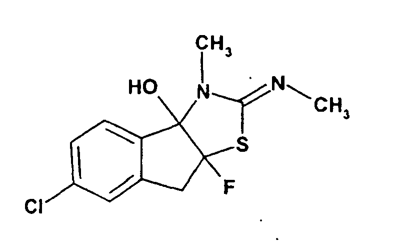

Insbesondere die Verbindung:

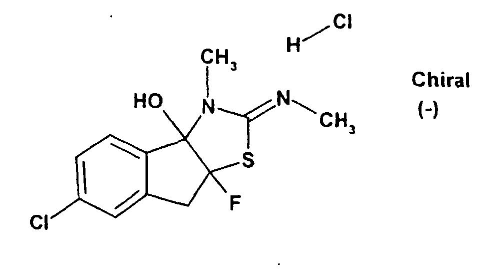

Die Erfindung bezieht sich auf Verbindungen der Formel 1, in Form ihrer Racemate, racemischen Mischungen und reinen Enantiomere sowie auf ihre Diastereomere und Mischungen davon.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindungen sind Salze anorganischer Säuren, wie Salzsäure, Bromwasserstoff-, Phosphor-, Metaphosphor-, Salpeter-, Sulfon- und Schwefelsäure sowie organischer Säuren, wie z.B. Essigsäure. Benzolsulfon-, Benzoe-, Zitronen-, Ethansulfon-, Fumar-, Glucon-, Glykol-, Isethion-,
Milch-, Lactobion-, Malein-, Äpfel-, Methansulfon-, Bernstein-, p-Toluolsulfon-, Wein- und Trifluoressigsäure. Für medizinische Zwecke wird in besonders bevorzugter Weise das Chlorsalz verwendet. Geeignete pharmazeutisch verträgliche basische Salze sind Ammoniumsalze, Alkalimetallsalze (wie Natrium- und Kaliumsalze) und Erdalkalisalze (wie Magnesium- und Calciumsalze). Bevorzugt sind die Hydrobromide und Hydrochloride, insbesondere die Hydrochloride.

Salze mit einem nicht pharmazeutisch verträglichen Anion gehören ebenfalls in den Rahmen der Erfindung als nützliche Zwischenprodukte für die Herstellung oder Reinigung pharmazeutisch verträglicher Salze und/oder für die Verwendung in nicht-therapeutischen, zum Beispiel in-vitro-Anwendungen.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Nachfolgend beziehen sich alle Verweise auf "Verbindung(en) gemäß Formel (I)" auf Verbindung(en) der Formel (I) wie vorstehend beschrieben, sowie ihre Salze, Solvate wie hierin beschrieben.

Die Menge einer Verbindung gemäß Formel (I), die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,3 mg bis 100 mg (typischerweise von 3 mg bis 50 mg) pro Tag pro Kilogramm Körpergewicht, z.B. 3-10 mg/kg/Tag. Eine intravenöse Dosis kann z.B. im Bereich von 0,3 mg bis 1,0 mg/kg liegen, die geeigneterweise als Infusion von 10 ng bis 100 ng pro Kilogramm pro Minute verabreicht werden kann. Geeignete Infusionslösungen für diese Zwecke können z.B. von 0,1 ng bis 10 mg, typischerweise von 1 ng bis 10 mg pro Milliliter, enthalten. Einzeldosen können z.B. von 1 mg bis 10 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, können beispielsweise von 1,0 bis 1000 mg, typischerweise von 10 bis 600 mg enthalten. Im Falle pharmazeutisch verträglicher Salze beziehen sich die vorgenannten Gewichtsangaben auf das Gewicht des vom Salz abgeleiteten Thiazolidin-2-yliden-lons. Zur Prophylaxe oder Therapie der oben genannten Zustände können die Verbindungen gemäß Formel (I) selbst als Verbindung verwendet werden, vorzugsweise liegen sie jedoch mit einem verträglichen Träger in Form einer pharmazeutischen Zusammensetzung vor. Der Träger muß natürlich verträglich sein, in dem Sinne, daß er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel (I). Die erfindungsgemäßen pharmazeutischen Zusammensetzungen können nach einer der bekannten pharmazeutischen Methoden hergestellt werden, die im wesentlichen darin bestehen, daß die Bestandteile mit pharmakologisch verträglichen Träger- und/oder Hilfsstoffen gemischt werden.

Erfindungsgemäße pharmazeutische Zusammensetzungen sind solche, die für orale, rektale, topische, perorale (z.B. sublinguale) und parenterale (z.B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel (1) abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Polyvinyfacetatphthalat, Hydroxypropylmethylcellulosephthalat und anionische Polymere von Methacrylsäure und Methacrylsäuremethylester.

Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel (1) enthalten; als Pulver oder Granulate; als Lösung oder Suspension in einer wäßrigen oder nicht-wäßrigen Flüssigkeit; oder als eine Öl-in-Wasser- oder Wasser-in-Öl-Emulsion. Diese Zusammensetzungen können, wie bereits erwähnt, nach jeder geeigneten pharmazeutischen Methode zubereitet werden, die einen Schritt umfaßt, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpreßt oder geformt wird, gegebenenfalls mit einem oder mehreren zusätzlichen Bestandteilen. Gepreßte Tabletten können durch Tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel (I) mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Geeignete pharmazeutische Zusammensetzungen für die parenterale Verabreichung umfassen vorzugsweise sterile wäßrige Zubereitungen einer Verbindung gemäß Formel (I), die vorzugsweise isotonisch mit dem Blut des vorgesehenen Empfängers sind. Diese Zubereitungen werden vorzugsweise intravenös verabreicht; wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im allgemeinen von 0,1 bis 5 Gew.-% der aktiven Verbindung.

Geeignete pharmazeutische Zusammensetzungen für die rektale Verabreichung liegen vorzugsweise als Einzeldosis-Zäpfchen vor. Diese können hergestellt werden, indem man eine Verbindung gemäß Formel (1) mit einem oder mehreren herkömmlichen festen Trägern, beispielsweise Kakaobutter mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor. Als Träger können Vaseline, Lanolin, Polyethylenglycole, Alkohole und Kombinationen von zwei oder mehreren dieser Substanzen verwendet werden. Der Wirkstoff ist im allgemeinen in einer Konzentration von 0,1 bis 15 Gew.-% der Zusammensetzung vorhanden, beispielsweise von 0,5 bis 2%.

Auch eine transdermale Verabreichung ist möglich. Geeignete pharmazeutische Zusammensetzungen für transdermale Anwendungen können als einzelne Pflaster vorliegen, die für einen langzeitigen engen Kontakt mit der Epidermis des Patienten geeignet sind. Solche Pflaster enthalten geeigneterweise den Wirkstoff in einer gegebenenfalls gepufferten wäßrigen Lösung, gelöst und/oder dispergiert in einem Haftmittel oder dispergiert in einem Polymer. Eine geeignete WirkstoffKonzentration beträgt ca. 1% bis 35%, vorzugsweise ca. 3% bis 15%. Als eine besondere Möglichkeit kann der Wirkstoff, wie beispielsweise in Pharmaceutical Research, 2(6): 318 (1986) beschrieben, durch Elektrotransport oder lontophorese freigesetzt werden.

Gegenstand der Erfindung ist weiterhin ein Verfahren zur Herstellung der Verbindungen der allgemeinen Formel I (R2 = H), dadurch gekennzeichnet, daß man
A) Verbindungen der allgemeinen Formel II, 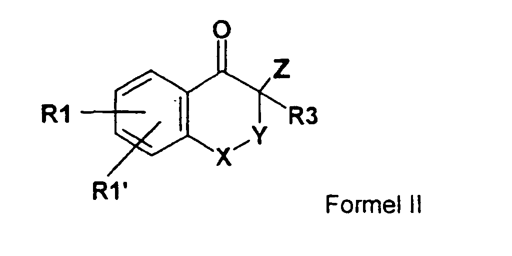 worin R1, R1', R3 und X und Y die angegebene Bedeutung besitzen, und Z für den Rest eines aktivierten Esters einer anorganischen oder organischen Säure steht, mit Thioharnstoffen der allgemeinen Formel III, die in den tautomeren Formen IIIa und IIIb und IIIc vorliegen können, 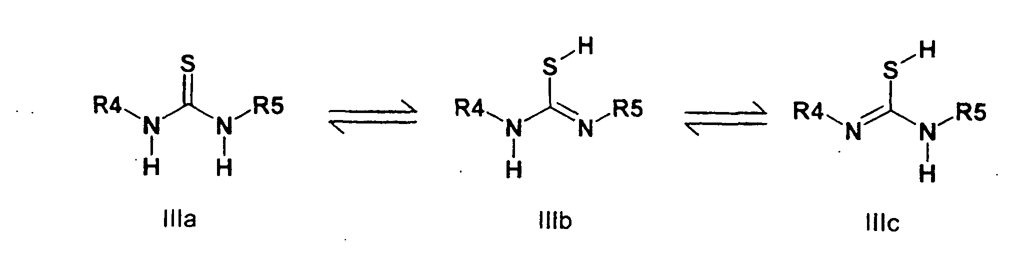 worin R4 und R5 die angegebene Bedeutung besitzen, umsetzt und
B) gegebenenfalls die Verbindungen der allgemeinen Formel I (R2 = H) mit organischen oder anorganischen Säuren in ihre Säureadditionssalze oder erhaltene Salze der Verbindungen der allgemeinen Formel I (R2 = H) mit organischen oder anorganischen Basen in die freien basischen Verbindungen der Formel I (R2 = H) überführt.

Als anorganische Säuren kommen beispielsweise in Betracht:
Halogenwasserstoffsäuren wie Chlorwasserstoffsäure und Bromwasserstoffsäure, sowie Schwefelsäure, Phosphorsäure und Amidosulfonsäure.
Als organische Säuren seien beispielsweise genannt: Ameisensäure, Essigsäure, Benzoesäure, p-Toluolsulfonsäure, Benzolsulfonsäure, Bernsteinsäure, Fumarsäure, Maleinsäure, Milchsäure, Weinsäure, Zitronensäure, L-Ascorbinsäure, Salizylsäure, Isethionsäure, Methansulfonsäure, Trifluormethansulfonsäure, 1,2-Benzisothiazol-3(2H)-on, 6-Methyl-1,2,3-oxathiazin-4(3H)-on-2,2-dioxid.

Die Verbindungen der Formel I (R2 = H) können auch in ihren tautomeren Formen vorliegen:

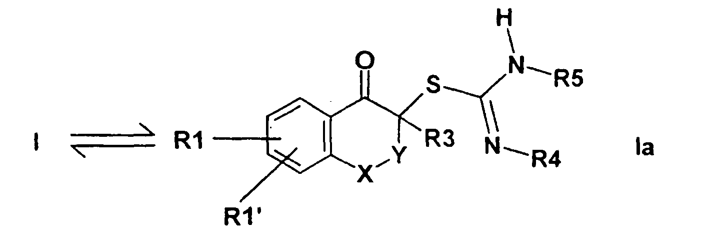

Die erfindungsgemäßen Verbindungen der Formel I (R2 = H) können außerdem in ihren möglichen geometrischen isomeren Strukturen vorliegen.

Die Alkyl-, Alkenyl- und Alkinylreste in den Substituenten R1, R1', R2, R3, R4 und R5 können sowohl geradkettig wie verzweigt sein.

Über die offenkettige tautomere Form la stehen die cyclischen Verbindungen der Formel I (R2 = H) bei unterschiedlichem R4 und R5 mit den stellungsisomeren Verbindungen der Formel Ib (R2 = H) und deren Säureadditionssalzen 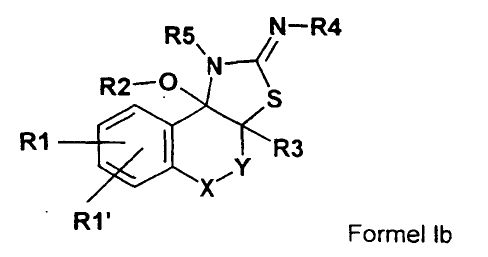 im Gleichgewicht. Welches der beiden cyclischen Isomeren I (R2 = H) oder Ib (R2 = H) bzw. deren Säureadditionssalze bevorzugt vorliegen, hängt in besonderem Maße von der unterschiedlichen Raumerfüllung der Substituenten R4 bzw. R5 in der Weise ab, daß sich der räumlich kleinere Substituent bevorzugt in Stellung 3 des Thiazolidin-Ringsystems befindet.

Bei den erfindungsgemäßen Verbindungen wird im folgenden nur eine der möglichen isomeren bzw. tautomeren Formen einer jeweiligen Substanz angegeben.

Die oben beschriebene Verfahrensweise wird vorteilhaft so ausgeführt, daß man die Verbindungen II mit den Thioharnstoffen III im molaren Verhältnis von 1:1 bis 1:1,5 umsetzt. Die Reaktion wird vorteilhaft in einem inerten Lösemittel. z.B. in polaren organischen Lösemitteln wie Dimethylsulfoxid, Dimethylformamid. Dimethylacetamid, N-Methyl-2-pyrrolidon, Dioxan, Tetrahydrofuran, Acetonitril, Nitromethan oder Diethylenglykoldimethylether. Als besonders vorteilhafte Lösemittel erweisen sich jedoch Essigsäuremethylester und Essigsäureethylester, kurzkettige Alkohole wie Methanol, Ethanol, Propanol, Isopropanol, sowie niedere Dialkylketone, wie z.B. Aceton, Butan-2-on oder Hexan-2-on. Auch Gemische der angeführten Reaktionsmedien können angewandt werden; so wie auch Gemische der aufgeführten Lösemittel mit Solventien, die für sich alleine genommen weniger geeignet sind, verwendet werden können, wie z.B. Gemische aus Methanol mit Benzol, Ethanol mit Toluol, Methanol mit Diethylether oder mit tert. Butylmethylether, Ethanol mit Tetrachlormethan, Aceton mit Chloroform, Dichlormethan oder 1,2-Dichlorethan, wobei das jeweils polarere Lösemittel zweckmäßigerweise im Überschuß verwendet werden soll. Die Reaktionspartner können im jeweiligen Reaktionsmedium suspendiert oder gelöst vorliegen. Grundsätzlich können die Reaktionspartner auch ohne Lösemittel umgesetzt werden, insbesondere dann, wenn der jeweilige Thioharnstoff einen möglichst tiefen Schmelzpunkt hat. Die Reaktion verläuft nur wenig exotherm und kann zwischen -10°C und 150°C, bevorzugt zwischen 0°C und 50°C, durchgeführt werden. Als besonders günstig erwies sich in der Regel ein Temperaturbereich zwischen 20°C und 40°C.
Die Reaktionsdauer ist weitgehend von der Reaktionstemperatur abhängig und liegt zwischen 2 Minuten und 3 Tagen bei höheren bzw. niedrigeren Temperaturen, Im günstigen Temperaturbereich liegt die Reaktionsdauer im allgemeinen zwischen 5 Minuten und 48 Stunden.

Häufig scheiden sich die Verbindungen I (R2 = H) in Form ihrer schwerlöslichen Säureadditionssalze im Verlauf der Reaktion ab, zwechmäßig wird nachträglich noch ein geeignetes Fällungsmittel zugesetzt. Als solches verwendet man z.B. Kohlenwasserstoffe wie Benzol, Toluol, Cyclohexan oder Heptan oder Tetrachlormethan; insbesondere erwiesen sich Essigsäurealkylester wie Essigsäureethylester oder Essigsäure-n-butylester oder Dialkylether wie Diethylether, Diisopropylether, Di-n-butylether oder tert. Butylmethylether als besonders geeignet. Bleibt nach Ende der Reaktion das Reaktionsgemisch in Lösung, so kann man die Salze der Verbindungen I (R2 = H), ggf, nach Konzentrierung der Reaktionslösung, mit einem der genannten Fällungsmittel ausfällen. Ferner kann man die Lösung des Reaktionsgemisches auch vorteilhaft in die Lösung eines der genannten Fällungsmittel unter Rühren einfiltrieren. Da die Reaktion der Verbindungen II mit den Thioharnstoffen III praktisch quantitativ abläuft, sind die erhaltenen Rohprodukte meistens bereits analytisch rein. Die Aufarbeitung des Reaktionsgemisches kann auch so durchgeführt werden, daß die Reaktionsmischung unter Zusatz einer organischen Base, wie z.B. Triethylamin oder Diisobutylamin oder Ammoniak oder Morpholin oder Piperidin oder 1,8-Diazabicyclo[5.4.0]undec-7-en alkalisch gestellt wird, und das Reaktionsrohprodukt nach Konzentrierung chromatographisch, z.B. über eine Kieselgelsäule, gereinigt wird. Als geeignete Elutionsmedien dafür erweisen sich z.B. Gemische von Essigsäureethylester mit Methanol. Gemische von Dichlormethan mit Methanol, Gemische von Toluol mit Methanol oder Essigsäureethylester oder Gemische von Essigsäurethylester mit Kohlenwasserstoffen wie Heptan. Erfolgt die Reinigung des Rohproduktes auf die zuletzt beschriebene Weise, kann aus der so gewonnenen reinen Base der Formel I (R2 = H) ein Säureadditionsprodukt der Formel I (R2 = H) so gewonnen werden, daß man die Base in einem organischen protischen Lösemittel wie Methanol, Ethanol, Propanol oder Isopropanol oder in einem organischen aprotischen Lösemittel wie Essigsäureethylester, Diethylether, Diisopropylether, tert.Butylmethylether, Dioxan, Tetrahydrofuran, Aceton oder Butan-2-on löst oder suspendiert und diese Mischung anschließend mit einer wenigstens äquimolaren Menge einer anorganischen Säure wie z.B. Chlorwasserstoffsäure, gelöst in einem inerten Lösemittel wie z.B. Diethylether oder Ethanol, oder einer anderen der weiter oben genannten anorganischen oder organischen Säuren versetzt. Die Verbindungen der Formel I (R2 = H) können aus einem inerten, geeigneten Lösemittel wie z.B. Aceton, Butan-2-on, Acetonitril, Nitromethan umkristallisiert werden. Besonders vorteilhaft ist aber die Umfällung aus einem Lösemittel wie z.B. Dimethylformamid, Dimethylacetamid, Nitromethan, Acetonitril, vorzugsweise Methanol oder Ethanol.

Verbindungen der Formel I oder Ib mit R2 = (C₁-C₆)-Alkyl, (C₃-C₆)-Cycloalkyl oder (CH₂)ₙ-Aryl, wobei n = 0-5 sein kann, und Aryl wie weiter oben bestimmt ist, lassen sich gewinnen, indem man entweder
aa) Die Säureadditionssalze der Formel I oder Ib mit R2 = H in einem Lösemittel der Formel R2-OH, wobei R2 die oben beschriebene Bedeutung hat, bei einer Temperatur von -20°C bis 120°C, vorzugsweise bei -5°C bis 50°C, 2 Stunden bis 4 Tage, vorzugsweise 4 Stunden bis 2 Tage, reagieren läßt
   oder
ab) die freien Basen der Formel I oder lb mit R2 = H in einem Lösemittel der Formel R2-OH, wobei R2 die oben beschriebene Bedeutung hat, mit äquimolaren, unterschüssigen oder katalytischen, vorzugsweise katalytischen Mengen einer anorganischen oder organischen Säure, wie sie weiter oben beschrieben sind, oder unter Zugabe eines sauren Ionenaustauschers bei einer Temperatur von -20°C bis 120°C, vorzugsweise bei -5°C bis 50°C, 2 Stunden bis 4 Tage, vorzugsweise 4 Stunden bis 2 Tage, umsetzt
   oder
ac) die Umsetzungen nach aa) und ab) in einem inerten aprotischem Lösemittel wie Dichlormethan, Chloroform, 1,2-Dichlorethan, Heptan, Benzol, Toluol, Acetonitril, Nitromethan, Dioxan, Tetrahydrofuran, Ethylenglykoldimethylether, Diethylether, Diisopropylether, tert.Butylmethylether, Aceton, Butan-2-on oder Essigsäureniederalkylester, wie z.B. Essigsäureethylester, durch Zugabe von 1 bis 5, vorzugsweise 1,5 -2 Äquivalenten einer Verbindung der Formel R2-OH durchführt
   oder
ad) Verbindungen der Formel I oder lb mit R2 = H in einem polaren aprotischen Lösemittel, wie z.B. Tetrahydrofuran, Dioxan, Ethylenglykoldimethylether, Nitromethan. Acetonitril oder Dimethylformamid, Dimethylacetamid oder N- -Methyl-2-pyrrolidon, mit Hilfe einer Base, wie z.B. Natriumhydrid, Lithiumdiisopropylamid, KOH oder Kaliumcarbonat, in ihr Alkoholat überführt und dieses dann unter Zugabe eines Alkylierungsmittels der Formel R2-X, wobei X = Chlor, Brom, Jod, O-C(O)-CH₃, O-C(O)-CF₃, O-C(O)-C₆H₄-4-NO₂, O-SO₂-CH₃, O-SO₂-CF₃, O-SO₂-C₆H₄-4-CH₃, O-SO₂-C₆H₄-4-NO₂, bei -20 bis 150°C, bevorzugt bei -15 bis 50°C, für 10 Minuten bis 2 Tage, bevorzugt für 20 Minuten bis 12 Stunden, reagieren läßt.

Die Verbindungen der Formel I oder lb mit R2 ≠ H, die nach aa) bis ad) wie oben beschrieben, erhalten werden, fallen entweder als Säureadditionssalz schwerlöslich aus - dann werden sie abgesaugt, mit wenig des eingesetzten Lösemittels gewaschen und getrocknet - oder sie bleiben in Lösung. Eine Reinigung kann so erfolgen, daß der Reaktionsansatz nach erfolgter Bildung der Ether der Formel I oder Ib mit R2 ≠ H mit einer anorganischen oder organischen Base, z.B. mit Triethylamin, neutralisiert und die resultierende Mischung konzentriert und anschließend an Kieselgel chromatographiert wird. Die so erhaltene reine Base der Formel I oder Ib mit R2 ≠ H kann, wie oben für Verbindungen der Formel I oder Ib mit R2 = H beschrieben, in ein Säureadditionssalz überführt werden.

Verbindungen der Formel I oder Ib mit R2 = C(O)-(C₁-C₆)-Alkyl, C(O)-(C₃-C₆)-Cycloalkyl oder C(O)-(CH₂)ₙ-Aryl, wobei Aryl gleich Phenyl, Thienyl, Pyridyl oder Furyl sein kann und n wie weiter oben definiert ist, lassen sich gewinnen, indem man entweder
ba) wie unter aa) - ac) beschrieben vorgeht, mit dem Unterschied, daß man statt einer Verbindung R2-OH eine Verbindung R2-COOH einsetzt, wobei R2 (C₁-C₆)-Alkyl, (C₃-C₆)-Cycloalkyl oder (CH₂)ₙ-Aryl ist und n und Aryl die weiter oben beschriebenen Bedeutungen haben, und man 1 bis 2 Äquivalente der Verbindung R2-COOH, vorzugswesie 1,5 Äquivalente der Verbindung R2-COOH einsetzt und auf den Zusatz des unter aa) - ac) beschriebenen sauren anorganischen oder organischen Katalysators verzichtet, den sauren Kationenaustauscher jedoch vorteilhaft einsetzt
   oder
bb) eine Verbindung der Formel I oder Ib mit R2 =H (freie Base) mit einer Verbindung der Formel R2-COOH, wobei R2 = C(O)-(C₁-C₆)-Alkyl, C(O)-(C₃-C₆)-Cycloalkyl oder C(O)-(CH₂)ₙ-Aryl ist und Aryl und n wie weiter oben definiert sind, z.B im Sinne einer Mitsunobu Reaktion (O Mitsunobu, Synthesis 1981, 1) zu einer Verbindung der Formel I oder Ib mit R2 ungleich H umsetzt
   oder
bc) eine Verbindung der Formel R2-C(O)-Cl oder R2-C(O)-Br oder R2-C(O)-O-(O)-C-R2 mit einer Verbindung der Formel I oder Ib mit R2 = H im Sinne einer Veresterung eines Alkohols (Houben-Weyl, Methoden der Organischen Chemie, Georg Thieme Verlag Stuttgart, Band E5, S. 656-715) umsetzt.

Die Verbindungen der Formel I oder Ib mit R2 ≠ H, die nach ba) bis bc) wie oben beschrieben erhalten werden, fallen entweder als Säureadditionssalz schwerlöslich aus - dann werden sie abgesaugt, mit wenig des eingesetzten Lösemittels gewaschen und getrocknet - oder sie bleiben in Lösung. Eine Reinigung kann so erfolgen, daß der Reaktionsansatz nach erfolgter Bildung der Ester der Formel I oder Ib mit R2 ungleich H mit einer anorganischen oder organischen Base, z.B. mit Kaliumcarbonat oder Triethylamin, neutralisiert und die resultierende Mischung konzentriert und anschließend an Kieselgel chromatographiert wird. Die so erhaltene reine Base der Formel I oder Ib mit R2 ungleich H kann, wie oben für Verbindungen der Formel I oder Ib mit R2 = H beschrieben, in ein Säureadditionssalz überführt werden.

Die Ausgangsstoffe der Formel III sind größtenteils in der Literatur beschrieben (vgl. Houben-Weyl, Methoden der organischen Chemie, Bd. 9, S. 384; 4. Aufl.; 1955 oder DOS 2640358 (16.03.1978)).

In den Verbindungen der Formel II kommen als Rest eines aktivierten Esters Z beispielsweise in Frage: Cl, Br, J, O-C(O)-C₆H₄-4-NO₂, O-SO₂-CH₃, O-SO₂-CF₃, O-SO₂-C₆H₄-4-CH₃, O-SO₂-C₆H₄. Sie können nach mehreren Methoden gewonnen werden:
ca) Es können Diazoketone der allgemeinen Formel VIII 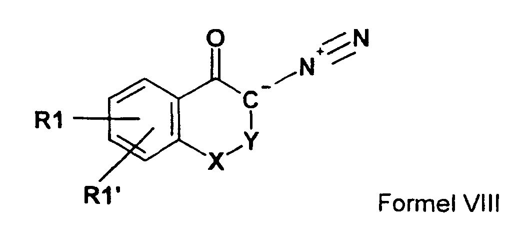 mit Halogenwasserstoffsäuren in die Verbindungen der allgemeinen Formel II überführt werden (Z = Cl, Br, J; R3 = H). Dieses Verfahren sowie einige Verbindungen der Formel II sind literaturbekannt (z.B. J. Am. Chem. Soc 80, 2255 (1958); J. Indian Chem. Soc. 42, 115 (1965)), die weiteren Verbindungen der Formel II lassen sich entsprechend herstellen und umsetzen. Die Diazoketone der allgemeinen Formel VIII können weiterhin nach literaturbekannten Verfahren über die Hydroxyverbindungen der allgemeinen Formel IX 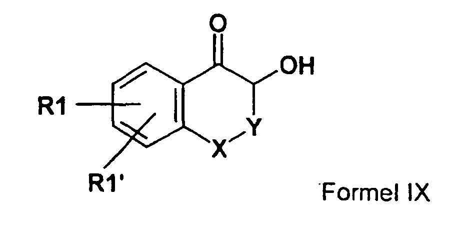 in die entsprechenden Verbindungen der Formel II (R3 = H) überführt werden.
cb) Da die unter ca) genannten Verfahren nur zu Verbindungen der allgemeinen Formel 11 führen, in denen R3 auf Wasserstoff beschränkt bleibt, stellt man Verbindungen der Formel II vorteilhaft dadurch her, daß man Verbindungen der allgemeinen Formel X 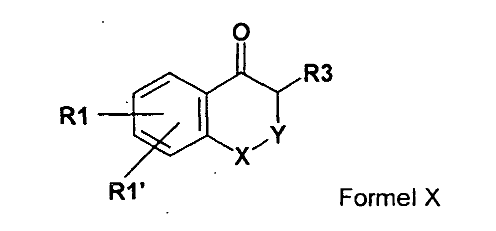 mit einem geeigneten Halogenierungsmittel, wie z.B. mit elementarem Chlor oder Brom, Sulfurylchlorid, Monochlorharnstoff, N-Chlor-Succinimid, Kupfer-II-bromid, Brom-Dioxan-Komplex, N-Brom-Succinimid unter literaturbekannten Bedingungen zur Reaktion bringt. Die bequem zugänglichen Verbindungen der Formel X sind entweder bekannt oder nach Literaturverfahren herstellbar.
   Als Halogenierungsmittel kommen beispielsweise elementares Chlor, Sulfurylchlorid, Monochlorharnstoff, N-Chlor-Succinimid, Brom-Dioxan-Komplex, N-Brom-Succinimid, insbesondere aber elementares Brom oder Kupfer-II-bromid in Betracht. Bei der Halogenierung mit Brom tropft man vorteilhaft Brom, ggf. in inerten Lösemitteln verdünnt, zu einer Lösung oder Suspension der äquimolaren Menge der Verbindung der Formel X in einem inerten Lösemittel. Als solche kommen beispielsweise Halogenkohlenwasserstoffe wie Di- oder Trichlormethan oder 1,2-Dichlorethan, bevorzugt aber Eisessig oder Essigsäureniederalkylester, oder Gemische der genannten Lösemittel in Betracht. Die Reaktionstemperatur liegt zwischen 0° und 50° C, bevorzugt zwischen 10° und 35° C. Halogenierungen von Ketonen werden durch Säuren katalysiert; deswegen ist es vorteilhaft, wenn man den Reaktionsansatz mit katalytischen Mengen einer Säure, z.B. mit Bromwasserstoffsäure, versetzt oder man nach Zutropfen von wenig Brom das Reaktionsgemisch zunächst bis zur Entfärbung des Halogens erwärmt und dann weiterbromiert.
   Bei der Bromierung der Verbindungen der Formel X mit Kupfer-II-bromid kann man analog den in J. Org. Chem. 29, 3459 (1964) oder J. Org. Chem. 40, 1990 (1975) beschriebenen Methoden arbeiten.
   Als Chlorierungsmittel eignet sich insbesondere Sulfurylchlorid, das man wie üblich mit einer Lösung oder Suspension der Verbindung der Formel X in einem Lösungsmittel, wie z.B. Tri- oder Tetrachlorethan oder einem Ether wie Diethylether oder tert.-Butylmethylether, in einem Temperaturbereich zwischen 20° und 80° C, zur Reaktion bringt. Man versetzt dann mit Eiswasser und arbeitet wie üblich auf. Bei Verwendung von Chlor als Halogenierungsmittel wird in die Lösung einer Verbindung der Formel X in einem polaren Lösungsmittel, z.B. Eisessig oder Dimethylformamid oder N-Methyl-2-pyrrolidon, zunächst HCI-Gas als Katalysator, dann in einem Temperaturbereich von 0° bis 25°C eine äquivalente Menge Chlor eingeleitet. Die Reaktionszeit beträgt 2 - 24 Stunden. Man versetzt dann mit Eiswasser und arbeitet wie üblich auf. Setzt man in die Chlorierungsreaktion N-Chlor-Succinimid als Chlorierungsmittel ein, so werden die Verbindungen der Formel X vorteilhaft in einem polaren Lösemittel, wie z.B. Eisessig, mit 1 - 2 Äquivalenten N-Chlor-Succinimid nach Zugabe einer katalytischen Menge Salzsäure bei ca. 50°C für 2 - 12 Stunden zur Reaktion gebracht. Man versetzt danach mit Eiswasser und arbeitet wie üblich auf.
cc) Die Verbindungen der Formel II können schließlich noch dadurch gewonnen werden, daß man die α-Hydroxyketone der allgemeinen Formel XI 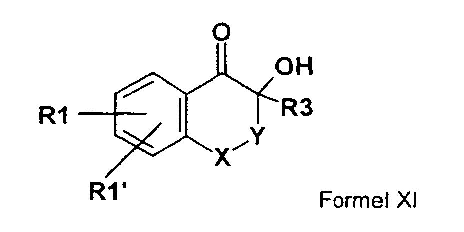 die entweder bekannt sind (Chem. Ber. 83, 390) oder nach gängigen Verfahren hergestellt werden können, in an sich bekannter Weise mit den aktivierten Derivaten organischer und anorganischer Säuren wie Methansulfonsäurechlorid, Trifluormethansulfonsäurechlorid, Ethansulfonsäurechlorid, Benzolsulfonsäurechlorid, p-Toluolsulfonsäurechlorid, Thionylbromid, Phosphortrichlorid, Phosphortribromid. Phosphoroxidchlorid, p-Nitrobenzoylchlorid zur Reaktion bringt.

Die so nach der jeweiligen Methode erhaltene Lösung oder Suspension der Verbindungen der Formel II engt man zweckmäßig unter vermindertem Druck ein und reinigt die Verbindungen der Formel II durch Kristallisation in inerten Lösemitteln wie z.B. Benzol, Toluol, Tetrachlormethan, Dichlormethan, 1,2-Dichlorethan, Cyclohexan, Hexan, Heptan. Eine andere Methode der Reinigung besteht darin, daß die Reaktionsgemische über eine Kieselgelsäule chromatographiert werden, wobei man zweckmäßigerweise als Elutionsmittel Heptan, Diethylether, tert.-Butylmethylether, Toluol, Essigsäureethylester oder Gemische davon verwendet.

Die erhaltenen Verbindungen der Formel II können auch ohne weitere Reinigungsoperation in die nächste Stufe eingesetzt werden.

Verbindungen der Formel X, 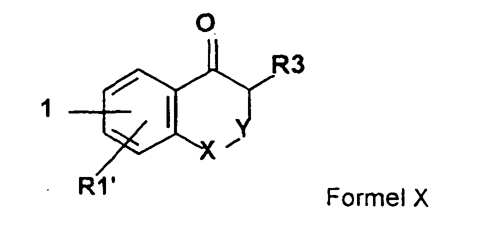 worin R3 ungleich Wasserstoff ist, sind auf unterschiedliche Weise zugänglich:
da) Verbindungen der Formel X, worin R3 = Fluor ist, können z.B. hergestellt werden, indem man Verbindungen der Formel X, worin R3 = H ist, mit einem elektrophilen Fluorierungsregenz umsetzt. Als elektrophile Fluorierungsreagenzien eignen sich (siehe auch: W. E. Barnette, J. Am. Chem. Soc. 106, 452-454 (1984)) z.B. 1-Fluor-2,4,6-Trimethylpyridiniumtriflat, 3,5-Dichlor-1-Fluorpyridiniumtriflat, 1-Fluorpyridiniumtriflat, 1-Fluorpyridiniumtetrafluoroborat, 1-Fluorpyridinium Pyridin Heptafluorodiborat, N-Fluor-N-Methyl-p-Toluolsulfonamid, N-Fluor-N-Propyl-p-Toluolsulfonamid, N-Fluorbenzolsulfonamid, N-Fluorbenzolsulfonimid [NFSi], 1-Fluor-4-Hydroxy-1,4-Diazoniabicyclo[2.2.2]oktan Bis(tetrafluoroborat) (NFTh). Die Fluorierungsreaktion wird z.B. so durchgeführt, daß zu einer Lösung oder Suspension einer Verbindung der Formel X, worin R3 = H ist, in einem unpolaren aprotischen Lösemittel wie Benzol, Toluol, Hexan oder Heptan oder in einem polaren aprotischen Lösemittel wie Tetrahydrofuran, Dimethylformamid, Acetonitril, Diethylether oder tert.-Butylmethylether oder in Gemischen davon, in äquivalenten Mengen eine Base zugefügt wird, mit deren Hilfe eine Verbindung der Formel X (R3 = H) in das Enolat der Verbindung der Formel X (R3 = H) überführt wird. Als Basen für diese Reaktion eignen sich n-Butyllithium, Kalium- oder Natriumhexamethyldisilazan, Natriumhydrid, Kaliumhydrid, Kalium-tert.butoxid, Methyllithium, Tetra-n-Butylammoniumhydroxid. Eine geeignete Reaktionstemperatur ist -78° bis 25°C. Zur Lösung oder Suspension des so gebildeten Enolats der Verbindung der Formel X (R3 = H) werden dann bei einer Temperatur von -78° bis +100° C, vorzugsweise bei einer Temperatur von -50° bis +80° C, 1 bis 2 , vorzugsweise 1,5 Äquivalente eines der o.g. Fluorierungsreagenzien, gelöst in einem der o.g. Lösemittel oder Lösemittelgemische, vorzugsweise gelöst in Toluol oder Dichlormethan, zugetropft. Die Reihenfolge der Zugabe der Reaktionspartner kann auch invers erfolgen, d.h., daß die Lösung oder Suspension des Enolates der Verbindung der Formel X (R3 = H) zu einer Lösung des Fluorierungsreagenzes bei den angegebenen Temperaturen zugetropft wird. Abhängig von der gewählten Reaktionstemperatur ist die Reaktion nach 15 Minuten bis 48 Stunden beendet. Die Umsetzung besonders mit N-Fluorbenzolsulfonimid kann in Analogie zu E. Differding und H. Ofner, Synlett 187-189 (1991) vorteilhaft auch so ausgeführt werden, daß man zuerst den Trimethylsilylenolether der Verbindung der Formel X, worin R3 = H ist, herstellt (z.B. mit Trifnethylsilylbromid oder Trifluormethansulfonsäuretrimethylsilylester in Toluol bei -78 bis 80°C unter Hinzufügung eines Äquivalents einer Base wie z.B. Triethylamin) und anschließend das Fluorierungsreagenz, gelöst in Dichlormethan oder Toluol, bei Raumtemperatur zugibt und nach einer ca. 12-stündigen Reaktionszeit (Raumtemperatur) aufarbeitet. Die Aufarbeitung des Reaktionsgemisches kann in der Art erfolgen, daß nach Neutralisation eines Überschusses der eingesetzten Base die Reaktionsmischung eingeengt wird und danach mit einem Lösemittel wie z.B. Essigsäureethylester oder Heptan versetzt und mit halbkonzentrierter Natriumhydrogencarbonatlösung ausgeschüttelt wird. Die organische Phase wird nach Trocknung über Magnesiumsulfat eingeengt und das Reaktionsprodukt kann dann zur weiteren Reinigung entweder aus einem Lösemittel wie Hexan oder Heptan umkristallisiert werden, oder aber auch einer chromatographischen Reinigung über eine Kieselgelsäule unter Elution mit z.B. Gemischen von Dichlormethan mit Heptan oder Essigsäureethylester mit Heptan unterzogen werden.
   Die Umsetzung mit 1-Fluor-4-Hydroxy-1,4-Diazoniabicyclo[2.2.2]oktan Bis(tetrafluoroborat) (NFTh) kann auch so durchgeführt werden, daß das Keton als solches, ohne Überführung in ein Enolat oder einen Enolsilylether, verwendet wird.
db) Verbindungen der Formel X, worin R3 = (C₁-C₆)-Alkyl oder (CH₂)ₙ-Aryl, und wobei n = 1-5 sein kann und Aryl wie weiter vorn definiert ist, können z.B. hergestellt werden, indem man Verbindungen der Formel X, worin R3 = H ist, mit einer starken Base und mit einem Alkylierungsreagenz der allgemeinen Formel R3-X umsetzt, wobei X = Br, J, O-C(O)-C₆H₄-4-NO₂, O-SO₂-CH₃, O-SO₂-CF₃, O-SO₂-C₆H₄-4-CH₃, O-SO₂-C₆H₄ sein kann. Um sicherzustellen, daß bei dieser Alkylierung nur die gewünschte Monoalkylierung stattfindet, wandelt man die Verbindungen der Formel X (R3 = H) zuvor vorteilhaft in Verbindungen z.B. der Formel XII, XIII oder XIV um. 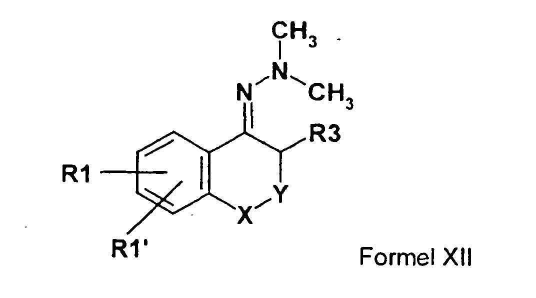 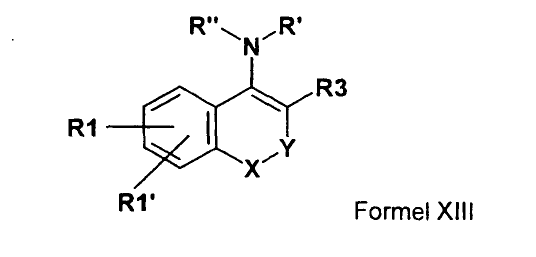 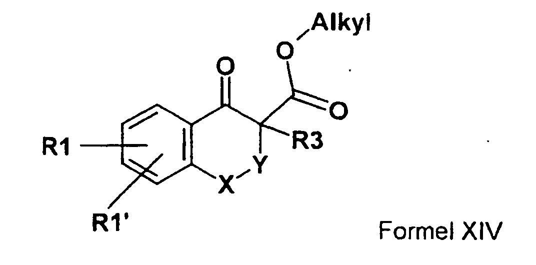
   Verbindungen vom Typ der Formel XII (R3 = H) können nach F. Henin et al., Tetrahedron, 50, 2849-2864 (1994) hergestellt werden. Solche vom Typ der Formel XIII (R3 = H), wobei R'-R" = -(CH₂)₄-, -(CH₂)₅-, -CH₂-CH₂-O-CH₂-CH₂- sein kann, lassen sich z.B. nach Stork et al., J. Am. Chem. Soc. 85, 207 (1963) herstellen. Verbindungen der Formel XIV (R3 = H) sind nach literaturbekannten Methoden, z.B. P. W. Hickmott, Chem. Ind. (London), 731 (1974), zugänglich. Verbindungen vom Typ der Formeln XII und XIV (R3 = H) können dann nach Überführung in ihr Anion mittels einer starken Base mit einem Alkylierungsmittel der allgemeinen Formel R3-X, wobei R3 und X wie weiter oben beschrieben definiert sind, zu den gewünschten Verbindungen der Formeln XII und XIV (R3 = H) umgesetzt und nach saurer Hydrolyse des Hydrazons (XII) bzw. Hydrolyse und Decarboxylierung des β-Ketoesters (XIV) in die erfindungsgemäßen Verbindungen der Formel X, worin R3 = H ist, umgewandelt werden. Verbindungen der Formel XIII (R3 = H) können in einem inerten Lösemittel wie Trichlormethan oder Toluol nach Zugabe einer Base wie Triethylamin mit Hilfe eines Alkylierungsmittels der allgemeinen Formel R3-X nach saurer Hydrolyse des α-alkylierten Enamins der Formel XIII (R3 = H) in Verbindungen der Formel X, worin R3 = H ist, umgewandelt werden. Verbindungen der Formel X, worin R3 = COOCH₃, COOCH₂CH₃, COOH, CONH₂, CONHCH₃, CON(CH₃)₂ ist, lassen sich über Verbindungen der Formel XII und der Formel XIII nach literaturbekannten Methoden durch Umsatz mit z.B. Cl-C(O)-O-CH₂-CH₃ darstellen.
dd) Verbindungen der allgemeinen Formel X, worin R3 = CN ist, können nach literaturbekannten Methoden (M. E. Kuehne, J. Org. Chem. 81, 5400-5404 (1959) durch Reaktion einer Verbindung der Formel XIII mit Chlorcyan hergestellt werden.
de) Verbindungen der allgemeinen Formel X, worin R3 = O-(C₁-C₆)-Alkyl ist, lassen sich nach literaturbekannten Methoden (J. Chan Lee et al., Synth. Commun. 27, 4085-90 (1997)) ausgehend von Verbindungen der allgemeinen Formel X, wobei R3 = H ist, so darstellen, daß man eine Verbindung der Formel X (R3 = H) mit [Hydroxy(p-Nitrobenzolsulfonyloxy)Jod]Benzol [HNJB] in Acetonitril für 2 - 6 Stunden unter Rückfluß kocht und das entstandene Zwischenprodukt nach Entfernung des Lösemittels direkt mit einem Alkohol der allgemeinen Formel R3-OH, wobei R3 die oben definierte Bedeutung hat, bei erhöhter Temperatur umsetzt.
df) In analoger Weise lassen sich auch Verbindungen der allgemeinen Formel X, worin R3 = O(O)CCH₃ ist, darstellen. Dabei wird das in de) beschriebene Zwischenprodukt in Essigsäure unter Hinzufügung katalytischer Mengen Silbercarbonats in eine Verbindung der Formel X, worin R3 = O(O)CCH₃ ist, überführt.
dg) Verbindungen der allgemeinen Formel X, worin R3 = N₃ ist, lassen sich entweder herstellen durch nukleophilen Austausch mit Azid aus den entsprechenden Verbindungen der Formel X, worin R3 = Cl oder Br ist (K. Van Sant, M. S. South; Tetrahedron Lett. 28, 6019 (1987)) oder aber besser in Analogie zu T. Patonay und R. V. Hoffman, J. Org. Chem. 59, 2902-2905 (1994) aus Enolacetaten der Verbindungen X (R3 = H) oder aus Enaminen der Verbindungen X (Verbindungen der Formel XIII (R3 = H)) über ein α-Tosyloxyketon und nachfolgender Reaktion mit Natriumazid.
dh) Verbindungen der Formel X, worin R3 = (C₂-C₆)-Alkinyl ist, können über 1,3-Dicarbonylverbindungen der allgemeinen Formel XIV hergestellt werden. In Analogie zu einer literaturbekannten Methode (M. Ochiai, T. Ito, Y. Takaoka, Y. Masaki, M. Kunishima, S. Tani, Y. Nagao; J. Chem. Soc., Chem. Commun. 118-119 (1990)) können dazu Verbindungen der Formel XIV (R3 = H) zuerst mit einer starken Base wie Kalium tert.-Butoxid in tert.-Butanol oder wie Kalium tert.-Butoxid in Tetrahydrofuran oder wie Natriumhydrid in Tetrahydrofuran in ihr Enolatanion überführt und dann mit Ethinyl(Phenyl)lodoniumtetrafluoroborat unter Bildung von Verbindungen der Formel XIV, worin R3 = (C₂-C₆)-Alkinyl ist, umgesetzt werden.
di) Verbindungen der allgemeinen Formel X, worin R3 = SO₂-(C₁-C₆)-Alkyl, SO₂-(CH₂)ₙ-Phenyl, SO-(C₁-C₆)-Alkyl, SO-(CH₂)ₙ-Phenyl, S-(C₁-C₆)-Alkyl, S-(CH₂)ₙ-Phenyl, wobei n = 0 -3 sein kann, ist, können hergestellt werden, indem man nach literaturbekannten Methoden das mittels einer starken Base wie Lithiumdiisopropylamid in einem Lösemittelgemisch wie Hexan/Pyridin generierte Enolatanion der Verbindungen der Formel X, worin R3 = H ist, mit dem entsprechenden Dialkyl-, Di(aralkyl)- oder Diphenyldisuifid bei tiefen Temperaturen umsetzt. Die auf diese Weise gewonnenen Verbindungen der Formel X, worin R3 = S-(C₁-C₆)-Alkyl oder S-(CH₂)ₙ-Phenyl mit n = 0 -3 ist, können mit Perselensäure zu den entsprechenden Sulfoxiden (R3 = SO-(C₁-C₆)-Alkyl, SO-(CH₂)n-Phenyl) (J. Drabowicz, M. Mikolajczyk; Synthesis 1978, 758) und mit einer Lösung von 30%igem Wasserstoffperoxid oder durch Reaktion mit m-Chlorperoxybenzoesäure in Dichlormethan zu den entsprechenden Sulfonen (R3 = SO₂-(C₁-C₆)-Alkyl, SO₂-(CH₂)ₙ-Phenyl) weiterverarbeitet werden.

Verbindungen der Formel X (R3 = H), die als Ausgangsprodukte für die Herstellung von Verbindungen der Formel II dienen, worin R3 = H, F, CN, N₃, O-(C₁-C₆)-Alkyl, (C₁-C₆)-Alkyl, (CH₂)n-Aryl, wobei n = 0-5 sein kann und Aryl gleich Phenyl, Thienyl, Pyridyl oder Furyl sein kann, (C₂-C₆)-Alkinyl, SO₂-(C₁-C₆)-Alkyl, SO₂-(CH₂)ₙ-Phenyl, SO-(C₁-C₆)-Alkyl, SO-(CH₂)ₙ-Phenyl, S-(C₁-C₆)-Alkyl, S-(CH₂)n-Phenyl, wobei n = 0 -3 sein kann, C(O)OCH₃, C(O)OCH₂CH₃, C(O)OH, C(O) NH₂, C(O)NHCH₃, C(O)N(CH₃)₂, O(O)CCH₃ ist, und Z die weiter oben genannten Bedeutungen hat, sind käuflich bzw. in Analogie zu literaturbekannten Verfahren auf unterschiedliche Weise zugänglich (Schema 1): 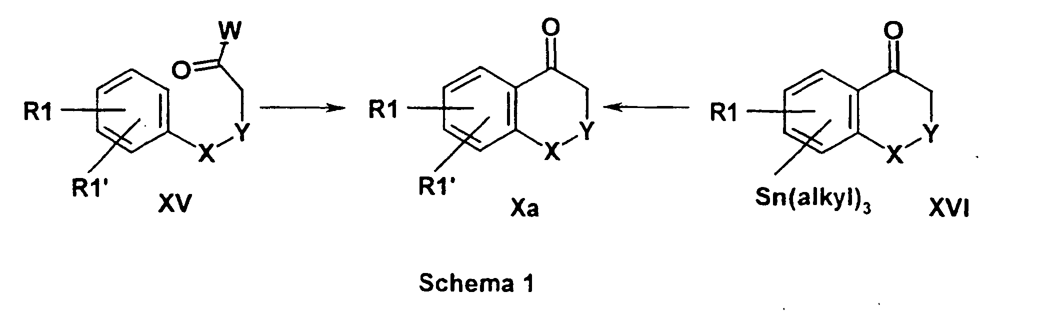
ea) Verbindungen der allgemeinen Formel XV, worin R1, R1', X und Y die eingangs genannten Bedeutungen haben, und worin Weine carbonylaktivierende Gruppe wie Halogen, z.B. Cl oder Br oder O-R" oder O-C(O)-R", wobei R" = Alkyl, z.B. CH₃ oder Aryl, z.B. Phenyl ist, darstellt, können nach literaturbekannten Methoden, z.B. Houben-Weyl, Methoden der Organischen Chemie, Thieme Verlag Stuttgart, 1973, Band VII/2a, S. 111 ff., mit Protonsäuren oder Lewis-Säuren zu den entsprechenden cyclisierten Verbindungen der allgemeinen Formel Xa ringgeschlossen werden.
eb) Verbindungen der allgemeinen Formeln X und Xa, worin R1 und/oder R1' = S-(C₁-C₆)-Alkyl, S-Phenyl, SO-(C₁-C₆)-Alkyl, SO-Phenyl, SO₂-(C₁-C₆)-Alkyl, SO₂-Phenyl, S-(CH₂)ₙ-Phenyl, SO-(CH₂)ₙ-Phenyl, SO₂-(CH₂)ₙ-Phenyl, wobei n = 0 - 6 sein kann, SO₂-NH₂, SO₂NH(C₁-C₆)-Alkyl, SO₂N[(C₁-C₆)-Alkyl]₂ bedeutet, können nach literaturbekannten Methoden (Houben-Weyl, Methoden der Organischen Chemie, Band XI/1, S. 422 ff. und 475 ff., Thieme Verlag Stuttgart) dadurch hergestellt werden, daß die entsprechend substituierten Verbindungen der Formel X oder Xa, worin R1 und/oder R1' = NO₂ ist, mit z.B. Raney-Nickel und Wasserstoff in einem Lösemittel wie z.B. Ethanol oder Eisessig, oder mit Zink in Eisessig, oder mit Zinn oder Zinn-(II)-chlorid in Salzsäure einer Reduktion zu den Aminen der Formel X oder Xa, worin R1 und/oder R1' = NH₂ ist, unterzogen und anschließend unter Umsatz mit Natriumnitrit in Salzsäure diazotiert und weiter mit CuCl₂ und SO₂ in Eisessig zu den entsprechend substituierten Sulfonsäurechloriden umgewandelt werden. Diese können nach Standardbedingungen im alkalischen Medium reduktiv in die entsprechenden Sulfinsäuren umgewandelt werden, welche dann ihrerseits zu Verbindungen der Formel X oder Xa, worin R1 und/oder R1' = S-(C₁-C₆)-Alkyl, S-Phenyl, SO-(C₁-C₆)-Alkyl, SO-Phenyl, SO₂-(C₁-C₆)-Alkyl, SO₂-Phenyl, S-(CH₂)n-Phenyl, SO-(CH₂)ₙ-Phenyl, SO₂-(CH₂)ₙ-Phenyl, wobei n = 0 - 6 sein kann, SO₂-NH₂, SO₂NH(C₁-C₆)-Alkyl, SO₂N[(C₁-C₆)-Alkyl]₂ ist, nach literaturbekannten Verfahren weiterverarbeitet werden.
   Verbindungen der Formel X oder Xa, worin R1 und/oder R1' = O-(C₁-C₆)-Alkyl, O-CH₂-CF₃, O-CH₂-CF₂-CF₃, O-(C₄-C₆)-Alkyl, wobei in den Alkylresten ein, mehrere, oder alle Wasserstoff(e) durch Fluor ersetzt sein können, oder ein Wasserstoff durch OH, OC(O)CH₃, O-CH₂-Ph, NH₂ oder N(COOCH₂Ph)₂ ersetzt sein kann, O-(CH₂)n-Phenyl, wobei n = 0 - 6 sein kann, S-(C₁-C₆)-Alkyl, S-Phenyl, S-(CH₂)ₙ-Phenyl, wobei n = 0 - 6 sein kann, ist, sind nach Standardmethoden aus Verbindungen der Formel X oder Xa, worin R1 und/oder R1' = F oder Br ist, durch nukleophilen Austausch mit Verbindungen der allgemeinen Formel R1-M oder R1'-M, worin R1 und R1' die oben genannten Bedeutungen haben und M für ein Alkalimetallatom, wie z.B. Na, oder ein vierfach substituiertes Stickstoffatom, wie z.B. (n-Bu)₄N, steht, zugänglich. Dazu wird z.B. eine Verbindung der Formel X oder Xa, worin R1 und/oder R1' = F ist, vorteilhaft in einem polaren aprotischen Lösemittel wie Dimethylformamid, N,N-Dimethylacetamid, N-Methyl-2-Pyrrolidon oder Dimethylsulfoxid mit einer Verbindung der Formel R1-H oder R1'-H, wobei R1 und R1' wie oben definiert sind, unter Hinzufügung einer Base wie z.B. Natrium- oder Kaliumcarbonat, Natriumhydrid, Kaliumhexamethyldisilazan, Kalium- oder Natriumhydroxid, Cäsiumcarbonat oder Tetra-n-butylammoniumhydroxid bei 50 bis 150°C zu Reaktion gebracht und danach wie üblich aufgearbeitet.
   Solche Verbindungen sind auch zugänglich, indem man Verbindungen der Formel X oder Xa, worin R1 und/oder R1' = Br ist, unter Bedingungen der Phasentransferkatalyse (E. V. Dehmlow, S. S. Dehmlow, Phase Transfer Catalysis, 2nd. Ed., Verlag Chemie, Weinheim, 1983) mit Verbindungen der Formel R1-H und/oder R1'-H mit z.B. einem Katalysator wie Aliquat 336 in einer Mischung aus Toluol mit 50%iger wässriger Natronlauge oder mit 15-Krone-5 in Toluol mit 50%iger wässriger Natronlauge für ca. 2 h zur Reaktion bringt und dann wie üblich aufarbeitet (A. J. Serio Duggan, E. J. J. Grabowski, W. K. Russ: Synthesis 573-5 (1980); A. Ohta, Y. Iwasaki, Y. Akita: Synthesis 828-9 (1982); W. Chin-Hsien, L. Xiang-Te, C. Xiao-Hun: Synthesis 858-61 (1982); H. Alsaidi, R. Gallo, J. Metzger: Synthesis 921-4 (1980)).
   Nach üblicher Aufarbeitung können so Verbindungen der allgemeinen Formeln X oder Xa, worin R1 und/oder R1' = O-(C₁-C₆)-Alkyl, O-CH₂-CF₃, O-CH₂-CF₂-CF₃, O-(C₄-C₆)-Alkyl, wobei in den Alkylresten ein, mehrere, oder alle Wasserstoff(e) durch Fluor ersetzt sein können durch Fluor ersetzt sein können, oder ein Wasserstoff durch OH, OC(O)CH₃, O-CH₂-Ph, NH₂ oder N(COOCH₂Ph)₂ ersetzt sein kann, O-(CH₂)ₙ-Phenyl, wobei n = 0 - 6 sein kann, S-(C₁-C₆)-Alkyl, S-Phenyl, S-(CH₂)ₙ-Phenyl, wobei n = 0 - 6 sein kann, ist, isoliert werden.
   Verbindungen der allgemeinen Formeln X und Xa, worin R1 und/oder R1' = CN ist, können nach Standardmethoden (L. Friedman, H. Shechter, J. Org. Chem. 26, 2522-24 (1961) aus z.B. Verbindungen der Formeln X und Xa, worin R1 und/oder R1' = Cl oder Br ist, durch nukleophilen Austausch mit einem Metallcyanid, wie z.B. NaCN, KCN, Cu-(I)-CN gewonnen werden. Als Lösemittel verwendet man dafür vorteilhaft ein polares, aprotisches Medium, wie z.B. Dimethylformamid, Dimethylacetamid, N-Methyl-2-Pyrrolidon oder Dimethylsulfoxid. Eine geeignete Reaktionstemperatur ist 150 bis 200°C.
ec) Verbindungen der allgemeinen Formeln XV und Xa, worin R1 und/oder R1' einen gegebenenfalls substituierten Aryl- oder Heteroarylrest bedeuten, können aus den entsprechenden Verbindungen der Formeln XV und Xa, worin R1 und/oder R1' Brom, Jod oder Trifluormethansulfonyloxy ist, nach literaturbekannten Methoden hergestellt werden. In Analogie zu N. Miyaura und A. Suzuki, Chem. Rev. 95, 2457-83 (1995) oder T. Oh-e, N. Miyaura und A. Suzuki, J. Org. Chem. 58, 2201-08 (1993) können solche Verbindungen ausgehend von Brom- oder Jod(hetero)arylen oder von (Hetero)Aryltriflaten der Formeln XV oder Xa, worin R1 und/oder R1' Brom, Jod, Trifluormethansulfonyloxy ist, durch Umsatz mit Arylboronsäuren oder -estern oder Arylborondialkylen der allgemeinen Formeln R1-B oder R1'-B, worin R1 und/oder R1' ein, gegebenenfalls substituierter, Arylrest wie z.B. Phenyl, Thienyl, Pyridyl oder Furyl sein kann und B ein borhaltiger Rest wie B(OH)₂, B(OCH₃)₂, B(O-(CH₂)₃-O), B(OC(CH₃)₂-C(CH₃)₂-O) oder B(CH₂-CH₃)₂ bedeutet, so gewonnen werden, daß man die Kupplungsreaktion unter Zusatz einer organischen Base wie z.B. Triethylamin oder einer anorganischen Base wie z.B. Natriumhydroxid, Natriumcarbonat, Natriumhydrogencarbonat, Kaliumcarbonat, Kaliumphosphat, Bariumhydroxid, Cäsiumfluorid oder Tetrabutylammoniumfluorid in einem Lösungsmittelgemisch aus z.B. Toluol und Wasser oder Aceton und Wasser oder Dimethoxyethan und Wasser oder in Lösemitteln wie Toluol, Benzol, Dimethoxyethan, Tetrahydrofuran, Dioxan, Aceton oder Dimethylformamid unter Hinzufügung eines Palladiumkatalysators bei 20 bis 150°C durchführt. Als Palladiumkatalysatoren kommen z.B. in Betracht: Pd(OAc)₂, Pd(PPh₃)₄, PdCl₂(PPh₃)₂, Palladium(II)-[1,1'-bis-(diphenylphosphino)-ferrocen]-chlorid-Methylenchlorid-Komplex, Pd[P(2-methoxyphenyl)₃]₄ oder Pd(DBA)₂/[(MeO)₃C₆H₂]PPh₂ ([DBA = Dibenzylidenaceton]).
ed) Außerdem können die Verbindungen der Formeln XV und Xa, worin R1 und/oder R1' einen, gegebenenfalls substituierten, Aryl- oder Heteroarylrest bedeuten, auch aus den entsprechenden Verbindungen der Formeln XV und Xa, worin R1 und/oder R1' einen borhaltigen Rest wie B(OCH₃)₂ oder B(OC(CH₃)₂-C(CH₃)₂-O) darstellen, gewonnen werden. Diese werden in Analogie zu T. lshiyama et al., J. Org. Chem. 60, 7508-10 (1995) oder M. Murata et al., J. Org. Chem. 62, 6458-59 (1997) aus den Verbindungen der Formeln XV und Xa, worin R1 und/oder R1' Brom oder Jod ist, durch Umsatz mit dem Pinakolester der Diboronsäure [(Me₄C₂O₂)BB(O₂C₂Me₄)] oder durch Umsatz mit 4,4,5,5-Tetramethyl-1,3,2-Dioxaborolan hergestellt. Als Lösungsmittel werden vorteilhaft Dimethylsulfoxid, Dimethylformamid, Dioxan oder Toluol oder Gemische davon eingesetzt. Die Reaktionstemperatur beträgt ca. 80 bis 100°C. Dem Raktionsgemisch werden weiter eine schwache Base wie z.B. Kaliumacetat und ein Palladiumkatalysator wie z. B. Palladium(II)-[1,1'-bis-(diphenylphosphino)-ferrocen]-chlorid-Methylenchlorid-Komplex oder PdCl₂(PPh₃)₂ zugesetzt. Die so erhaltenen Verbindungen der Formeln XV und Xa, worin R1 und/oder R1' ein borhaltiger Rest wie B(OCH₃)₂ oder B(OC(CH₃)₂-C(CH₃)₂-O) bedeutet, können dann wie unter ec) beschrieben mit einer Verbindung R1-Br, R1-J, R1-OTf zu Verbindungen der Formeln XV und Xa, worin R1 und/oder R1' einen, gegebenenfalls substituierten, Aryl- oder Heteroarylrest bedeuten, gekuppelt werden.
   Verbindungen der Formeln XV und Xa, worin R1 und/oder R1' einen borhaltigen Rest wie B(OH)₂, B(OCH₃)₂, B(O-(CH₂)₃-O), B(OC(CH₃)₂-C(CH₃)₂-O) oder B(CH₂-CH₃)₂ bedeutet, lassen sich auch in anderer Weise aus Verbindungen der Formeln XV oder Xa, worin R1 und oder R1' Brom oder Jod bedeutet, herstellen. Dazu werden nach literaturbekannten Methoden (z.B. M. Ishikura et al., Chem. Pharm. Bull. 33, 4755-63 (1985)) die Aryl- bzw. Heteroarylhalogenide der Formeln XV oder Xa, eventuell nach Schutz einer vorhandenen Carbonylfunktion als z.B. Acetal, mit Butyllithium oder Lithiumdiisopropylamid in Tetrahydrofuran bei -78°C in die entsprechenden Lithiumverbindungen überführt, welche dann ihrerseits mit einem Borsäureester wie z.B. Borsäuretrimethylester oder einem Alkoxydialkylboran wie z.B. Methoxydiethylboran zu einer borhaltigen Verbindung der Formeln XV oder Xa umgesetzt werden.
ee) Eine weitere Methode zur Gewinnung von Verbindungen der Formel Xa, worin R1 und/oder R1' für einen, gegebenenfalls substituierten, Aryl- oder Heteroarylrest stehen, besteht darin, daß Verbindungen der Formel XVI, worin R1 die eingangs genannten Bedeutungen hat, und Sn(alkyl)₃ z.B. gleich Sn(n-butyl)₃ ist, im Sinne einer Stille-Kupplung nach literaturbekannten Methoden, wie sie z.B. bei J. K. Stille, Angewandte Chemie, 98, 504-519 (1986), bei T. N. Mitchell, Synthesis 803-815 (1992) oder bei T. Gan et al., Tetrahedron Lett. 38, 8453-56 (1997) beschrieben sind, mit einer Verbindung R1'-Br oder R1'-J, wobei R1' ein, gegebenenfalls substituierter, Aryl- oder Heteroarylrest ist, unter Palladiumkatalyse mit z.B. Pd(PPh₃)₄, PdCl₂(PPh₃)₂, PdCl₂(PhCN)₂ oder PdCl₂(CH₃CN)₂ in einem Lösungsmittel wie z.B. Tetrahydrofuran, Toluol oder Dimethylformamid bei Temperaruren zwischen 20 und 150°C umgesetzt werden.
   Die zinnorganischen Verbindungen der Formel XVI ihrerseits sind z.B. aus ringbromierten oder -jodierten Vorstufen durch Umsatz mit Bistrialkylzinnverbindungen, z.B. Bistributylzinn (Bu₃SnSnBu₃), nach literaturbekannten Verfahren zugänglich (J. K. Stille, Angewandte Chemie, 98, 504-519 (1986); T. Gan et al., Tetrahedron Lett. 38, 8453-56 (1997)). Dabei wird ein Palladiumkatalysator wie z.B. Pd(PPh₃)₄ zugesetzt. Als Lösungsmittel kann z.B. Toluol dienen; die Reaktionstemperatur beträgt zwischen 20 und 110°C. Außerdem können die zinnorganischen Verbindungen der Formel XVI, wobei Sn(alkyl)₃ z.B. Sn(CH₃)₃ sein kann, in Analogie zu literaturbekannten Methoden (M. Gielen et al., Rev. Silicon Germanium Tin Lead Compd. 3, 9 (1977); M. Gielen, Rev. Silicon Germanium Tin Lead Compd. 5, 6 (1981)) durch Umsatz der entsprechenden ringbromierten Vorstufe mit Natriumtrimethylstannat bei ca. 0°C gewonnen werden.
ef) Ebenso können Verbindungen der Formel Xa, worin R1 und/oder R1' für einen, gegebenenfalls substituierten, Aryl- oder Heteroarylrest stehen, so gewonnen werden, daß Verbindungen der Formel Xa, worin R1 und/oder R1' für Brom oder Jod stehen, mit Aryl- oder Heteroarylzinntrialkylen der allgemeinen Formel R1-Sn(alkyl)₃ oder R1'-Sn(alkyl)₃, worin R1 und R1' für einen, gegebenenfalls substituierten, Aryl- oder Heteroarylrest stehen, unter den unter ee) beschriebenen Bedingungen zur Reaktion gebracht werden.
eg) Verbindungen der Formel Xa, worin R1 und/oder R1' = (C₂-C₆)-Alkinyl oder (C₂-C₆)-Alkenyl sind, können nach literaturbekannten Methoden, wie bei ec) und ee) beschrieben, durch palladiumkatalysierte Umsetzung von z. B. Trimethylsilylacetylen oder Alkinen (K. Sonagashira et al., Tetrahedron Lett. 4467 (1975); S. Takahashi et al., Synthesis 627 (1980)), Alkinylzinkbromiden (E. Negishi et al., J. Org. Chem. 62, 8957-60 (1997)) oder Trialkylzinnalkinen, von Trialkylzinnvinyl- oder allylverbindungen, von 1-Alkenylborverbindungen oder Vinylverbindungen (A. Hassner et al., J. Org. Chem. 49, 2546 (1984)) mit Verbindungen der Formel Xa, worin R1 und/oder R1' gleich Brom, Jod oder OTf ist, erhalten werden.

Die so nach der jeweiligen Methode erhaltene Lösung oder Suspension der Verbindungen der Formel II dampft man zweckmäßig unter vermindertem Druck ein und reinigt die Verbindungen der Formel II durch Kristallisation aus inerten Lösemitteln wie z.B. Benzol, Toluol, Tetrachlormethan, Dichlormethan, 1,2-Dichlorethan, Cyclohexan, Pentan, Heptan. Vorteilhaft können die so erhaltenen Verbindungen der Formel II auch ohne weitere Reinigungsoperationen in einem geeigneten inerten Lösemittel mit der 1 bis 1,5-fachen Menge eines Thioharnstoffs der Formel III in der oben unter a) beschriebenen Weise umgesetzt werden.

Für die in Verfahrensweise b) beschriebene Umsetzung der Verbindungen der Formel IV arbeitet man in einem Lösemittel mit den bekannten Verbindungen der Formel V. Als solche Lösemittel sind niedere Alkohole mit 1 bis 4 Kohlenstoffatomen sowie niedere Alkylester der Essigsäure mit 1 bis 4 Kohlenstoffatomen im Alkylteil wie beispielsweise Essigsäuremethylester und Essigsäureethylester besonders geeignet. Die Umsetzungen werden im allgemeinen in einem Temperaturbereich zwischen 0° und 60° C, vorzugsweise zwischen 15° und 35° C, durchgeführt, wobei die Reaktionsdauer zwischen 5 und 60 Stunden liegt.

Die Verbindungen der Formel I können aus einem inerten, geeigneten Lösungsmittel, wie beispielsweise Aceton, Methylethylketon, Acetonitril, Nitromethan umkristallisiert werden. Besonders vorteilhaft ist aber die Umfällung aus einem Lösungsmittel, wie beispielsweise in Dimethylformamid, Dimethylacetamid, Nitromethan, Acetonitril, Methanol, Ethanol, Isopropanol. Die freien Basen der allgemeinen Formel I können auch vorteilhaft chromatographisch über Kieselgel gereinigt werden. Als Elutionsmittel eignen sich Gemische von Dichlormethan mit Methanol, Essigsäurethylester mit Heptan, Essigsäureethylester mit Methanol.

Die Verbindungen der Formel I können ggf. mit einer Säure der Formel H-Z in ihre Salze überführt werden. Man kann dabei die Verbindungen I in die reinen Säuren bei Temperaturen zwischen 0° und 40°C eintragen, sofern diese flüssig sind bzw. einen nicht wesentlich höheren Schmelzpunkt als 40°C besitzen. Vorteilhaft arbeitet man aber in einem Lösungsmittel, wie beispielsweise in Wasser oder einem organischen Lösungsmittel, wie z.B. in Dioxan, Tetrahydrofuran, Diethylether, Diisopropylether, tert.-Butylmethylether, einem Essigsäureniederalkylester mit 1 - 4 Kohlenstoffatomen im Alkylteil, Acetonitril, Nitromethan, Aceton, Methylethylketon oder niederen Alkohol mit 1 - 4 Kohlenstoffatomen. Dabei werden pro Mol der Verbindungen I 1 - 1,5 Mol der Säure H-Z angewendet; man kann aber auch größere Mengen an Säure verwenden. Zweckmäßigerweise arbeitet man bei Temperaturen zwischen 0° und 40°C, bevorzugt zwischen 10° und 25°C.

Beim Arbeiten in wässriger Lösung kommt es nach Zugabe von Säuren H-Z im allgemeinen zur sofortigen Auflösung der Verbindungen der Formel I und nur selten zur Abscheidung der entsprechenden Säureadditionsverbindungen. Zweckmäßig isoliert man die erfindungsgemäßen Salze beim Erhalten einer Lösung durch schonendes Verdampfen des Wassers, vorzugsweise durch Gefriertrocknung. Beim Arbeiten in organischen Lösungsmitteln scheiden sich die Säureadditionssalze vielfach nach Zugabe der jeweiligen Säure H-Z schwerlöslich ab. Andernfalls scheidet man die Säureadditionsverbindungen, ggf. nach vorangegangener Konzentrierung, mit einem der genannten Fällungsmittel ab.

Die Säureadditionsprodukte fallen auch bei sehr hohem Reinigungsgrad gelegentlich in Form zäher Öle oder amorpher glasartiger Produkte an. Diese amorphen Produkte lassen sich unter Behandlung mit einem organischen Lösungsmittel bei 40° bis 80°C zur Kristallisation bringen. Hierfür eignen sich insbesondere Dialkylketone, wie Aceton oder Methylethylketon, niedere Dialkylether sowie Acetonitril, Nitromethan und ggf. auch niedere Alkohole.

Die Säureadditionsprodukte können durch Behandlung mit Basen zu den Verbindungen der allgemeinen Formel I deprotoniert werden. Als Basen kommen beispielsweise Lösungen anorganischer Hydroxide, wie Lithium-, Natrium-, Kalium-, Calcium- oder Bariumhydroxid. Carbonate oder Hydrogencarbonate, wie Natrium- oder Kaliumcarbonat, Natrium- oder Kaliumhydrogencarbonat, Ammoniak und Amine, wie Triethylamin, Diisopropylamin, Dicyclohexylamin, Piperidin, Morpholin, Methyldicyclohexylamin in Frage.

Beim Arbeiten im wässrigen Medium scheiden sich die freien basischen Verbindungen I häufig schwerlöslich ab und können durch Filtration oder Extraktion mit einem organischen Lösungsmittel, vorzugsweise mit Essigsäureethylester, abgetrennt und isoliert werden. Als organische Reaktionsmedien eignen sich besonders niedere Alkohole mit 1 - 4 Kohlenstoffatomen, vorzugsweise Methanol, Ethanol und Isopropanol; es können jedoch auch Essigsäureethylester, Diethylether, Tetrahydrofuran, Dioxan, Ethylenglykoldimethylether, Dimethylformamid verwendet werden. Man arbeitet bei -35° bis 60°C, bevorzugt zwischen 0° und 25°C. Wird ein mit Wasser mischbares organische Lösungsmittel verwendet, so fällt man, ggf. nach vorangehender Konzentrierung des Reaktionsgemisches, die freien Basen der Formel I durch Zugabe von Wasser aus. Bei Verwendung eines mit Wasser nicht mischbaren Lösemittels wäscht man nach der Umsetzung das Reaktionsgemisch mit Wasser und verdampft das organische Lösungsmittel.

Die nachfolgend aufgeführten Beispiele dienen zur Erläuterung der Erfindung, ohne diese jedoch einzuschränken. Die gemessenen Fest-, bzw. Zersetzungspunkte (Fp.) wurden nicht korrigiert und sind generell von der Aufheizgeschwindigkeit abhängig.

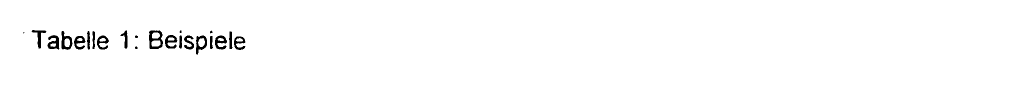

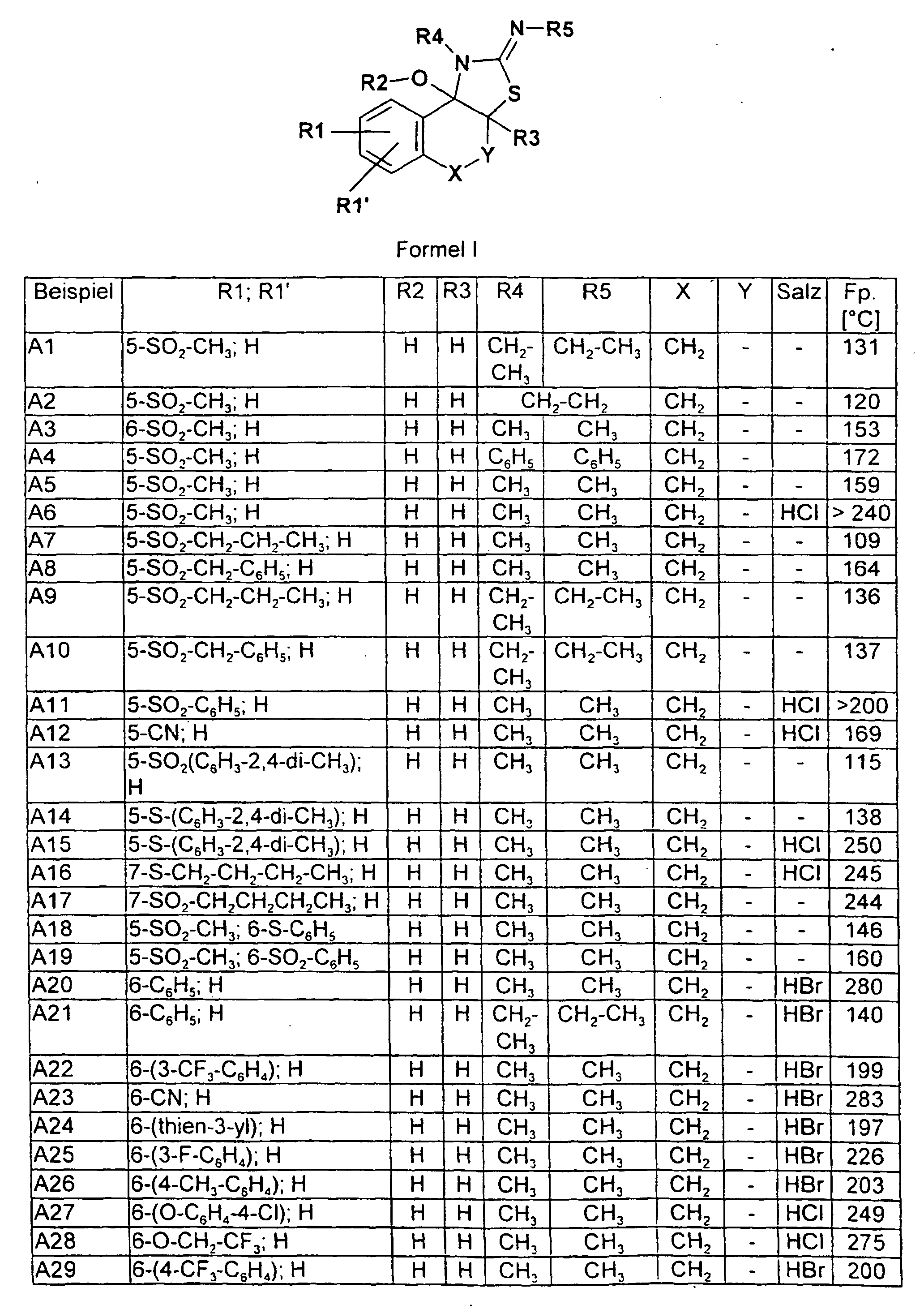

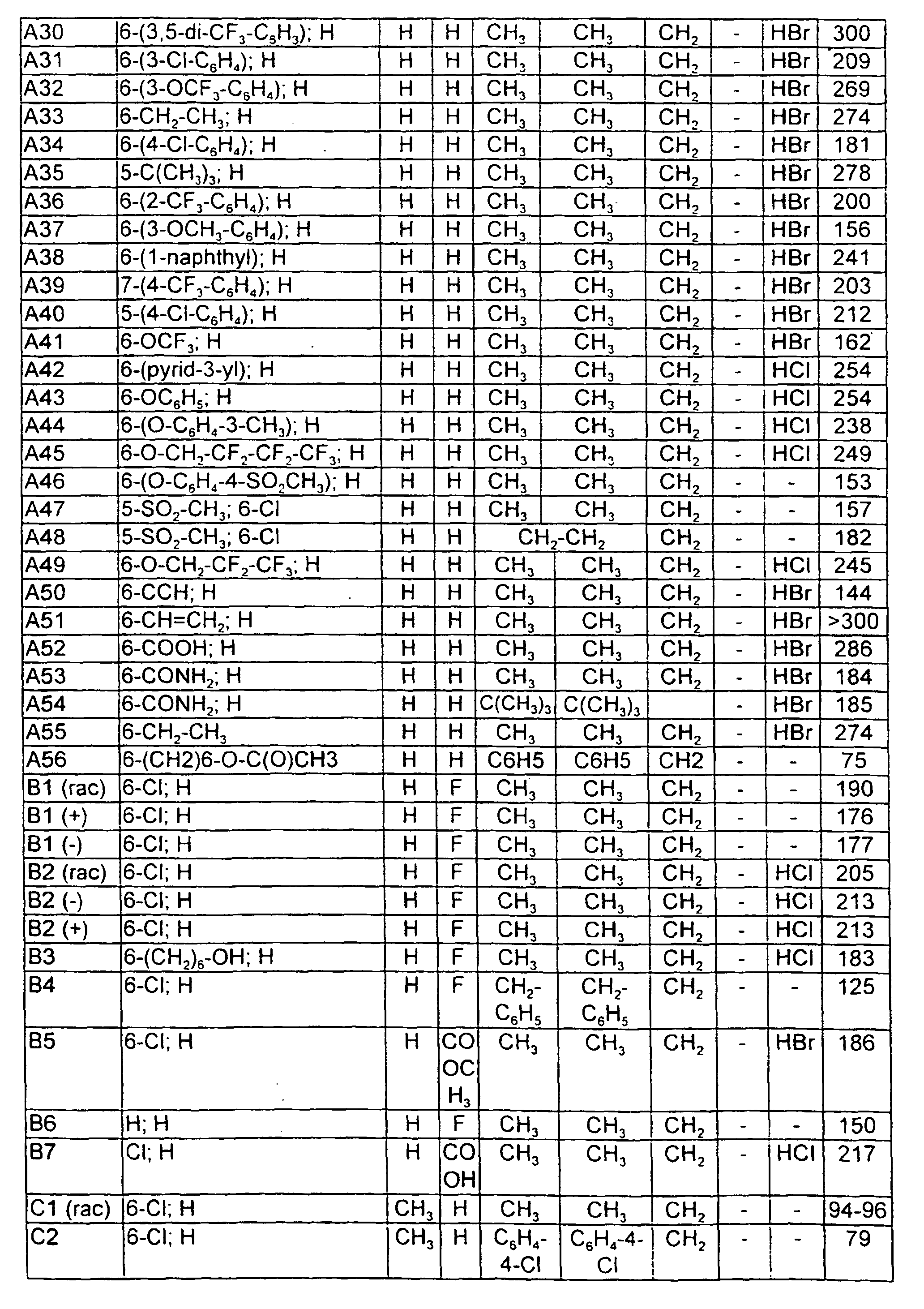

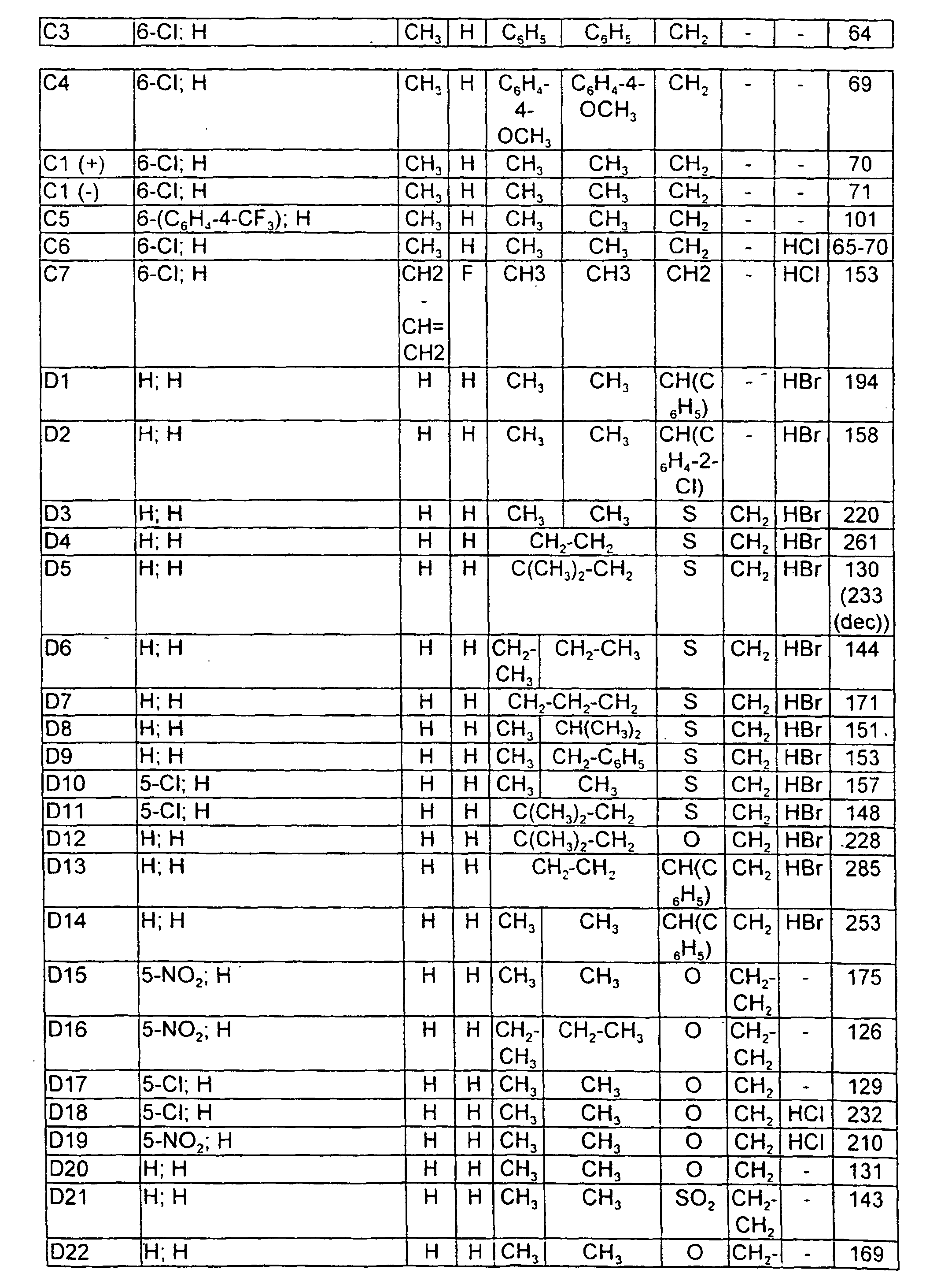

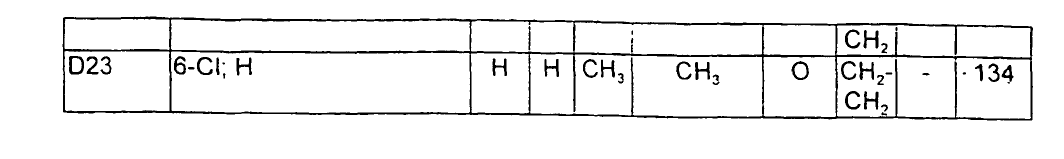

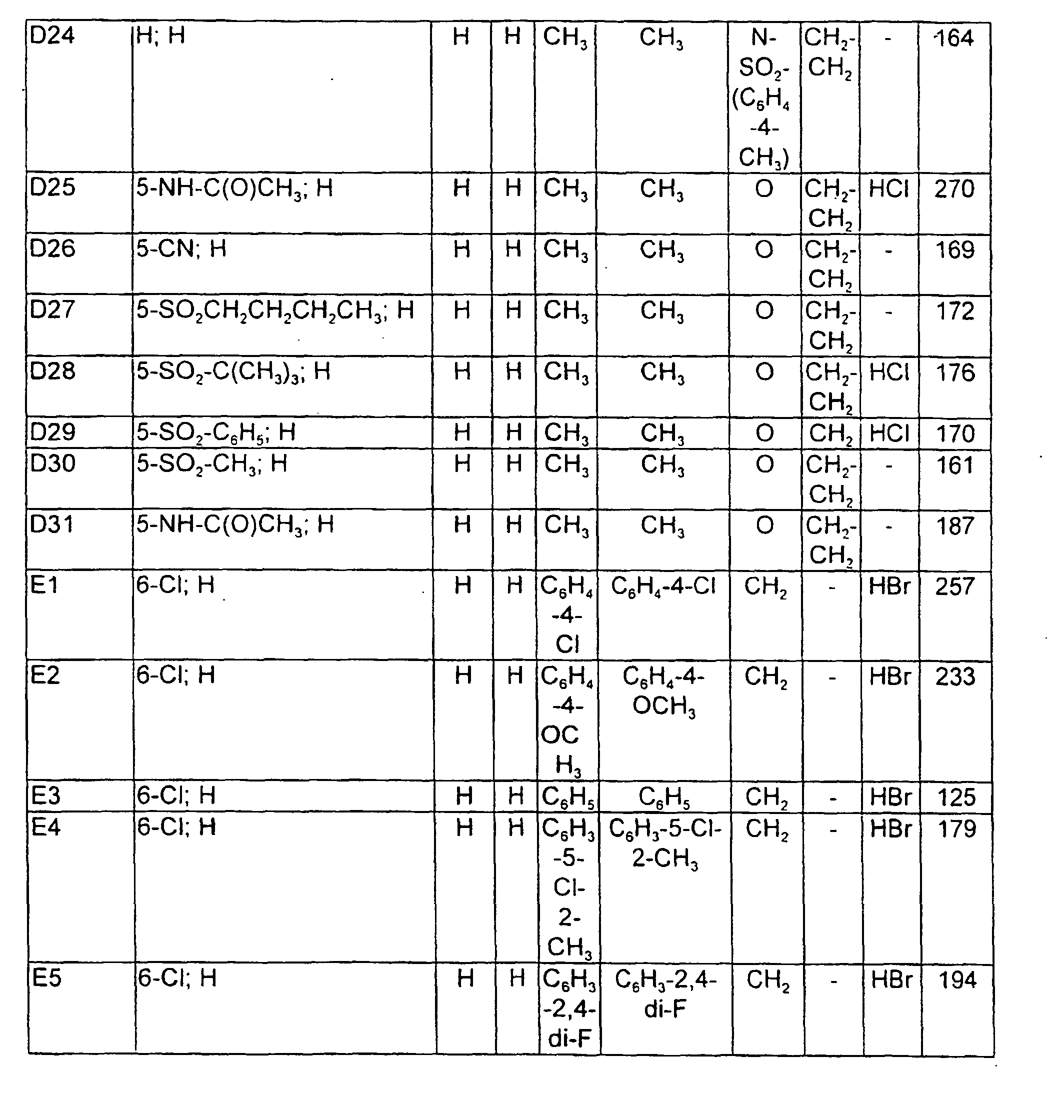

Die Verbindungen der Formel I zeichnen sich durch günstige Wirkungen auf den Fettstoffwechsel aus, insbesondere sind sie als Anorektika/Abmagerungsmittel geeignet. Die Verbindungen können allein oder in Kombination mit weiteren Abmagerungsmitteln (wie sie z.B. in Kapitel 01 der Roten Liste beschrieben sind) eingesetzt werden. Die Verbindungen eignen sich weiterhin zur Prophylaxe sowie zur Behandlung von Typ II Diabetes.

Die Wirksamkeit der Verbindungen wurde wie folgt getestet:

### Biologisches Prüfmodell:

Die Prüfung der anorektischen Wirkung erfolgte an männlichen NMRI Mäusen. Nach 24stündigem Futterentzug wurde über eine Schlundsonde das Testpräparat verabreicht. In Einzelhaltung und bei freiem Zugang zu Trinkwasser wurde den Tieren 30 Minuten nach Präparatgabe Kondensmilch angeboten. Der Kondensmilchverbrauch wurde halbstündlich 7 Stunden lang bestimmt und das Allgemeinbefinden der Tiere beobachtet. Der gemessene Milchverbrauch wurde mit dem unbehandelter Kontrolltieren verglichen.

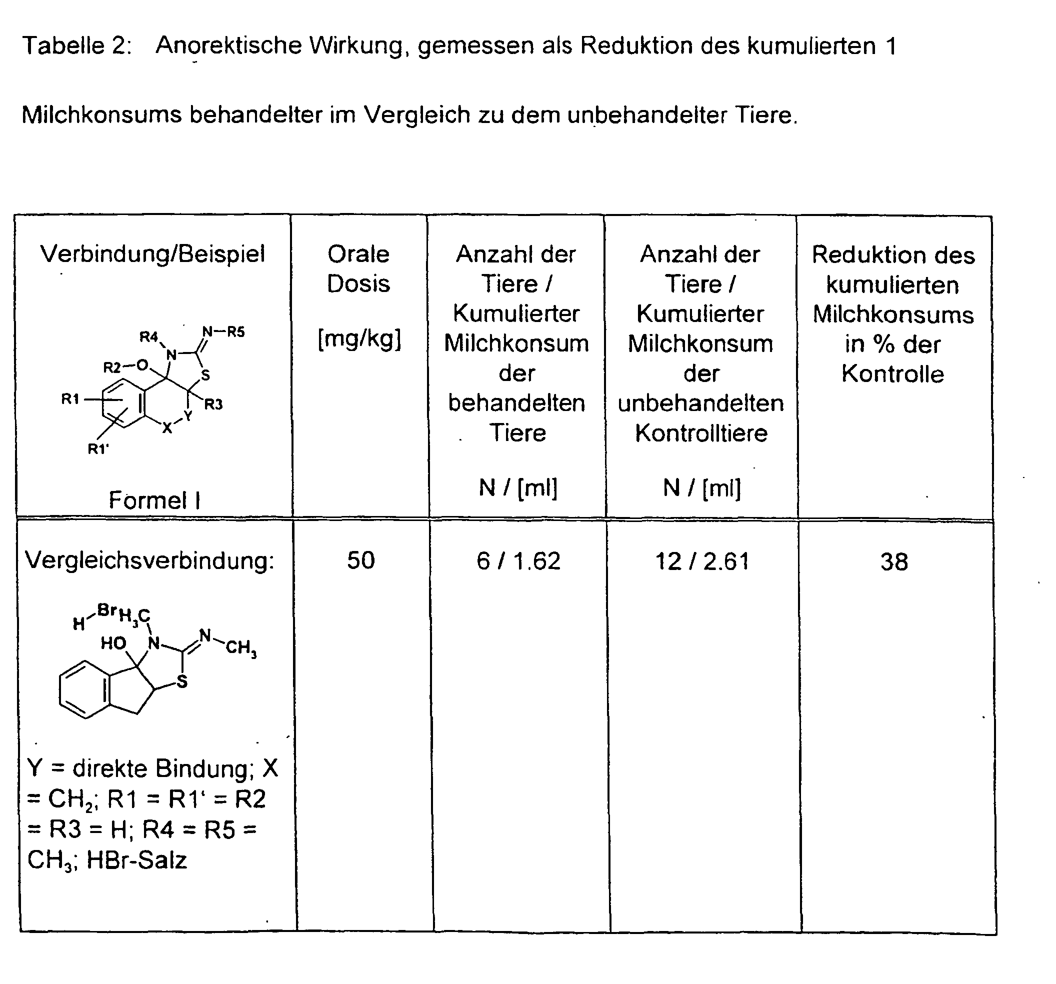

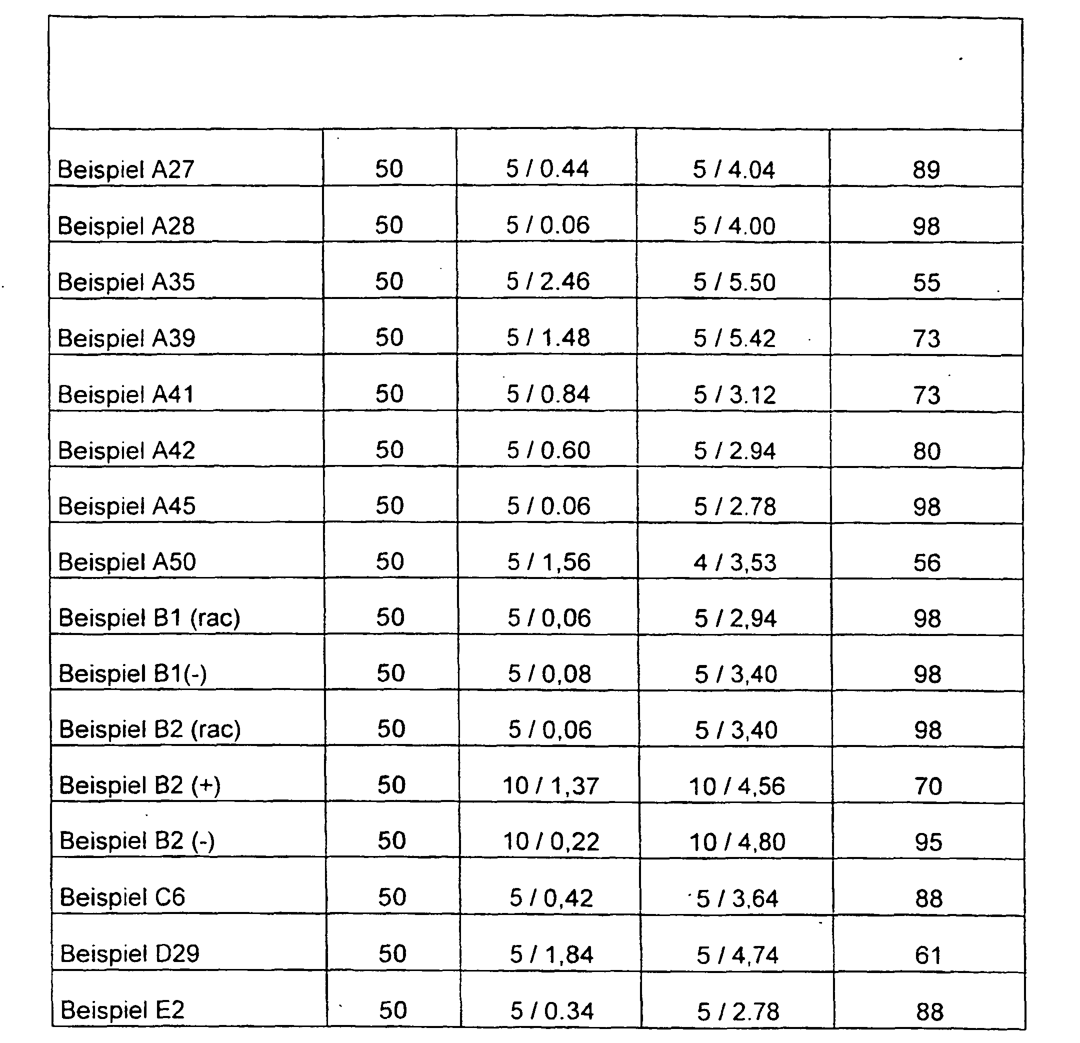

Aus der Tabelle ist abzulesen, daß die Verbindungen der Formel I eine sehr gute anorektische Wirkung zeigen. Die anorektische Wirkung ist auch gegenüber dem Vergleichsbeispiel deutlich verbessert.

Nachfolgend wird die Herstellung einiger Beispiele detailliert beschrieben, die übrigen Verbindungen der Formel I wurden analog erhalten:

### Beispiel 1 (Verbindung A4):

### 5-Methansulfonyl-3-phenyl-2-phenylimino-2,3,8,8a-tetrahydro-indeno[1,2-d]thiazol-3a-ol

a) Indan-1-on-6-sulfonsäurechlorid:
   Zu einer Suspension von 10.7 g kommerziell erhältlichem 6-Aminoindan-1-on in 100 ml halbkonzentrierter Salzsäure gibt man bei 0-5°C tropfenweise 5.2 g Natriumnitrit in 11 ml Wasser und rührt 15 Min nach. Die entstandene Lösung wird tropfenweise zu einer Mischung von 6.4 g CuCl2 x 2H2O in 20 ml Wasser und 195 ml einer gesättigten Lösung von SO2 in Eisessig gegeben. Nach dem Abklingen der Stickstoffentwicklung rührt man 1 Std bei Raumtemp. nach. Durch Zugabe von 400 ml Wasser erreicht man die Kristallisation des gewünschten Produkts (Indan-1-on-6-sulfonsäurechlorid), das abgesaugt und mit wenig kaltem Wasser nachgewaschen wird. Es hat einen Schmelzpunkt von 75°C.
b) Indan-1-on-6-sulfinsäure:
   Man löst 2.1g festes NaOH und 18.1 g NaHSO₃ in 53 ml Wasser. Dazu gibt man gleichzeitig 2N NaOH und trägt 8g Indan-1-on-6-sulfonsäurechlorid portionsweise ein, so daß der pH bei 7-7.2 bleibt. Die Temp. steigt dabei auf ca. 35°C. Man rührt 2 Std bei Raumtemp. nach, säuert mit conc. Salzsäure vorsichtig an und engt zur Trockne ein, kocht den Rückstand zweimal mit ca. 200 ml Methanol aus und engt das Filtrat wiederum ein. Der Rückstand wird mit wenig Aceton verrieben, abgesaugt und i. Vak. getrocknet. Man erhält Indan-1-on-6-sulfinsäure, die sich bei 275°C zersetzt.
c) 6-Methansulfonyl-indan-1-on:
   0.75 g Natrium werden in 75 ml wasserfreiem Methanol gelöst. Dazu gibt man portionsweise 5.9 g Indan-1-on-6-sulfinsäure und rührt 30 Min bei Raumtemp. Anschließend gibt man 7.5 ml Methyliodid dazu und rührt 4 Std bei Rückflusstemp. Nach Stehen über Nacht wird eingeengt, der Rückstand mit Wasser und CH₂Cl₂ ausgeschüttelt, die org. Phase getrocknet, eingeengt und der Rückstand mit Diisopropylether zur Kristallisation gebracht. Nach dem Absaugen und Trocknen erhält man 6-Methansulfonyl-indan-1-on mit dem Schmelzpunkt 155°C.
d) 2-Brom-6-methansulfonyl-indan-1-on:
   3.15 g 6-Methansulfonyl-indan-1-on werden in 50 ml Eisessig suspendiert und mit 0.4 ml 48proz. HBr-Lösung versetzt. Dazu gibt man bei Raumtemp. 0.92 ml Brom in 10 ml Eisessig und rührt 2 Std nach. Man gießt auf Eis, saugt den gebildeten Niederschlag ab und wäscht mit kaltem Wasser nach. Das Produkt wird durch Säulenchromatographie an SiO₂ mit Dichlormethan gereinigt. Als Vorfraktion erhält man etwas Dibromderivat und als Hauptprodukt 2-Brom-6-methansuffonyl-indan-1-on mit dem Schmelzpunkt 120°C.
e) 5-Methansulfonyl-3-phenyl-2-phenylimino-2,3,8,8a-tetrahydro-indeno-[1,2-d]thiazol-3a-ol:
   1.45 g 2-Brom-6-methansulfonyl-indan-1-on werden in 15 ml Aceton gelöst und unter Rühren mit 1.25 g N,N'-Diphenylthioharnstoff in 25 ml Aceton versetzt. Aus der klaren Lösung kristallisiert nach ca. 2 Std das Hydrobromid des 5-Methansulfonyl-3-phenyl-2-phenylimino-2,3,8,8a-tetrahydro-indeno[1,2-d]thiazol-3a-ols aus. Nach Stehen über Nacht wird abgesaugt und mit wenig Aceton nachgewaschen. 1.7 g Hydrobromid (Schmp. 247°C) werden in 10 ml Methanol gelöst und mit 0.8 ml Triethylamin versetzt. Nach 15 Min gibt man dazu 150 ml Wasser und rührt 30 Min unter Eiskühlung. Das gebildete Produkt wird abgesaugt und mit wenig kaltem Wasser nachgewaschen. Man erhält 5-Methansulfonyl-3-phenyl-2-phenylimino-2,3,8,8a-tetrahydro-indeno[1,2-d]thiazol-3a-ol mit dem Schmelzpunkt 172°C.

### Beispiel 2 (Verbindung A9):

### 3-Ethyl-2-ethylimino-5-(propan-1-sulfonyl)-2,3,8,8a-tetrahydro-indeno[1,2-d]thiazol-3a-ol

a) 6-(Propan-1-sulfonyl)-indan-1-on:
   0.25 g Natrium werden in 50 ml wasserfreiem Methanol gelöst. Dazu gibt man portionsweise 1,96 g Indan-1-on-6-sulfinsäure (siehe oben) und rührt 30 Min bei Raumtemp. Anschließend gibt man 1.87 g 1-lodpropan dazu und rührt 4 Std bei Rückflußtemp. Zur Vervollständigung der Umsetzung wird i. Vak. eingeengt, mit 5 ml lodpropan und 10 ml Toluol versetzt und 2 Std. zum Rückfluss erwärmt. Dann wird eingeengt, der Rückstand mit Wasser und CH₂Cl₂ ausgeschüttelt, die org. Phase getrocknet, eingeengt und der Rückstand durch Säulenfiltration (SiO₂; Cyclohexan/Essigester = 2/1) gereinigt. Man erhält 6-(Propan-1-sulfonyl)-indan-1-on mit dem Schmelzpunkt 100°C.
b) 2-Brom-6-(propan-1-sulfonyl)-indan-1-on:
   Die Umsetzung von 6-(Propan-1-sulfonyl)-indan-1-on zu 2-Brom-6-(propan-1-sulfonyl)-indan-1-on erfolgt analog zur Herstellung von 2-Brom-6-methansulfonyl-indan-1-on. Das Rohprodukt läßt sich durch Kristallisation und Nachwaschen mit niedrigsiedendem Petrolether reinigen. Man erhält 2-Brom-6-(propan-1-sulfonyl)-indan-1-on vom Schmelzpunkt 87-89°C.
c) 3-Ethyl-2-ethylimino-5-(propan-1-sulfonyl)-2,3,8,8a-tetrahydro-indeno(1,2-d]thiazol-3a-ol:
   951 mg 2-Brom-6-(propan-1-sulfonyl)-indan-1-on werden in 20 ml Aceton gelöst und unter Rühren mit 528 mg N,N'-Diethylharnstoff in 10 ml Aceton versetzt. Nach ca. 10 Min kristallisiert das Hydrobromid von 3-Ethyl-2-ethylimino-5-(propan-1-sulfonyl)-2,3,8,8a-tetrahydro-indeno[1,2-d]thiazol-3a-ol aus. Man rührt noch 1 Std. bei Raumtemp., saugt ab und wäscht mit wenig Aceton nach. Nach dem Trocknen erhält man das Hydrobromid von 3-Ethyl-2-ethylimino-5-(propan-1-sulfonyl)-2,3,8,8a-tetrahydro-indeno[1,2-d]thiazol-3a-ol mit dem Schmelzpunkt 146°C. Dieses wird in 10 ml Methanol gelöst und mit 0.4 ml Triethylamin versetzt. Nach 15 Min gibt man 50 ml Wasser dazu und rührt 1 Std. im Eisbad. Das gebildete Kristallisat wird abgesaugt, mit Wasser nachgewaschen und durch Säulenfiltration gereinigt (Si02; Ethylacetat/Methanol 3/1). Man erhält 3-Ethyl-2-ethylimino-5-(propan-1-sulfonyl)-2,3,8,8a-tetrahydro-indeno[1,2-d]thiazol-3a-ol mit dem Schmelzpunkt 136°C.

Die Verbindungen der Beispiele A1, A2 (Einsatz von lmidazolidin-2-thion), A3 (Einsatz von 5-Amino-indan-1-on), A5, A6, A7, A8, A10, A17 (Einsatz von 4-Amino-indan-1-on), A 47, A48 (Einsatz von 5-Chlor-6-nitro-indan-1-on) wurden in analoger Weise hergestellt.

### Beispiel 3 (Verbindung A21)

### 3-Ethyl-2-ethylimino-6-phenyl-2,3,8,8a-tetrahydro-indeno[1,2-d]thiazol-3a-ol Hydrobromid:

a) 5-Phenyl-indan-1-on:
   4,22 g 5-Brom-indan-1-on, 2,44 g Phenylboronsäure und 4,24 g Natriumcarbonat werden in einer Mischung aus 100 ml Toluol mit 20 ml Ethanol und 20 ml Wasser suspendiert. Nach der Zugabe von 450 mg Palladium-II-acetat und 1,05 g Triphenylphosphin wird die Mischung 5 h unter Rückfluß erhitzt. Danach wird der Ethanolanteil der Reaktionsmischung im Vakuum abdestilliert, die Reaktionsmischung mit 50 ml 0,5 N Natronlauge versetzt und 15 Minuten bei Raumtemperatur gerührt. Die organische Phase wird abgetrennt und der wässrige Teil noch zweimal mit Toluol ausgeschüttelt. Die vereinigten organischen Phasen werden mit Wasser, danach mit ges. Kochsalzlösung gewaschen, über Magnesiumsulfat getrocknet und im Vakuum eingeengt. Der Rückstand wird mit n-Heptan verrührt, abgesaugt, mit n-Pentan gewaschen und im Vakuum bei Raumtemperatur getrocknet. Man erhält 5-Phenyl-indan-1-on mit dem Schmelzpunkt 75°C.
b) 2-Brom-5-phenyl-indan-1-on:
   3,19 g 5-Phenyl-indan-1-on werden in 25 ml Eisessig gelöst. Man fügt 10 µl einer 48%igen Lösung von HBr in Eisessig hinzu und tropft während 15 Minuten eine Lösung von 0,592 ml Brom in 5 ml Eisessig unter Rühren bei Raumtemperatur zu. Die Reaktionsmischung wird zwei weitere Stunden bei Raumtemperatur gerührt bevor man 0, 057 ml Brom nachgibt, eine weitere Stunde rührt und dann den Reaktionsansatz auf Eiswasser (15 g Wasser, 45 g Eis, 150 mg Natriumhydrogencarbonat) gibt. Der ausgefallene Niederschlag wird abgesaugt und an Kieselgel mit Toluol /Essigsäureethylester 10/1 chromatographiert. Man erhält 2-Brom-5-phenyl-indan-1-on mit dem Schmelzpunkt 99-100°C.
c) 3-Ethyl-2-ethylimino-6-phenyl-2,3,8,8a-tetrahydro-indeno[1,2-d]thiazol-3a-ol Hydrobromid
   718 mg 2-Brom-5-phenyl-indan-1-on werden in 20 ml trockenem Aceton gelöst. Zu dieser Lösung wird bei 5°C eine Lösung von 397 mg N,N'-Diethylthioharnstoff in 10 ml trockenem Aceton während 5 Minuten zugetropft. Die Reaktionsmischung wird 2 Stunden bei Raumtemperatur gerührt, der Niederschlag anschließend abgesaugt und mit Aceton gewaschen. Man erhält 3-Ethyl-2-ethylimino-6-phenyl-2,3,8,8a-tetrahydro-indeno[1,2-d]thiazol-3a-ol Hydrobromid mit dem Schmelzpunkt 140-141°C (Zersetzung).

Ausgehend von 5-Brom-indan-1-on wurden in analoger Weise die Verbindungen A22, A24, A25, A26, A29, A30, A31, A32, A34, A36, A37, A38 hergestellt.

### Beispiel 4 (Verbindung A39):

### 3-Methyl-2-methylimino-7-(4-trifluormethyl-phenyl)-2,3,8,8a-tetrahydro-indeno[1,2-d]thiazol-3a-ol Hydrobromid

a) 4-(4-Trifluormethylphenyl)-indan-1-on: 6,33 g 4-Brom-indan-1-on, 5,7 g 4-(Trifluormethyl)-phenylboronsäure und 6,36 g Natriumcarbonat werden in einer Mischung aus 100 ml Toluol mit 20 ml Ethanol und 20 ml Wasser unter Rühren suspendiert. Unter einer Schutzgasatmosphäre (Argon) gibt man 320 mg Palladium-II-acetat und 787 mg Triphenylphosphin zu und rührt die Mischung 3 Stunden unter Rückfluß. Nach einer weiteren Stunde gibt man nochmals 1.45 g 4-(Trifluormethyl)-phenylboronsäure und 320 mg Palladium-II-acetat zu und kocht weitere 2 Stunden. Von der abgekühlten Reaktionsmischung wird das Ethanol im Vakuum abdestilliert, und der Rückstand wird mit 50 ml 0,5 N Natronlauge versetzt, gerührt und filtriert. Die organische Phase des Filtrats wird mehrmals mit jeweils 50 ml Wasser und schließlich mit 50 ml ges. Kochsalzlösung gewaschen, über Magnesiumsulfat getrocknet und eingeengt. Der Rückstand wird über Kieselgel mit n-Heptan /Essigsäureethylester 3/1 chromatographiert und man erhält 4-(4-Trifluormethylphenyl)-indan-1-on mit dem Schmelzpunkt 81°C.
b) 2-Brom-4-(4-Trifluormethylphenyl)-indan-1 -on:
   2,76 g 4-(4-Trifluormethylphenyl)-indan-1-on werden in 20 ml Eisessig gelöst. Man fügt 10 µl einer 48%igen Lösung von Bromwasserstoffsäure in Eisessig zu und tropft anschließendlangsam eine Lösung von 0,516 ml Brom in 5 ml Eisessig zu. Die Reaktionsmischung wird 3 Stunden bei Raumtemperatur gerührt und danach in eine Mischung aus 100 ml Wasser mit 100 g Eis und 100 mg Natriumhydrogencarbonat eingegossen. Die wässrige Suspension wird mit Dichlormethan ausgeschüttelt, die organische Phase dreimal mit Wasser gewaschen, über Magnesiumsulfat getrocknet, eingeengt und über Kieselgel mit Dichlormethan / n-Heptan 3/1 chromatographiert. Man erhält 2-Brom-4-(4-Trifluoromethylphenyl)-indan-1-on mit dem Schmelzpunkt 94-97°C.
c) 3-Methyl-2-methylimino-7-(4-trifluormethyl-phenyl)-2,3,8,8a-tetrahydro-indeno[1,2-d]thiazol-3a-ol Hydrobromid:
   426 mg 2-Brom-4-(4-Trifluormethylphenyl)-indan-1-on und 131 mg N,N'-Dimethylthioharnstoff werden in 10 ml Aceton bei Raumtemperatur zusammengegeben und 4 Stunden gerührt. Der ausgefallene Niederschlag wird abgesaugt, mit Aceton nachgewaschen und im Vakuum getrocknet.
Man erhält 3-Methyl-2-methylimino-7-(4-trifluormethyl-phenyl)-2,3,8,8a-tetrahydro-indeno[1,2-d]thiazol-3a-ol Hydrobromid mit dem Schmelzpunkt 202-204°C.

Ausgehend von 4-Brom-indan-1-on wurde die Verbindung A40 in analoger Weise hergestellt.

### Beispiel 5 (Verbindung A42)

### 3-Methyl-2-methylimino-6-pyridin-3-yl-2,3,8,8a-tetrahydro-indeno[1,2-d]thiazol-3a-ol Hydrochlorid

a) 5-Pyridin-3-yl-indan-1-on:
   13,26 g 3-Brompyridin werden in 160 ml Diethylether gelöst und auf -60°C abgekühlt. Zu dieser Lösung werden während 30 Minuten 52 ml einer 1,6 molaren Lösung von n-Butyllithium in n-Hexan zugetropft. Man läßt die Lösung sich auf -30°C erwärmen und fügt bei dieser Temperatur tropfenweise unter Rühren 9,5 ml Borsäuretrimethylester zu. Das Reaktionsgemisch wird anschließend 3 Stunden unter Rückfluß erhitzt, dann auf 0°C abgekühlt und tropfenweise mit 6,1 ml 1,3-Propandiol versetzt. Diese Mischung wird 30 Minuten bei 0°C gerührt, bevor 5,46 ml Methansulfonsäure zugetropft werden und weitere 30 Minuten gerührt wird. Danach fügt man 20 g Celite zu, erwärmt das Gemisch auf Raumtemperatur, filtriert, engt das Filtrat ein, verrührt den Rückstand in 700 ml Toluol, filtriert erneut und destilliert das Lösemittel im Vakuum ab. 4,1 g des Rückstands (3-[1,3,2]Dioxaborinan-2-yl-pyridin) werden ohne weitere Reinigung zusammen mit 4,22 g 5-Bromindan-1-on und 4,24 g Natriumcarbonat in einer Mischung aus 100 ml Toluol mit 20 ml Ethanol und 20 ml Wasser gelöst. Die Lösung wird mit Argon entgast, und anschließend werden 112 mg Palladium-II-acetat und 262 mg Triphenylphosphin zugegeben. Das Reaktionsgemisch wird 4 Stunden unter Rückfluß gekocht, auf Raumtemperatur abgekühlt und der Ethanolanteil des Gemisches wird im Vakuum abdestilliert. Sodann werden unter Rühren 50 ml einer 0,5 N Natronlauge zugesetzt, die organische Phase abgetrennt und die wässrige Phase mit Toluol ausgeschüttelt. Die vereinigten organischen Phasen werden nacheinander mit Wasser und ges. Kochsalzlösung ausgeschüttelt, über Magnesiumsulfat getrocknet, im Vakuum eingeengt und über Kieselgel mit Essigsäureethylester / n-Heptan 1/1 chromatographisch gereinigt. Man erhält 5-pyridin-3-yl-indan-1-on mit dem Schmelzpunkt 103 - 106°C.
b) 2-Chlor-5-pyridin-3-yl-indan-1-on:
   3,22 g 5-pyridin-3-yl-indan-1-on werden in 160 ml Dichlormethan gelöst und bei 0°C während 15 Minuten tropfenweise mit einer Lösung von 1,34 ml Sulfurylchlorid in 40 ml Dichlormethan versetzt. Es wird 30 Minuten bei 0°C und dann 60 Minuten bei Raumtemperatur nachgerührt, bevor man langsam 50 ml einer ges. Natriumhydrogencarbonatlösung zufügt. Die organische Phase wird abgetrennt, mit Wasser gewaschen, über Magnesiumsulfat getrocknet, im Vakuum eingeengt und über Kieselgel mit Dichlormethan / Methanol 50/1 chromatographisch gereinigt. Man erhält (neben 2,2-Dichlor-5-pyridin-3-yl-indan-1-on mit dem Schmelzpunkt 109°C) 2-Chlor-5-pyridin-3-yl-indan-1-on mit dem Schmelzpunkt 103-105°C.
c) 3-Methyl-2-methylimino-6-pyridin-3-yl-2,3,8,8a-tetrahydro-indeno [1,2-d]thiazol-3a-ol Hydrochlorid:
   366 mg 2-Chlor-5-pyridin-3-yl-indan-1-on und 235 mg N,N'-Dimethylthioharnstoff werden in 5 ml Methanol gelöst und 7 Stunden unter Rückfluß gekocht. Die Reaktionsmischung wird abgekühlt und im Vakuum eingeengt. Der Rückstand wird mit 5 ml Aceton versetzt, 30 Minuten im Ultraschallbad gerührt und dann abgesaugt. Der Rückstand wird mit Aceton gewaschen und im Vakuum getrocknet. Man erhält das Hydrochlorid des 3-Methyl-2-methylimino-6-pyridin-3-yl-2,3,8,8a-tetrahydro-indeno [1,2-d]thiazol-3a-ols mit einem Schmelzpunkt von 253 - 255°C.

### Beispiel 6 (Verbindung A41):

### 3-Methyl-2-methylimino-6-trifluormethoxy-2,3,8,8a-tetrahydro-indeno [1,2-d]thiazol-3a-ol Hydrobromid:

a) 3-Trifluormethoxy-zimtsäuremethylester:
   9.15 g 3-Trifluormethoxy-zimtsäure werden in 90 ml Methanol gelöst. Man fügt 0,25 ml konz. Schwefelsäure zu und kocht die Mischung 5 Stunden unter Rückfluß. Die abgekühlte Lösung wird vorsichtig mit 0,9 g Natriumhydrogencarbonat versetzt, 5 Minuten gerührt und dann im Vakuum eingeengt. Der Rückstand wird in 250 ml Essigsäureethylester aufgenommen, zweimal mit jeweils 50 ml Wasser gewaschen und über Magnesiumsulfat getrocknet. Die Lösung wird im Vakuum eingeengt und das zurückbleibende helle Öl wird ohne weitere reinigung weiter umgesetzt.
b) 3-(3-Trifluormethoxy-phenyl)-propionsäuremethylester:
   9,6 g 3-Trifluormethoxy-zimtsäuremethylester werden in 200 ml Methanol gelöst. Man fügt 750 mg Palladium auf Kohle (10%ig) zu und hydriert bei Normaldruck. Nach üblicher Aufarbeitung wird 3-(3-Trifluormethoxy-phenyl)-propionsäuremethylester als helles Öl erhalten.
c) 5-Trifluormethoxy-indan-1-on:
   9,37 g 3-(3-Trifluormethoxy-phenyl)-propionsäuremethylester werden in 50 ml Ethanol mit 25 ml Wasser gelöst. Man fügt 3,74 g Kaliumhydroxid zu und kocht 45 Minuten unter Ruckfluß. Die abgekühlte Lösung wird eingeengt, der Rückstand mit 25 ml Wasser versetzt, und unter Rühren wird so lange konz. Salzsäure zugefügt bis der pH-Wert 1 beträgt. Die wässrige Reaktionsmischung wird zweimal mit jeweils 75 ml Dichlormethan ausgeschüttelt. Die organische Phase wird mit Wasser gewaschen, über Magnesiumsulfat getrocknet, eingeengt und im Vakuum getrocknet. Man erhält 3-(3-Trifluormethoxy-phenyl)-propionsäure als ein farbloses Öl. 6,24 g der Säure werden in 40 ml Toluol gelöst, mit 2,1 ml Thionylchlorid versetzt und 1 Stunde unter Rückfluß gekocht. Die abgekühlte Reaktionsmischung wird eingeengt, der Rückstand in 5 ml Toluol aufgenommen und erneut eingeengt. Man erhält 3-(3-Trifluormethoxy-phenyl)-propionsäurechlorid, welches ohne weitere Reinigung in 30 ml Dichlormethan gelöst und bei 0 - 5°C während 15 Minuten zu einer Suspension von 5,49 g wasserfreiem Aluminiumtrichlorid in 40 ml Dichlormethan unter Rühren zugetropft wird. Die Reaktionsmischung wird eine Stunde bei 0°C gerührt und anschließend auf 40 ml Eiswasser gegeben. Die organische Phase wird abgetrennt und die wässrige Phase nochmals mit 40 ml Dichlormethan ausgeschüttelt. Die vereinigten organischen Phasen werden mit 40 ml ges. Natriumhydrogencarbonatlösung und Wasser gewaschen, über Magnesiumsulfat getrocknet, im Vakuum eingeengt und zur Reinigung über Kieselgel mit Toluol / Essigsäureethylester 20/1 chromatographisch gereinigt. Man erhält 5-Trifluormethoxy-indan-1-on als ein hellgelbes Öl.
d) 2-Brom-5-trifluormethoxy-indan-1-on:
   5,3 g 5-Trifluormethoxy-indan-1-on werden in 50 ml Eisessig gelöst, mit 110 µl Bromwasserstoffsäure (48%ig in Eisessig) versetzt und bei Raumtemperatur tropfenweise mit einer Lösung von 1,305 ml Brom in 12 ml Essigsäure versetzt. Die Reaktionsmischung wird 90 Minuten bei Raumtemperatur gerührt, anschließend mit 70 ml Wasser versetzt und zweimal mit jeweils 100 ml Dichlormethan ausgeschüttelt. Die organischen Phasen werden mit 50 ml Wasser gewaschen, über Magnesiumsulfat getrocknet, im Vakuum eingeengt und über Kieselgel mit Toluol /Essigsäureethylester 50/1 chromatographiert. Man erhält 2-Brom-5-trifluormethoxy-indan-1-on als ein Wachs.
e) 3-Methyl-2-methylimino-6-trifluormethoxy-2,3,8,8a-tetrahydro-indeno [1,2-d]thiazol-3a-ol Hydrobromid:
   0,197 g 2-Brom-5-trifluormethoxy-indan-1-on werden mit 0,104 g N,N'-Dimethylthioharnstoff in 5 ml Aceton gelöst und 5 Stunden bei Raumtemperatur gerührt. Die erhaltene Suspension wird noch 1 Stunde bei 0°C gerührt und dann abgesaugt; der Rückstand wird mit Aceton gewaschen und im Vakuum getrocknet. Das Hydrobromid des 3-Methyl-2-methylimino-6-trifluormethoxy-2,3,8,8a-tetrahydro-indeno [1,2-d]thiazol-3a-ols schmilzt bei 162°C unter Zersetzung.

### Beispiel 7 (Verbindung A35):

### 5-tert-Butyl-3-methyl-2-methylimino-2,3,8,8a-tetrahydro-indeno[1,2-d]thiazol-3a-ol Hydrobromid:

a) 3-(4-tert-Butyl-phenyl)-acrylsäuremethylester:
   3-(4-tert-Butyl-phenyl)-acrylsäure wird analog dem Beispiel 6a) in Methanol mit Schwefelsäure zum 3-(4-tert-Butyl-phenyl)-acrylsäuremethylester umgestzt. Der Ester hat eine wachsartige Konsistenz mit einem Schmelzpunkt von ca. 35°C.
b) 3-(4-tert-Butyl-phenyl)-propionsäuremethylester:
   10,0 g des 3-(4-tert-Butyl-phenyl)-acrylsäuremethylesters werden in 75 ml absolutem methanol gelöst. Portionsweise werden innerhalb einer Stunde 2,45 g Magnesiumspäne eingetragen, und die Mischung wird drei Stunden bei Raumtemperatur gerührt. Danach werden 380 mg Magnesiumspäne nachgegeben und man rührt eine weitere Stunde. Unter Kühlung (Eisbad) werden unter Rühren vorsichtig 90 ml 2N Salzsäure zugetropft. Sodann wird das Methanol im Vakuum entfernt, der Rückstand zweimal mit jeweils 200 ml Dichlormethan extrahiert, die Dichlormethanphase mit Wasser neutral gewaschen (2 x 50 ml), über Magnesiumsulfat getrocknet und eingeengt. Man erhält 3-(4-tert-Butyl-phenyl)-propionsäuremethylester als eine farblose, wachsartige Verbindung.
c) 3-(4-tert-Butyl-phenyl)-propionsäure:
   9,90 g 3-(4-tert-Buty)-phenyl)-propionsäuremethylester werden un einer Mischung aus 30 ml Ethanol mit 15 ml Wasser gelöst, mit 4,46 g Kaliumhydroxid versetzt, 45 Minuten unter Rückfluß erhitzt, danach im Vakuum eingeengt, mit 30 ml Wasser versetzt und unter Kühlung mit dem Eisbad mit konz. Salzsäure auf pH 1 gestellt. Die wässrige Lösung wird dreimal mit jeweils 100 ml Dichlormethan ausgeschüttelt, mit Wasser neutral gewaschen, über Magnesiumsulfat getrocknet und im Vakuum eingeengt. Der Rückstand wird mit 100 ml 25%iger Essigsäure verrührt, auf 10°C abgekühlt und abgesaugt und im Vakuum getrocknet. Man erhält 3-(4-tert-Butyl-phenyl)-propionsäure, welche ohne weitere Reinigung in die nächste Stufe eingesetzt wird.
d) 3-(4-tert-Butyl-phenyl)-propionylchlorid:
   Das Säurechlorid wird wie bei Beispiel 6c) beschrieben hergestellt und ohne weitere Reinigung in die nächste Stufe eingesetzt.
e) 6-tert-Butyl-indan-1-on:
   Aus dem Säurechlorid 7d wird das 6-tert-Butyl-indan-1-on wie bei 6c) beschrieben hergestellt. Das 6-tert-Butyl-indan-1-on hat einen Schmelzpunkt von 94 - 96°C.
f) 2-Brom-6-tert-butyl-indan-1-on:
   Nach dem Verfahren wie es bei 6d) beschrieben ist, wird 6-tert-Butyl-indan-1-on in 2-Brom-6-tert-butyl-indan-1-on überführt; es hat einen Schmelzpunkt von 58 - 61°C.
g) 5-tert-Butyl-3-methyl-2-methylimino-2,3,8,8a-tetrahydro-indeno[1,2-d]thiazol-3a-ol Hydrobromid:
   Die Umsetzung von 267 mg 2-Brom-6-tert-butyl-indan-1-on mit 156 mg N,N'-Dimethylthioharnstoff in 10 ml Aceton liefert das Hydrobromid des 5-tert-Butyl-3-methyl-2-methylimino-2,3,8,8a-tetrahydro-indeno[1,2-d]thiazol-3a-ols mit dem Schmelzpunkt 277 - 279°C.

### Beispiel 8 (Verbindung A23):

### 3a-Hydroxy-3-methyl-2-methylimino-3,3a,8,8a-tetrahydro-2H-indeno[1,2-d]thiazol-6-carbonitril Hydrobromid:

a) 1-Oxo-indan-5-carbonitril:
   9,5 g 5-Brom-indan-1-on und 4,93 g CuCN werden in 10 ml Dimethylformamid suspendiert und 4 Stunden unter Rückfluß gekocht. Zur abgekühlten, dunkelbraunen, viskosen Suspension wird unter Rühren eine Lösung von 18 g Eisen-III-chlorid in 5 ml konz. Salzsäure mit 30 ml Wasser zugetropft und anschließend für 30 Minuten bei 70°C gerührt. Die Reaktionsmischung wird dreimal mit 50 ml Toluol ausgeschüttelt, und die vereinigten organischen Phasen werden mit 50 ml 2N Salzsäure und 50 ml 2 N Natronlauge ausgeschüttelt und dann mit Wasser neutral gewaschen. Der Toluolextrakt wird über Magnesiumsulfat getrocknet, im Vakuum eingeengt, und der Rückstand wird aus n-Heptan umkristallisiert. Man erhält 1-Oxo-indan-5-carbonitril mit dem Schmelzpunkt 123 - 125°C.
b) 2-Brom-1-oxo-indan-5-carbonitril:
   Die Bromierung des 1-Oxo-indan-5-carbonitrils erfolgt so wie beim Beispiel 6d) beschrieben und liefert 2-Brom-1-oxo-indan-5-carbonitril mit dem Schmelzpunkt 115 - 118°C.
c) 3a-Hydroxy-3-methyl-2-methylimino-3,3a,8,8a-tetrahydro-2H-indeno[1,2-d]thiazole-6-carbonitril Hydrobromid:
   236 mg 2-Brom-1-oxo-indan-5-carbonitril werden in 10 ml Aceton gelöst und bei 0 - 5°C mit 210 mg N,N'-Dimethylthioharnstoff versetzt. Die Mischung wird 3 Stunden bei Raumtemperatur und 1 Stunde bei 0°C gerührt. Das Reaktionsprodukt wird abgesaugt, mit Aceton gewaschen und im Vakuum getrocknet. Man erhält das Hydrobromid des 3a-Hydroxy-3-methyl-2-methylimino-3,3a,8,8a-tetrahydro-2H-indeno[1,2-d]thiazol-6-carbonitrils mit dem Schmelzpunkt 282 - 284°C (Zersetzung).

In analoger Weise wurde die Verbindung A12, ausgehend von 6-Brom-indan-1-on, hergestellt.

### Beispiel 9 (Verbindung A18):

### 3-Methyl-2-methylimino-5-methylsulfonyl-6-phenylthio-2,3,8,8a-tetrahydro-indeno[1,2-d]thiazol-3a-ol

a) 6-Methylsulfonyl-5-phenylthio-1-indanon:
   Zu einer Suspension, hergestellt aus 4,9 g 5-Chlor-6-methylsulfonyl-1-indanon, 1,4 g wasserfreies gemahlenes Kaliumcarbonat und 50 ml DMF, tropft man unter Argonatmosphäre und Rührung 2,55 ml Thiophenol und erwärmt unter Beibehaltung der Rührung für ca 10 Stunden auf 80°C. Nach Zugabe von 500ml Wasser bei Raumtemperatur filtriert man die Kristalle ab und reinigt durch Auflösung in Aceton, Behandlung mit Aktivkohle und anschließender Ausfällung mit Wasser. Hellgelbe kristalline Substanz, Schmelzpunkt 211-212°C.
b) 2-Brom-6-methylsulfonyl-5-phenylthio-1-indanon
   erhält man durch Zutropfen einer Mischung aus 0,39 ml Brom in 10 ml Eisessig zu einer gerührten Lösung aus 2,4 g 6-Methylsulfonyl-5-phenylthio-1-indanon, 0,2 ml einer konzentrierten wäßrigen Bromwasserstoffsäure und 30 ml Eisessig, anschließendem Eingießen des Reaktionsgemisches in eine Suspension aus Wasser und Eis und nachfolgendem Filtrieren des kristallinen Produkts. Schwach gelbe kristalline Substanz, Schmelzpunkt 158-160°C.
c) 3-Methyl-2-methylimino-5-methylsulfonyl-6-phenylthio-2,3,8,8a-tetrahydro-indeno[1,2-d]thiazol-3a-ol Hydrobromid
   In eine Lösung von 2,7 g 2-Brom-6-methylsulfonyl-5-phenylthio-1-indanon in 30 ml Aceton gibt man 0, 72g N,N'-Dimethylthioharnstoff, rührt eine Stunde bei Raumtemperatur, filtriert die kristalline Verbindung vom Schmelzpunkt unter Zersetzung 268-270 °C.
d) 3-Methyl-2-methylimino-5-methylsulfonyl-6-phenylthio-2,3,8,8a-tetrahydro-indeno[1,2-d]thiazol-3a-ol
   3-Methyl-2-methylimino-5-methylsulfonyl-6-phenylthio-2,3,8,8a-tetrahydro-indeno[1,2-d]thiazol-3a-ol Hydrobromid wird in Methanol suspendiert und mit 3-fachen molaren Überschuß an Triethylamin versetzt. Die entstehende Lösung wird filtriert und durch Anreiben mit einem Glasstab zur Kristallisation gebracht. Farblose kristalline Verbindung, Schmelzpunkt unter Zersetzung 142 - 143 °C.

### Beispiel 10 (Verbindung A48):

### 8-Chlor-5a-hydroxy-3,4-dihydro-7-methylsulfonyl-indano[2,1-b]imidazo[1,2-d]thiazolidin Hydrobromid

a) 5-Chlor-6-nitro-1-indanon:
   86g 5-Chlor-1-indanon werden unter Rührung und äußerer Kühlung in 540 ml rauchende Salpertersäure mit einer Dichte von 1.52 g/ml so eingetragen, daß die Innentemperatur zwischen - 15 und - 10°C gehalten wird. Man gießt das Reaktionsgemisch in eine gerührte Wasser/Eis Suspension ein und filtriert die kristalline gelbe Substanz ab. Reinigung durch Auflösung in einem Gemisch aus 2 Teilen Aceton und 5 Teilen Ethanol, Behandlung mit Aktivkohle und weitgehende Entfernung des Acetonanteils durch Destillation unter vermindertem Druck. Schmelzpunkt 126 - 128°C.
b) 6-Amino-5-chlor-1-indanon:
   Zu einer kochenden Suspension aus 30 g 5-Chlor-6-nitro-1-indanon in 400 ml Wasser tropft man im Verlauf einer Stunde unter Beibehaltung des Siedens eine Lösung aus 77,5 g Natriumhydrogensulfit in 250 ml Wasser und kocht weitere 2 Stunden unter Rückfluß. Man stellt vorsichtig mit konzentrierter Salzsäure auf pH 1 - 2 und erhitzt eine weitere Stunde zum Sieden. Nach dem Abkühlen werden die Kristalle abfiltriert, mit Wasser gewaschen und aus Isopropanol umkristallisiert. Farblose bis bräunliche kristalline Substanz, Schmelzpunkt 210 - 212 °C unter Zersetzung.
c) 5-Chlor-6-chlorsulfonyl-1-indanon:
   Zu einer Suspension von 26,8 g 6-Amino-5-chlor-1-indanon in 200 ml 20%iger Salzsäure tropft man unter guter Rührung und Kühlung eine Lösung von 10,4 g Natriumnitrit in 30 ml Wasser, wobei die Temperatur zwischen 0 und 5 °C gehalten wird. Dabei wird eine Lösung erhalten, die nach weiterem Rührenlassen bei 0°C portionsweise unter Rührung in eine Lösung aus 12,7g Kupfer-II-chlorid Dihydrat, 50 ml Wasser und 400 ml mit Schwefeldioxid gesättigtem Eisessig bei 0 - 5°C gegeben wird. Man rührt eine weitere Stunde ohne externe Kühlung, fügt anschließend 600 ml Wasser zu und filtriert die Kristalle ab, die mehrmals auf dem Filter mit kaltem Wasser gewaschen und anschließend im Vakuum getrocknet werden. Farblose kristalline Substanz; Schmelzpunkt 147 - 148°C.
d) 5-Chlor-1-indanon-6-sulfinsäure:
   Zu einer Lösung aus 26g Natriumhydrogensulfit und 3 g NaOH in 75 ml Wasser trägt man portionsweise unter Rührung 13,3 g 5-Chlor-6-chlorsulfonyl-1-indanon ein, wobei gleichzeitig aus einem Tropftrichter 2N Natromlauge so zugetropft wird, daß der pH zwischen 7 und 7,5 gehalten wird. Sodann stellt man mit konzentrierter Salzsäure auf pH 1 - 2, kühlt auf 0 bis -5°C, filtriert die Kristalle ab und wäscht mit Wasser nach. Schmelzpunkt > 300°C mit Schwarzfärbung ab 170°C.
e) 5-Chlor-6-methylsulfonyl-1-indanon:
   Zu einer Natriummethylatlösung, die aus 150ml Methanol und 1 g Natrium hergestellt wurde, gibt man zuerst 9,2 g 5-Chlor-1-indanon-6-sulfinsäure und anschließend 15g Methyljodid und erhitzt das Gemisch unter dem aufgesetzten Intensivkühler 10 Stunden zum Sieden. Nach Abdestillieren des Lösungsmittels versetzt man den Rückstand mit 200 ml Wasser, filtriert die Kristalle ab und wäscht das Produkt sorgfältig mit Wasser. Farblose kristalline Substanz aus Methanol nach Behandlung mit Aktivkohle, Schmelzpunkt 197-198 °C.
f) 2-Brom-5-chlor-6-methylsulfonyl-1-indanon:
   erhält man aus 6,8 g 5-Chlor-6-methylsulfonyl-1-indanon und 1.45g Brom in Eisessig. Farblose bis leicht bräunliche kristalline Substanz, Schmelzpunkt 144°C.
g) 8-Chlor-5a-hydroxy-3,4-dihydro-7-methylsulfonyl-indano[2,1-b]imidazo[1,2-d]thiazolidin Hydrobromid
   Zu einer Lösung aus 3,24 g 2-Brom-5-chlor-6-methylsulfonyl-1-indanon in 50 ml Aceton fürgt man unter Rührung eine warme Lösung aus 1,02 g 2-Imidazolidinthion in 7,5 ml Dimethylacetamid und rührt zwei weitere Stunden bei Raumtemperatur nach. Die farblose kristalline Verbindung wird abfiltriert und mehrmals mit Aceton gewaschen. Zersetzungspunkt 135°C.
h) 8-Chlor-5a-hydroxy-3,4-dihydro-7-methylsulfonyl-indano[2,1-b]imidazo[1,2-d]thiazolidin
   erhält man analog der in Beispiel 9 angegebenen Vorschrift aus 8-Chlor-5a-hydroxy-3,4-dihydro-7-methylsulfonyl-indano[2, 1-b]imidazo[1,2-d]thiazolidin Hydrobromid und Triethylamin in Methanol. Farblose kristalline Substanz, Zersetzungspunkt 192 °C.

### Beispiel 11 (Verbindung A27):

### 6-(4-Chlorphenoxy)-3-methyl-2-methylimino-2,3,8,8a-tetrahydro-indeno[1,2-d]thiazol-3a-ol Hydrochlorid

a) 5-(4-Chlorphenoxy)-1-indanon:
   2,82 g 4-Chlorphenol werden nach Auflösung in 60 ml wasserfreiem Dimethylacetamid mit 8,2 g wasserfreien und gemahlenen Kaliumkarbonat ½ Stunde bei Raumtemperatur gerührt. Nach Zugabe von 1,5 g 5-Fluorindanon rührt man 10 Stunden bei 120 - 130°C und destilliert nach Abkühlung das Lösungsmittel unter vermindertem Druck ab. Man versetzt den Rückstand mit Wasser und extrahiert mehrmals mit Ethylacetat. Die organische Phase wird mit 2N NaOH und nachfolgend mit Wasser gewaschen, sodann über 15 Minuten nach Zugabe von Aktivkohle gerührt und das Lösungsmittel nach dem Trocknen über wasserfreiem Magnesiumsulfat unter vermindertem Druck abdestilliert. Der teilweise kristalline dunkle Rückstand wird durch Säulenchromatografie an Kieselgel mit einem Laufmittel, bestehend aus gleichen Teilen Ethylacetat und Toluol, gereinigt. Braune Kristalle, Schmelzpunkt 75 - 80 °C.
b) 2-Brom-5-(4-chlorphenoxy)-1-indanon:
   Zu einer Lösung von 1,3 g 5-(4-Chlorphenoxy)-1-indanon in 30 ml Essigsäureethylester tropft man etwa ½ ml einer Lösung aus 0,25 ml Brom in 5 ml Essigsäureethylester und erwärmt langsam bis zur Entfärbung des Broms bzw. bis zur beginnenden HBr-Entwicklung. Sodann kühlt man ab und tropft bei Raumtemperatur die restliche Brommenge zu, läßt weitere 2 Stunden rühren und destilliert das Lösungsmittel unter vermindertem Druck ab. Das zurückbrleibende dunkle Öl wird ohne weitere Reinigung verwendet.
c) 6-(4-Chlorphenoxy)-3-methyl-2-methylimino-2,3,8,8a-tetrahydro-indeno[1,2-d]thiazol-3a-ol Hydrobromid
   erhält man durch Umsetzung von 1,69 g 2-Brom-5-(4-chlorphenoxy)-1-indanon und 0,52 g N,N'-Dimethylthioharnstoff in 25 ml Ethylacetat als hellgelben bis farblosen kristallinen Niederschlag. Schmelzpunkt 252 - 255 °C.
d) 6-(4-Chlorphenoxy)-3-methyl-2-methylimino-2,3,8,8a-tetrahydro-indeno[1,2-d]thiazol-3a-ol
   entsteht durch Behandlung einer Lösung von 1,5 g 6-(4-Chlorphenoxy)-3-methyl-2-methylimino-2,3,8,8a-tetrahydro-indeno[1,2-d]thiazol-3a-ol Hydrobromid in 40 ml Methanol mit 2,3 ml Triethylamin. Man destilliert das Lösungsmittel ab und verfestigt den Rückstand unter Wasser. Amorpher Feststoff, Schmelzpunkt 85 - 90 °C.
e) 6-(4-Chlorphenoxy)-3-methyl-2-methylimino-2,3,8,8a-tetrahydro-indeno[1,2-d]thiazol-3a-ol Hydrochlorid
   erhält man durch Zugabe einer Lösung von HCl-Gas in Diethylether zu einer Lösung von 1,2 g 6-(4-Chlorphenoxy)-3-methyl-2-methylimino-2,3,8,8a-tetrahydro-indeno[1,2-d]thiazol-3a-ol in 30 ml Ethylacetat bis zur stark sauren Reaktion als kristallinen farblosen Niederschlag. Schmelzpunkt 247 - 250°C.

### Beispiel 12 (Verbindung A 28):

### 6-(2,2,2-Trifluorethoxy)-3-methyl-2-methylimino-2,3,8,8a-tetrahydro-indeno[1,2-d]thiazol-3a-ol Hydrobromid

a) 5-(2,2,2-Trifluorethoxy)-1-indanon:
   2,2 ml 2,2,2-Trifluorethanol werden zu einem rührenden Gemisch aus 3,5 g 5-Fluorindanon, 20 ml wasserfreiem Dimethylformamid und 4,1 g wasserfreien und gemahlenen Kaliumkarbonat gegeben und 10 Stunden bei 80°C gerührt. Man destilliert das Lösungsmittel unter verminderten Druck ab, löst den Rückstand in Ethylacetat und wäscht die organische Phase mehrmals mit Wasser. Man erhält das Indanonderivat als bräunlich kristallinen Feststoff nach Chromatographie an Kieselgel mit einem Gemisch aus gleichen Teilen Ethylacetat und Toluol als Elutionsmittel. Schmelzpunkt 93 - 97 °C.
b) 2-Brom-5-(2,2,2-trifluorethoxy)-1-indanon:
   erhält man durch Umsetzung von 0,9 g 5-(2,2,2-Trifluorethoxy)-1-indanon mit 0,2 ml Brom in 25 ml Essigester. Die Verbindung wird ohne weitere Reinigung weiter verwendet.
c) 6-(2,2,2-Trifluorethoxy)-3-methyl-2-methylimino-2,3,8,8a-tetrahydro-indeno[1,2-d]thiazol-3a-ol Hydrobromid
   erhält man durch Umsetzung von 1,2 g 2-Brom-5-(2,2,2-trifluorethoxy)-1-indanon und 0,4 g N,N'-Dimethylthioharnstoff in 25 ml Ethylacetat als hellgelben bis farblosen kristallinen Niederschlag. Schmelzpunkt 278 - 280 °C.
d) 6-(2,2,2-Trifluorethoxy)-3-methyl-2-methylimino-2,3,8,8a-tetrahydro-indeno[1,2-d]thiazol-3a-ol
   erhält man aus 6-(2,2,2-Trifluorethoxy)-3-methyl-2-methylimino-2,3,8,8a-tetrahydro-indeno[1,2-d]thiazol-3a-ol Hydrobromid und Triethylamin. Farbloser kristalliner Feststoff, Schmelzpunkt 138 - 140°C.
e) 6-(2,2,2-Trifluorethoxy)-3-methyl-2-methylimino-2,3,8,8a-tetrahydro-indeno[1,2-d]thiazol-3a-ol Hydrochlorid
   erhält man aus 6-(2,2,2- Trifluorethoxy)-3-methyl-2-methylimino-2,3,8,8a-tetrahydro-indeno[1,2-d]thiazol-3a-ol und einer Lösung von HCl-Gas in Ether. Farblose Kristalle, Schmelzpunkt 274 - 276 °C.

### Beispiel 13 (Verbindung A45):

### 5-(2,2,3,3,4,4,4-Heptafluorbutoxy)-3-methyl-2-methylimino-2,3,8,8a-tetrahydro-indeno[1,2-d]thiazol-3a-ol Hydrochlorid

a) 5-(2,2,3,3,4,4,4-Heptafluorbutoxy)-1-indanon:
   erhält man aus 6,5 g 5-Fluorindanon und 35,6 g wasserfreien und gemahlenen Kaliumkarbonat in 50 ml wasserfreiem Dimethylacetamid als honigfarbenes Öl.
b) 2-Brom-5-(2,2,3,3,4,4,4-heptafluorbutoxy)-1-indanon:
   erhält man durch Umsetzung von 4,16 g 5-(2,2,3,3,4,4,4-Heptafluorbutoxy)-1-indanon mit 0.69 ml Brom in 110 ml Ethylacetat. Die Verbindung wird als braunes Öl isoliert und wird ohne weitere Reinigung verwendet.
c) 6-(2,2,3,3,4,4,4-Heptafluorbutoxy)-3-methyl-2-methylimino-2,3,8,8a-tetrahydro-indeno[1,2-d]thiazol-3a-ol Hydrobromid
   erhält man durch Umsetzung von 1,4 g 2-Brom-5-(2,2,3,3,4,4,4-heptafluorbutoxy)-1-indanon und 0,36 g N,N'-Dimethylthioharnstoff in 40 ml Ethylacetat als hellgelben bis farblosen kristallinen Niederschlag. Zersetzungspunkt 253 °C.
d) 6-(2,2,3,3,4,4,4-Heptafluorbutoxy)-3-methyl-2-methylimino-2,3,8,8a-tetrahydro-indeno[1,2-d]thiazol-3a-ol
   erhalt man aus 6-(2,2,3,3,4,4,4-Heptafluorbutoxy)-3-methyl-2-methylimino-2,3,8,8a-tetrahydro-indeno[1,2-d]thiazol-3a-ol Hydrobromid und Triethylamin. Farbloser kristalliner Feststoff, Schmelzpunkt 138 - 140°C.
e) 6-(2,2,3,3,4,4,4-Heptafluorbutoxy)-3-methyl-2-methylimino-2,3,8,8a-tetrahydro-indeno[1,2-d]thiazol-3a-ol Hydrochlorid
   erhält man aus 6-(2,2,3,3,4,4,4-Heptafluorbutoxy)--3-methyl-2-methylimino-2,3,8,8a-tetrahydro-indeno[1,2-d]thiazol-3a-ol und etherischer Salzsäure. Farblose Kristalle, Schmelzpunkt 248-250 °C.

### Beispiel 14 (Verbindung B1):

### 6-Chlor-8a-fluor-3-methyl-2-methylimino-2,3,8,8a-tetrahydro-indeno[1,2-d]thiazol-3a-ol:

a) 5-Chlor-2-fluor-indan-1-on:
   Zu einer Lösung von 5,24 ml Diisopropylamin in 60 ml trockenem Tetrahydrofuran werden bei einer Temperatur von < -50°C 25 ml einer 1,6 molaren Lösung von n-Butyllithium in n-Hexan langsam zugetropft; die Mischung wird anschließend noch 10 Minuten bei -50°C gerührt. Sodann wird eine Lösung von 6,33 g 5-Chlor-indan-1-on in 60 ml trockenem Tetrahydrofuran langsam zugegeben und die Mischung weitere 20 Minuten bei -50°C gerührt. Schließlich werden 11,4 g N-Fluordibenzolsulfimid, gelöst in 60 ml trockenem Tetrahydrofuran, zugetropft. Unter Rühren läßt man die Mischung sich innerhalb von 2 Stunden auf 0°C erwärmen, tropft 120 ml einer ges. Natriumhydrogencarbonatlösung zu, destilliert das Tetrahydrofuran im Vakuum ab und schüttelt den Rückstand zweimal mit 150 ml Essigsäureethylester aus. Die organische Phase wird mit Wasser und ges. Kochsalzlösung gewaschen, über Magnesiumsulfat getrocknet, eingeengt und über Kieselgel mit Diisopropylether / n-Heptan 1/1 chromatographisch gereinigt. Neben 5-Chlor-2,2-difluor-indan-1-on erhält man 5-Chlor-2-fluor-indan-1-on mit dem Schmelzpunkt 102 - 104°C.
b) 5-Chlor-2-brom-2-fluor-indan-1-on:
   Die Bromierung des 5-Chlor-2-fluor-indan-1-ons erfolgt analog wie bei 6d) beschrieben und liefert 5-Chlor-2-brom-2-fluor-indan-1-on mit dem schmelzpunkt 104 - 105°C.
c) 6-Chlor-8a-fluor-3-methyl-2-methylimino-2,3,8,8a-tetrahydro-indeno[1,2-d]thiazol-3a-ol:
   263 mg 5-Chlor-2-brom-2-fluor-indan-1-on und 156 mg N,N'-Dimethylthioharnstoff werden in 5 ml Aceton gelöst und erst 90 Minuten bei Raumtemperatur und dann 2 Stunden bei 50°C gerührt. Die Reaktionsmischung wird abgekühlt und im Vakuum eingeengt. Zum Rückstand werden 5 ml Toluol gegeben und die Mischung wird 2 Stunden unter Rückfluß erhitzt, abgekühlt, mit 152 mg Triethylamin versetzt und 2 Stunden bei Raumtemperatur gerührt. Der Rückstand wird abgesaugt, mit wenig Wasser gewaschen und im Vakuum getrocknet. Man erhält 6-Chlor-8a-fluor-3-methyl-2-methylimino-2,3,8,8a-tetrahydro-indeno[1,2-d]thiazol-3a-ol mit dem Schmelzpunkt 189 - 190°C.

### Beispiel 15 (Verbindung B2):

### 6-Chlor-8a-fluor-3-methyl-2-methylimino-2.3,8,8a-tetrahydro-indeno[1,2-d]thiazol-3a-ol Hydrochlorid:

1 g (4,47 mMol) 6-Chlor-8a-fluor-3-methyl-2-methylimino-2,3,8,8a-tetrahydro-indeno[1,2-d]thiazol-3a-ol wird in 120 ml warmem Isopropanol gelöst und mit 1,2 g ca. 20%iger etherischer HCI-Lösung versetzt. Nach 1 h bei Raumtemperatur wird das Reaktionsgemisch eingeengt und der Rückstand mit Aceton verrührt, abgesaugt und der Rückstand im Vakuum getrocknet. Man erhält das Hydrochlorid des 6-Chlor-8a-fluor-3-methyl-2-methylimino-2,3,8,8a-tetrahydro-indeno[1,2-d]thiazol-3a-ols mit dem Schmelzpunkt 205°C (Zers.).

### Beispiel 16 (Verbindungen B1(-) und B1(+)):

100 mg des Racemates der Verbindung B1 werden über eine HPLC Säule (CSP Chiralpak AD 250x4.6) mit n-Hexan / Ethanol 10+1 in die Enantiomere aufgetrennt. Man erhält das (-)-drehende Enantiomer B1(-) mit einer Retentionszeit von 7.9 Minuten mit dem Schmelzpunkt 175 - 79°C (Zers.) und das (+)-drehende Enantiomer B1(+) mit einer Retentionszeit von 8.84 Minuten mit einem Schmelzpunkt von 172 - 77°C (Zers.).

### Beispiel 17 (Verbindungen C1 und C6):

### (6-Chlor-3a-methoxy-3-methyl-3,3a,8,8a-tetrahydro-indeno[1,2-d]thiazol-2-yliden)-methylamin (Hydrochlorid):

5 g des Hydrochlorides von 6-Chlor-3-methyl-2-methylimino-2,3,8,8a-tetrahydro-indeno[1,2-d]thiazol-3a-ol werden in 200 ml Methanol gelöst und drei Tage unter Rückfluß erhitzt. Die abgekühlte Reaktionsmischung wird im Vakuum eingeengt, der Rückstand mit 50 ml Aceton verrührt und filtriert. Das Filtrat wird eingeengt, in 100 ml Essigsäureethylester suspendiert und mit 100 ml einer ges. Natriumhydrogencarbonatlösung versetzt. Die organische Phase wird abgetrennt, mit Wasser gewaschen, über Magnesiumsulfat getrocknet, eingeengt und über Kieselgel mit Essigsäureethylester / Methanol 9/1 chromatographiert. Man erhält 6-Chlor-3-methyl-2-methylimino-2,3,8,8a-tetrahydro-indeno[1,2-d]thiazol-3a-ol mit dem Schmelzpunkt 94-96°C.
Das Hydrochlorid dieser Verbindung (Verbindung C6) wird gewonnen, indem man die freie Base in Methyl-tert.Butylether löst und unter Rühren und Kühlung mit dem Eisbad solange etherische Salzsäure zufügt bis ein pH von ca. 1 erreicht ist. Die Reaktionsmischung wird anschließend noch 3 Stunden bei Raumtemperatur gerührt, das Lösemittel abdestilliert, der Rückstand mit Aceton vesetzt und filtriert. Das Filrat wird im Vakuum eingeengt, der Rückstand im Vakuum getrocknet. Man erhält das Hydrochlorid des 6-Chlor-3-methyl-2-methylimino-2,3,8,8a-tetrahydro-indeno[1,2-d]thiazol-3a-ols mit dem Schmelzpunkt 65 - 70°C.

Die Verbindungen C2 - C5 wurden in analoger Weise hergestellt.

### Beispiel 18 a und b (Verbindung C1(+) und C1(-)):

100 mg des Racemates der Verbindung C1 (freie Base) werden über eine HPLC Säule (CSP Chiralpak AD 250x4.6) mit n-Hexan / 2-Propanol 25/1 mit 0,1 % Diethylamin in die Enantiomere aufgetrennt. Man erhält das (+)-drehende Enantiomer C1(+) mit einer Retentionszeit von 7.38 Minuten und einem spezifischen Drehwert von 237.5° (c = 10,3 mg/2ml in Trichlormethan) mit dem Schmelzpunkt 70 - 71 °C und das (-)-drehende Enantiomer C1(-) mit einer Retentionszeit von 8.06 Minuten und einem spezifischen Drehwert von -229.1° (c = 9.9 mg/2ml in Trichlormethan) mit einem Schmelzpunkt von 71 - 72°C.

### Beispiel 19 (Verbindung D2):

### 8-(2-Chlorphenyl)-3-methyl-2-methylimino-2,3,8,8a-tetrahydro-indeno[1,2-d]thiazol-3a-ol Hydrobromid

a) 2,3-Dibrom-3-(2-chlorphenyl)propionsäure
   erhält man durch Zutropfen einer Lösung von 7,8g Brom zu einer Suspension von 9 g 2-Chlorzimtsäure in 250 ml Chloroform unter Bestrahlung mit einer 500 W Tageslichtlampe und nachfolgendem Verdampfen des Lösungsmittels. Schmelzpunkt 183-185 °C.
b) 2,3-Dibrom-3-(2-chlorphenyl)propionsäurechlorid erhält man durch
   Kochen von 10 g 2,3-Dibrom-3-(2-chlorphenyl)propionsäure in 70 ml Thionylchlorid unter Rückflußbedingungen und Abdestillieren der Flüssigkeit als ölig-amorphen Rückstand.
c) 2-Brom-3-(2-chlorphenyl)-1-indanon
   In ein Gemisch aus 4,1 g wasserfreies Benzol und 30 ml Schwefelkohlenstoff trägt man unter Argonathmosphäre 6,6 g wasserfreies aktives Aluminiumchlorid ein und kühlt sodann auf - 20°C ab. Unter Beibehaltung der Kühlung tropft man in diese Suspension eine Lösung von 15 g 2,3-Dibrom-3-(2-chlorphenyl)propionsäurechlorid in 50 ml Schwefelkohlenstoff, hält sodann 5 Stunden bei 0°C und läßt weitere 16 Stunden im Kühlschrank bei einer Temperatur von 4-8°C stehen. Man gießt die Reaktionsmischung unter Rührung auf ein Eis-Wasser-Gemisch, das mit konz HCl stark sauer gestellt wurde, extrahiert mit Chloroform, wäscht die organische Phase mit Wasser und trocknet anschließend über Magnesiumsulfat. Nach Abdestillieren des Lösungsmittels wird der zähe amorphe Rückstand mit Diethylether aufgenommen, mit einer natriumbicarbonat-alkalischen wäßrigen Lösung mehrere Stunden gerührt und das organische Lösungsmittel nach Trocknung über Magnesiumsulfat abdestilliert. Viskose amorphe Substanz.
d) 8-(2-Chlorphenyl)-3-methyl-2-methylimino-2,3,8,8a-tetrahydro-indeno[1,2-d]thiazol-3a-ol Hydrobromid
   4 g 2-Brom-3-(2-chlorphenyl)-1-indanon werden in Essigester mit 1,2 g N,N'-Dimethylthioharnstoff versetzt und 2 Tage bei Raumtemperatur gerührt. Blaßgelbe kristalline Substanz, Schmelzpunkt 163 °C

### Beispiel 20 (Verbindung D14):

### 3a-Hydroxy-3-methyl-2-methylimino-8-phenyl-3a,8,9,9a-tetrahydronaphtho[2,1-b] thiazolidin Hydrobromid

a) 2-Brom-4-phenyl-1-tetralon
   erhält man durch Reaktion von 4,43 g 4-Phenyl-1-tetralon und 3,2 g Brom in HBr-enthaltendem Ethylacetat als viskoses amorphes Produkt.
b) 3a-Hydroxy-3-methyl-2-methylimino-8-phenyl-3a,8,9,9a-tetrahydronaphtho[2,1-b] thiazolidin Hydrobromid
   erhält man durch Umsetzung von 26 g 2-Brom-4-phenyl-1-tetralon und 5 g N,N'-Dimethylthioharnstoff in 60 ml Ethylacetat als hellgelben bis farblosen kristallinen Niederschlag. Schmelzpunkt 229-231 °C (unter Zersetzung).

Die Verbindung D1 wurde in analoger Weise hergestellt.

### Beispiel 21 (Verbindung D16):

### 1-Ethyl-2-ethylimino-9-nitro-1,2,4,5-tetrahydro-3aH-6-oxa-3-thia-1-aza-benzo[e]azulen-10b-ol:

a) 4-Phenoxybuttersäurechlorid:
   504,6 g 4-Phenoxybuttersäure werden mit 308 ml Thionylchlorid und 1,8 ml Dimethylformamid 3 Stunden unter Rückfluß gekocht. Anschließend wird die Mischung im Vakuum destilliert und man erhält 4-Phenoxybuttersäurechlorid mit dem Siedepunkt 145-147°C bei einem Druck von 10-12 mmHg.
b) 6,7,8,9-Tetrahydro-benzocyclohepten-5-on:
   Man suspendiert 192 g Aluminium-III-chlorid in 1,6 l trockenem 1,2-Dichlorethan und tropft unter einer Argonschutzgasatmosphäre unter Rühren eine Lösung von 238 g 4-Phenoxybuttersäurechlorid in 300 ml 1,2-Dichlorethan innerhalb von 5,5 Stunden bei einer Temperatur von -5°C langsam zu. Das Reaktionsprodukt wird durch Eingießen in eine Mischung aus 2 Wasser mit 2 l konz. Salzsäure der Hydrolyse unterworfen. Man rührt 30 Minuten, läßt sich den Niederschlag absetzen und saugt ab. Die organische Phase wird abgetrennt und die wässrige Phase wird dreimal mit 1,2-Dichlorethan ausgeschüttelt. Die organischen Extrakte werden mit Wasser, mit verd. Hydrogencarbonatlösung und schließlich mit ges. Kochsalzlösung gewaschen, über Natriumsulfat getrocknet und im Vakuum eingeengt. Der Rückstand wird im Vakuum destilliert. Man erhält 6,7,8,9-Tetrahydro-benzocyclohepten-5-on mit einem Siedepunkt von 79 - 84°C bei einem Druck von 0,001 mmHg als eine klare Flüssigkeit.
c) 7-Nitro-3,4-dihydro-2H-benzo[b]oxepin-5-on:
   25 g 6,7,8,9-Tetrahydro-benzocyclohepten-5-on werden in 280 ml konz. Schwefelsäure bei -10°C gelöst. Unter kräftigem Rühren werden nach und nach bei -10°C insgesamt 16,75 g Natriumnitrat eingetragen. Die dunkle Reaktionsmischung wird 45 Minuten bei 0°C gerührt; dabei geht der Rest Natriumnitrat langsam in Lösung. Das Reaktionsgemisch wird sodann unter Rühren auf Eiswasser gegeben und 30 Minuten gerührt. Der Niederschlag wird abgesaugt und mit Wasser neutral gewaschen. Der lufttrockene Rückstand wird zur Reinigung aus Isopropanol umkristallisiert. Man erhält 7-Nitro-3,4-dihydro-2H-benzo[b]oxepin-5-on, welches gleich weiterverarbeitet wird.
d) 4-Brom-7-nitro-3,4-dihydro-2H-benzo[b]oxepin-5-on:
   2.07 g 7-Nitro-3,4-dihydro-2H-benzo[b]oxepin-5-on werden in 10 ml Dichlormethan gelöst und unter Kühlung mit dem Eisbad unter Rühren mit 1,7 g Brom, gelöst in 10 ml Dichlormethan, versetzt (Zugabe über 3 Stunden). Die Reaktionsmischung wird in 30 ml einer gesättigten Natriumhydrogencarbonatlösung eingetragen, die organische Phase abgetrennt und die wässrige Phase mehrmals mit Dichlormethan extrahiert. Die vereinigten organischen Extrakte werden mit ges. Kochsalzlösung gewaschen, über Natriumsulfat getrocknet, eingeengt und der Rückstand wird aus Essigsäurebutylester umkristallisiert und liefert 4-Brom-7-nitro-3,4-dihydro-2H-benzo[b]oxepin-5-on mit dem Schmelzpunkt 115 - 118°C.
e) 1-Ethyl-2-ethylimino-9-nitro-1,2,4,5-tetrahydro-3aH-6-oxa-3-thia-1-aza-benzo[e]azulen-10b-ol:
   5 g 4-Brom-7-nitro-3,4-dihydro-2H-benzo[b]oxepin-5-on werden mit 2,4 g N,N'-Diethylthioharnstoff in 30 ml Butan-2-on 30 Minuten zum Rückfluß erhitzt. Der Niederschlag wird abgesaugt, mit ges. Kaliumhydrogen carbonatlösung verrührt und mit Essigsäureethylester extrahiert. Die organische Phase wird mit ges. Kochsalzlösung gewaschen, über Natriumsulfat getrocknet, eingeengt und der Rückstand aus Methanol /Wasser umkristallisiert. Man erhält 1-Ethyl-2-ethylimino-9-nitro-1,2,4,5-tetrahydro-3aH-6-oxa-3-thia-1-aza-benzo[e]azulen-10b-ol mit dem Schmelzpunkt 125 - 127°C.

In analoger Weise wurden die Verbindungen D12, D15, D17-D20, D22-D23, D26-D31, ausgehend von den entsprechenden Benzocyclohexanon- bzw. Benzocydoheptanon-Derivaten, hergestellt.

### Beispiel 22 (Verbindung D21):

### 1-Methyl-2-methylimino-6,6-dioxo-1,2,3a,4,5,6-hexahydro-dithia-1-aza-benzo[e]azulen-10b-ol:

a) 4-Phenylsulfanylbuttersäurechlorid:
   54,9 g 4-Phenylsulfanylbuttersäure werden mit 0,5 ml Dimethylformamid in 280 ml Toluol gelöst. Man tropft 36,5 ml Oxalylchlorid zu und rührt die Reaktionsmsichung 2 Stunden bei Raumtemperatur und eine weitere Stunde bei 65 - 70°C. Im Vakuum werden sodann Toluol und überschüssiges Oxalylchlorid abdestilliert. Der braune, ölige Rückstand wird im Hochvakuum destilliert und liefert 4-Phenylsulfanylbuttersäurechlorid mit einem Siedepunkt von 116 - 119°C bei einem Druck von 0,008 mmHg.
b) 3,4-Dihydro-2H-benzo[b]thiepin-5-on:
   38,2 g wasserfreies Aluminium-lll-chlorid werden in 260 ml Dichlormethan suspendiert. Bei 0°C fügt man unter Rühren langsam eine Lösung von 51,1 g 4-Phenylsulfanylbuttersäurechlorid in 70 ml Dichlormethan zu (2 h). Die Reaktionsmischung rührt über Nacht, und man erhält eine braungelbe Lösung. Diese Lösung wird unter kräftigem Rühren in eine eiskalte Mischung aus 1 I Wasser mit 1 I konz. Salzsäure gegeben. Nachdem 30 Minuten gerührt wurde, wird die Mischung dreimal mit jeweils 200 ml Diethylether extrahiert. Die vereinigten organischen Phasen werden mit Wasser und mit ges. Kochsalzlösung ausgeschüttelt, über Natriumsulfat getrocknet und im Vakuum eingeengt. Das erhaltene Öl wird destilliert und man erhält 3,4-Dihydro-2H-benzo[b]thiepin-5-on mit einem Siedpunkt von 93 - 94°C bei einem Druck von 0,002 mmHg.
c) 1,1-Dioxo-1,2,3,4-tetrahydro-benzo[b]thiepin-5-on:
   10 g 3,4-Dihydro-2H-benzo[b]thiepin-5-on werden in 125 ml Eisessig gelöst. Bei Raumtemperatur werden unter Rühren langsam 11,2 ml 35%iges Wasserstoffperoxid zugetropft. Nach 2stündigem Rühren bei Raumtemperatur werden nochmals 11 ml 35%iges Wasserstoffperoxid zugetropft. Die Reaktionsmischung wird sodann über Nacht gerührt. Im Vakuum wird das Lösemittel vorsichtig entfernt, und der Rückstand wird mit Eis versetzt und unter Rühren vorsichtig mit 1N Kaliumhydrogencarbonatlösung versetzt. Die wässrige Suspension wird mit Kochsalz gesättigt, abgesaugt, mit Wasser gewaschen und im Vakuum getrocknet. Man erhält 1.1-Dioxo-1,2,3,4-tetrahydro-benzo[b]thiepin-5-on mit dem Schmelzpunkt 142 - 146°C.
d) 4-Brom-1,1-dioxo-1,2,3,4-tetrahydro-benzo[b]thiepin-5-on:
   9,45 g 1,1-Dioxo-1,2,3,4-tetrahydro-benzo[b]thiepin-5-on werden in 200 ml Eisessig unter Rühren gelöst. Man fügt 8,9 g N-Bromsuccinimid zu und erwärmt die Mischung 8 Stunden lang auf 75 - 80°C. Nach Ende der Reaktion wird das Lösemittel im Vakuum abgedampft, und der ölige Rückstand wird unter Rühren mit eiskalter Natriumhydrogencarbonatlösung versetzt. Nach 30minütigem Rühren wird abgesaugt und der Rückstand mit Wasser gewaschen. Nach Trocknung im Vakuum wird 4-Brom-1,1-dioxo-1,2,3,4-tetrahydro-benzo[b]thiepin-5-on mit einem Schmelzpunkt von 132 - 136°C erhalten.
e) 1-Methyl-2-methylimino-6,6-dioxo-1,2,3a,4,5,6-hexahydro-dithia-1-aza-benzo[e]azulen-10b-ol:
   4,34 g 4-Brom-1,1-dioxo-1,2,3,4-tetrahydro-benzo[b]thiepin-5-on werden in 15 ml Butan-2-on suspendiert. Man erwärmt die Mischung auf 60 - 70°C und gibt portionsweise 1,8 g N,N'-Dimethylthioharnstoff zu. Die Lösung wird 2 Stunden unter Rückfluß gekocht, danach abgekühlt und der Niederschlag wird abgesaugt und mit Butan-2-on gewaschen. Der Rückstand wird mit 100 ml ges. Natiumhydrogencarbonatlösung verrührt und mit Essigsäureethylester extrahiert. Die vereinigten organischen Phasen werden mit ges. Kochsalzlösung gewaschen, über Natriumsulfat getrocknet und im Vakuum eingeengt. Man nimmt mit wenig Essigsäureethylester auf, saugt ab und trocknet im Vakuum. Man erhält 1-Methyl-2-methylimino-6,6-dioxo-1,2,3a,4,5,6-hexahydro-dithia-1-aza-benzo[e]azulen-10b-ol mit dem Schmelzpunkt 142 - 144°C (Zersetzung).

### Beispiel 23 (Verbindung D25):

### N-(10b-Hydroxy-1-methyl-2-methylimino-1,2,3a,4,5,10b-hexahydro-6-oxa-3-thia-1-aza-benzo[e]azulen-9-yl)-acetamid Hydrochlorid:

a) N-(5-Oxo-2,3,4,5-tetrahydro-benzo[b]oxepin-7-yl)-acetamid:
   16 g 7-Nitro-3,4-dihydro-2H-benzo[b]oxepin-5-on werden in 400 ml Methanol gelöst, mit 270 mg Palladium auf Aktivkohle (10%ig) versetzt und bei Normaldruck hydriert. Nach Filtration und Entfernen des Lösemittels im Vakuum erhält man das sauerstoffempfindliche 7-Amino-3,4-dihydro-2H-benzo[b]oxepin-5-on als gelbes Öl. Dieses Öl wird in 150 ml Dichlormethan gelöst; man fügt 8 g Triethylamin zu und tropft dann unter Eiskühlung langsam 8,1 g Essigsäureanhydrid unter kräftigem Rühren langsam zu. Die Reaktionsmischung wird noch eine Stunde bei Raumtemperatur gerührt. Die Lösung wird im Vakuum eingeengt und der Rückstand mit Essigsäureethylester aufgenommen, mit Wasser und mit ges. Kochsalzlösung gewaschen, über Natriumsulfat getrocknet und erneut eingeengt. Nach der Kristallisation aus Essigsäurebutylester schmilzt das N-(5-Oxo-2,3,4,5-tetrahydro-benzo[b]oxepin-7-yl)-acetamid bei 130 - 132°C.
b) N-(4-Brom-5-oxo-2,3,4,5-tetrahydro-benzo[b]oxepin-7-yl)-acetamid:
   1 g N-(5-Oxo-2,3,4,5-tetrahydro-benzo[b]oxepin-7-yl)-acetamid wird in 6 ml Dichlormethan gelöst. Bei 0°C werden unter Rühren langsam 0,8075 g Brom, gelöst in 5 ml Dichlormethan, zugetropft. Die Reaktionsmischung wird über Nacht bei Raumtemperatur gerührt, danach auf eine eiskalte ges. Natriumhydrogencarbonatlösung gegossen und mehrmals mit Dichlormethan extrahiert. Die organischen Phasen werden mit Wasser und mit ges. Kochsalzlösung gewaschen, über Natriumsulfat getrocknet und im Vakuum eingeengt. Der Rückstand wird aus Isopropanol umkristallisiert und liefert N-(4-Brom-5-oxo-2,3,4,5-tetrahydro-benzo[b]oxepin-7-yl)-acetamid mit einem Schmelzpunkt von 157 - 160°C.
c) N-(10b-Hydroxy-1-methyl-2-methylimino-1,2,3a,4,5,10b-hexahydro-6-oxa-3-thia-1-aza-benzo[e]azulen-9-yl)-acetamid Hydrochlorid:
   7 g N-(4-Brom-5-oxo-2,3,4,5-tetrahydro-benzo[b]oxepin-7-yl)-acetamid und 2,5 g N,N'-Dimethylthioharnstoff werden in 60 ml Butan-2-on 4 Stunden unter Rückfluß gekocht. Die abgekühlte Suspension wird filtriert, und der Rückstand wird mit 200 ml ges. Kaliumhydrogencarbonatlösung verrührt. Man fügt wenig Essigsäureethylester zu, saugt ab, wäscht den Rückstand mit Wasser und erhält N-(10b-Hydroxy-1-methyl-2-methylimino-1,2,3a,4,5,10b-hexahydro-6-oxa-3-thia-1-aza-benzo[e]azulen-9-yl)-acetamid mit dem Schmelzpunkt 186 - 188°C. Die freie Base wird in 30 ml 2 N Salzsäure gelöst. Nach etwa 10minütigem Rühren fällt das Hydrochlorid des N-(10b-Hydroxy-1-methyl-2-methylimino-1,2,3a,4,5,10b-hexahydro-6-oxa-3-thia-1-aza-benzo[e]azulen-9-yl)-acetamids aus. Es schmilzt unter Zersetzung bei 270°C.

### Beispiel 24 (Verbindung E2):

### 6-Chlor-3-(4-methoxy-phenyl)-2-(4-methoxy-phenylimino)-2,3,8,8a-tetrahydro-indeno[1,2-d]thiazol-3a-ol Hydrobromid:

1 g 2-Brom-5-chlor-indan-1-on und 1,17 g 1,3-Bis-(4-methoxy-phenyl)-thioharnstoff werden in 50 ml trockenem Dichlormethan supendiert, 4 Stunden bei Raumtemperatur und eine Stunde bei Eisbadtemperatur gerührt. Der Niederschlag wird abgesaugt, mir Dichlormethan gewaschen und im Vakuum getrocknet. Man erhält das Hydrobromid des 6-Chlor-3-(4-methoxy-phenyl)-2-(4-methoxy-phenylimino)-2,3,8,8a-tetrahydro-indeno[1,2-d]thiazol-3a-ols, welches bei 230-235°C unter Zersetzung schmilzt.

Die Verbindungen E1 und E3-E5 wurden in analoger Weise hergestellt.

## Patentansprüche

1. Polycyclische Thiazolidin-2-yliden Amine der Formel I, 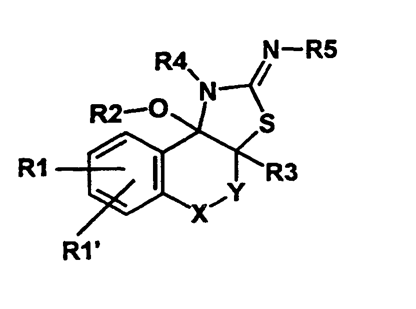 worin bedeuten
A)
Y eine direkte Bindung, -CH₂-, -CH₂-CH₂-;
X CH₂, CH(CH₃), CH(C₂H₅), CH(C₃H₇);
R1 CN, COOH, COO(C₁-C₆)Alkyl, CONH₂, CONH(C₁-C₆)Alkyl, CON[(C₁-C₆)Alkyl]₂, (C₂-C₆)-Alkyl; (C₂-C₆)-Alkenyl; (C₂-C₆)-Alkinyl; O-CH₂-CF₃, O-CH₂-CF₂-CF₃, O-(C₄-C₆)-Alkyl, wobei in den Alkylresten ein, mehrere, oder alle Wasserstoff(e) durch Fluor ersetzt sein können, oder ein Wasserstoff durch OH, OC(O)CH₃, O-CH₂-Ph, NH₂ oder N(COOCH₂Ph)₂ ersetzt sein kann;
S-(C₁-C₆)-Alkyl, S-(CH₂)ₙ-Phenyl, SO-(C₁-C₆)-Alkyl, SO-(CH₂)ₙ-Phenyl, SO₂-(C₁-C₆)-Alkyl, SO₂-(CH₂)ₙ-Phenyl, wobei n = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl, NH₂ substituiert sein kann; NH₂, NH-(C₁-C₆)-Alkyl, N((C₁-C₆)-Alkyl)₂, NH(C₁-C₇)-Acyl, Phenyl, Biphenyl, O-(CH₂)ₙ-Phenyl, wobei n = 0 - 6 sein kann, 1- oder 2-Naphthyl, 2-, 3- oder 4-Pyridyl, 2- oder 3-Furanyl oder 2- oder 3-Thienyl, wobei die Phenyl-, Biphenyl-, Naphthyl-, Pyridyl-, Furanyl - oder Thienylringe jeweils ein bis 3-fach substituiert sein können mit F, CI, Br, J, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C1-C₆)-Alkyl, CONH₂;
1,2,3-Triazol-5-yl, wobei der Triazolring in 1-, 2- oder 3-Stellung mit Methyl oder Benzyl substituiert sein kann;
Tetrazol-5-yl, wobei der Tetrazolring in 1- oder 2-Stellung mit Methyl oder Benzyl substituiert sein kann;
R1' H, F, Cl, Br, J, CH₃, CF₃, O-(C₁-C₃)-Alkyl, NO₂, SO₂-NH₂, SO₂NH(C₁-C₆)-Alkyl, SO₂N[(C₁-C₆)-Alkyl]₂ oder R1;
R2 H, (C₁-C₆)-Alkyl, (C₃-C₆)-Cycloalkyl, (CH₂)ₙ-Phenyl, (CH₂)ₙ-Thienyl, (CH₂)ₙ-Pyridyl, (CH₂)ₙ-Furyl, C(O)-(CH₂)ₙ-Phenyl, C(O)-(CH₂)ₙ-Thienyl, C(O)-(CH₂)ₙ-Pyridyl, C(O)-(CH₂)ₙ-Furyl, wobei n = 0 - 5 sein kann und worin Phenyl, Thienyl, Pyridyl, Furyl jeweils bis zu zweimal mit Cl, F, CN, CF₃, (C₁-C₃)-Alkyl, OH, O-(C1-C₆)-Alkyl substituiert sein kann, C(O)-(C₁-C₆)-Alkyl, C(O)-(C₃-C₆)-Cycloalkyl;
R3 H, (C₁-C₆)-Alkyl, F, CN, N₃, O-(C₁-C₆)-Alkyl, (CH₂)ₙ-Phenyl, CH₂-COO(C₁-C₆ Alkyl), CH₂-COO(C₃-C₈ Cycloalkyl), CH₂-COOH, CH₂-CONH₂, CH₂-CONHCH₃, CH₂-CON(CH₃)₂ (CH₂)ₙ-Thienyl, (CH₂)ₙ-Pyridyl, (CH₂)ₙ-Furyl, wobei n = 0 - 5 sein kann und worin Phenyl, Thienyl, Pyridyl, Furyl jeweils bis zu zweimal mit Cl, F, CN, CF₃, (C₁-C₃)-Alkyl, OH, O-(C1-C₆)-Alkyl substituiert sein kann, (C₂-C₆)-Alkinyl, (C₂-C₆)-Alkenyl, C(O)OCH₃, C(O)OCH₂CH₃, C(O)OH, C(O)NH₂, C(O)NHCH₃, C(O)N(CH₃)₂, OC(O)CH_{3;}
R4 (C₁-C₆)-Alkyl, (C₃-C₆)-Cycloalkyl, (CH₂)ₙ-Phenyl, (CH₂)ₙ-Thienyl, (CH₂)ₙ-Pyridyl, (CH₂)ₙ-Furyl, wobei n = 0 - 5 sein kann und worin Phenyl, Thienyl, Pyridyl, Furyl jeweils bis zu zweimal mit Cl, F, CN, CF₃, (C₁-C₃)-Alkyl, OH, O-(C₁-C₆)-Alkyl substituiert sein kann;
R5 (C₁-C₆)-Alkyl, (C₃-C₆)-Cycloalkyl, (CH₂)ₙ-Phenyl, (CH₂)ₙ-Thienyl, (CH₂)ₙ-Pyridyl, (CH₂)ₙ-Furyl, wobei n = 0 - 5 sein kann und worin Phenyl, Thienyl, Pyridyl, Furyl jeweils bis zu zweimal mit Cl, F, CN, CF₃, (C₁-C₃)-Alkyl, OH, O-(C1-C₆)-Alkyl substituiert sein kann;
oder
R4 und R5 bilden gemeinsam eine -CH₂-CH₂-, -CH₂-C(CH₃)₂-, -CH₂-CH₂-CH₂-, oder -CH₂-CH₂-CH₂-CH₂- Gruppe;
oder
B)
Y eine direkte Bindung, -CH₂- oder -CH₂-CH₂-;
X CH₂, CH(CH₃), CH(C₂H₅), CH(C₃H₇);
R1 und R1' unabhängig voneinander
H, F, Cl, Br, J, NO₂, CN, COOH, COO(C₁-C₆)Alkyl, CONH₂, CONH(C₁-C₆)Alkyl, CON[(C₁-C₆)Alkyl]₂, (C₁-C₆)-Alkyl, (C₂-C₆)-Alkenyl, (C₂-C₆)-Alkinyl, O-(C₁-C₆)-Alkyl, O-CH₂-CF₃, O-CH₂-CF₂-CF₃, O-(C₄-C₆)-Alkyl, wobei in den Alkylresten ein, mehrere, oder alle Wasserstoff(e) durch Fluor ersetzt sein können oder ein Wasserstoff durch OH, OC(O)CH₃, O-CH₂-Ph, NH₂ oder N(COOCH₂Ph)₂ ersetzt sein kann;
S-(C₁-C₆)-Alkyl, S-(CH₂)n-Phenyl, SO-(C₁-C₆)-Alkyl, SO-(CH₂)ₙ-Phenyl, SO₂-(C₁-C₆)-Alkyl, SO₂-(CH₂)ₙ-Phenyl, wobei n = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl, NH₂ substituiert sein kann; SO₂-NH₂, SO₂NH(C₁-C₆)-Alkyl, SO₂N[(C₁-C₆)-Alkyl]₂, NH₂, NH-(C₁-C₆)-Alkyl, N((C₁-C₆)-Alkyl)₂, NH(C₁-C₇)-Acyl, Phenyl, Biphenyl, O-(CH₂)ₙ-Phenyl, wobei n = 0 - 6 sein kann, 1- oder 2-Naphthyl, 2-, 3- oder 4-Pyridyl, 2- oder 3-Furanyl oder 2- oder 3-Thienyl, wobei die Phenyl-, Biphenyl-, Naphthyl-, Pyridyl-, Furanyl- oder Thienylringe jeweils ein bis 3-fach substituiert sein können mit F, Cl, Br, J, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂; 1,2,3-Triazol-5-yl, wobei der Triazolring in 1-, 2- oder 3-Stellung mit Methyl oder Benzyl substituiert sein kann;
Tetrazol-5-yl, wobei der Tetrazolring in 1- oder 2-Stellung mit Methyl oder Benzyl substituiert sein kann;
R2 H, (C₁-C₆)-Alkyl, (C₃-C₆)-Cycloalkyl, (CH₂)ₙ-Phenyl, (CH₂)ₙ-Thienyl, (CH₂)ₙ-Pyridyl, (CH₂)ₙ-Furyl, C(O)-(CH₂)ₙ-Phenyl, C(O)-(CH₂)ₙ-Thienyl, C(O)-(CH₂)ₙ-Pyridyl, C(O)-(CH₂)ₙ-Furyl, wobei n = 0 - 5 sein kann und worin Phenyl, Thienyl, Pyridyl, Furyl jeweils bis zu zweimal mit Cl, F, CN, CF₃, (C₁-C₃)-Alkyl, OH, O-(C1-C₆)-Alkyl substituiert sein kann, C(O)-(C₁-C₆)-Alkyl, C(O)-(C₃-C₆)-Cycloalkyl;
R3 (C₄-C₆)-Alkyl, F, CN, N₃, O-(C₁-C₆)-Alkyl, CH₂-COO(C₁-C₆ Alkyl), CH₂-COO(C₃-C₈ Cycloalkyl), CH₂-COOH, CH₂-CONH₂, CH₂-CONHCH₃, CH₂-CON(CH₃)₂ (CH₂)n-Phenyl, (CH₂)ₙ-Thienyl, (CH₂)ₙ-Pyridyl, (CH₂)ₙ-Furyl, wobei n = 0 - 5 sein kann und worin Phenyl, Thienyl, Pyridyl, Furyl jeweils bis zu zweimal mit Cl, F, CN, CF₃, (C₁-C₃)-Alkyl, OH, O-(C1-C₆)-Alkyl substituiert sein kann, (C₂-C₆)-Alkinyl, (C₂-C₆)-Alkenyl, C(O)OCH₃, C(O)OCH₂CH₃, C(O)OH, C(O)NH₂, C(O)NHCH₃, C(O)N(CH₃)₂, OC(O)CH_{3;}
R4 (C₁-C₆)-Alkyl, (C₃-C₆)-Cycloalkyl, (CH₂)ₙ-Phenyl, (CH₂)ₙ-Thienyl, (CH₂)ₙ-Pyridyl, (CH₂)ₙ-Furyl, wobei n = 0 - 5 sein kann und worin Phenyl, Thienyl, Pyridyl, Furyl jeweils bis zu zweimal mit Cl, F, CN, CF₃, (C₁-C₃)-Alkyl, OH, O-(C1-C₆)-Alkyl substituiert sein kann;
R5 (C₁-C₆)-Alkyl, (C₃-C₆)-Cycloalkyl, (CH₂)ₙ-Phenyl, (CH₂)ₙ-Thienyl, (CH₂)ₙ-Pyridyl, (CH₂)ₙ-Furyl, wobei n = 0 - 5 sein kann und worin Phenyl, Thienyl, Pyridyl, Furyl jeweils bis zu zweimal mit Cl, F, CN, CF₃, (C₁-C₃)-Alkyl, OH, O-(C1-C₆)-Alkyl substituiert sein kann;
oder
R4 und R5 bilden gemeinsam eine -CH₂-CH₂-, -CH₂-C(CH₃)₂-, -CH₂-CH₂-CH₂-, oder -CH₂-CH₂-CH₂-CH₂- Gruppe;
oder
C)
Y eine direkte Bindung, -CH₂- oder -CH₂-CH₂-;
X CH₂, CH(CH₃), CH(C₂H₅), CH(C₃H₇);
R1 und R1' unabhängig voneinander
H, F, Cl, Br, J, NO₂, CN, COOH, COO(C₁-C₆)Alkyl, CONH₂, CONH(C₁-C₆)Alkyl, CON[(C₁-C₆)Alkyl]₂, (C₁-C₆)-Alkyl, (C₂-C₆)-Alkenyl, (C₂-C₆)-Alkinyl, O-(C₁-C₆)-Alkyl, O-CH₂-CF₃, O-CH₂-CF₂-CF₃, O-(C₄-C₆)-Alkyl, wobei in den Alkylresten ein, mehrere, oder alle Wasserstoff(e) durch Fluor ersetzt sein können oder ein Wasserstoff durch OH, OC(O)CH₃, O-CH₂-Ph, NH₂ oder N(COOCH₂Ph)₂ ersetzt sein kann;
S-(C₁-C₆)-Alkyl, S-(CH₂)n-Phenyl, SO-(C₁-C₆)-Alkyl, SO-(CH₂)ₙ-Phenyl, SO₂-(C₁-C₆)-Alkyl, SO₂-(CH₂)n-Phenyl, wobei n = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl, NH₂ substituiert sein kann; SO₂-NH₂, SO₂NH(C₁-C₆)-Alkyl, SO₂N[(C₁-C₆)-Alkyl]₂, NH₂, NH-(C₁-C₆)-Alkyl, N((C₁-C₆)-Alkyl)₂, NH(C₁-C₇)-Acyl, Phenyl, Biphenyl, O-(CH₂)ₙ-Phenyl, wobei n = 0 - 6 sein kann, 1- oder 2-Naphthyl, 2-, 3- oder 4-Pyridyl, 2- oder 3-Furanyl oder 2- oder 3-Thienyl, wobei die Phenyl-, Biphenyl-, Naphthyl-, Pyridyl-, Furanyl- oder Thienylringe jeweils ein bis 3-fach substituiert sein können mit F, Cl, Br, J, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂; 1,2,3-Triazol-5-yl, wobei der Triazolring in 1-, 2- oder 3-Stellung mit Methyl oder Benzyl substituiert sein kann;
Tetrazol-5-yl, wobei der Tetrazolring in 1- oder 2-Stellung mit Methyl oder Benzyl substituiert sein kann;
R2 (C₁-C₆)-Alkyl, (C₃-C₆)-Cycloalkyl, C₂-C₈ Alkenyl, C₂-C₈ Alkinyl, (CH₂)ₙ-Phenyl, (CH₂)ₙ-Thienyl, (CH₂)ₙ-Pyridyl, (CH₂)ₙ-Furyl, C(O)-(CH₂)ₙ-Phenyl, C(O)-(CH₂)ₙ-Thienyl, C(O)-(CH₂)ₙ-Pyridyl, C(O)-(CH₂)ₙ-Furyl, wobei n = 0 - 5 sein kann und worin Phenyl, Thienyl, Pyridyl, Furyl jeweils bis zu zweimal mit Cl, F, CN, CF₃, (C₁-C₃)-Alkyl, OH, O-(C1-C₆)-Alkyl substituiert sein kann, C(O)-(C₁-C₆)-Alkyl, C(O)-(C₃-C₆)-Cycloalkyl;
R3 H, (C₁-C₆)-Alkyl, F, CN, N₃, O-(C₁-C₆)-Alkyl, CH₂-COO(C₁-C₆ Alkyl), CH₂-COO(C₃-C₈ Cycloalkyl), CH₂-COOH, CH₂-CONH₂, CH₂-CONHCH₃, CH₂-CON(CH₃)₂ (CH₂)ₙ-Phenyl, (CH₂)ₙ-Thienyl, (CH₂)ₙ-Pyridyl, (CH₂)ₙ-Furyl, wobei n = 0 - 5 sein kann und worin Phenyl, Thienyl, Pyridyl, Furyl jeweils bis zu zweimal mit Cl, F, CN, CF₃, (C₁-C₃)-Alkyl, OH, O-(C1-C₆)-Alkyl substituiert sein kann, (C₂-C₆)-Alkinyl, (C₂-C₆)-Alkenyl, C(O)OCH₃, C(O)OCH₂CH₃, C(O)OH, C(O)NH₂, C(O)NHCH₃, C(O)N(CH₃)₂, OC(O)CH_{3;}
R4 (C₁-C₆)-Alkyl, (C₃-C₆)-Cycloalkyl, (CH₂)ₙ-Phenyl, (CH₂)ₙ-Thienyl, (CH₂)ₙ-Pyridyl, (CH₂)ₙ-Furyl, wobei n = 0 - 5 sein kann und worin Phenyl, Thienyl, Pyridyl, Furyl jeweils bis zu zweimal mit Cl, F, CN, CF₃, (C₁-C₃)-Alkyl, OH, O-(C1-C₆)-Alkyl substituiert sein kann;
R5 (C₁-C₆)-Alkyl, (C₃-C₆)-Cycloalkyl, (CH₂)ₙ-Phenyl, (CH₂)ₙ-Thienyl, (CH₂)ₙ-Pyridyl, (CH₂)ₙ-Furyl, wobei n = 0 - 5 sein kann und worin Phenyl, Thienyl, Pyridyl, Furyl jeweils bis zu zweimal mit Cl, F, CN, CF₃, (C₁-C₃)-Alkyl, OH, O-(C1-C₆)-Alkyl substituiert sein kann;
oder
R4 und R5 bilden gemeinsam eine -CH₂-CH₂-, -CH₂-C(CH₃)₂-, -CH₂-CH₂-CH₂-, oder -CH₂-CH₂-CH₂-CH₂- Gruppe;
oder
D)
Y eine direkte Bindung, -CH₂- oder -CH₂-CH₂-;
X CH(Phenyl), wobei der Phenylrest mit F, Cl, Br oder J substituiert sein kann,
O, S, SO, SO₂ oder N-R6;
R1 und R1' unabhängig voneinander
H, F, Cl, Br, J, NO₂, CN, COOH, COO(C₁-C₆)Alkyl, CONH₂, CONH(C₁-C₆)Alkyl, CON[(C₁-C₆)Alkyl]₂, (C₁-C₆)-Alkyl, (C₂-C₆)-Alkenyl, (C₂-C₆)-Alkinyl, O-(C₁-C₆)-Alkyl, O-CH₂-CF₃, O-CH₂-CF₂-CF₃, O-(C₄-C₆)-Alkyl, wobei in den Alkylresten ein, mehrere, oder alle Wasserstoff(e) durch Fluor ersetzt sein können oder ein Wasserstoff durch OH, OC(O)CH₃, O-CH₂-Ph, NH₂ oder N(COOCH₂Ph)₂ ersetzt sein kann;
S-(C₁-C₆)-Alkyl, S-(CH₂)n-Phenyl, SO-(C₁-C₆)-Alkyl, SO-(CH₂)ₙ-Phenyl, SO₂-(C₁-C₆)-Alkyl, SO₂-(CH₂)n-Phenyl, wobei n = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl, NH₂ substituiert sein kann; SO₂-NH₂, SO₂NH(C₁-C₆)-Alkyl, SO₂N[(C₁-C₆)-Alkyl]₂, NH₂, NH-(C₁-C₆)-Alkyl, N((C₁-C₆)-Alkyl)₂, NH(C₁-C₇)-Acyl, Phenyl, Biphenyl, O-(CH₂)ₙ-Phenyl, wobei n = 0 - 6 sein kann, 1- oder 2-Naphthyl, 2-, 3- oder 4-Pyridyl, 2- oder 3-Furanyl oder 2- oder 3-Thienyl, wobei die Phenyl-, Biphenyl-, Naphthyl-, Pyridyl-, Furanyl- oder Thienylringe jeweils ein bis 3-fach substituiert sein können mit F, Cl, Br, J, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, COOH, COO-(C₁-C₆)-Alkyl, CONH₂;
1,2,3-Triazol-5-yl, wobei der Triazolring in 1-, 2- oder 3-Stellung mit Methyl oder Benzyl substituiert sein kann;
Tetrazol-5-yl, wobei der Tetrazolring in 1- oder 2-Stellung mit Methyl oder Benzyl substituiert sein kann;
R2 H, (C₁-C₆)-Alkyl, (C₃-C₆)-Cycloalkyl, (CH₂)ₙ-Phenyl, (CH₂)ₙ-Thienyl, (CH₂)ₙ-Pyridyl, (CH₂)ₙ-Furyl, C(O)-(CH₂)ₙ-Phenyl, C(O)-(CH₂)ₙ-Thienyl, C(O)-(CH₂)ₙ-Pyridyl, C(O)-(CH₂)ₙ-Furyl, wobei n = 0 - 5 sein kann und worin Phenyl, Thienyl, Pyridyl, Furyl jeweils bis zu zweimal mit Cl, F, CN, CF₃, (C₁-C₃)-Alkyl, OH, O-(C1-C₆)-Alkyl substituiert sein kann, C(O)-(C₁-C₆)-Alkyl, C(O)-(C₃-C₆)-Cycloalkyl;
R3 H, (C₁-C₆)-Alkyl, F, CN, N₃, O-(C₁-C₆)-Alkyl, CH₂-COO(C₁-C₆ Alkyl), CH₂-COO(C₃-C₈ Cycloalkyl), CH₂-COOH, CH₂-CONH₂, CH₂-CONHCH₃, CH₂-CON(CH₃)₂ (CH₂)ₙ-Phenyl, (CH₂)ₙ-Thienyl, (CH₂)ₙ-Pyridyl, (CH₂)ₙ-Furyl, wobei n = 0 - 5 sein kann und worin Phenyl, Thienyl, Pyridyl, Furyl jeweils bis zu zweimal mit Cl, F, CN, CF₃, (C₁-C₃)-Alkyl, OH, O-(C1-C₆)-Alkyl substituiert sein kann, (C₂-C₆)-Alkinyl, (C₂-C₆)-Alkenyl, C(O)OCH₃, C(O)OCH₂CH₃, C(O)OH, C(O)NH₂, C(O)NHCH₃, C(O)N(CH₃)₂, OC(O)CH₃;
R4 (C₁-C₆)-Alkyl, (C₃-C₆)-Cycloalkyl, (CH₂)ₙ-Phenyl, (CH₂)ₙ-Thienyl, (CH₂)ₙ-Pyridyl, (CH₂)ₙ-Furyl, wobei n = 0 - 5 sein kann und worin Phenyl, Thienyl, Pyridyl, Furyl jeweils bis zu zweimal mit Cl, F, CN, CF₃, (C₁-C₃)-Alkyl, OH, O-(C₁-C₆)-Alkyl substituiert sein kann;
R5 (C₁-C₈)-Alkyl, (C₃-C₆)-Cycloalkyl, (CH₂)ₙ-Phenyl, (CH₂)ₙ-Thienyl, (CH₂)ₙ-Pyridyl, (CH₂)ₙ-Furyl, wobei n = 0 - 5 sein kann und worin Phenyl, Thienyl, Pyridyl, Furyl jeweils bis zu zweimal mit Cl, F, CN, CF₃, (C₁-C₃)-Alkyl, OH, O-(C₁-C₆)-Alkyl substituiert sein kann;
oder
R4 und R5 bilden gemeinsam eine -CH₂-CH₂-, -CH₂-C(CH₃)₂-, -CH₂-CH₂-CH₂-, oder -CH₂-CH₂-CH₂-CH₂- Gruppe;
R6 SO₂-(C₆H₄-4-CH₃)
oder
E)
Y eine direkte Bindung, -CH₂- oder -CH₂-CH₂-;
X CH₂, CH(CH₃), CH(C₂H₅), CH(C₃H₇);
R1 H, F, Cl, Br, J, CH₃, CF₃, O-(C₁-C₃)-Alkyl;
R1' H, F, Cl, Br, J, NO₂;
R2 H;
R3 H, (C₁-C₃)-Alkyl;
R4 Phenyl, wobei der Phenylrest bis zu zweifach substituiert sein kann mit F, Cl, Br, J, (C₁-C₆)-Alkyl, (C₃-C₆)-Cycloalkyl, O-(C₁-C₃)-Alkyl, CF₃, OCF₃, O-CH₂-Phenyl, COOH, COO(C₁-C₆)Alkyl, COO(C₃-C₆)-Cycloalkyl, CONH₂;
R5 Phenyl, wobei der Phenylrest bis zu zweifach substituiert sein kann mit F, Cl, Br, J, (C₁-C₆)-Alkyl, (C₃-C₆)-Cycloalkyl, O-(C₁-C₃)-Alkyl, CF₃, OCF₃, O-CH₂-Phenyl, COOH, COO(C₁-C₆)Alkyl, COO(C₃-C₆)-Cycloalkyl, CONH₂;
sowie deren physiologisch verträgliche Salze.

2. Verbindungen der Formel I, gemäß Anspruch 1, **dadurch gekennzeichnet, daß** darin bedeuten
A)
Y eine direkte Bindung, -CH₂;
X CH₂, CH(CH₃), CH(C₂H₅), CH(C₃H₇);
R1 CN, COOH, COO(C₁-C₆)Alkyl, CONH₂, CONH(C₁-C₆)Alkyl, CON[(C₁-C₆)Alkyl]₂, (C₂-C₆)-Alkyl; (C₂-C₆)-Alkenyl; (C₂-C₆)-Alkinyl; O-CH₂-CF₃, O-CH₂-CF₂-CF₃, O-(C₄-C₆)-Alkyl, wobei in den Alkylresten ein, mehrere, oder alle Wasserstoff(e) durch Fluor ersetzt sein können, oder ein Wasserstoff durch OH, OC(O)CH₃, O-CH₂-Ph, NH₂ oder N(COOCH₂Ph)₂ ersetzt sein kann;
S-(C₁-C₆)-Alkyl, S-(CH₂)ₙ-Phenyl, SO-(C₁-C₆)-Alkyl, SO-(CH₂)ₙ-Phenyl, wobei der Phenylrest bis zu zweifach mit F, Cl, Br, OH, CF₃, NO₂, CN, OCF₃, O-(C₁-C₆)-Alkyl, (C₁-C₆)-Alkyl substituiert sein kann; NH-(C₁-C₆)-Alkyl, N((C₁-C₆)-Alkyl)₂, NH(C₁-C₇)-Acyl, Phenyl, O-(CH₂)ₙ-Phenyl, wobei n = 0 - 6 sein kann, 1- oder 2-Naphthyl, 2-, 3-oder 4-Pyridyl, 2- oder 3-Furanyl oder 2- oder 3-Thienyl, wobei die Phenyl-, Naphthyl-, Pyridyl-, Furanyl- oder Thienylringe jeweils ein bis 3-fach substituiert sein können mit F, Cl, J, OH, CF₃, CN, OCF₃, O-(C₁-C₆)-Alkyl, (C₁-C₆)-Alkyl, NH₂, NH(C₁-C₆)-Alkyl, N((C₁-C₆)-Alkyl)₂, COOH, COO-(C1-C₆)-Alkyl, CONH₂;
1,2,3-Triazol-5-yl, wobei der Triazolring in 1-, 2- oder 3-Stellung mit Methyl oder Benzyl substituiert sein kann;
Tetrazol-5-yl, wobei der Tetrazolring in 1- oder 2-Stellung mit Methyl oder Benzyl substituiert sein kann;
R1' H, F, Cl, Br, J, CH₃, CF₃, O-(C₁-C₃)-Alkyl, SO₂-NH₂, SO₂NH(C₁-C₆)-Alkyl, SO₂N[(C₁-C₆)-Alkyl]₂ oder R1;
R2 H, (C₁-C₆)-Alkyl, (CH₂)ₙ-Phenyl, (CH₂)ₙ-Thienyl, (CH₂)ₙ-Pyridyl, C(O)-(CH₂)ₙ-Phenyl, C(O)-(CH₂)ₙ-Thienyl, C(O)-(CH₂)ₙ-Pyridyl, wobei n = 0 - 3 sein kann und worin Phenyl, Thienyl, Pyridyl, Furyl jeweils bis zu zweimal mit Cl, F, CN, CF₃, (C₁-C₃)-Alkyl, OH, O-(C₁-C₆)-Alkyl substituiert sein kann;
R3 H, (C₁-C₆)-Alkyl, F, CN, O-(C₁-C₆)-Alkyl, CH₂-COO(C₁-C₆ Alkyl), CH₂-COO(C₃-C₈ Cycloalkyl), CH₂-COOH, CH₂-CONH₂, CH₂-CONHCH₃, CH₂-CON(CH₃)₂,(CH₂)ₙ-Phenyl, (CH₂)ₙ-Thienyl, (CH₂)ₙ-Pyridyl, wobei n = 0 - 3 sein kann und worin Phenyl, Thienyl, Pyridyl jeweils bis zu zweimal mit Cl, F, CN, CF₃, (C₁-C₃)-Alkyl, OH, O-(C1-C₆)-Alkyl substituiert sein kann, (C₂-C₆)-Alkinyl, (C₂-C₆)-Alkenyl, C(O)OCH₃, C(O)OCH₂CH₃, C(O)OH, C(O)NH₂, C(O)N(CH₃)₂, OC(O)CH₃;
R4 (C₁-C₆)-Alkyl, (C₃-C₆)-Cycloalkyl, (CH₂)ₙ-Phenyl, (CH₂)ₙ-Thienyl, (CH₂)ₙ-Pyridyl, wobei n = 0 - 3 sein kann und worin Phenyl, Thienyl, Pyridyl jeweils bis zu zweimal mit Cl, F, CN, CF₃, (C₁-C₃)-Alkyl, OH, O-(C1-C₆)-Alkyl substituiert sein kann;
R5 (C₁-C₆)-Alkyl, (C₃-C₆)-Cycloalkyl, (CH₂)ₙ-Phenyl, (CH₂)ₙ-Thienyl, (CH₂)ₙ-Pyridyl, wobei n = 0 - 3 sein kann und worin Phenyl, Thienyl, Pyridyl jeweils bis zu zweimal mit Cl, F, CN, CF₃, (C₁-C₃)-Alkyl, OH, O-(C1-C₆)-Alkyl substituiert sein kann;
oder
R4 und R5 bilden gemeinsam eine -CH₂-CH₂-, -CH₂-C(CH₃)₂- oder -CH₂-CH₂-CH₂- Gruppe;
oder
B)
Y eine direkte Bindung, -CH₂-;
X CH₂, CH(CH₃), CH(C₂H₅), CH(C₃H₇);
R1 und R1' unabhängig voneinander
H, F, Cl, Br, J, CN, COOH, COO(C₁-C₆)Alkyl, CONH₂, CONH(C₁-C₆)Alkyl, CON[(C₁-C₆)Alkyl]₂, (C₁-C₆)-Alkyl, (C₂-C₆)-Alkenyl, (C₂-C₈)-Alkinyl, O-(C₁-C₆)-Alkyl, O-CH₂-CF₃, O-CH₂-CF₂-CF₃, O-(C₄-C₆)-Alkyl, wobei in den Alkylresten ein, mehrere, oder alle Wasserstoff(e) durch Fluor ersetzt sein können oder ein Wasserstoff durch OH, OC(O)CH₃, O-CH₂-Ph, NH₂ oder N(COOCH₂Ph)₂ ersetzt sein kann; S-(C₁-C₆)-Alkyl, S-(CH₂)n-Phenyl, SO-(C₁-C₆)-Alkyl, SO-(CH₂)ₙ-Phenyl, wobei der Phenylrest bis zu zweifach mit F, Cl, Br, OH, CF₃, NO₂, CN, OCF₃, O-(C₁-C₆)-Alkyl, (C₁-C₆)-Alkyl substituiert sein kann; SO₂-NH₂, SO₂NH(C₁-C₆)-Alkyl, SO₂N[(C₁-C₆)-Alkyl]₂, NH-(C₁-C₆)-Alkyl, N((C₁-C₆)-Alkyl)₂, NH(C₁-C₇)-Acyl, Phenyl, O-(CH₂)ₙ-Phenyl, wobei n = 0 - 6 sein kann, 1- oder 2-Naphthyl, 2-, 3- oder 4-Pyridyl, 2- oder 3-Furanyl oder 2- oder 3-Thienyl, wobei die Phenyl-, Naphthyl-, Pyridyl-, Furanyl- oder Thienylringe jeweils ein bis 3-fach substituiert sein können mit F, Cl, J, OH, CF₃, CN, OCF₃, O-(C₁-C₆)-Alkyl, (C₁-C₆)-Alkyl, NH₂, NH(C₁-C₆)-Alkyl, N((C₁-C₆)-Alkyl)₂, COOH, COO-(C₁-C₆)-Alkyl, CONH₂; 1,2,3-Triazol-5-yl, wobei der Triazolring in 1-, 2- oder 3-Stellung mit Methyl oder Benzyl substituiert sein kann;
Tetrazol-5-yl, wobei der Tetrazolring in 1- oder 2-Stellung mit Methyl oder Benzyl substituiert sein kann;
R2 H, (C₁-C₆)-Alkyl, (CH₂)ₙ-Phenyl, (CH₂)ₙ-Thienyl, (CH₂)ₙ-Pyridyl, C(O)-(CH₂)ₙ-Phenyl, C(O)-(CH₂)ₙ-Thienyl, C(O)-(CH₂)ₙ-Pyridyl, wobei n = 0 - 3 sein kann und worin Phenyl, Thienyl, Pyridyl, Furyl jeweils bis zu zweimal mit Cl, F, CN, CF₃, (C₁-C₃)-Alkyl, OH, O-(C₁-C₆)-Alkyl substituiert sein kann;
R3 (C₄-C₆)-Alkyl, F, CN, N₃, O-(C₁-C₆)-Alkyl, CH₂-COO(C₁-C₆ Alkyl), CH₂-COO(C₃-C₈ Cycloalkyl), CH₂-COOH, CH₂-CONH₂, CH₂-CONHCH₃, CH₂-CON(CH₃)₂,(CH₂)ₙ-Phenyl, (CH₂)ₙ-Thienyl, (CH₂)ₙ-Pyridyl, wobei n = 0 - 3 sein kann und worin Phenyl, Thienyl, Pyridyl jeweils bis zu zweimal mit Cl, F, CN, CF₃, (C₁-C₃)-Alkyl, OH, O-(C1-C₆)-Alkyl substituiert sein kann, (C₂-C₆)-Alkinyl, (C₂-C₆)-Alkenyl, C(O)OCH₃, C(O)OCH₂CH₃, C(O)OH, C(O)NH₂, C(O)NHCH₃, C(O)N(CH₃)₂, OC(O)CH₃;
R4 (C₁-C₆)-Alkyl, (C₃-C₆)-Cycloalkyl, (CH₂)ₙ-Phenyl, (CH₂)ₙ-Thienyl, (CH₂)ₙ-Pyridyl, wobei n = 0 - 3 sein kann und worin Phenyl, Thienyl, Pyridyl jeweils bis zu zweimal mit Cl, F, CN, CF₃, (C₁-C₃)-Alkyl, OH, O-(C1-C₆)-Alkyl substituiert sein kann;
R5 (C₁-C₆)-Alkyl, (C₃-C₆)-Cycloalkyl, (CH₂)ₙ-Phenyl, (CH₂)ₙ-Thienyl, (CH₂)ₙ-Pyridyl, wobei n = 0 - 3 sein kann und worin Phenyl, Thienyl, Pyridyl jeweils bis zu zweimal mit Cl, F, CN, CF₃, (C₁-C₃)-Alkyl, OH, O-(C1-C₆)-Alkyl substituiert sein kann;
oder
R4 und R5 bilden gemeinsam eine -CH₂-CH₂-, -CH₂-C(CH₃)₂- oder -CH₂-CH₂-CH₂-, Gruppe;
oder
C)
Y eine direkte Bindung oder -CH₂-;
X CH₂, CH(CH₃), CH(C₂H₅), CH(C₃H₇);
R1 und R1' unabhängig voneinander
H, F, Cl, Br, J, CN, COOH, COO(C₁-C₆)Alkyl, CONH₂, CONH(C₁-C₆)Alkyl, CON[(C₁-C₆)Alkyl]₂, (C₁-C₆)-Alkyl, (C₂-C₆)-Alkenyl, (C₂-C₆)-Alkinyl, O-(C₁-C₆)-Alkyl, O-CH₂-CF₃, O-CH₂-CF₂-CF₃, O-(C₄-C₆)-Alkyl, wobei in den Alkylresten ein, mehrere, oder alle Wasserstoff(e) durch Fluor ersetzt sein können oder ein Wasserstoff durch OH, OC(O)CH₃, O-CH₂-Ph, NH₂ oder N(COOCH₂Ph)₂ ersetzt sein kann; S-(C₁-C₆)-Alkyl, S-(CH₂)ₙ-Phenyl, SO₂-(C₁-C₆)-Alkyl, SO₂-(CH₂)n-Phenyl, wobei n = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl substituiert sein kann; SO₂-NH₂, SO₂NH(C₁-C₆)-Alkyl, SO₂N[(C₁-C₆)-Alkyl]₂, NH-(C₁-C₆)-Alkyl, N((C₁-C₆)-Alkyl)₂, NH(C₁-C₇)-Acyl, Phenyl, O-(CH₂)ₙ-Phenyl, wobei n = 0 - 4 sein kann, 1- oder 2-Naphthyl, 2-, 3- oder 4-Pyridyl, 2- oder 3-Furanyl oder 2- oder 3-Thienyl, wobei die Phenyl-, Naphthyl-, Pyridyl-, Furanyl- oder Thienylringe jeweils ein bis 3-fach substituiert sein können mit F, Cl, J, OH, CF₃, CN, OCF₃, O-(C₁-C₆)-Alkyl, (C₁-C₆)-Alkyl, NH₂, NH(C₁-C₆)-Alkyl, N((C₁-C₆)-Alkyl)₂, COOH, COO-(C₁-C₆)-Alkyl, CONH₂;
1,2,3-Triazol-5-yl, wobei der Triazolring in 1-, 2- oder 3-Stellung mit Methyl oder Benzyl substituiert sein kann;
Tetrazol-5-yl, wobei der Tetrazolring in 1- oder 2-Stellung mit Methyl oder Benzyl substituiert sein kann;
R2 (C₁-C₆)-Alkyl, C₂-C₈ Alkenyl, C₂-C₈ Alkinyl, (CH₂)ₙ-Phenyl, (CH₂)ₙ-Thienyl, (CH₂)ₙ-Pyridyl, C(O)-(CH₂)ₙ-Phenyl, C(O)-(CH₂)ₙ-Thienyl, C(O)-(CH₂)ₙ-Pyridyl, wobei n = 0 - 3 sein kann und worin Phenyl, Thienyl, Pyridyl, Furyl jeweils bis zu zweimal mit Cl, F, CN, CF₃, (C₁-C₃)-Alkyl, OH, O-(C₁-C₆)-Alkyl substituiert sein kann;
R3 H, (C₁-C₆)-Alkyl, F, CN, O-(C₁-C₆)-Alkyl, CH₂-COO(C₁-C₆ Alkyl), CH₂-COO(C₃-C₈ Cycloalkyl), CH₂-COOH, CH₂-CONH₂, CH₂-CONHCH₃, CH₂-CON(CH₃)₂,(CH₂)ₙ-Phenyl, (CH₂)ₙ-Thienyl, (CH₂)ₙ-Pyridyl, wobei n = 0 - 3 sein kann und worin Phenyl, Thienyl, Pyridyl jeweils bis zu zweimal mit Cl, F, CN, CF₃, (C₁-C₃)-Alkyl, OH, O-(C1-C₆)-Alkyl substituiert sein kann, (C₂-C₆)-Alkinyl, (C₂-C₆)-Alkenyl, C(O)OCH₃, C(O)OCH₂CH₃, C(O)OH, C(O)NH₂, C(O)N(CH₃)₂, OC(O)CH₃;
R4 (C₁-C₆)-Alkyl, (C₃-C₆)-Cycloalkyl, (CH₂)ₙ-Phenyl, (CH₂)ₙ-Thienyl, (CH₂)ₙ-Pyridyl, wobei n = 0 - 3 sein kann und worin Phenyl, Thienyl, Pyridyl jeweils bis zu zweimal mit Cl, F, CN, CF₃, (C₁-C₃)-Alkyl, OH, O-(C₁-C₆)-Alkyl substituiert sein kann;
R5 (C₁-C₆)-Alkyl, (C₃-C₆)-Cycloalkyl, (CH₂)ₙ-Phenyl, (CH₂)ₙ-Thienyl, (CH₂)ₙ-Pyridyl, wobei n = 0 - 3 sein kann und worin Phenyl, Thienyl, Pyridyl jeweils bis zu zweimal mit Cl, F, CN, CF₃, (C₁-C₃)-Alkyl, OH, O-(C1-C₆)-Alkyl substituiert sein kann;
oder
R4 und R5 bilden gemeinsam eine -CH₂-CH₂-, -CH₂-C(CH₃)₂- oder -CH₂-CH₂-CH₂-, Gruppe;
oder
D)
Y eine direkte Bindung oder -CH₂-;
X CH(Phenyl), wobei der Phenylrest mit F, Cl oder Br substituiert sein kann,
O, S, SO, SO₂ oder N-R6;
R1 und R1' unabhängig voneinander
H, F, Cl, Br, J, CN, COOH, COO(C₁-C₆)Alkyl, CONH₂, CONH(C₁-C₆)Alkyl, CON[(C₁-C₆)Alkyl]₂, (C₁-C₆)-Alkyl, (C₂-C₆)-Alkenyl, (C₂-C₆)-Alkinyl, O-(C₁-C₆)-Alkyl, O-CH₂-CF₃, O-CH₂-CF₂-CF₃, O-(C₄-C₆)-Alkyl, wobei in den Alkylresten ein, mehrere, oder alle Wasserstoff(e) durch Fluor ersetzt sein können oder ein Wasserstoff durch OH, OC(O)CH₃, O-CH₂-Ph, NH₂ oder N(COOCH₂Ph)₂ ersetzt sein kann; S-(C₁-C₆)-Alkyl, S-(CH₂)n-Phenyl, SO₂-(C₁-C₆)-Alkyl, SO₂-(CH₂)n-Phenyl, wobei n = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl substituiert sein kann; SO₂-NH₂, SO₂NH(C₁-C₆)-Alkyl, SO₂N[(C₁-C₆)-Alkyl]₂, NH₂, NH-(C₁-C₆)-Alkyl, N((C₁-C₆)-Alkyl)₂, NH(C₁-C₇)-Acyl, Phenyl, O-(CH₂)ₙ-Phenyl, wobei n = 0 - 6 sein kann, 1- oder 2-Naphthyl, 2-, 3- oder 4-Pyridyl, 2- oder 3-Furanyl oder 2- oder 3-Thienyl, wobei die Phenyl-, Naphthyl-, Pyridyl-, Furanyl- oder Thienylringe jeweils ein bis 3-fach substituiert sein können mit F, Cl, J, OH, CF₃, CN, OCF₃, O-(C₁-C₆)-Alkyl, (C₁-C₆)-Alkyl, NH₂, NH(C₁-C₆)-Alkyl, N((C₁-C₆)-Alkyl)₂, COOH, COO-(C₁-C₆)-Alkyl, CONH₂;
1,2,3-Triazol-5-yl, wobei der Triazolring in 1-, 2- oder 3-Stellung mit Methyl oder Benzyl substituiert sein kann;
Tetrazol-5-yl, wobei der Tetrazolring in 1- oder 2-Stellung mit Methyl oder Benzyl substituiert sein kann;
R2 H, (C₁-C₆)-Alkyl, (CH₂)ₙ-Phenyl, (CH₂)ₙ-Thienyl, (CH₂)ₙ-Pyridyl, C(O)-(CH₂)ₙ-Phenyl, C(O)-(CH₂)ₙ-Thienyl, C(O)-(CH₂)ₙ-Pyridyl, wobei n = 0 - 3 sein kann und worin Phenyl, Thienyl, Pyridyl, Furyl jeweils bis zu zweimal mit Cl, F, CN, CF₃, (C₁-C₃)-Alkyl, OH, O-(C₁-C₆)-Alkyl substituiert sein kann;
R3 H, (C₁-C₆)-Alkyl, F, CN, O-(C₁-C₆)-Alkyl, CH₂-COO(C₁-C₆ Alkyl), CH₂-COO(C₃-C₈ Cycloalkyl), CH₂-COOH, CH₂-CONH₂, CH₂-CONHCH₃, CH₂-CON(CH₃)₂, (CH₂)ₙ-Phenyl, (CH₂)ₙ-Thienyl, (CH₂)ₙ-Pyridyl, wobei n = 0 - 3 sein kann und worin Phenyl, Thienyl, Pyridyl jeweils bis zu zweimal mit Cl, F, CN, CF₃, (C₁-C₃)-Alkyl, OH, O-(C1-C₆)-Alkyl substituiert sein kann, (C₂-C₆)-Alkinyl, (C₂-C₆)-Alkenyl, C(O)OCH₃, C(O)OCH₂CH₃, C(O)OH, C(O)NH₂, C(O)NHCH₃, C(O)N(CH₃)₂, OC(O)CH₃;
R4 (C₁-C₆)-Alkyl, (C₃-C₆)-Cycloalkyl, (CH₂)ₙ-Phenyl, (CH₂)ₙ-Thienyl, (CH₂)ₙ-Pyridyl, wobei n = 0 - 3 sein kann und worin Phenyl, Thienyl, Pyridyl jeweils bis zu zweimal mit Cl, F, CN, CF₃, (C₁-C₃)-Alkyl, OH, O-(C1-C₆)-Alkyl substituiert sein kann;
R5 (C₁-C₆)-Alkyl, (C₃-C₆)-Cycloalkyl, (CH₂)ₙ-Phenyl, (CH₂)ₙ-Thienyl, (CH₂)ₙ-Pyridyl, wobei n = 0 - 3 sein kann und worin Phenyl, Thienyl, Pyridyl jeweils bis zu zweimal mit Cl, F, CN, CF₃, (C₁-C₃)-Alkyl, OH, O-(C1-C₆)-Alkyl substituiert sein kann;
oder
R4 und R5 bilden gemeinsam eine -CH₂-CH₂-, -CH₂-C(CH₃)₂- oder -CH₂-CH₂-CH₂- Gruppe;
R6 SO₂-(C₆H₄-4-CH₃)
oder
E)
Y eine direkte Bindung oder -CH₂-;
X CH₂, CH(CH₃), CH(C₂H₅), CH(C₃H₇);
R1 H, F, Cl, Br, J, CH₃, CF₃, O-(C₁-C₃)-Alkyl;
R1' H, F, Cl, Br, J;
R2 H;
R3 H, (C₁-C₃)-Alkyl;
R4 Phenyl, wobei der Phenylrest bis zu zweifach substituiert sein kann mit F, Cl, Br, J, (C₁-C₆)-Alkyl, (C₃-C₆)-Cycloalkyl, O-(C₁-C₃)-Alkyl, CF₃, OCF₃, O-CH₂-Phenyl, COOH, COO(C₁-C₆)Alkyl, CONH₂;
R5 Phenyl, wobei der Phenylrest bis zu zweifach substituiert sein kann mit F, Cl, Br, J, (C₁-C₆)-Alkyl, (C₃-C₆)-Cycloalkyl, O-(C₁-C₃)-Alkyl, CF₃, OCF₃, O-CH₂-Phenyl, COOH, COO(C₁-C₆)Alkyl, CONH₂;
sowie deren physiologisch verträgliche Salze.

3. Verbindungen der Formel I, gemäß Anspruch 1 oder 2, **dadurch gekennzeichnet, daß** darin bedeuten
A)
Y eine direkte Bindung;
X CH₂, CH(CH₃), CH(C₂H₅), CH(C₃H₇);
R1 CN, COOH, COO(C₁-C₆)Alkyl, CONH₂, CON[(C₁-C₆)Alkyl]₂, (C₂-C₆)-Alkyl; (C₂-C₆)-Alkenyl; (C₂-C₆)-Alkinyl; O-CH₂-CF₃, O-CH₂-CF₂-CF₃, O-(C₄-C₆)-Alkyl, wobei in den Alkylresten ein, mehrere, oder alle Wasserstoff(e) durch Fluor ersetzt sein können, oder ein Wasserstoff durch OH, OC(O)CH₃, O-CH₂-Ph, NH₂ oder N(COOCH₂Ph)₂ ersetzt sein kann;
S-(C₁-C₆)-Alkyl, S-(CH₂)ₙ-Phenyl, SO-(C₁-C₆)-Alkyl, SO-(CH₂)ₙ-Phenyl, wobei der Phenylrest bis zu zweifach mit F, Cl, Br, OH, CF₃, NO₂, CN, OCF₃, O-(C₁-C₆)-Alkyl, (C₁-C₆)-Alkyl substituiert sein kann; NH-(C₁-C₆)-Alkyl, N((C₁-C₆)-Alkyl)₂, NH(C₁-C₇)-Acyl, Phenyl, O-(CH₂)ₙ-Phenyl, wobei n = 0 - 6 sein kann, 1- oder 2-Naphthyl, 2-, 3- oder 4-Pyridyl, 2- oder 3-Furanyl oder 2- oder 3-Thienyl, wobei die Phenyl-, Naphthyl-, Pyridyl-, Furanyl- oder Thienylringe jeweils ein bis 2-fach substituiert sein können mit F, Cl, OH, CF₃, CN, OCF₃, O-(C₁-C₆)-Alkyl, (C₁-C₆)-Alkyl, COOH, COO-(C1-C₆)-Alkyl, CONH₂;
1,2,3-Triazol-5-yl, wobei der Triazolring in 1-, 2- oder 3-Stellung mit Methyl oder Benzyl substituiert sein kann;
Tetrazol-5-yl, wobei der Tetrazolring in 1- oder 2-Stellung mit Methyl oder Benzyl substituiert sein kann;
R1' H, F, Cl, CH₃, CF₃, O-(C₁-C₃)-Alkyl, SO₂-NH₂, SO₂NH(C₁-C₆)-Alkyl, SO₂N[(C₁-C₆)-Alkyl]₂ oder R1;
R2 H, (C₁-C₆)-Alkyl, (CH₂)ₙ-Phenyl, (CH₂)n-Pyridyl, C(O)-(CH₂)ₙ-Phenyl, C(O)-(CH₂)ₙ-Pyridyl, wobei n = 0 - 3 sein kann und worin Phenyl, Pyridyl jeweils bis zu zweimal mit Cl, F, CN, CF₃, (C₁-C₃)-Alkyl, O-(C₁-C₆)-Alkyl substituiert sein kann, C(O)-(C₁-C₆)-Alkyl;
R3 H, (C₁-C₆)-Alkyl, F, CN, O-(C₁-C₆)-Alkyl, CH₂-COO(C₁-C₆ Alkyl), CH₂-COO(C₃-C₈ Cycloalkyl), CH₂-COOH, CH₂-CONH₂, CH₂-CONHCH₃, CH₂-CON(CH₃)₂, (CH₂)ₙ-Phenyl, (CH₂)ₙ-Pyridyl, wobei n = 0 - 3 sein kann und worin Phenyl, Pyridyl jeweils bis zu zweimal mit Cl, F, CN, CF₃, (C₁-C₃)-Alkyl, O-(C1-C₆)-Alkyl sein kann, (C₂-C₆)-Alkinyl, C(O)OCH₃, C(O)OCH₂CH₃, C(O)OH, C(O)NH₂, C(O)N(CH₃)₂, OC(O)CH₃;
R4 (C₁-C₆)-Alkyl, (C₃-C₆)-Cycloalkyl, (CH₂)ₙ-Phenyl, (CH₂)ₙ-Pyridyl, wobei n = 0 - 3 sein kann und worin Phenyl, Pyridyl jeweils bis zu zweimal mit Cl, F, CN, CF₃, (C₁-C₃)-Alkyl, O-(C1-C₆)-Alkyl substituiert sein kann;
R5 (C₁-C₆)-Alkyl, (C₃-C₆)-Cycloalkyl, (CH₂)n-Phenyl, (CH₂)ₙ-Pyridyl, wobei n = 0 - 3 sein kann und worin Phenyl, Pyridyl jeweils bis zu zweimal mit Cl, F, CN, CF₃, (C₁-C₃)-Alkyl, O-(C1-C₆)-Alkyl substituiert sein kann;
oder
R4 und R5 bilden gemeinsam eine -CH₂-CH₂-, -CH₂-C(CH₃)₂- oder -CH₂-CH₂-CH₂- Gruppe;
oder
B)
Y eine direkte Bindung, -CH₂-;
X CH₂, CH(CH₃), CH(C₂H₅), CH(C₃H₇);
R1 und R1' unabhängig voneinander
H, F, Cl, Br, J, CN, COOH, COO(C₁-C₆)Alkyl, CONH₂, CON[(C₁-C₆)Alkyl]₂, (C₁-C₆)-Alkyl, (C₂-C₆)-Alkenyl, (C₂-C₆)-Alkinyl, O-(C₁-C₆)-Alkyl, O-CH₂-CF₃, O-CH₂-CF₂-CF₃, O-(C₄-C₆)-Alkyl, wobei in den Alkylresten ein, mehrere, oder alle Wasserstoff(e) durch Fluor ersetzt sein können oder ein Wasserstoff durch OH, OC(O)CH₃, O-CH₂-Ph, NH₂ oder N(COOCH₂Ph)₂ ersetzt sein kann;
S-(C₁-C₆)-Alkyl, S-(CH₂)n-Phenyl, SO-(C₁-C₆)-Alkyl, SO-(CH₂)ₙ-Phenyl, wobei der Phenylrest bis zu zweifach mit F, Cl, OH, CF₃, OCF₃, O-(C₁-C₆)-Alkyl, (C₁-C₆)-Alkyl substituiert sein kann; SO₂-NH₂, SO₂NH(C₁-C₆)-Alkyl, SO₂N[(C₁-C₆)-Alkyl]₂, N((C₁-C₆)-Alkyl)₂, NH(C₁-C₇)-Acyl, Phenyl, O-(CH₂)ₙ-Phenyl, wobei n = 0 - 6 sein kann, 1- oder 2-Naphthyl, 2-, 3- oder 4-Pyridyl, 2- oder 3-Furanyl oder 2- oder 3-Thienyl, wobei die Phenyl-, Naphthyl-, Pyridyl-, Furanyl- oder Thienylringe jeweils ein bis 3-fach substituiert sein können mit F, Cl, J, OH, CF₃, CN, OCF₃, O-(C₁-C₆)-Alkyl, (C₁-C₆)-Alkyl, NH₂, N((C₁-C₆)-Alkyl)₂, COOH, COO-(C₁-C₆)-Alkyl, CONH₂;
1,2,3-Triazol-5-yl, wobei der Triazolring in 1-, 2- oder 3-Stellung mit Methyl oder Benzyl substituiert sein kann;
Tetrazol-5-yl, wobei der Tetrazolring in 1- oder 2-Stellung mit Methyl oder Benzyl substituiert sein kann;
R2 H, (C₁-C₆)-Alkyl, (CH₂)ₙ-Phenyl, (CH₂)ₙ-Pyridyl, C(O)-(CH₂)ₙ-Phenyl, C(O)-(CH₂)ₙ-Pyridyl, wobei n = 0 - 3 sein kann und worin Phenyl, Pyridyl jeweils bis zu zweimal mit Cl, F, CN, CF₃, (C₁-C₃)-Alkyl, OH, O-(C₁-C₆)-Alkyl substituiert sein kann, C(O)-(C₁-C₆)-Alkyl;
R3 (C₄-C₆)-Alkyl, F, CN, N₃, O-(C₁-C₆)-Alkyl, CH₂-COO(C₁-C₆ Alkyl), CH₂-COO(C₃-C₈ Cycloalkyl), CH₂-COOH, CH₂-CONH₂, CH₂-CONHCH₃, CH₂-CON(CH₃)₂, (CH₂)ₙ-Phenyl, (CH₂)ₙ-Pyridyl, wobei n = 0 - 3 sein kann und worin Phenyl, Pyridyl jeweils bis zu zweimal mit Cl, F, CN, CF₃, (C₁-C₃)-Alkyl, O-(C1-C₆)-Alkyl substituiert sein kann, (C₂-C₆)-Alkinyl, C(O)OCH₃, C(O)OCH₂CH₃, C(O)OH, C(O)NH₂, C(O)NHCH₃, C(O)N(CH₃)₂, OC(O)CH₃;
R4 (C₁-C₆)-Alkyl, (C₃-C₆)-Cycloalkyl, (CH₂)ₙ-Phenyl, (CH₂)ₙ-Pyridyl, wobei n = 0 - 3 sein kann und worin Phenyl, Pyridyl jeweils bis zu zweimal mit Cl, F, CN, CF₃, (C₁-C₃)-Alkyl, O-(C₁-C₆)-Alkyl substituiert sein kann;
R5 (C₁-C₆)-Alkyl, (C₃-C₆)-Cycloalkyl, (CH₂)ₙ-Phenyl, (CH₂)ₙ-Pyridyl, wobei n = 0 - 3 sein kann und worin Phenyl, Pyridyl jeweils bis zu zweimal mit Cl, F, CN, CF₃, (C₁-C₃)-Alkyl, O-(C1-C₆)-Alkyl substituiert sein kann;
oder
R4 und R5 bilden gemeinsam eine -CH₂-CH₂-, -CH₂-C(CH₃)₂- oder -CH₂-CH₂-CH₂-, Gruppe;
oder
C)
Y eine direkte Bindung oder -CH₂-;
X CH₂, CH(CH₃), CH(C₂H₅), CH(C₃H₇);
R1 und R1' unabhängig voneinander
H, F, Cl, Br, J, CN, COOH, COO(C₁-C₆)Alkyl, CONH₂, CON[(C₁-C₆)Alkyl]₂, (C₁-C₆)-Alkyl, (C₂-C₆)-Alkenyl, (C₂-C₆)-Alkinyl, O-(C₁-C₆)-Alkyl, O-CH₂-CF₃, O-CH₂-CF₂-CF₃, O-(C₄-C₆)-Alkyl, wobei in den Alkylresten ein, mehrere, oder alle Wasserstoff(e) durch Fluor ersetzt sein können oder ein Wasserstoff durch OH, OC(O)CH₃, O-CH₂-Ph, NH₂ oder N(COOCH₂Ph)₂ ersetzt sein kann; S-(C₁-C₆)-Alkyl, S-(CH₂)ₙ-Phenyl, SO₂-(C₁-C₆)-Alkyl, SO₂-(CH₂)ₙ-Phenyl, wobei n = 0 - 6 sein kann und der Phenylrest bis zu zweifach mit F, Cl, Br, OH, CF₃, NO₂, OCF₃, O-(C₁-C₆)-Alkyl, (C₁-C₆)-Alkyl substituiert sein kann; SO₂-NH₂, SO₂NH(C₁-C₆)-Alkyl, SO₂N[(C₁-C₆)-Alkyl]₂, N((C₁-C₆)-Alkyl)₂, NH(C₁-C₇)-Acyl, Phenyl, O-(CH₂)ₙ-Phenyl, wobei n = 0 - 4 sein kann, 1- oder 2-Naphthyl, 2-, 3- oder 4-Pyridyl, 2- oder 3-Furanyl oder 2- oder 3-Thienyl, wobei die Phenyl-, Naphthyl-, Pyridyl-, Furanyl- oder Thienylringe jeweils ein bis 3-fach substituiert sein können mit F, Cl, J, OH, CF₃, CN, OCF₃, O-(C₁-C₆)-Alkyl, (C₁-C₆)-Alkyl, NH₂, NH(C₁-C₆)-Alkyl, N((C₁-C₆)-Alkyl)₂, COOH. COO-(C₁-C₆)-Alkyl, CONH₂;
1,2,3-Triazol-5-yl, wobei der Triazolring in 1-, 2- oder 3-Stellung mit Methyl oder Benzyl substituiert sein kann;
Tetrazol-5-yl, wobei der Tetrazolring in 1- oder 2-Stellung mit Methyl oder Benzyl substituiert sein kann;
R2 (C₁-C₆)-Alkyl, C₂-C₈ Alkenyl, C₂-C₈ Alkinyl, (CH₂)ₙ-Phenyl, (CH₂)ₙ-Thienyl, (CH₂)ₙ-Pyridyl, C(O)-(CH₂)ₙ-Phenyl, C(O)-(CH₂)ₙ-Thienyl, C(O)-(CH₂)ₙ-Pyridyl, wobei n = 0 - 3 sein kann und worin Phenyl, Thienyl, Pyridyl, Furyl jeweils bis zu zweimal mit Cl, F, CN, CF₃, (C₁-C₃)-Alkyl, OH, O-(C₁-C₆)-Alkyl substituiert sein kann, C(O)-(C₁-C₆)-Alkyl;
R3 H, (C₁-C₆)-Alkyl, F, CN, O-(C₁-C₆)-Alkyl, CH₂-COO(C₁-C₆ Alkyl), CH₂-COO(C₃-C₈ Cycloalkyl), CH₂-COOH, CH₂-CONH₂, CH₂-CONHCH₃, CH₂-CON(CH₃)₂, (CH₂)ₙ-Phenyl, (CH₂)ₙ-Pyridyl, wobei n = 0 - 3 sein kann und worin Phenyl, Pyridyl jeweils bis zu zweimal mit Cl, F, CN, CF₃, (C₁-C₃)-Alkyl, O-(C1-C₆)-Alkyl substituiert sein kann, (C₂-C₆)-Alkinyl, C(O)OCH₃, C(O)OCH₂CH₃, C(O)OH, C(O)NH₂, C(O)N(CH₃)₂, OC(O)CH₃;
R4 (C₁-C₆)-Alkyl, (C₃-C₆)-Cycloalkyl, (CH₂)ₙ-Phenyl, (CH₂)ₙ-Pyridyl, wobei n = 0 - 3 sein kann und worin Phenyl, Pyridyl jeweils bis zu zweimal mit Cl, F, CN, CF₃, (C₁-C₃)-Alkyl, O-(C1-C₆)-Alkyl substituiert sein kann;
R5 (C₁-C₆)-Alkyl, (C₃-C₆)-Cycloalkyl, (CH₂)ₙ-Phenyl, (CH₂)ₙ-Pyridyl, wobei n = 0 - 3 sein kann und worin Phenyl, Pyridyl jeweils bis zu zweimal mit Cl, F, CN, CF₃, (C₁-C₃)-Alkyl, O-(C1-C₆)-Alkyl substituiert sein kann;
oder
R4 und R5 bilden gemeinsam eine -CH₂-CH₂-, -CH₂-C(CH₃)₂- oder -CH₂-CH₂-CH₂-, Gruppe;
oder
D)
Y eine direkte Bindung oder -CH₂-;
X CH(Phenyl), wobei der Phenylrest mit F oder Cl substituiert sein kann,
O, S, SO₂ oder N-R6;
R1 und R1' unabhängig voneinander
H, F, Cl, Br, J, CN, COOH, COO(C₁-C₆)Alkyl, CONH₂, CON[(C₁-C₆)Alkyl]₂, (C₁-C₆)-Alkyl, (C₂-C₆)-Alkenyl, (C₂-C₆)-Alkinyl, O-(C₁-C₆)-Alkyl, O-CH₂-CF₃, O-CH₂-CF₂-CF₃, O-(C₄-C₆)-Alkyl, wobei in den Alkylresten ein, mehrere, oder alle Wasserstoff(e) durch Fluor ersetzt sein können oder ein Wasserstoff durch OH, OC(O)CH₃, O-CH₂-Ph, NH₂ oder N(COOCH₂Ph)₂ ersetzt sein kann;
S-(C₁-C₆)-Alkyl, S-(CH₂)n-Phenyl, SO₂-(C₁-C₆)-Alkyl, SO₂-(CH₂)n-Phenyl, wobei n = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl substituiert sein kann; SO₂-NH₂, SO₂NH(C₁-C₆)-Alkyl, SO₂N[(C₁-C₆)-Alkyl]₂, NH₂, N((C₁-C₆)-Alkyl)₂, NH(C₁-C₇)-Acyl, Phenyl, O-(CH₂)ₙ-Phenyl, wobei n = 0 - 6 sein kann, 1- oder 2-Naphthyl, 2-, 3- oder 4-Pyridyl, 2- oder 3-Furanyl oder 2- oder 3-Thienyl, wobei die Phenyl-, Naphthyl-, Pyridyl-, Furanyl- oder Thienylringe jeweils ein bis 3-fach substituiert sein können mit F, Cl, J, OH, CF₃, CN, OCF₃, O-(C₁-C₆)-Alkyl, (C₁-C₆)-Alkyl, NH₂, N((C₁-C₆)-Alkyl)₂, COOH, COO-(C₁-C₆)-Alkyl, CONH₂;
1,2,3-Triazol-5-yl, wobei der Triazolring in 1-, 2- oder 3-Stellung mit Methyl oder Benzyl substituiert sein kann;
Tetrazol-5-yl, wobei der Tetrazolring in 1- oder 2-Stellung mit Methyl oder Benzyl substituiert sein kann;
R2 H, (C₁-C₆)-Alkyl, (CH₂)ₙ-Phenyl, (CH₂)ₙ-Thienyl, (CH₂)ₙ-Pyridyl, C(O)-(CH₂)ₙ-Phenyl, C(O)-(CH₂)ₙ-Thienyl, C(O)-(CH₂)ₙ-Pyridyl, wobei n =, 0 - 3 sein kann und worin Phenyl, Thienyl, Pyridyl, Furyl jeweils bis zu zweimal mit Cl, F, CN, CF₃, (C₁-C₃)-Alkyl, OH, O-(C₁-C₆)-Alkyl substituiert sein kann, C(O)-(C₁-C₆)-Alkyl;
R3 H, (C₁-C₆)-Alkyl, F, CN, O-(C₁-C₆)-Alkyl, CH₂-COO(C₁-C₆ Alkyl), CH₂-COO(C₃-C₈ Cycloalkyl), CH₂-COOH, CH₂-CONH₂, CH₂-CONHCH₃, CH₂-CON(CH₃)₂, (CH₂)ₙ-Phenyl, (CH₂)ₙ-Pyridyl, wobei n = 0 - 3 sein kann und worin Phenyl, Pyridyl jeweils bis zu zweimal mit Cl, F, CN, CF₃, (C₁-C₃)-Alkyl, O-(C₁-C₆)-Alkyl substituiert sein kann, (C₂-C₆)-Alkinyl, C(O)OCH₃, C(O)OCH₂CH₃, C(O)OH, C(O)NH₂, C(O)NHCH₃, C(O)N(CH₃)₂, OC(O)CH₃;
R4 (C₁-C₆)-Alkyl, (C₃-C₆)-Cycloalkyl, (CH₂)ₙ-Phenyl, (CH₂)ₙ-Pyridyl, wobei n = 0 - 3 sein kann und worin Phenyl, Pyridyl jeweils bis zu zweimal mit Cl, F, CN, CF₃, (C₁-C₃)-Alkyl, O-(C₁-C₆)-Alkyl substituiert sein kann;
R5 (C₁-C₆)-Alkyl, (C₃-C₆)-Cycloalkyl, (CH₂)ₙ-Phenyl, (CH₂)ₙ-Pyridyl, wobei n = 0 - 3 sein kann und worin Phenyl, Pyridyl jeweils bis zu zweimal mit Cl, F, CN, CF₃, (C₁-C₃)-Alkyl, O-(C1-C₆)-Alkyl substituiert sein kann;
oder
R4 und R5 bilden gemeinsam eine -CH₂-CH₂-, -CH₂-C(CH₃)₂- oder -CH₂-CH₂-CH₂- Gruppe;
R6 SO₂-(C₆H₄-4-CH₃)
oder
E)
Y eine direkte Bindung oder -CH₂-;
X CH₂, CH(CH₃), CH(C₂H₅), CH(C₃H₇);
R1 H, F, Cl, CH₃, CF₃, O-(C₁-C₃)-Alkyl;
R1' H, F, Cl;
R2 H;
R3 H, (C₁-C₃)-Alkyl;
R4 Phenyl, wobei der Phenylrest bis zu zweifach substituiert sein kann mit F, Cl, (C₁-C₆)-Alkyl, (C₃-C₆)-Cycloalkyl, O-(C₁-C₃)-Alkyl, CF₃, O-CH₂-Phenyl, COOH, COO(C₁-C₆)Alkyl, CONH₂;
R5 Phenyl, wobei der Phenylrest bis zu zweifach substituiert sein kann mit F, Cl, (C₁-C₆)-Alkyl, (C₃-C₆)-Cycloalkyl, O-(C₁-C₃)-Alkyl, CF₃, O-CH₂-Phenyl, COOH, COO(C₁-C₆)Alkyl, CONH₂;
sowie deren physiologisch verträgliche Salze.

4. Verbindungen der Formel I, gemäß Anspruch 1, 2 oder 3, **dadurch gekennzeichnet, daß** darin bedeuten
Y eine direkte Bindung;
X CH₂
R1 und R1' unabhängig voneinander H, F, Cl, CN, COOH, CONH₂, COO(C₁-C₃)-Alkyl, (C₁-C₆)-Alkyl, (C₂-C₆)-Alkenyl, (C₂-C₆)-Alkinyl, wobei in den Alkyl-, Alkenyl- und Alkinresten ein Wasserstoff durch OH, OC(O)CH₃, O-CH₂-Ph, NH₂ oder N(COOCH2Ph)2 ersetzt sein kann; OCF₃, OCH₂CF₃ ; O-(C1-C4)-Alkyl, wobei in den Alkylresten ein, mehrere, oder alle Wasserstoff(e) durch Fluor ersetzt sein können oder ein Wasserstoff durch OH, OC(O)CH₃, O-CH₂-Ph, NH₂ oder N(COOCH₂Ph)₂ ersetzt sein kann;
SO₂-(C₁-C₆)-Alkyl, SO₂-(CH₂)ₙ-Phenyl, wobei n = 0 - 3 sein kann und der Phenylrest mit F, Cl, OH, CF₃, O-(C₁-C₄)-Alkyl substituiert sein kann;
NH-(CO)-(C₁-C₃)-Alkyl; (CH₂)ₙ-Phenyl, S-(CH₂)ₙ-Phenyl, O-(CH₂)ₙ-Phenyl, wobei n = 0 - 3 sein kann, 1- oder 2-Naphthyl, 2-, 3- oder 4-Pyridyl, 2- oder 3- Furanyl oder 2- oder 3-Thienyl, wobei die Phenyl-, Naphthyl-, Pyridyl-, Furanyl- oder Thienylringe jeweils substituiert sein können mit F, Cl, CF₃, (C₁-C₆)-Alkyl, O-(C1-C6)-Alkyl und wobei in den Alkylresten ein Wasserstoff durch OH, OC(O)CH₃, O-CH₂-Ph, NH₂ oder N(COOCH₂Ph)₂ ersetzt sein kann;
1,2,3-Triazol-5-yl, wobei der Triazolring in 1-, 2- oder 3-Stellung mit Methyl oder Benzyl substituiert sein kann;
Tetrazol-5-yl, wobei der Tetrazolring in 1- oder 2-Stellung mit Methyl oder Benzyl substituiert sein kann;
R2 H, (C₁-C₄)-Alkyl, (C₅-C₆)-Cycloalkyl; (CH₂)ₙ-Phenyl, wobei n = 0 - 3 sein kann, C(O)-(C₁-C₄)-Alkyl oder C(O)-Phenyl;
R3 F, (C₄-C₆)-Alkyl, CH₂-Phenyl, wobei Phenyl bis zu zweimal mit F, Cl, CF₃, O-(C₁-C₃)-Alkyl, (C₁-C₃)-Alkyl, COOH, CO-O-(C₁-C₃)-Alkyl oder CONH₂ substituiert sein kann;
R4 (C₁-C₆)-Alkyl, (C₃-C₆)-Cycloalkyl, (CH₂)ₙ-Phenyl, wobei n = 0 - 3 sein kann und der Phenylrest bis zu zweimal mit F, Cl, O-(C₁-C₄)-Alkyl oder OH substituiert sein kann;
R5 (C₁-C₆)-Alkyl, (C₃-C₆)-Cycloalkyl, (CH₂)ₙ-Phenyl, wobei n = 0 - 3 sein kann und der Phenylrest bis zu zweimal mit F, Cl, O-(C₁-C₄)-Alkyl oder OH substituiert sein kann;
sowie deren physiologisch verträgliche Salze.

5. Verbindungen der Formel I, gemäß einem oder mehreren der Ansprüche 1 bis 4, **dadurch gekennzeichnet, daß** darin bedeuten
| | |
|---|---|
| R1 | 6-Cl |
| R1' | H |
| R2 | H |
| R3 | F |
| R4 | CH₃ |
| R5 | CH₃ |
| X | CH₂ |
| Y | - |
sowie deren physiologisch verträgliche Salze.

6. Hydrochloride der Verbindungen der Formel I gemäß einem oder mehreren der Ansprüche 1 bis 5.

7. Arzneimittel enthaltend eine oder mehrere der Verbindungen gemäß einem oder mehreren der Ansprüche 1 bis 6.

8. Arzneimittel enthaltend eine oder mehrere der Verbindungen gemäß einem oder mehreren der Ansprüche 1 bis 6 und ein oder mehrere Abmagerungsmittel.

9. Verbindungen gemäß einem oder mehreren der Ansprüche 1 bis 6 zur Anwendung als Medikament zur Behandlung der Obesitas.

10. Verbindungen gemäß einem oder mehreren der Ansprüche 1 bis 6 zur Anwendung als Medikament zur Prophylaxe oder Behandlung des Typ II Diabetes.

11. Verfahren zur Herstellung eines Arzneimittels enthaltend eine oder mehrere der Verbindungen gemäß einem oder mehreren der Ansprüche 1 bis 6, **dadurch gekennzeichnet, daß** der Wirkstoff mit einem pharmazeutisch geeigneten Träger vermischt wird und diese Mischung in eine für die Verabreichung geeignete Form gebracht wird.

12. Verwendung der Verbindungen gemäß einem oder mehreren der Ansprüche 1 bis 6 zur Herstellung eines Medikaments zur Behandlung der Obesitas.

13. Verwendung der Verbindungen gemäß einem oder mehreren der Ansprüche 1 bis 6 zur Herstellung eines Medikaments zur Prophylaxe oder Behandlung des Typ 11 Diabetes.

## Claims

1. A polycyclic thiazolidin-2-ylidene amine of the formula I 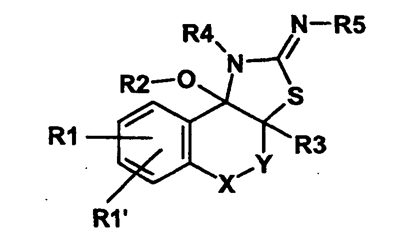 in which
A)
Y is a direct bond, -CH₂-, -CH₂-CH₂-;
X is CH₂, CH(CH₃), CH(C₂H₅), CH(C₃H₇);
R1 is CN, COOH, COO(C₁-C₆)-alkyl, CONH₂, CONH(C₁-C₆)-alkyl, CON[(C₁-C₆)-alkyl]₂, (C₂-C₆)-alkyl, (C₂-C₆)-alkenyl, (C₂-C₆)-alkynyl, O-CH₂-CF₃, O-CH₂-CF₂-CF₃, O-(C₄-C₆)-alkyl, where in the alkyl radicals one or more, or all hydrogen(s) can be replaced by fluorine, or one hydrogen can be replaced by OH, OC(O)CH₃, O-CH₂-Ph, NH₂ or N(COOCH₂Ph)₂;
S-(C₁-C₆)-alkyl, S-(CH₂)ₙ-phenyl, SO-(C₁-C₆)-alkyl, SO-(CH₂)ₙ-phenyl, SO₂-(C₁-C₆)-alkyl, SO₂-(CH₂)ₙ-phenyl, where n can be = 0 - 6 and the phenyl radical can be substituted up to two times by F, Cl, Br, OH, CF₃, NO₂, CN, OCF₃, O-(C₁-C₆)-alkyl, (C₁-C₆)-alkyl, NH₂; NH₂, NH-(C₁-C₆)-alkyl, N((C₁-C₆)-alkyl)₂, NH(C₁-C₇)-acyl, phenyl, biphenyl, O-(CH₂)ₙ-phenyl, where n can be = 0 - 6, 1- or 2-naphthyl, 2-, 3- or 4-pyridyl, 2- or 3-furanyl or 2- or 3-thienyl, where the phenyl, biphenyl, naphthyl, pyridyl, furanyl or thienyl rings in each case can be substituted one to 3 times by F, Cl, Br, I, OH, CF₃, NO₂, CN, OCF₃, O-(C₁-C₆)-alkyl, (C₁-C₆)-alkyl, NH₂, NH(C₁-C₆)-alkyl, N((C₁-C₆)-alkyl)₂, SO₂-CH₃, COOH, COO-(C₁-C₆)-alkyl, CONH₂;
1,2,3-triazol-5-yl, where the triazole ring can be substituted in the 1-, 2- or 3-position by methyl or benzyl; tetrazol-5-yl, where the tetrazole ring can be substituted in the 1- or 2-position by methyl or benzyl;
R1' is H, F, Cl, Br, I, CH₃, CF₃, O-(C₁-C₃)-alkyl, NO₂, SO₂-NH₂, SO₂NH(C₁-C₆)-alkyl, SO₂N[(C₁-C₆)-alkyl]₂ or R1;
R2 is H, (C₁-C₆)-alkyl, (C₃-C₆)-cycloalkyl, (CH₂)ₙ-phenyl, (CH₂)ₙ-thienyl, (CH₂)ₙ-pyridyl, (CH₂)ₙ-furyl, C(O)-(CH₂)ₙ-phenyl, C(O)-(CH₂)ₙ-thienyl, C(O)-(CH₂)ₙ-pyridyl, C(O)-(CH₂)ₙ-furyl, where n can be = 0 - 5 and in which phenyl, thienyl, pyridyl, furyl in each case can be substituted up to two times by Cl, F, CN, CF₃, (C₁-C₃)-alkyl, OH, O-(C₁-C₆)-alkyl; C(O)-(C₁-C₆)-alkyl, C(O)-(C₃-C₆)-cycloalkyl;
R3 is H, (C₁-C₆)-alkyl, F, CN, N₃, O-(C₁-C₆)-alkyl, (CH₂)ₙ-phenyl, CH₂-COO(C₁-C₆ alkyl), CH₂-COO(C₃-C₈ cycloalkyl), CH₂-COOH, CH₂-CONH₂, CH₂-CONHCH₃, CH₂-CON(CH₃)₂, (CH₂)ₙ-thienyl, (CH₂)ₙ-pyridyl, (CH₂)ₙ-furyl, where n can be = 0 - 5 and in which phenyl, thienyl, pyridyl, furyl in each case can be substituted up to two times by Cl, F, CN, CF₃, (C₁-C₃)-alkyl, OH, O-(C₁-C₆)-alkyl; (C₂-C₆)-alkynyl, (C₂-C₆)-alkenyl, C(O)OCH₃, C(O)OCH₂CH₃, C(O)OH, C(O)NH₂, C(O)NHCH₃, C(O)N(CH₃)₂, OC(O)CH₃;
R4 is (C₁-C₆)-alkyl, (C₃-C₆)-cycloalkyl, (CH₂)ₙ-phenyl, (CH₂)ₙ-thienyl, (CH₂)ₙ-pyridyl, (CH₂)ₙ-furyl, where n can be = 0 - 5 and in which phenyl, thienyl, pyridyl, furyl in each case can be substituted up to two times by Cl, F, CN, CF₃, (C₁-C₃)-alkyl, OH, O-(C₁-C₆)-alkyl;
R5 is (C₁-C₆)-alkyl, (C₃-C₆)-cycloalkyl, (CH₂)ₙ-phenyl, (CH₂)ₙ-thienyl, (CH₂)ₙ-pyridyl, (CH₂)ₙ-furyl, where n can be = 0 - 5 and in which phenyl, thienyl, pyridyl, furyl in each case can be substituted up to two times by Cl, F, CN, CF₃, (C₁-C₃)-alkyl, OH, O-(C₁-C₆)-alkyl;
or
R4 and R5 together form a -CH₂-CH₂-, -CH₂-C(CH₃)₂-, -CH₂-CH₂-CH₂-, or -CH₂-CH₂-CH₂-CH₂- group;
or
B)
Y is a direct bond, -CH₂- or -CH₂-CH₂-;
X is CH₂, CH(CH₃), CH(C₂H₅), CH(C₃H₇);
R1 and R1' independently of one another are
H, F, Cl, Br, I, NO₂, CN, COOH, COO(C₁-C₆)-alkyl, CONH₂, CONH(C₁-C₆)-alkyl, CON[(C₁-C₆)-alkyl]₂, (C₁-C₆)-alkyl, (C₂-C₆)-alkenyl, (C₂-C₆)-alkynyl, O-(C₁-C₆)-alkyl, O-CH₂-CF₃, O-CH₂-CF₂-CF₃, O-(C₄-C₆)-alkyl, where in the alkyl radicals one or more, or all hydrogen(s) can be replaced by fluorine, or one hydrogen can be replaced by OH, OC(O)CH₃, O-CH₂-Ph, NH₂ or N(COOCH₂Ph)₂;
S-(C₁-C₆)-alkyl, S-(CH₂)ₙ-phenyl, SO-(C₁-C₆)-alkyl, SO-(CH₂)ₙ-phenyl, SO₂-(C₁-C₆)-alkyl, SO₂-(CH₂)ₙ-phenyl, where n can be = 0 - 6 and the phenyl radical can be substituted up to two times by F, Cl, Br, OH, CF₃, NO₂, CN, OCF₃, O-(C₁-C₆)-alkyl, (C₁-C₆)-alkyl, NH₂; SO₂-NH₂, SO₂NH(C₁-C₆)-alkyl, SO₂N[(C₁-C₆)-alkyl]₂, NH₂, NH-(C₁-C₆)-alkyl, N((C₁-C₆)-alkyl)₂, NH(C₁-C₇)-acyl, phenyl, biphenyl, O-(CH₂)ₙ-phenyl, where n can be = 0 - 6, 1- or 2-naphthyl, 2-, 3- or 4-pyridyl, 2- or 3-furanyl or 2- or 3-thienyl, where the phenyl, biphenyl, naphthyl, pyridyl, furanyl or thienyl rings in each case can be substituted one to 3 times by F, Cl, Br, I, OH, CF₃, NO₂, CN, OCF₃, O-(C₁-C₆)-alkyl, (C₁-C₆)-alkyl, NH₂, NH(C₁-C₆)-alkyl, N((C₁-C₆)-alkyl)₂, SO₂-CH₃, COOH, COO-(C₁-C₆)-alkyl, CONH₂;
1,2,3-triazol-5-yl, where the triazole ring can be substituted in the 1-, 2- or 3-position by methyl or benZyl;
tetrazol-5-yt, where the tetrazole ring can be substituted in the 1- or 2-position by methyl or benzyl;
R2 is H, (C₁-C₆)-alkyl, (C₃-C₆)-cycloalkyl, (CH₂)ₙ-phenyl, (CH₂)ₙ-thienyl, (CH₂)ₙ-pyridyl, (CH₂)ₙ-furyl, C(O)-(CH₂)ₙ-phenyl, C(O)-(CH₂)ₙ-thienyl, C(O)-(CH₂)ₙ-pyridyl, C(O)-(CH₂)ₙ-furyl, where n can be = 0 - 5 and in which phenyl, thienyl, pyridyl, furyl in each case can be substituted up to two times by Cl, F, CN, CF₃, (C₁-C₃)-alkyl, OH, O-(C₁-C₆)-alkyl; C(O)-(C₁-C₆)-alkyl, C(O)-(C₃-C₆)-cycloalkyl;
R3 is (C₄-C₆)-alkyl, F, CN, N₃, O-(C₁-C₆)-alkyl, CH₂-COO(C₁-C₆ alkyl), CH₂-COO(C₃-C₈ cycloalkyl), CH₂-COOH, CH₂-CONH₂, CH₂-CONHCH₃, CH₂-CON(CH₃)₂, (CH₂)ₙ-phenyl, (CH₂)ₙ-thienyl, (CH₂)ₙ-pyridyl, (CH₂)ₙ-furyl, where n can be = 0 - 5 and in which phenyl, thienyl, pyridyl, furyl in each case can be substituted up to two times by Cl, F, CN, CF₃, (C₁-C₃)-alkyl, OH, O-(C₁-C₆)-alkyl; (C₂-C₆)-alkynyl, (C₂-C₆)-alkenyl, C(O)OCH₃, C(O)OCH₂CH₃, C(O)OH, C(O)NH₂, C(O)NHCH₃, C(O)N(CH₃)₂, OC(O)CH₃;
R4 is (C₁-C₆)-alkyl, (C₃-C₆)-cycloalkyl, (CH₂)ₙ-phenyl, (CH₂)ₙ-thienyl, (CH₂)ₙ-pyridyl, (CH₂)ₙ-furyl, where n can be = 0 - 5 and in which phenyl, thienyl, pyridyl, furyl in each case can be substituted up to two times by Cl, F, CN, CF₃, (C₁-C₃)-alkyl, OH, O-(C₁-C₆)-alkyl;
R5 is (C₁-C₆)-alkyl, (C₃-C₆)-cycloalkyl, (CH₂)ₙ-phenyl, (CH₂)ₙ-thienyl, (CH₂)ₙ-pyridyl, (CH₂)ₙ-furyl, where n can be = 0 - 5 and in which phenyl, thienyl, pyridyl, furyl in each case can be substituted up to two times by Cl, F, CN, CF₃, (C₁-C₃)-alkyl, OH, O-(C₁-C₆)-alkyl;
or
R4 and R5 together form a -CH₂-CH₂-, -CH₂-C(CH₃)₂-, -CH₂-CH₂-CH₂-, or -CH₂-CH₂-CH₂-CH₂- group;
or
C)
Y is a direct bond, -CH₂- or-CH₂-CH₂-;
X is CH₂, CH(CH₃), CH(C₂H₅), CH(C₃H₇);
R1 and R1' independently of one another are
H, F, Cl, Br, 1, NO₂, CN, COOH, COO(C₁-C₆)-alkyl, CONH₂, CONH(C₁-C₆)-alkyl, CON[(C₁-C₆)-alkyl]₂, (C₁-C₆)-alkyl, (C₂-C₆)-alkenyl, (C₂-C₆)-alkynyl, O-(C₁-C₆)-alkyl, O-CH₂-CF₃, O-CH₂-CF₂-CF₃, O-(C₄-C₆)-alkyl, where in the alkyl radicals one or more, or all hydrogen(s) can be replaced by fluorine, or one hydrogen can be replaced by OH, OC(O)CH₃, O-CH₂-Ph, NH₂ or N(COOCH₂Ph)₂;
S-(C₁-C₆)alkyl, S-(CH₂)ₙ-phenyl, SO-(C₁-C₆)-alkyl, SO-(CH₂)ₙ-phenyl, SO₂-(C₁-C₆)-alkyl, SO₂-(CH₂)ₙ-phenyl, where n can be = 0 - 6 and the phenyl radical can be substituted up to two times by F, Cl, Br, OH, CF₃, NO₂, CN, OCF₃, O-(C₁-C₆)-alkyl, (C₁-C₆)-alkyl, NH₂; SO₂-NH₂, SO₂NH(C₁-C₆)alkyl, SO₂N[(C₁-C₆)-alkyl]₂, NH₂, NH-(C₁-C₆)-alkyl, N((C₁-C₆)-alkyl)₂, NH(C₁-C₇)-acyl, phenyl, biphenyl, O-(CH₂)ₙ-phenyl, where n can be = 0 - 6, 1- or 2-naphthyl, 2-, 3- or 4-pyridyl, 2- or 3-furanyl or 2- or 3-thienyl, where the phenyl, biphenyl, naphthyl, pyridyl, furanyl or thienyl rings in each case can be substituted one to 3 times by F, Cl, Br, I, OH, CF₃, NO₂, CN, OCF₃, O-(C₁-C₆)-alkyl, (C₁-C₆)-alkyl, NH₂, NH(C1-Cs)-alkyl, N((C₁-C₆)-alkyl)₂, SO₂-CH₃, COOH, COO-(C₁-C₆)-alkyl, CONH₂;
1,2,3-triazol-5-yl, where the triazole ring can be substituted in the 1-, 2- or 3-position by methyl or benzyl;
tetrazol-5-yl, where the tetrazole ring can be substituted in the 1- or 2-position by methyl or benzyl;
R2 is (C₁-C₆)-alkyl, (C₃-C₆)-cycloalkyl, C₂-C₈ alkenyl, C₂-C₈ alkynyl, (CH₂)ₙ-phenyl, (CH₂)ₙ-thienyl, (CH₂)ₙ-pyridyl, (CH₂)ₙ-furyl, C(O)-(CH₂)ₙ-phenyl, C(O)-(CH₂)ₙ-thienyl, C(O)-(CH₂)ₙ-pyridyl, C(O)-(CH₂)ₙ-furyl, where n can be = 0 - 5 and in which phenyl, thienyl, pyridyl, furyl in each case can be substituted up to two times by Cl, F, CN, CF₃, (C₁-C₃)-alkyl, OH, O-(C₁-C₆)-alkyl; C(O)-(C₁-C₆)-alkyl, C(O)-(C₃-C₆)-cycloalkyl;
R3 is H, (C₁-C₆)-alkyl, F, CN, N₃, O-(C₁-C₆)-alkyl, CH₂-COO(C₁-C₆ alkyl), CH₂-COO(C₃-C₈ cycloalkyl), CH₂-COOH, CH₂-CONH₂, CH₂-CONHCH₃, CH₂-CON(CH₃)₂, (CH₂)ₙ-phenyl, (CH₂)ₙ-thienyl, (CH₂)ₙ-pyridyl, (CH₂)ₙ-furyl, where n can be = 0 - 5 and in which phenyl, thienyl, pyridyl, furyl in each case can be substituted up to two times by Cl, F, CN, CF₃, (C₁-C₃)-alkyl, OH, O-(C₁-C₆)-alkyl; (C₂-C₆)-alkynyl, (C₂-C₆)-alkenyl, C(O)OCH₃, C(O)OCH₂CH₃, C(O)OH, C(O)NH₂, C(O)NHCH₃, C(O)N(CH₃)₂, OC(O)CH₃;
R4 is (C₁-C₆)-alkyl, (C₃-C₆)-cycloalkyl, (CH₂)ₙ-phenyl, (CH₂)ₙ-thienyl, (CH₂)ₙ-pyridyl, (CH₂)ₙ-furyl, where n can be = 0 - 5 and in which phenyl, thienyl, pyridyl, furyl in each case can be substituted up to two times by Cl, F, CN, CF₃, (C₁-C₃)-alkyl, OH, O-(C₁-C₆)-alkyl;
R5 is (C₁-C₆)-alkyl, (C₃-C₆)-cycloalkyl, (CH₂)ₙ-phenyl, (CH₂)ₙ-thienyl, (CH₂)ₙ-pyridyl, (CH₂)ₙ-furyl, where n can be = 0 - 5 and in which phenyl, thienyl, pyridyl, furyl in each case can be substituted up to two times by Cl, F, CN, CF₃, (C₁-C₃)-alkyl, OH, O-(C₁-C₆)-alkyl;
or
R4 and R5 together form a -CH₂-CH₂-, -CH₂-C(CH₃)₂-, -CH₂-CH₂-CH₂-, or -CH₂-CH₂-CH₂-CH₂- group;
or
D)
Y is a direct bond, -CH₂- or -CH₂-CH₂-;
X is CH(phenyl), where the phenyl radical can be substituted by F, Cl, Br or I,
O, S, SO, SO₂ or N-R6;
R1 and R1' independently of one another are
H, F, Cl, Br, I, NO₂, CN, COOH, COO(C₁-C₆)-alkyl, CONH₂, CONH(C₁-C₆)-alkyl, CON[(C₁-C₆)-alkyl]₂, (C₁-C₆)-alkyl, (C₂-C₆)-alkenyl, (C₂-C₆)-alkynyl, O-(C₁-C₆)-alkyl, O-CH₂-CF₃, O-CH₂-CF₂-CF₃, O-(C₄-C₆)-alkyl, where in the alkyl radicals one or more, or all hydrogen(s) can be replaced by fluorine, or one hydrogen can be replaced by OH, OC(O)CH₃, O-CH₂-Ph, NH₂ or N(COOCH₂Ph)₂;
S-(C₁-C₆)-alkyl, S-(CH₂)ₙ-phenyl, SO-(C₁-C₆)-alkyl, SO-(CH₂)ₙ-phenyl, SO₂-(C₁-C₆)-alkyl, SO₂-(CH₂)ₙ-phenyl, where n can be = 0 - 6 and the phenyl radical can be substituted up to two times by F, Cl, Br, OH, CF₃, NO₂, CN, OCF₃, O-(C₁-C₆)-alkyl, (C₁-C₆)-alkyl, NH₂; SO₂-NH₂, SO₂NH(C₁-C₆)-alkyl, SO₂N[(C₁-C₆)-alkyl]₂, NH₂, NH-(C₁-C₆)-alkyl, N((C₁-C₆)-alkyl)₂, NH(C₁-C₇)-acyl, phenyl, biphenyl, O-(CH₂)ₙ-phenyl, where n can be = 0 - 6, 1- or 2-naphthyl, 2-, 3- or 4-pyridyl, 2- or 3-furanyl or 2- or 3-thienyl, where the phenyl, biphenyl, naphthyl, pyridyl, furanyl or thienyl rings in each case can be substituted one to 3 times by F, Cl, Br, I, OH, CF₃, NO₂, CN, OCF₃, O-(C₁-C₆)-alkyl, (C₁-C₆)-alkyl, NH₂, NH(C₁-C₆)-alkyl, N((C₁-C₆)-alkyl)₂, COOH, COO-(C₁-C₆)-alkyl, CONH₂;
1,2,3-triazol-5-yl, where the triazole ring can be substituted in the 1-, 2- or 3-position by methyl or benzyl;
tetrazol-5-yl, where the tetrazole ring can be substituted in the 1- or 2-position by methyl or benzyl;
R2 is H, (C₁-C₆)-alkyl, (C₃-C₆)-cycloalkyl, (CH₂)ₙ-phenyl, (CH₂)ₙ-thienyl, (CH₂)ₙ-pyridyl, (CH₂)ₙ-furyl, C(O)-(CH₂)ₙ-phenyl, C(O)-(CH₂)ₙ-thienyl, C(O)-(CH₂)ₙ-pyridyl, C(O)-(CH₂)ₙ-furyl, where n can be = 0 - 5 and in which phenyl, thienyl, pyridyl, furyl in each case can be substituted up to two times by Cl, F, CN, CF₃, (C₁-C₃)-alkyl, OH, O-(C₁-C₆)-alkyl; C(O)-(C₁-C₆)-alkyl, C(O)-(C₃-C₆)-cycloalkyl;
R3 is H, (C₁-C₆)-alkyl, F, CN, N₃, O-(C₁-C₆)-alkyl, CH₂-COO(C₁-C₆ alkyl), CH₂-COO(C₃-C₈ cycloalkyl), CH₂-COOH, CH₂-CONH₂, CH₂-CONHCH₃, CH₂-CON(CH₃)₂, (CH₂)ₙ-phenyl, (CH₂)ₙ-thienyl, (CH₂)ₙ-pyridyl, (CH₂)ₙ-furyl, where n can be = 0 - 5 and in which phenyl, thienyl, pyridyl, furyl in each case can be substituted up to two times by Cl, F, CN, CF₃, (C₁-C₃)-alkyl, OH, O-(C₁-C₆)-alkyl; (C₂-C₆)-alkynyl, (C₂-C₆)-alkenyl, C(O)OCH₃, C(O)OCH₂CH₃, C(O)OH, C(O)NH₂, C(O)NHCH₃, C(O)N(CH₃)₂, OC(O)CH₃;
R4 is (C₁-C₆)-alkyl, (C₃-C₆)-cycloalkyl, (CH₂)ₙ-phenyl, (CH₂)ₙ-thienyl, (CH₂)ₙ-pyridyl, (CH₂)ₙ-furyl, where n can be = 0 - 5 and in which phenyl, thienyl, pyridyl, furyl in each case can be substituted up to two times by Cl, F, CN, CF₃, (C₁-C₃)-alkyl, OH, O-(C₁-C₆)-alkyl;
R5 is (C₁-C₆)-alkyl, (C₃-C₆)-cycloalkyl, (CH₂)ₙ-phenyl, (CH₂)ₙ-thienyl, (CH₂)ₙ-pyridyl, (CH₂)ₙ-furyl, where n can be = 0 - 5 and in which phenyl, thienyl, pyridyl, furyl in each case can be substituted up to two times by Cl, F, CN, CF₃, (C₁-C₃)-alkyl, OH, O-(C₁-C₆)-alkyl;
or
R4 and R5 together form a -CH₂-CH₂-, -CH₂-C(CH₃)₂-, -CH₂-CH₂-CH₂-, or -CH₂-CH₂-CH₂-CH₂- group;
R6 is SO₂-(C₆H₄-4-CH₃)
or
E)
Y is a direct bond, -CH₂- or -CH₂-CH₂-;
X is CH₂, CH(CH₃), CH(C₂H₅), CH(C₃H₇);
R1 is H, F, Cl, Br, I, CH₃, CF₃, O-(C₁-C₃)-alkyl;
R1' is H, F, Cl, Br, I, NO₂;
R2 is H;
R3 is H, (C₁-C₃)-alkyl;
R4 is phenyl, where the phenyl radical can be substituted up to two times by F, Cl, Br, I, (C₁-C₆)-alkyl, (C₃-C₆)-cycloalkyl, O-(C₁-C₃)-alkyl, CF₃, OCF₃, O-CH₂-phenyl, COOH, COO(C₁-C₆)-alkyl, COO(C₃-C₆)-cycloalkyl, CONH₂;
R5 is phenyl, where the phenyl radical can be substituted up to two times by F, Cl, Br, I, (C₁-C₆)-alkyl, (C₃-C₆)-cycloalkyl, O-(C₁-C₃)-alkyl, CF₃, OCF₃, O-CH₂-phenyl, COOH, COO(C₁-C₆)-alkyl, COO(C₃-C₆)-cycloalkyl, CONH₂;
or its physiologically tolerable salts.

2. A compound of the formula I, as claimed in claim 1, wherein
A)
Y is a direct bond, -CH₂;
X is CH₂, CH(CH₃), CH(C₂H₅), CH(C₃H₇);
R1 is CN, COOH, COO(C₁-C₆)-alkyl, CONH₂, CONH(C₁-C₆)-alkyl, CON[(C₁-C₆)-alkyl]₂, (C₂-C₆)-alkyl, (C₂-C₆)-alkenyl, (C₂-C₆)-alkynyl, O-CH₂-CF₃, O-CH₂-CF₂-CF₃, O-(C₄-C₆)-alkyl, where in the alkyl radicals one or more, or all hydrogen(s) can be replaced by fluorine, or one hydrogen can be replaced by OH, OC(O)CH₃; O-CH₂-Ph, NH₂ or N(COOCH₂Ph)₂;
S-(C₁-C₆)-alkyl, S-(CH₂)ₙ-phenyl, SO-(C₁-C₆)-alkyl, SO-(CH₂)ₙ-phenyl, where the phenyl radical can be substituted up to two times by F, Cl, Br, OH, CF₃, NO₂, CN, OCF₃, O-(C₁-C₆)-alkyl, (C₁-C₆)-alkyl; NH-(C₁-C₆)-alkyl, N((C₁-C₆)-alkyl)₂, NH(C₁-C₇)-acyl, phenyl, O-(CH₂)ₙ-phenyl, where n can be = 0-6, 1- or 2-naphthyl, 2-, 3- or 4-pyridyl, 2 - or 3-furanyl or 2- or 3-thienyl, where the phenyl, naphthyl, pyridyl, furanyl or thienyl rings in each case can be substituted one to 3 times by F, Cl, I, OH, CF₃, CN, OCF₃, O-(C₁-C₆)-alkyl, (C₁-C₆)-alkyl, NH₂, NH(C₁-C₆)-alkyl, N((C₁-C₆)-alkyl)₂, COOH, COO-(C₁-C₆)-alkyl, CONH₂;
1,2,3-triazol-5-yl, where the triazole ring can be substituted in the 1-, 2- or 3-position by methyl or benzyl;
tetrazol-5-yl, where the tetrazole ring can be substituted in the 1- or 2-position by methyl or benzyl;
R1' is H, F, Cl, Br, I, CH₃, CF₃, O-(C₁-C₃)-alkyl, SO₂-NH₂, SO₂NH(C₁-C₆)-alkyl, SO₂N[(C₁-C₆)-alkyl]₂ or R1;
R2 is H, (C₁-C₆)-alkyl, (CH₂)ₙ-phenyl, (CH₂)ₙ-thienyl, (CH₂)ₙ-pyridyl, C(O)-(CH₂)ₙ-phenyl, C(O)-(CH₂)ₙ-thienyl, C(O)-(CH₂)ₙ-pyridyl, where n can be = 0 - 3 and in which phenyl, thienyl, pyridyl, furyl in each case can be substituted up to two times by Cl, F, CN, CF₃, (C₁-C₃)-alkyl, OH, O-(C₁-C₆)-alkyl;
R3 is H, (C₁-C₆)-alkyl, F, CN, O-(C₁-C₆)-alkyl, CH₂-COO(C₁-C₆ alkyl), CH₂-COO(C₃-C₈ cycloalkyl), CH₂-COOH, CH₂-CONH₂, CH₂-CONHCH₃, CH₂-CON(CH₃)₂, (CH₂)ₙ-phenyl, (CH₂)n-thienyl, (CH₂)ₙ-pyridyl, where n can be = 0 - 3 and in which phenyl, thienyl, pyridyl in each case can be substituted up to two times by Cl, F, CN, CF₃, (C₁-C₃)-alkyl, OH, O-(C₁-C₆)-alkyl; (C₂-C₆)-alkynyl, (C₂-C₆)-alkenyl, C(O)OCH₃, C(O)OCH₂CH₃, C(O)OH, C(O)NH₂, C(O)N(CH₃)₂, OC(O)CH₃;
R4 is (C₁-C₆)-alkyl, (C₃-C₆)-cycloalkyl, (CH₂)ₙ-phenyl, (CH₂)ₙ-thienyl, (CH₂)ₙ-pyridyl, where n can be = 0 - 3 and in which phenyl, thienyl, pyridyl in each case can be substituted up to two times by Cl, F, CN, CF₃, (C₁-C₃)-alkyl, OH, O-(C₁-C₆)-alkyl;
R5 is (C₁-C₆)-alkyl, (C₃-C₆)-cycloalkyl, (CH₂)ₙ-phenyl, (CH₂)ₙ-thienyl, (CH₂)ₙ-pyridyl, where n can be = 0 - 3 and in which phenyl, thienyl, pyridyl in each case can be substituted up to two times by Cl, F, CN, CF₃, (C₁-C₃)-alkyl, OH, O-(C₁-C₆)-alkyl;
or
R4 and R5 together form a -CH₂-CH₂-, -CH₂-C(CH₃)₂- or -CH₂-CH₂-CH₂- group;
or
B)
Y is a direct bond, -CH₂-;
X is CH₂, CH(CH₃), CH(C₂H₅), CH(C₃H₇);
R1 and R1' independently of one another are
H, F, Cl, Br, I, CN, COOH, COO(C₁-C₆)-alkyl, CONH₂, CONH(C₁-C₆)-alkyl, CON[(C₁-C₆)-alkyl]₂, (C₁-C₆)-alkyl, (C₂-C₆)-alkenyl, (C₂-C₆)-alkynyl, O-(C₁-C₆)-alkyl, O-CH₂-CF₃, O-CH₂-CF₂-CF₃, O-(C₄-C₆)-alkyl, where in the alkyl radicals one or more, or all hydrogen(s) can be replaced by fluorine, or one hydrogen can be replaced by OH, OC(O)CH₃, O-CH₂-Ph, NH₂ or N(COOCH₂Ph)₂;
S-(C₁-C₆)-alkyl, S-(CH₂)ₙ-phenyl, SO-(C₁-C₆)-alkyl, SO-(CH₂)ₙ-phenyl, where the phenyl radical can be substituted up to two times by F, Cl, Br, OH, CF₃, NO₂, CN, OCF₃, O-(C₁-C₆)-alkyl, (C₁-C₆)-alkyl; SO₂-NH₂, SO₂NH(C₁-C₆)-alkyl, SO₂N[(C₁-C₆)-alkyl]₂, NH-(C₁-C₆)-alkyl, N((C₁-C₆)-alkyl)₂, NH(C₁-C₇)-acyl, phenyl, O-(CH₂)ₙ-phenyl, where, n can be = 0 - 6, 1- or 2-naphthyl, 2-, 3- or 4-pyridyl, 2- or 3-furanyl or 2- or 3-thienyl, where the phenyl, naphthyl, pyridyl, furanyl or thienyl rings in each case can be substituted one to 3 times by F, Cl, I, OH, CF₃, CN, OCF₃, O-(C₁-C₆)-alkyl, (C₁-C₆)-alkyl, NH₂, NH(C₁-C₆)-alkyl, N((C₁-C₆)-alkyl)₂, COOH, COO-(C₁-C₆)-alkyl, CONH₂;
1,2,3-triazol-5-yl, where the triazole ring can be substituted in the 1-, 2- or 3-position by methyl or benzyl;
tetrazol-5-yl, where the tetrazole ring can be substituted in the 1- or 2-position by methyl or benzyl;
R2 is H, (C₁-C₆)-alkyl, (CH₂)ₙ-phenyl, (CH₂)ₙ-thienyl, (CH₂)ₙ-pyridyl, C(O)-(CH₂)ₙ-phenyl, C(O)-(CH₂)ₙ-thienyl, C(O)-(CH₂)ₙ-pyridyl, where n can be = 0 - 3 and in which phenyl, thienyl, pyridyl, furyl in each case can be substituted up to two times by Cl, F, CN, CF₃, (C₁-C₃)-alkyl, OH, O-(C₁-C₆)-alkyl;
R3 is (C₄-C₆)-alkyl, F, CN, N₃, O-(C₁-C₆)-alkyl, CH₂-COO(C₁-C₆ alkyl), CH₂-COO(C₃-C₈ cycloalkyl), CH₂-COOH, CH₂-CONH₂, CH₂-CONHCH₃, CH₂-CON(CH₃)₂, (CH₂)ₙ-phenyl, (CH₂)ₙ-thienyl, (CH₂)ₙ-pyridyl, where n can be = 0 - 3 and in which phenyl, thienyl, pyridyl in each case can be substituted up to two times by Cl, F, CN, CF₃, (C₁-C₃)-alkyl, OH, O-(C₁-C₆)-alkyl; (C₂-C₆)-alkynyl, (C₂-C₆)-alkenyl, C(O)OCH₃, C(O)OCH₂CH₃, C(O)OH, C(O)NH₂, C(O)NHCH₃, C(O)N(CH₃)₂, OC(O)CH₃;
R4 is (C₁-C₆)-alkyl, (C₃-C₆)-cycloalkyl, (CH₂)ₙ-phenyl, (CH₂)ₙ-thienyl, (CH₂)ₙ-pyridyl, where n can be = 0 - 3 and in which phenyl, thienyl, pyridyl in each case can be substituted up to two times by Cl, F, CN, CF₃, (C₁-C₃)-alkyl, OH, O-(C₁-C₆)-alkyl;
R5 is (C₁-C₆)-alkyl, (C₃-C₆)-cycloalkyl, (CH₂)ₙ-phenyl, (CH₂)ₙ-thienyl, (CH₂)ₙ-pyridyl, where n can be = 0 - 3 and in which phenyl, thienyl, pyridyl in each case can be substituted up to two times by Cl, F, CN, CF₃, (C₁-C₃)-alkyl, OH, O-(C₁-C₆)-alkyl;
or
R4 and R5 together form a -CH₂-CH₂-, -CH₂-C(CH₃)₂- or -CH₂-CH₂-CH₂- group;
or
C)
Y is a direct bond, or -CH₂-;
X is CH₂, CH(CH₃), CH(C₂H₅), CH(C₃H₇);
R1 and R1' independently of one another are
H, F, Cl, Br, I, CN, COOH, COO(C₁-C₆)-alkyl, CONH₂, CONH(C₁-C₆)-alkyl, CON[(C₁-C₆)-alkyl]₂, (C₁-C₆)-alkyl, (C₂-C₆)-alkenyl, (C₂-C₆)-alkynyl, O-(C₁-C₆)-alkyl, O-CH₂-CF₃, O-CH₂-CF₂-CF₃, O-(C₄-C₆)-alkyl, where in the alkyl radicals one or more, or all hydrogen(s) can be replaced by fluorine, or one hydrogen can be replaced by OH, OC(O)CH₃, O-CH₂-Ph, NH₂ or N(COOCH₂Ph)₂;
S-(C₁-C₆)-alkyl, S-(CH₂)ₙ-phenyl, SO₂-(C₁-C₆)-alkyl, SO₂-(CH₂)ₙ-phenyl, where n can be = 0 - 6 and the phenyl radical can be substituted up to two times by F, Cl, Br, OH, CF₃, NO₂, CN, OCF₃, O-(C₁-C₆)-alkyl, (C₁-C₆)-alkyl; SO₂-NH₂, SO₂NH(C₁-C₆)-alkyl, SO₂N[(C₁-C₆)-alkyl]₂, NH-(C₁-C₆)-alkyl, N((C₁-C₆)-alkyl)₂, NH(C₁-C₇)-acyl, phenyl, O-(CH₂)ₙ-phenyl, where n can be = 0 - 4, 1- or 2-naphthyl, 2-, 3- or 4-pyridyl, 2- or 3-furanyl or 2- or 3-thienyl, where the phenyl, naphthyl, pyridyl, furanyl or thienyl rings in each case can be substituted one to 3 times by F, Cl, I, OH, CF₃, CN, OCF_{3,} O-(C₁-C₆)-alkyl, (C₁-C₆)-alkyl, NH₂, NH(C₁-C₆)-alkyl, N((C₁-C₆)-alkyl)₂, COOH, COO-(C₁-C₆)-alkyl, CONH₂;
1,2,3-triazol-5-yl, where the triazole ring can be substituted in the 1-, 2- or 3-position by methyl or benzyl;
tetrazol-5-yl, where the tetrazole ring can be substituted in the 1- or 2-position by methyl or benzyl;
R2 is (C₁-C₆)-alkyl, C₂-C₈ alkenyl, C₂-C₈ alkynyl, (CH₂)ₙ-phenyl, (CH₂)ₙ-thienyl, (CH₂)ₙ-pyridyl, C(O)-(CH₂)ₙ-phenyl, C(O)-(CH₂)ₙ-thienyl, C(O)-(CH₂)ₙ-pyridyl, where n can be = 0 - 3 and in which phenyl, thienyl, pyridyl, furyl in each case can be substituted up to two times by Cl, F, CN, CF₃, (C₁-C₃)-alkyl, OH, O-(C₁-C₆)-alkyl;
R3 is H, (C₁-C₆)-alkyl, F, CN, O-(C₁-C₆)-alkyl, CH₂-COO(C₁-C₆ alkyl), CH₂-COO(C₃-C₈ cycloalkyl), CH₂-COOH, CH₂-CONH₂, CH₂-CONHCH₃, CH₂-CON(CH₃)₂, (CH₂)ₙ-phenyl, (CH₂)ₙ-thienyl, (CH₂)ₙ-pyridyl, where n can be = 0 - 3 and in which phenyl, thienyl, pyridyl in each case can be substituted up to two times by Cl, F, CN, CF₃, (C₁-C₃)-alkyl, OH, O-(C₁-C₆)-alkyl; (C₂-C₆)-alkynyl, (C₂-C₆)-alkenyl, C(O)OCH₃, C(O)OCH₂CH₃, C(O)OH, C(O)NH₂, C(O)N(CH₃)₂, OC(O)CH₃;
R4 is (C₁-C₆)-alkyl, (C₃-C₆)-cycloalkyl, (CH₂)ₙ-phenyl, (CH₂)ₙ-thienyl, (CH₂)ₙ-pyridyl, where n can be = 0 - 3 and in which phenyl, thienyl, pyridyl in each case can be substituted up to two times by Cl, F, CN, CF₃, (C₁-C₃)-alkyl, OH, O-(C₁-C₆)-alkyl;
R5 is (C₁-C₆)-alkyl, (C₃-C₆)-cycloalkyl, (CH₂)ₙ-phenyl, (CH₂)ₙ-thienyl, (CH₂)ₙ-pyridyl, where n can be = 0 - 3 and in which phenyl, thienyl, pyridyl in each case can be substituted up to two times by Cl, F, CN, CF₃, (C₁-C₃)-alkyl, OH, O-(C₁-C₆)-alkyl;
or
R4 and R5 together form a -CH₂-CH₂-, -CH₂-C(CH₃)₂- or -CH₂-CH₂-CH₂- group;
or
D)
Y is a direct bond, or -CH₂-;
X is CH(phenyl), where the phenyl radical can be substituted by F, Cl or Br,
O, S, SO, SO₂ or N-R6;
R1 and R1' independently of one another are
H, F, Cl, Br, I, CN, COOH, COO(C₁-C₆)-alkyl, CONH₂, CONH(C₁-C₆)-alkyl, CON[(C₁-C₆)-alkyl]₂, (C₁-C₆)-alkyl, (C₂-C₆)-alkenyl, (C₂-C₆)-alkynyl, O-(C₁-C₆)-alkyl, O-CH₂-CF₃, O-CH₂-CF₂-CF₃, O-(C₄-C₆)-alkyl, where in the alkyl radicals one or more, or all hydrogen(s) can be replaced by fluorine, or one hydrogen can be replaced by OH, OC(O)CH₃, O-CH₂-Ph, NH₂ or N(COOCH₂Ph)₂;
S-(C₁-C₆)-alkyl, S-(CH₂)ₙ-phenyl, SO₂-(C₁-C₆)-alkyl, SO₂-(CH₂)ₙ-phenyl, where n can be = 0 - 6 and the phenyl radical can be substituted up to two times by F, Cl, Br, OH, CF₃, NO₂, CN, OCF₃, O-(C₁-C₆)-alkyl, (C₁-C₆)-alkyl; SO₂-NH₂, SO₂NH(C₁-C₆)-alkyl, SO₂N[(C₁-C₆)-alkyl]₂, NH₂, NH-(C₁-C₆)-alkyl, N((C₁-C₆)-alkyl)₂, NH(C₁-C₇)-acyl, phenyl, O-(CH₂)ₙ-phenyl, where n can be = 0 - 6, 1- or 2-naphthyl, 2-, 3- or 4-pyridyl, 2- or 3-furanyl or 2- or 3-thienyl, where the phenyl, naphthyl, pyridyl, furanyl or thienyl rings in each case can be substituted one to 3 times by F, Cl, I, OH, CF₃, CN, OCF₃, O-(C₁-C₆)-alkyl, (C₁-C₆)-alkyl, NH₂, NH(C₁-C₆)-alkyl, N((C₁-C₆)-alkyl)₂, COOH, COO-(C₁-C₆)-alkyl, CONH₂;
1,2,3-triazol-5-yl, where the triazole ring can be substituted in the 1-, 2- or 3-position by methyl or benzyl;
tetrazol-5-yl, where the tetrazole ring can be substituted in the 1- or 2-position by methyl or benzyl;
R2 is H, (C₁-C₆)-alkyl, (CH₂)ₙ-phenyl, (CH₂)ₙ-thienyl, (CH₂)ₙ-pyridyl, C(O)-(CH₂)ₙ-phenyl, C(O)-(CH₂)ₙ-thienyl, C(O)-(CH₂)ₙ-pyridyl, where n can be = 0 - 3 and in which phenyl, thienyl, pyridyl, furyl in each case can be substituted up to two times by Cl, F, CN, CF₃, (C₁-C₃)-alkyl, OH, O-(C₁-C₆)-alkyl;
R3 is H, (C₁-C₆)-alkyl, F, CN, O-(C₁-C₆)-alkyl, CH₂-COO(C₁-C₆ alkyl), CH₂-COO(C₃-C₈ cycloalkyl), CH₂-COOH, CH₂-CONH₂, CH₂-CONHCH₃, CH₂-CON(CH₃)₂, (CH₂)ₙ-phenyl, (CH₂)ₙ-thienyl, (CH₂)ₙ-pyridyl, where n can be = 0 - 3 and in which phenyl, thienyl, pyridyl in each case can be substituted up to two times by Cl, F, CN, CF₃, (C₁-C₃)-alkyl, OH, O-(C₁-C₆)-alkyl; (C₂-C₆)-alkynyl, (C₂-C₆)-alkenyl, C(O)OCH₃, C(O)OCH₂CH₃, C(O)OH, C(O)NH₂, C(O)NHCH₃, C(O)N(CH₃)₂, OC(O)CH₃;
R4 is (C₁-C₆)-alkyl, (C₃-C₆)-cycloalkyl, (CH₂)ₙ-phenyl, (CH₂)ₙ-thienyl, (CH₂)ₙ-pyridyl, where n can be = 0 - 3 and in which phenyl, thienyl, pyridyl in each case can be substituted up to two times by Cl, F, CN, CF₃, (C₁-C₃)-alkyl, OH, O-(C₁-C₆)-alkyl;
R5 is (C₁-C₆)-alkyl, (C₃-C₆)-cycloalkyl, (CH₂)ₙ-phenyl, (CH₂)ₙ-thienyl, (CH₂)ₙ-pyridyl, where n can be = 0 - 3 and in which phenyl, thienyl, pyridyl in each case can be substituted up to two times by Cl, F, CN, CF₃, (C₁-C₃)-alkyl, OH, O-(C₁-C₆)-alkyl;
or
R4 and R5 together form a -CH₂-CH₂-, -CH₂-C(CH₃)₂- or -CH₂-CH₂-CH₂- group;
R6 is SO₂-(C₆H₄-4-CH₃)
or
E)
Y is a direct bond or -CH₂-;
X is CH₂, CH(CH₃), CH(C₂H₅), CH(C₃H₇);
R1 is H, F, Cl, Br, I, CH₃, CF₃, O-(C₁-C₃)-alkyl;
R1' is H, F, Cl, Br, I;
R2 is H;
R3 is H, (C₁-C₃)-alkyl;
R4 is phenyl, where the phenyl radical can be substituted up to two times by F, Cl, Br, I, (C₁-C₆)-alkyl, (C₃-C₆)-cycloalkyl, O-(C₁-C₃)-alkyl, CF₃, OCF₃, O-CH₂-phenyl, COOH, COO(C₁-C₆)-alkyl, CONH₂;
R5 is phenyl, where the phenyl radical can be substituted up to two times by F, Cl, Br, I, (C₁-C₆)-alkyl, (C₃-C₆)-cycloalkyl, O-(C₁-C₃)-alkyl, CF₃, OCF₃, O-CH₂-phenyl, COOH, COO(C₁-C₆)-alkyl, CONH₂;
or its physiologically tolerable salts.

3. A compound of the formula I, as claimed in claim 1 or 2, wherein
A)
Y is a direct bond;
X is CH₂, CH(CH₃), CH(C₂H₅), CH(C₃H₇);
R1 is CN, COOH, COO(C₁-C₆)-alkyl, CONH₂, CON[(C₁-C₆)-alkyl]₂, (C₂-C₆)-alkyl, (C₂-C₆)-alkenyl, (C₂-C₆)-alkynyl, O-CH₂-CF₃, O-CH₂-CF₂-CF₃, O-(C₄-C₆)-alkyl, where in the alkyl radicals one or more, or all hydrogen(s) can be replaced by fluorine, or one hydrogen can be replaced by OH, OC(O)CH₃, O-CH₂-Ph, NH₂ or N(COOCH₂Ph)₂;
S-(C₁-C₆)-alkyl, S-(CH₂)ₙ-phenyl, SO-(C₁-C₆)-alkyl, SO-(CH₂)ₙ-phenyl, where the phenyl radical can be substituted up to two times by F, Cl, Br, OH, CF₃, NO₂, CN, OCF₃, O-(C₁-C₆)-alkyl, (C₁-C₆)-alkyl; NH-(C₁-C₆)-alkyl, N((C₁-C₆)-alkyl)₂, NH(C₁-C₇)-acyl, phenyl, O-(CH₂)ₙ-phenyl, where n can be = 0 - 6, 1- or 2-naphthyl, 2-, 3- or 4-pyridyl, 2- or 3-furanyl or 2- or 3-thienyl, where the phenyl, naphthyl, pyridyl, furanyl or thienyl rings in each case can be substituted one to 2 times by F, Cl, OH, CF₃, CN, OCF₃, O-(C₁-C₆)-alkyl, (C₁-C₆)-alkyl, COOH, COO-(C₁-C₆)-alkyl, CONH₂;
1,2,3-triazol-5-yl, where the triazole ring can be substituted in the 1-, 2- or 3-position by methyl or benzyl;
tetrazol-5-yl, where the tetrazole ring can be substituted in the 1- or 2-position by methyl or benzyl;
R1' is H, F, Cl, CH₃, CF₃, O-(C₁-C₃)-alkyl, SO₂-NH₂, SO₂NH(C₁-C₆)-alkyl, SO₂N[(C₁-C₆)-alkyl]₂ or R1;
R2 is H, (C₁-C₆)-alkyl, (CH₂)ₙ-phenyl, (CH₂)ₙ-pyridyl, C(O)-(CH₂)ₙ-phenyl, C(O)-(CH₂)ₙ-pyridyl, where n can be = 0 - 3 and in which phenyl, pyridyl in each case can be substituted up to two times by Cl, F, CN, CF₃, (C₁-C₃)-alkyl, O-(C₁-C₆)-alkyl; C(O)-(C₁-C₆)-alkyl;
R3 is H, (C₁-C₆)-alkyl, F, CN, O-(C₁-C₆)-alkyl, CH₂-COO(C₁-C₆ alkyl), CH₂-COO(C₃-C₈ cycloalkyl), CH₂-COOH, CH₂-CONH₂, CH₂-CONHCH₃, CH₂-CON(CH₃)₂, (CH₂)ₙ-phenyl, (CH₂)n-pyridyl, where n can be = 0 - 3 and in which phenyl, pyridyl in each case can be substituted up to two times by Cl, F, CN, CF₃, (C₁-C₃)-alkyl, O-(C₁-C₆)-alkyl; (C₂-C₆)-alkynyl, C(O)OCH₃, C(O)OCH₂CH₃, C(O)OH, C(O)NH₂, C(O)N(CH₃)₂, OC(O)CH₃;
R4 is (C₁-C₆)-alkyl, (C₃-C₆)-cycloalkyl, (CH₂)ₙ-phenyl, (CH₂)ₙ--pyridyl, where n can be = 0 - 3 and in which phenyl, pyridyl in each case can be substituted up to two times by Cl, F, CN, CF₃, (C₁-C₃)-alkyl, O-(C₁-C₆)-alkyl;
R5 is (C₁-C₆)-alkyl, (C₃-C₆)-cycloalkyl, (CH₂)ₙ-phenyl, (CH₂)ₙ-pyridyl, where n can be = 0 - 3 and in which phenyl, pyridyl in each case can be substituted up to two times by Cl, F, CN, CF₃, (C₁-C₃)-alkyl, O-(C₁-C₆)-alkyl;
or
R4 and R5 together form a -CH₂-CH₂-, -CH₂-C(CH₃)₂- or -CH₂-CH₂-CH₂- group;
or
B)
Y is a direct bond, -CH₂-;
X is CH₂, CH(CH₃), CH(C₂H₅), CH(C₃H₇);
R1 and R1' independently of one another are
H, F, Cl, Br, I, CN, COOH, COO(C₁-C₆)-alkyl, CONH₂, CON[(C₁-C₆)-alkyl]₂, (C₁-C₆)-alkyl, (C₂-C₆)-alkenyl, (C₂-C₆)-alkynyl, O-(C₁-C₆)-alkyl, O-CH₂-CF₃, O-CH₂-CF₂-CF₃, O-(C₄-C₆)-alkyl, where in the alkyl radicals one or more, or all hydrogen(s) can be replaced by fluorine, or one hydrogen can be replaced by OH, OC(O)CH₃, O-CH₂-Ph, NH₂ or N(COOCH₂Ph)₂;
S-(C₁-C₆)-alkyl, S-(CH₂)ₙ-phenyl, SO-(C₁-C₆)-alkyl, SO-(CH₂)ₙ-phenyl, where the phenyl radical can be substituted up to two times by F, Cl, OH, CF₃, OCF₃, O-(C₁-C₆)-alkyl, (C₁-C₆)-alkyl; SO₂-NH₂, SO₂NH(C₁-C₆)-alkyl, SO₂N[(C₁-C₆)-alkyl]₂, N((C₁-C₆)-alkyl)₂, NH(C₁-C₇)-acyl, phenyl, O-(CH₂)ₙ-phenyl, where n can be = 0 - 6, 1- or 2-naphthyl, 2-, 3- or 4-pyridyl, 2- or 3-furanyl or 2- or 3-thienyl, where the phenyl, naphthyl, pyridyl, furanyl or thienyl rings in each case can be substituted one to 3 times by F, Cl, I, OH, CF₃, CN, OCF₃, O-(C₁-C₆)-alkyl, (C₁-C₆)-alkyl, NH₂, N((C₁-C₆)-alkyl)₂, COOH, COO-(C₁-C₆)-alkyl, CONH₂;
1,2,3-triazol-5-yl, where the triazole ring can be substituted in the 1-, 2- or 3-position by methyl or benzyl;
tetrazol-5-yl, where the tetrazole ring can be substituted in the 1- or 2-position by methyl or benzyl;
R2 is H, (C₁-C₆)-alkyl, (CH₂)ₙ-phenyl, (CH₂)ₙ-pyridyl, C(O)-(CH₂)ₙ-phenyl, C(O)-(CH₂)ₙ-pyridyl, where n can be = 0 - 3 and in which phenyl, pyridyl in each case can be substituted up to two times by Cl, F, CN, CF₃, (C₁-C₃)-alkyl, OH, O-(C₁-C₆)-alkyl; C(O)-(C₁-C₆)-alkyl;
R3 is (C₄-C₆)-alkyl, F, CN, N₃, O-(C₁-C₆)-alkyl, CH₂-COO(C₁-C₆ alkyl), CH₂-COO(C₃-C₈ cycloalkyl), CH₂-COOH, CH₂-CONH₂, CH₂-CONHCH₃, CH₂-CON(CH₃)₂, (CH₂)ₙ-phenyl, (CH₂)n-pyridyl, where n can be = 0 - 3 and in which phenyl, pyridyl in each case can be substituted up to two times by Cl, F, CN, CF₃, (C₁-C₃)-alkyl, O-(C₁-C₆)-alkyl; (C₂-C₆)-alkynyl, C(O)OCH₃, C(O)OCH₂CH₃, C(O)OH, C(O)NH₂, C(O)NHCH₃, C(O)N(CH₃)₂, OC(O)CH₃;
R4 is (C₁-C₆)-alkyl, (C₃-C₆)-cycloalkyl, (CH₂)ₙ-phenyl, (CH₂)ₙ-pyridyl, where n can be = 0 - 3 and in which phenyl, pyridyl in each case can be substituted up to two times by Cl, F, CN, CF₃, (C₁-C₃)-alkyl, O-(C₁-C₆)-alkyl;
R5 is (C₁-C₆)-alkyl, (C₃-C₆)-cycloalkyl, (CH₂)ₙ-phenyl, (CH₂)n-pyridyl, where n can be = 0 - 3 and in which phenyl, pyridyl in each case can be substituted up to two times by Cl, F, CN, CF₃, (C₁-C₃)-alkyl, O-(C₁-C₆)-alkyl;
or
R4 and R5 together form a -CH₂-CH₂-, -CH₂-C(CH₃)₂- or -CH₂-CH₂-CH₂- group;
or
C)
Y is a direct bond or -CH₂-;
X is CH₂, CH(CH₃), CH(C₂H₅), CH(C₃H₇);
R1 and R1' independently of one another are
H, F, Cl, Br, I, CN, COOH, COO(C₁-C₆)-alkyl, CONH₂, CON[(C₁-C₆)-alkyl]₂, (C₁-C₆)-alkyl, (C₂-C₆)-alkenyl, (C₂-C₆)-alkynyl, O-(C₁-C₆)-alkyl, O-CH₂-CF₃, O-CH₂-CF₂-CF₃, O-(C₄-C₆)-alkyl, where in the alkyl radicals one or more, or all hydrogen(s) can be replaced by fluorine, or one hydrogen can be replaced by OH, OC(O)CH₃, O-CH₂-Ph, NH₂ or N(COOCH₂Ph)₂;
S-(C₁-C₆)-alkyl, S-(CH₂)ₙ-phenyl, SO₂-(C₁-C₆)-alkyl, SO₂-(CH₂)ₙ-phenyl, where n can be = 0 - 6 and the phenyl radical can be substituted up to two times by F, Cl, Br, OH, CF₃, NO₂, OCF₃, O-(C₁-C₆)-alkyl, (C₁-C₆)-alkyl; SO₂-NH₂, SO₂NH(C₁-C₆)-alkyl, SO₂N[(C₁-C₆)-alkyl]₂, N((C₁-C₆)-alkyl)₂, NH(C₁-C₇)-acyl, phenyl, O-(CH₂)ₙ-phenyl, where n can be = 0 - 4, 1- or 2-naphthyl, 2-, 3- or 4-pyridyl, 2- or 3-furanyl or 2- or 3-thienyl, where the phenyl, naphthyl, pyridyl, furanyl or thienyl rings in each case can be substituted one to 3 times by F, Cl, I, OH, CF₃, CN, OCF₃, O-(C₁-C₆)-alkyl, (C₁-C₆)-alkyl, NH₂, NH(C₁-C₆)-alkyl, N((C₁-C₆)-alkyl)₂, COOH, COO-(C₁-C₆)-alkyl, CONH₂;
1,2,3-triazol-5-yl, where the triazole ring can be substituted in the 1-, 2- or 3-position by methyl or benzyl;
tetrazol-5-yl, where the tetrazole ring can be substituted in the 1- or 2-position by methyl or benzyl;
R2 is (C₁-C₆)-alkyl, C₂-C₈ alkenyl, C₂-C₈ alkynyl, (CH₂)ₙ-phenyl, (CH₂)ₙ-thienyl, (CH₂)ₙ-pyridyl, C(O)-(CH₂)ₙ-phenyl, C(O)-(CH₂)ₙ-thienyl, C(O)-(CH₂)ₙ-pyridyl, where n can be = 0 - 3 and in which phenyl, thienyl, pyridyl, furyl in each case can be substituted up to two times by Cl, F, CN, CF₃, (C₁-C₃)-alkyl, OH, O-(C₁-C₆)-alkyl; C(O)-(C₁-C₆)-alkyl;
R3 is H, (C₁-C₆)-alkyl, F, CN, O-(C₁-C₆)-alkyl, CH₂-COO(C₁-C₆ alkyl), CH₂-COO(C₃-C₈ cycloalkyl), CH₂-COOH, CH₂-CONH₂, CH₂-CONHCH₃, CH₂-CON(CH₃)₂, (CH₂)ₙ-phenyl, (CH₂)ₙ-pyridyl, where n can be = 0 - 3 and in which phenyl, pyridyl in each case can be substituted up to two times by Cl, F, CN, CF₃, (C₁-C₃)-alkyl, O-(C₁-C₆)-alkyl; (C₂-C₆)-alkynyl, C(O)OCH₃, C(O)OCH₂CH₃, C(O)OH, C(O)NH₂, C(O)N(CH₃)₂, OC(O)CH₃;
R4 is (C₁-C₆)-alkyl, (C₃-C₆)-cycloalkyl, (CH₂)ₙ-phenyl, (CH₂)ₙ-pyridyl, where n can be = 0 - 3 and in which phenyl, pyridyl in each case can be substituted up to two times by Cl, F, CN, CF₃, (C₁-C₃)-alkyl, O-(C₁-C₆)-alkyl;
R5 is (C₁-C₆)-alkyl, (C₃-C₆)-cycloalkyl, (CH₂)ₙ-phenyl, (CH₂)ₙ-pyridyl, where n can be = 0 - 3 and in which phenyl, pyridyl in each case can be substituted up to two times by Cl, F, CN, CF₃, (C₁-C₃)-alkyl, O-(C₁-C₆)-alkyl;
or
R4 and R5 together form a -CH₂-CH₂-, -CH₂-C(CH₃)₂- or -CH₂-CH₂-CH₂- group;
or
D)
Y is a direct bond or -CH₂-;
X is CH(phenyl), where the phenyl radical can be substituted by F or Cl,
O, S, SO₂ or N-R6;
R1 and R1' independently of one another are
H, F, Cl, Br, I, CN, COOH, COO(C₁-C₆)-alkyl, CONH₂, CON[(C₁-C₆)-alkyl]₂, (C₁-C₆)-alkyl, (C₂-C₆)-alkenyl, (C₂-C₆)-alkynyl, O-(C₁-C₆)-alkyl, O-CH₂-CF₃, O-CH₂-CF₂-CF₃, O-(C₄-C₆)-alkyl, where in the alkyl radicals one or more, or all hydrogen(s) can be replaced by fluorine, or one hydrogen can be replaced by OH, OC(O)CH₃, O-CH₂-Ph, NH₂ or N(COOCH₂Ph)₂;
S-(C₁-C₆)-alkyl, S-(CH₂)ₙ-phenyl, SO₂-(C₁-C₆)-alkyl, SO₂-(CH₂)ₙ-phenyl, where n can be = 0 - 6 and the phenyl radical can be substituted up to two times by F, Cl, Br, OH, CF₃, NO₂, CN, OCF₃, O-(C₁-C₆)-alkyl, (C₁-C₆)-alkyl; SO₂-NH₂, SO₂NH(C₁-C₆)-alkyl, SO₂N[(C₁-C₆)-alkyl]₂, NH₂, N((C₁-C₆)-alkyl)₂, NH(C₁-C₇)-acyl, phenyl, O-(CH₂)ₙ-phenyl, where n can be = 0 - 6, 1- or 2-naphthyl, 2-, 3- or 4-pyridyl, 2- or 3-furanyl or 2- or 3-thienyl, where the phenyl, naphthyl, pyridyl, furanyl or thienyl rings in each case can be substituted one to 3 times by F, Cl, I, OH, CF₃, CN, OCF₃, O-(C₁-C₆)-alkyl, (C₁-C₆)-alkyl, NH₂, N((C₁-C₆)-alkyl)₂, COOH, COO-(C₁-C₆)-alkyl, CONH₂;
1,2,3-triazol-5-yl, where the triazole ring can be substituted in the 1-, 2- or 3-position by methyl or benzyl;
tetrazol-5-yl, where the tetrazole ring can be substituted in the 1- or 2-position by methyl or benzyl;
R2 is H, (C₁-C₆)-alkyl, (CH₂)ₙ-phenyl, (CH₂)ₙ-thienyl, (CH₂)ₙ-pyridyl, C(O)-(CH₂)ₙ-phenyl, C(O)-(CH₂)ₙ-thienyl, C(O)-(CH₂)ₙ-pyridyl, where n can be = 0 - 3 and in which phenyl, thienyl, pyridyl, furyl in each case can be substituted up to two times by Cl, F, CN, CF₃, (C₁-C₃)-alkyl, OH, O-(C₁-C₆)-alkyl; C(O)-(C₁-C₆)-alkyl;
R3 is H, (C₁-C₆)-alkyl, F, CN, O-(C₁-C₆)-alkyl, CH₂-COO(C₁-C₆ alkyl), CH₂-COO(C₃-C₈ cycloalkyl), CH₂-COOH, CH₂-CONH₂, CH₂-CONHCH₃, CH₂-CON(CH₃)₂, (CH₂)ₙ-phenyl, (CH₂)ₙ-pyridyl, where n can be = 0 - 3 and in which phenyl, pyridyl in each case can be substituted up to two times by Cl, F, CN, CF₃, (C₁-C₃)-alkyl, O-(C₁-C₆)-alkyl; (C₂-C₆)-alkynyl, C(O)OCH₃, C(O)OCH₂CH₃, C(O)OH, C(O)NH₂, C(O)NHCH₃, C(O)N(CH₃)₂, OC(O)CH₃;
R4 is (C₁-C₆)-alkyl, (C₃-C₆)-cycloalkyl, (CH₂)ₙ-phenyl, (CH₂)ₙ-pyridyl, where n can be = 0 - 3 and in which phenyl, pyridyl in each case can be substituted up to two times by Cl, F, CN, CF₃, (C₁-C₃)-alkyl, O-(C₁-C₆)-alkyl;
R5 is (C₁-C₆)-alkyl, (C₃-C₆)-cycloalkyl, (CH₂)ₙ-phenyl, (CH₂)ₙ-pyridyl, where n can be = 0 - 3 and in which phenyl, pyridyl in each case can be substituted up to two times by Cl, F, CN, CF₃, (C₁-C₃)-alkyl, O-(C₁-C₆)-alkyl;
or
R4 and R5 together form a -CH₂-CH₂-, -CH₂-C(CH₃)₂- or -CH₂-CH₂-CH₂- group;
R6 is SO₂-(C₆H₄-4-CH₃)
or
E)
Y is a direct bond or -CH₂-;
X is CH₂, CH(CH₃), CH(C₂H₅), CH(C₃H₇);
R1 is H, F, Cl, CH₃, CF₃, O-(C₁-C₃)-alkyl;
R1' is H, F, Cl;
R2 is H;
R3 is H, (C₁-C₃)-alkyl;
R4 is phenyl, where the phenyl radical can be substituted up to two times by F, Cl, (C₁-C₆)-alkyl, (C₃-C₆)-cycloalkyl, O-(C₁-C₃)-alkyl, CF₃, O-CH₂-phenyl, COOH, COO(C₁-C₆)-alkyl, CONH₂;
R5 is phenyl, where the phenyl radical can be substituted up to two times by F, Cl, (C₁-C₆)-alkyl, (C₃-C₆)-cycloalkyl, O-(C₁-C₃)-alkyl, CF₃, O-CH₂-phenyl, COOH, COO(C₁-C₆)-alkyl, CONH₂;
or its physiologically tolerable salts.

4. A compound of the formula I, as claimed in claim 1, 2 or 3, wherein
Y is a direct bond;
X is CH₂
R1 and R1' independently of one another are H, F, Cl, CN, COOH, CONH₂, COO(C₁-C₃)-alkyl, (C₁-C₆)-alkyl, (C₂-C₆)-alkenyl, (C₂-C₆)-alkynyl, where in the alkyl, alkenyl and alkynyl radicals one hydrogen can be replaced by OH, OC(O)CH₃, O-CH₂-Ph, NH₂ or N(COOCH₂Ph)₂;
OCF₃, OCH₂CF₃, O-(C₁-C₄)-alkyl, where in the alkyl radicals one or more, or all hydrogen(s) can be replaced by fluorine, or one hydrogen can be replaced by OH, OC(O)CH₃, O-CH₂-Ph, NH₂ or N(COOCH₂Ph)₂;
SO₂-(C₁-C₆)-alkyl, SO₂-(CH₂)ₙ-phenyl, where n can be = 0 - 3 and the phenyl radical can be substituted by F, Cl, OH, CF₃, O-(C₁-C₄)-alkyl;
NH-(CO)-(C₁-C₃)-alkyl; (CH₂)ₙ-phenyl, S-(CH₂)ₙ-phenyl, O-(CH₂)ₙ-phenyl, where n can be = 0 - 3, 1- or 2-naphthyl, 2-, 3- or 4-pyridyl, 2- or 3-furanyl or 2- or 3-thienyl, where the phenyl, naphthyl, pyridyl, furanyl or thienyl rings in each case can be substituted by F, Cl, CF₃, (C₁-C₆)-alkyl, O-(C₁-C₆)-alkyl and where in the alkyl radicals one hydrogen can be replaced by OH, OC(O)CH₃, O-CH₂-Ph, NH₂ or N(COOCH₂Ph)₂;
1,2,3-triazol-5-yl, where the triazole ring can be substituted in the 1-, 2- or 3-position by methyl or benzyl;
tetrazol-5-yl, where the tetrazole ring can be substituted in the 1- or 2-position by methyl or benzyl;
R2 is H, (C₁-C₄)-alkyl, (C₅-C₆)-cycloalkyl; (CH₂)ₙ-phenyl, where n can be = 0 - 3, C(O)-(C₁-C₄)-alkyl or C(O)-phenyl;
R3 is F, (C₄-C₆)-alkyl, CH₂-phenyl, where phenyl can be substituted up to two times by F, Cl, CF₃, O-(C₁-C₃)-alkyl, (C₁-C₃)-alkyl, COOH, CO-O-(C₁-C₃)-alkyl or CONH₂;
R4 is (C₁-C₆)-alkyl, (C₃-C₆)-cycloalkyl, (CH₂)ₙ-phenyl, where n can be = 0 - 3 and the phenyl radical can be substituted up to two times by F, Cl, O-(C₁-C₄)-alkyl or OH;
R5 is (C₁-C₆)-alkyl, (C₃-C₆)-cycloalkyl, (CH₂)ₙ-phenyl, where n can be = 0 - 3 and the phenyl radical can be substituted up to two times by F, Cl, O-(C₁-C₄)-alkyl or OH;
or its physiologically tolerable salts.

5. A compound of the formula I as claimed in one or more of claims 1 to 4, wherein
R1 denotes 6-Cl
R1' denotes H
R2 denotes H
R3 denotes F
R4 denotes CH₃
R5 denotes CH₃
X denotes CH₂
Y denotes -
or its physiologically tolerable salts.

6. A hydrochloride of the compounds of the formula I as claimed in one or more of claims 1 to 5.

7. A pharmaceutical comprising one or more of the compounds as claimed in one or more of claims 1 to 6.

8. A pharmaceutical comprising one or more of the compounds as claimed in one or more of claims 1 to 6 and one or more slimming agents.

9. A compound as claimed in one or more of claims 1 to 6 for use as a medicament for the treatment of obesity.

10. A compound as claimed in one or more of claims 1 to 6 for use as a medicament for the prophylaxis or treatment of type II diabetes.

11. A process for the production of a pharmaceutical comprising one or more of the compounds as claimed in one or more of claims 1 to 6, which comprises mixing the active compound with a pharmaceutically suitable vehicle and bringing this mixture into a form suitable for administration.

12. The use of the compounds as claimed in one or more of claims 1 to 6 for the production of a medicament for the treatment of obesity.

13. The use of the compounds as claimed in one or more of claims 1 to 6 for the production of a medicament for the prophylaxis or treatment of type II diabetes.

## Revendications

1. Thiazolidin-2-ylidèneamines polycycliques de formule I, 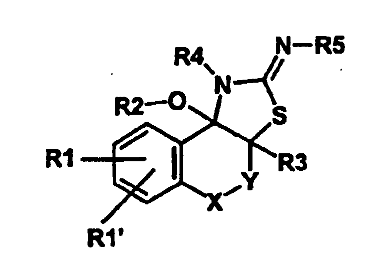 dans laquelle
A)
Y signifie une liaison directe, -CH₂-, -CH₂-CH₂ ;
X signifie CH₂, CH(CH₃), CH(C₂H₅), CH(C₃H₇) ;
R1 signifie CN, COOH, COO(alkyle en C₁ à C₆), CONH₂, CONH(alkyle en C₁ à C₆), CON (alkyle en C₁ à C₆)₂, alkyle en C₂ à C₆ ; alcényle en C₂ à C₆ ; alcynyle en C₂ à C₆ ; O-CH₂-CF₃, O-CH₂-CF₂-CF₃, 0-(alkyle en C₄ à C₆), où, dans les radicaux alkyle, un, plusieurs ou tous les hydrogènes peuvent être remplacés par fluor ou un hydrogène peut être remplacé par OH, OC(O)CH₃, O-CH₂-Ph, NH₂ ou N(COOCH₂Ph)₂ ;
S-(alkyle en C₁ à C₆), S-(CH₂)ₙ-phényle, SO-(alkyle en C₁ à C₆), SO- (CH₂)ₙ-phényle, SO₂-(alkyle en C₁ à C₆), SO₂-(CH₂)ₙ-phényle, où n peut être égal à 0 - 6 et le radical phényle peut être jusqu'à disubstitué par F, Cl, Br, OH, CF₃, NO₂, CN, OCF₃, O-(alkyle en C₁ à C₆), alkyle en C₁ à C₆, NH₂ ; NH₂, NH-(alkyle en C₁ à C₆), N(alkyle en C₁ à C₆)₂, NH(acyle en C₁ à C₇), phényle, biphényle, O-(CH₂)ₙ-phényle, où n peut être égal à 0 - 6, 1-naphtyle ou 2-naphtyle, 2-pyridyle, 3-pyridyle ou 4-pyridyle, 2-furannyle ou 3-furannyle ou 2-thiényle ou 3-thiényle, où les cycles phényle, biphényle, naphtyle, pyridyle, furannyle ou thiényle peuvent être à chaque fois monosubstitués à trisubstitués par F, Cl, Br, I, OH, CF₃, NO₂, CN, OCF₃, O-(alkyle en C₁ à C₆), alkyle en C₁ à C₆, NH₂, NH(alkyle en C₁ à C₆), N(alkyle en C₁ à C₆)₂, SO₂-CH₃, COOH, COO-(alkyle en C₁ à C₆), CONH₂ ;
1,2,3-triazol-5-yle, où le cycle triazole peut être substitué en 1ère, 2ème ou 3ème position par méthyle ou benzyle ;
tétrazol-5-yle, où le cycle tétrazole peut être substitué en 1ère ou 2ème position par méthyle ou benzyle ;
R1' signifie H, F, Cl, Br, I, CH₃, CF₃, O-(alkyle en C₁ à C₃), NO₂, SO₂-NH₂, SO₂NH(alkyle en C₁ à C₆), SO₂N(alkyle en C₁ à C₆)₂ ou R1 ;
R2 signifie H, alkyle en C₁ à C₆, cycloalkyle en C₃ à C₆, (CH₂)ₙ-phényle, (CH₂)ₙ-thiényle, (CH₂)ₙ-pyridyle, (CH₂)ₙ-furyle, C(O)-(CH₂)ₙ-phényle, C(O)-(CH₂)ₙ-thiényle, C(O)-(CH₂)ₙ-pyridyle, C(O)-(CH₂)ₙ-furyle, où n peut être égal à 0 - 5 et où phényle, thiényle, pyridyle, furyle peuvent être à chaque fois jusqu'à disubstitués par Cl, F, CN, CF₃, alkyle en C₁ à C₃, OH, O-(alkyle en C₁ à C₆) ; C(O)-(alkyle en C₁ à C₆), C(O)-(cycloalkyle en C₃ à C₆) ;
R3 signifie H, alkyle en C₁ à C₆, F, CN, N₃, 0-(alkyle en C₁ à C₆), (CH₂)ₙ-phényle, CH₂-COO(alkyle en C₁ à C₆), CH₂-COO(cycloalkyle en C₃ à C₈), CH₂-COOH, CH₂-CONH₂, CH₂-CONHCH₃, CH₂-CON(CH₃)₂ (CH₂)ₙ-thiényle, (CH₂)ₙ-pyridyle, (CH₂)ₙ-furyle, où n peut être égal à 0 - 5 et où phényle, thiényle, pyridyle, furyle peuvent être jusqu'à disubstitués par Cl, F, CN, CF₃, alkyle en C₁ à C₃, OH, O-(alkyle en C₁ à C₆) ; alcynyle en C₂ à C₆, alcényle en C₂ à C₆, C(O)OCH₃, C(O)OCH₂CH₃, C(O)OH, C(O)NH₂, C(O)NHCH₃, C(O)N(CH₃)₂, OC(O)CH₃ ;
R4 signifie alkyle en C₁ à C₆, cycloalkyle en C₃ à C₆, (CH₂)ₙ-phényle, (CH₂)ₙ-thiényle, (CH₂)ₙ-pyridyle, (CH₂)ₙ-furyle, où n peut être égal à 0 - 5 et où phényle, thiényle, pyridyle, furyle peuvent être à chaque fois jusqu'à disubstitués par Cl, F, CN, CF₃, alkyle en C₁ à C₃, OH, O-(alkyle en C₁ à C₆) ;
R5 signifie alkyle en C₁ à C₆, cycloalkyle en C₃ à C₆, (CH₂)ₙ-phényle, (CH₂)ₙ-thiényle, (CH₂)ₙ-pyridyle, (CH₂)ₙ-furyle, où n peut être égal à 0 - 5 et où phényle, thiényle, pyridyle, furyle peuvent être à chaque fois jusqu'à disubstitués par Cl, F, CN, CF₃, alkyle en C₁ à C₃, OH, 0-(alkyle en C₁ à C₆) ;
ou
R4 et R5 forment ensemble un groupe -CH₂-CH₂-, -CH₂-C(CH₃)₂-, -CH₂-CH₂-CH₂- ou -CH₂-CH₂-CH₂-CH₂-;
ou
B)
Y signifie une liaison directe, -CH₂- ou -CH₂-CH₂- ;
X signifie CH₂, CH(CH₃), CH(C₂H₅), CH(C₃H₇) ;
R1 et R1' signifient, indépendamment l'un de l'autre,
H, F, Cl, Br, I, NO₂, CN, COOH, COO(alkyle en C₁ à C₆), CONH₂, CONH(alkyle en C₁ à C₆), CON(alkyle en C₁ à C₆)₂, alkyle en C₁ à C₆, alcényle en C₂ à C₆, alcynyle en C₂ à C₆, O-(alkyle en C₁ à C₆), O-CH₂-CF₃, O-CH₂-CF₂-CF₃, O-(alkyle en C₄ à C₆), où dans les radicaux alkyle un, plusieurs ou tous les hydrogènes peuvent être remplacés par fluor ou un hydrogène peut être remplacé par OH, OC(O)CH₃, O-CH₂-Ph, NH₂ ou N(COOCH₂Ph)₂ ;
S-(alkyle en C₁ à C₆), S-(CH₂)ₙ-phényle, SO-(alkyle en C₁ à C₆). SO-(CH₂)ₙ-phényle, SO₂-(alkyle en C₁ à C₆), SO₂-(CH₂)ₙ-phényle, où n peut être égal à 0 - 6 et le radical phényle peut être jusqu'à disubstitué par F, Cl, Br, OH, CF₃, NO₂, CN, OCF₃, O-(alkyle en C₁ à C₆), alkyle en C₁ à C₆, NH₂ ; SO₂-NH₂, SO₂NH(alkyle en C₁ à C₆), SO₂N(alkyle en C₁ à C₆)₂, NH₂, NH-(alkyle en C₁ à C₆), N(alkyle en C₁ à C₆)₂, NH(acyle en C₁ à C₇), phényle, biphényle, 0-(CH₂)ₙ-phényle, où n peut être égal à 0 - 6, 1-naphtyle ou 2-naphtyle, 2-pyridyle, 3-pyridyle ou 4-pyridyle, 2-furannyle ou 3-furannyle ou 2-thiényle ou 3-thiényle, où les cycles phényle, biphényle, naphtyle, pyridyle, furannyle ou thiényle peuvent être à chaque fois monosubstitués à trisubstitués par F, Cl, Br, I, OH, CF₃, NO₂, CN, OCF₃, O-(alkyle en C₁ à C₆), alkyle en C₁ à C₆, NH₂, NH(alkyle en C₁ à C₆), N(alkyle en C₁ à C₆)₂, SO₂-CH₃, COOH, COO-(alkyle en C₁ à C₆), CONH₂ ;
1,2,3-triazol-5-yle, où le cycle triazole peut être substitué en 1ère, 2ème ou 3ème position par méthyle ou benzyle ;
tétrazol-5-yle, où le cycle tétrazole peut être substitué en 1ère ou 2ème position par méthyle ou benzyle ;
R2 signifie H, alkyle en C₁ à C₆, cycloalkyle en C₃ à C₆, (CH₂)ₙ-phényle, (CH₂)ₙ-thiényle, (CH₂)ₙ-pyridyle, (CH₂)ₙ-furyle, C(O)-(CH₂)ₙ-phényle, C(O)-(CH₂)ₙ- thiényle, C(O)-(CH₂)ₙ-pyridyle, C(O)-(CH₂)ₙ-furyle, où n peut être égal à 0 - 5 et où phényle, thiényle, pyridyle, furyle peuvent être à chaque fois jusqu'à disubstitués par Cl, F, CN, CF₃, alkyle en C₁ à C₃, OH, O-(alkyle en C₁ à C₆) ; C(O)-(alkyle en C₁ à C₆), C(O)-cycloalkyle en C₃ à C₆ ;
R3 signifie alkyle en C₄ à C₆, F, CN, N₃, 0-(alkyle en C₁ à C₆), CH₂-COO (alkyle en C₁ à C₆), CH₂-COO(cycloalkyle en C₃ à C₈), CH₂-COOH, CH₂-CONH₂, CH₂-CONHCH₃, CH₂-CON(CH₃)₂, (CH₂)ₙ-phényle, (CH₂)ₙ-thiényle, (CH₂)ₙ-pyridyle, (CH₂)ₙ-furyle, où n peut être égal à 0 - 5 et où phényle, thiényle, pyridyle, furyle peuvent être à chaque fois jusqu'à disubstitués par Cl, F, CN, CF₃, alkyle en C₁ à C₃, OH, O-(alkyle en C₁ à C₆) ; alcynyle en C₂ à C₆, alcényle en C₂ à C₆, C(O)OCH₃, C(O)OCH₂CH₃, C(O)OH, C(O)NH₂, C(O)NHCH₃, C(O)N(CH₃)₂, OC(O)CH₃ ;
R4 signifie alkyle en C₁ à C₆, cycloalkyle en C₃ à C₆, (CH₂)ₙ-phényle, (CH₂)ₙ-thiényle, (CH₂)ₙ-pyridyle, (CH₂)ₙ-furyle, où n peut être égal à 0 - 5 et où phényle, thiényle, pyridyle, furyle peuvent être à chaque fois jusqu'à disubstitués par Cl, F, CN, CF₃, alkyle en C₁ à C₃, OH, O-(alkyle en C₁ à C₆) ;
R5 signifie alkyle en C₁ à C₆, cycloalkyle en C₃ à C₆, (CH₂)ₙ-phényle, (CH₂)ₙ-thiényle, (CH₂)ₙ-pyridyle, (CH₂)ₙ-furyle, où n peut être égal à 0 - 5 et où phényle, thiényle, pyridyle, furyle peuvent être à chaque fois jusqu'à disubstitués par Cl, F, CN, CF₃, alkyle en C₁ à C₃, OH, O-(alkyle en C₁ à C₆) ;
ou
R4 et R5 forment ensemble un groupe -CH₂-CH₂-, -CH₂-C(CH₃)₂-, -CH₂-CH₂-CH₂- ou -CH₂-CH₂-CH₂-CH₂- ;
ou
C)
Y signifie une liaison directe, -CH₂- ou -CH₂-CH₂-;
X signifie CH₂, CH(CH₃), CH(C₂H₅), CH(C₃H₇) ;
R1 et R1' signifient, indépendamment l'un de l'autre,
H, F, Cl, Br, I, NO₂, CN, COOH, COO(alkyle en C₁ à C₆), CONH₂, CONH(alkyle en C₁ à C₆), CON(alkyle en C₁ à C₆)₂, alkyle en C₁ à C₆, alcényle en C₂ à C₆, alcynyle en C₂ à C₆, O-(alkyle en C₁ à C₆), O-CH₂-CF₃, O-CH₂-CF₂-CF₃, O-(alkyle en C₄ à C₆), où dans les radicaux alkyle un, plusieurs ou tous les hydrogènes peuvent être remplacés par fluor ou un hydrogène peut être remplacé par OH, OC(O)CH₃, O-CH₂-Ph, NH₂ ou N(COOCH₂Ph)₂ ;
S-(alkyle en C₁ à C₆), S-(CH₂)ₙ-phényle, SO-(alkyle en C₁ à C₆), SO-(CH₂)ₙ-phényle, SO₂-(alkyle en C₁ à C₆), SO₂-(CH₂)ₙ-phényle, où n peut être égal à 0 - 6 et le radical phényle peut être jusqu'à disubstitué par F, Cl, Br, OH, CF₃, NO₂, CN, OCF₃, O-(alkyle en C₁ à C₆), alkyle en C₁ à C₆, NH₂ ;
SO₂-NH₂, SO₂NH(alkyle en C₁ à C₆), SO₂N(alkyle en C₁ à C₆)₂, NH₂, NH-(alkyle en C₁ à C₆), N(alkyle en C₁ à C₆)₂, NH(acyle en C₁ à C₇), phényle, biphényle, O-(CH₂)ₙ-phényle, où n peut être égal à 0 - 6, 1-naphtyle ou 2-naphtyle, 2-pyridyle, 3-pyridyle ou 4-pyridyle, 2-furannyle ou 3-furannyle ou 2-thiényle ou 3-thiényle, où les cycles phényle, biphényle, naphtyle, pyridyle, furannyle ou thiényle peuvent être à chaque fois monosubstitués à trisubstitués par F, Cl, Br, I, OH, CF₃, NO₂, CN, OCF₃, O-(alkyle en C₁ à C₆), alkyle en C₁ à C₆, NH₂, NH(alkyle en C₁ à C₆), N(alkyle en C₁ à C₆)₂, SO₂-CH₃, COOH, COO- (alkyle en C₁ à C₆), CONH₂ ;
1,2,3-triazol-5-yle, où le cycle triazole peut être substitué en 1ère, 2ème ou 3ème position par méthyle ou benzyle ;
tétrazol-5-yle, où le cycle tétrazole peut être substitué en 1ère ou 2ème position par méthyle ou benzyle ;
R2 signifie alkyle en C₁ à C₆, cycloalkyle en C₃ à C₆, alcényle en C₂ à C₈, alcynyle en C₂ à C₈, (CH₂)ₙ-phényle, (CH₂)ₙ-thiényle, (CH₂)ₙ-pyridyle, (CH₂)ₙ-furyle, C(O)-(CH₂)ₙ-phényle, C(O)-(CH₂)ₙ-thiényle, C(O)-(CH₂)ₙ-pyridyle, C(O)-(CH₂)ₙ-furyle, où n peut être égal à 0 - 5 et où phényle, thiényle, pyridyle, furyle peuvent être à chaque fois jusqu'à disubstitués par Cl, F, CN, CF₃, alkyle en C₁ à C₃, OH, O-(alkyle en C₁ à C₆) ; C(O)-(alkyle en C₁ à C₆), C(O)-cycloalkyle en C₃ à C₆ ;
R3 signifie H, alkyle en C₁ à C₆, F, CN, N₃, O-(alkyle en C₁ à C₆), CH₂-COO (alkyle en C₁ à C₆), CH₂-COO (cycloalkyle en C₃ à C₈), CH₂-COOH, CH₂-CONH₂, CH₂-CONHCH₃, CH₂-CON(CH₃)₂, (CH₂)ₙ-phényle, (CH₂)ₙ-thiényle, (CH₂)ₙ-pyridyle, (CH₂)ₙ-furyle, où n peut être égal à 0 - 5 et où phényle, thiényle, pyridyle, furyle peuvent être à chaque fois jusqu'à disubstitués par Cl, F, CN, CF₃, alkyle en C₁ à C₃, OH, O-(alkyle en C₁ à C₆) ; alcynyle en C₂ à C₆ , alcényle en C₂ à C₆ , C(O)OCH₃, C(O)OCH₂CH₃, C(O)OH, C(O)NH₂, C(O)NHCH₃, C(O)N(CH₃)₂, OC(O)CH₃ ;
R4 signifie alkyle en C₁ à C₆, cycloalkyle en C₃ à C₆, (CH₂)ₙ-phényle, (CH₂)ₙ-thiényle, (CH₂)ₙ-pyridyle, (CH₂)ₙ-furyle, où n peut être égal à 0 - 5 et où phényle, thiényle, pyridyle, furyle peuvent être à chaque fois jusqu'à disubstitués par Cl, F, CN, CF₃, alkyle en C₁ à C₃, OH, O-(alkyle en C₁ à C₆) ;
R5 signifie alkyle en C₁ à C₆, cycloalkyle en C₃ à C₆, (CH₂)ₙ-phényle, (CH₂)n-thiényle, (CH₂)ₙ-pyridyle, (CH₂)ₙ-furyle, où n peut être égal à 0 - 5 et où phényle, thiényle, pyridyle, furyle peuvent être à chaque fois jusqu'à disubstitués par Cl, F, CN, CF₃, alkyle en C₁ à C₃, OH, O-(alkyle en C₁ à C₆) ;
ou
R4 et R5 forment ensemble un groupe -CH₂-CH₂-, - CH₂-C(CH₃)₂-, -CH₂-CH₂-CH₂- ou -CH₂-CH₂-CH₂-CH₂- ;
ou
D)
Y signifie une liaison directe, -CH₂- ou -CH₂-CH₂- ;
X signifie CH(phényle), où le radical phényle peut être substitué par F, Cl, Br ou I ; O, S, SO, SO₂ ou N-R6 ;
R1 et R1' signifient, indépendamment l'un de l'autre,
H, F, Cl, Br, I, NO₂, CN, COOH, COO(alkyle en C₁ à C₆), CONH₂, CONH(alkyle en C₁ à C₆), CON(alkyle en C₁ à C₆)₂, alkyle en C₁ à C₆, alcényle en C₂ à C₆, alcynyle en C₂ à C₆, O-(alkyle en C₁ à C₆), O-CH₂-CF₃, O-CH₂-CF₂-CF₃, O-(alkyle en C₄ à C₆), où dans les radicaux alkyle un, plusieurs ou tous les hydrogènes peuvent être remplacés par fluor ou un hydrogène peut être remplacé par OH, OC(O)CH₃, O-CH₂-Ph, NH₂ ou N(COOCH₂Ph)₂ ;
S-(alkyle en C₁ à C₆), S-(CH₂)ₙ-phényle, SO-(alkyle en C₁ à C₆), SO-(CH₂)ₙ-phényle, SO₂-(alkyle en C₁ à C₆), SO₂-(CH₂)ₙ-phényle, où n peut être égal à 0 - 6 et le radical phényle peut être jusqu'à disubstitué par F, Cl, Br, OH, CF₃, NO₂, CN, OCF₃, O-(alkyle en C₁ à C₆), alkyle en C₁ à C₆, NH₂ ; SO₂-NH₂, SO₂NH(alkyle en C₁ à C₆), SO₂N(alkyle en C₁ à C₆)₂, NH₂, NH-(alkyle en C₁ à C₆), N(alkyle en C₁ à C₆)₂, NH(acyle en C₁ à C₇), phényle, biphényle, O-(CH₂)ₙ-phényle, où n peut être égal à 0 - 6, 1-naphtyle ou 2-naphtyle, 2-pyridyle, 3-pyridyle ou 4-pyridyle, 2-furannyle ou 3-furannyle ou 2-thiényle ou 3-thiényle, où les cycles phényle, biphényle, naphtyle, pyridyle, furannyle ou thiényle peuvent être à chaque fois monosubstitués à trisubstitués par F, Cl, Br, I, OH, CF₃, NO₂, CN, OCF₃, O-(alkyle en C₁ à C₆), alkyle en C₁ à C₆, NH₂, NH (alkyle en C₁ à C₆), N (alkyle en C₁ à C₆)₂, COOH, COO-(alkyle en C₁ à C₆), CONH₂ ;
1,2,3-triazol-5-yle, où le cycle triazole peut être substitué en 1ère, 2ème ou 3ème position par méthyle ou benzyle ;
tétrazol-5-yle, où le cycle tétrazole peut être substitué en 1ère ou 2ème position par méthyle ou benzyle ;
R2 signifie H, alkyle en C₁ à C₆, cycloalkyle en C₃ à C₆, (CH₂)ₙ-phényle, (CH₂)ₙ-thiényle, (CH₂)ₙ-pyridyle, (CH₂)ₙ-furyle, C(O)-(CH₂)ₙ-phényle, C(O)-(CH₂)ₙ-thiényle, C(O)-(CH₂)ₙ-pyridyle, C(O)-(CH₂)ₙ-furyle, où n peut être égal à 0 - 5 et où phényle, thiényle, pyridyle, furyle peuvent être à chaque fois jusqu'à disubstitués par Cl, F, CN, CF₃, alkyle en C₁ à C₃, OH, O-(alkyle en C₁ à C₆), C(O)-(alkyle en C₁ à C₆), C (O) - (cycloalkyle en C₃ à C₆) ;
R3 signifie H, alkyle en C₁ à C₆, F, CN, N₃, O-(alkyle en C₁ à C₆), CH₂-COO(alkyle en C₁ à C₆), CH₂-COO(cycloalkyle en C₃ à C₈), CH₂-COOH, CH₂-CONH₂, CH₂-CONHCH₃, CH₂-CON(CH₃)₂, (CH₂)ₙ-phényle, (CH₂)n-thiényle, (CH₂)ₙ-pyridyle, (CH₂)ₙ-furyle, où n peut être égal à 0 - 5 et où phényle, thiényle, pyridyle, furyle peuvent être à chaque fois jusqu'à disubstitués par Cl, F, CN, CF₃, alkyle en C₁ à C₃, OH, O- (alkyle en C₁ à C₆) ; alcynyle en C₂ à C₆, alcényle en C₂ à C₆, C(O)OCH₃, C(O)OCH₂CH₃, C(O)OH, C(O)NH₂, C(O)NHCH₃, C(O)N(CH₃)₂, OC(O)CH₃ ;
R4 signifie alkyle en C₁ à C₆, cycloalkyle en C₃ à C₆, (CH₂)ₙ-phényle, (CH₂)ₙ-thiényle, (CH₂)ₙ-pyridyle, (CH₂)ₙ-furyle, où n peut être égal à 0 - 5 et où phényle, thiényle, pyridyle, furyle peuvent être à chaque fois jusqu'à disubstitués par Cl, F, CN, CF₃, alkyle en C₁ à C₃, OH, O- (alkyle en C₁ à C₆) ;
R5 signifie alkyle en C₁ à C₆, cycloalkyle en C₃ à C₆, (CH₂)ₙ-phényle, (CH₂)ₙ-thiényle, (CH₂)n-pyridyle, (CH₂)ₙ-furyle, où n peut être égal à 0 - 5 et où phényle, thiényle, pyridyle, furyle peuvent être à chaque fois jusqu'à disubstitués par Cl, F, CN, CF₃, alkyle en C₁ à C₃, OH, O-(alkyle en C₁ à C₆) ;
ou
R4 et R5 forment ensemble un groupe -CH₂-CH₂-, -CH₂-C(CH₃)₂, -CH₂-CH₂-CH₂- ou -CH₂-CH₂-CH₂-CH₂- ; R6 signifie SO₂-(C₆H₄-4-CH₃)
ou
E)
Y signifie une liaison directe, -CH₂- ou -CH₂-CH₂- ;
X signifie CH₂, CH(CH₃), CH(C₂H₅), CH(C₃H₇) ;
R1 signifie H, F, Cl, Br, I, CH₃, CF₃, O-(alkyle en C₁ à C₃) ;
R1' signifie H, F, Cl, Br, I, NO₂;
R2 signifie H ;
R3 signifie H, alkyle en C₁ à C₃ ;
R4 signifie phényle, où le radical phényle peut être jusqu'à disubstitué par F, Cl, Br, I, alkyle en C₁ à C₆, cycloalkyle en C₃ à C₆, O-alkyle en C₁ à C₃, CF₃, OCF₃, O-CH₂-phényle, COOH, COO(alkyle en C₁ à C₆), COO(cycloalkyle en C₃ à C₆), CONH₂ ;
R5 signifie phényle, où le radical phényle peut être jusqu'à disubstitué par F, Cl, Br, I, alkyle en C₁ à C₆, cycloalkyle en C₃ à C₆, O-alkyle en C₁ à C₃, CF₃, OCF₃, O-CH₂-phényle, COOH, COO(alkyle en C₁ à C₆), COO(cycloalkyle en C₃ à C₆), CONH₂ ;
ainsi que leurs sels physiologiquement acceptables.

2. Composés de formule I, selon la revendication 1, **caractérisés en ce que**
A)
Y signifie une liaison directe, -CH₂ ;
X signifie CH₂, CH(CH₃), CH(C₂H₅), CH(C₃H₇) ;
R1 signifie CN, COOH, COO(alkyle en C₁ à C₆), CONH₂, CONH(alkyle en C₁ à C₆), CON(alkyle en C₁ à C₆)₂, alkyle en C₂ à C₆ ; alcényle en C₂ à C₆ ; alcynyle en C₂ à C₆ ; O-CH₂-CF₃, O-CH₂-CF₂-CF₃, 0-(alkyle en C₄ à C₆), où dans les radicaux alkyle un, plusieurs ou tous les hydrogènes peuvent être remplacés par fluor ou un hydrogène peut être remplacé par OH, OC(O)CH₃, O-CH₂-Ph, NH₂ ou N(COOCH₂Ph)₂ ;
S-(alkyle en C₁ à C₆), S-(CH₂)ₙ-phényle, SO-(alkyle en C₁ à C₆), SO-(CH₂)ₙ-phényle, où le radical phényle peut être jusqu'à disubstitué par F, Cl, Br, OH, CF₃, NO₂, CN, OCF₃, O-(alkyle en C₁ à C₆), alkyle en C₁ à C₆ ; NH-(alkyle en C₁ à C₆), N(alkyle en C₁ à C₆)₂, NH(acyle en C₁ à C₇), phényle, O-(CH₂)ₙ-phényle, où n peut être égal à 0 - 6, 1-naphtyle ou 2-naphtyle, 2-pyridyle, 3-pyridyle ou 4-pyridyle, 2-furannyle ou 3-furannyle ou 2-thiényle ou 3-thiényle, où les cycles phényle, naphtyle, pyridyle, furannyle ou thiényle peuvent être à chaque fois monosubstitués à trisubstitués par F, Cl, I, OH, CF₃ , CN, OCF₃, O-(alkyle en C₁ à C₆), alkyle en C₁ à C₆, NH₂, NH(alkyle en C₁ à C₆), N (alkyle en C₁ à C₆)₂, COOH, COO-(alkyle en C₁ à C₆), CONH₂ ;
1,2,3-triazol-5-yle, où le cycle triazole peut être substitué en 1ère, 2ème ou 3ème position par méthyle ou benzyle ;
tétrazol-5-yle, où le cycle tétrazole peut être substitué en 1ère ou 2ème position par méthyle ou benzyle ;
R1' signifie H, F, Cl, Br, I, CH₃, CF₃, O-(alkyle en C₁ à C₃), SO₂-NH₂, SO₂NH(alkyle en C₁ à C₆), SO₂N(alkyle en C₁ à C₆)₂ ou R1 ;
R2 signifie H, alkyle en C₁ à C₆, (CH₂)ₙ-phényle, (CH₂)ₙ-thiényle, (CH₂)ₙ-pyridyle, C(O)-(CH₂)ₙ-phényle, C(O)-(CH₂)ₙ-thiényle, C(O)-(CH₂)ₙ-pyridyle, où n peut être égal à 0 - 3 et où phényle, thiényle, pyridyle, furyle peuvent être à chaque fois jusqu'à disubstitués par Cl, F, CN, CF₃, alkyle en C₁ à C₃, OH, O-(alkyle en C₁ à C₆) ;
R3 signifie H, alkyle en C₁ à C₆, F, CN, O-(alkyle en C₁ à C₆), CH₂-COO (alkyle en C₁ à C₆), CH₂-COO(cycloalkyle en C₃ à C₈), CH₂-COOH, CH₂-CONH₂, CH₂-CONHCH₃, CH₂-CON(CH₃)₂, (CH₂)ₙ-phényle, (CH₂)ₙ-thiényle, (CH₂)ₙ-pyridyle, où n peut être égal à 0 - 3 et où phényle, thiényle, pyridyle peuvent être à chaque fois jusqu'à disubstitués par Cl, F, CN, CF₃, alkyle en C₁ à C₃, OH, O-(alkyle en C₁ à C₆), alcynyle en C₂ à C₆, alcényle en C₂ à C₆, C(O)OCH₃, C(O)OCH₂CH₃, C(O)OH, C(O)NH₂, C(O)N(CH₃)₂, OC(O)CH₃ ;
R4 signifie alkyle en C₁ à C₆, cycloalkyle en C₃ à C₆, (CH₂)ₙ-phényle, (CH₂)ₙ-thiényle, (CH₂)ₙ-pyridyle, où n peut être égal à 0 - 3 et où phényle, thiényle, pyridyle peuvent être à chaque fois jusqu'à disubstitués par Cl, F, CN, CF₃, alkyle en C₁ à C₃, OH, O-(alkyle en C₁ à C₆) ;
R5 signifie alkyle en C₁ à C₆, cycloalkyle en C₃ à C₆, (CH₂)n-phényle, (CH₂)ₙ-thiényle, (CH₂)n-pyridyle, où n peut être égal à 0 - 3 et où phényle, thiényle, pyridyle peuvent être à chaque fois jusqu'à disubstitués par Cl, F, CN, CF₃, alkyle en C₁ à C₃, OH, O-(alkyle en C₁ à C₆) ;
ou
R4 et R5 forment ensemble un groupe -CH₂-CH₂-, -CH₂-C(CH₃)₂- ou -CH₂-CH₂-CH₂- ;
ou
B)
Y signifie une liaison directe, -CH₂- ;
X signifie CH₂, CH(CH₃), CH(C₂H₅), CH(C₃H₇) ;
R1 et R1' signifient, indépendamment l'un de l'autre,
H, F, Cl, Br, I, CN, COOH, COO(alkyle en C₁ à C₆), CONH₂, CONH(alkyle en C₁ à C₆), CON(alkyle en C₁ à C₆)₂, alkyle en C₁ à C₆, alcényle en C₂ à C₆, alcynyle en C₂ à C₆, O-(alkyle en C₁ à C₆), O-CH₂-CF₃, O-CH₂-CF₂-CF₃, O-(alkyle en C₄ à C₆), où dans les radicaux alkyle un, plusieurs ou tous les hydrogènes peuvent être remplacés par fluor ou un hydrogène peut être remplacé par OH, OC(O)CH₃, 0-CH₂-Ph, NH₂ ou N(COOCH₂Ph)₂ ;
S-(alkyle en C₁ à C₆), S-(CH₂)ₙ-phényle, SO-(alkyle en C₁ à C₆), SO-(CH₂)ₙ-phényle, où le radical phényle peut être jusqu'à disubstitué par F, Cl, Br, OH, CF₃, NO₂, CN, OCF₃, O-(alkyle en C₁ à C₆), alkyle en C₁ à C₆ ; SO₂-NH₂, SO₂NH (alkyle en C₁ à C₆), SO₂N(alkyle en C₁ à C₆)₂, NH-(alkyle en C₁ à C₆), N(alkyle en C₁ à C₆)₂, NH(acyle en C₁ à C₇), phényle, O-(CH₂)ₙ-phényle, où n peut être égal à 0 - 6, 1-naphtyle ou 2-naphtyle, 2-pyridyle, 3-pyridyle ou 4-pyridyle, 2-furannyle ou 3-furannyle ou 2-thiényle ou 3-thiényle, où les cycles phényle, naphtyle, pyridyle, furannyle ou thiényle peuvent être à chaque fois monosubstitués à trisubstitués par F, Cl, I, OH, CF₃, CN, OCF₃, O-(alkyle en C₁ à C₆), alkyle en C₁ à C₆, NH₂, NH(alkyle en C₁ à C₆), N(alkyle en C₁ à C₆)₂, COOH, COO-(alkyle en C₁ à C₆), CONH₂ ;
1,2,3-triazol-5-yle, où le cycle triazole peut être substitué en 1ère, 2ème ou 3ème position par méthyle ou benzyle ;
tétrazol-5-yle, où le cycle tétrazole peut être substitué en 1ère ou 2ème position par méthyle ou benzyle ;
R2 signifie H, alkyle en C₁ à C₆, (CH₂)ₙ-phényle, (CH₂)ₙ-thiényle, (CH₂)ₙ-pyridyle, C(O)-(CH₂)ₙ-phényle, C(O)-(CH₂)ₙ-thiényle, C(O)-(CH₂)ₙ-pyridyle, où n peut être égal à 0 - 3 et où phényle, thiényle, pyridyle, furyle peuvent être à chaque fois jusqu'à disubstitués par Cl, F, CN, CF₃, alkyle en C₁ à C₃, OH, O-(alkyle en C₁ à C₆) ;
R3 signifie alkyle en C₄ à C₆, F, CN, N₃, O-(alkyle en C₁ à C₆), CH₂-COO(alkyle en C₁ à C₆), CH₂-COO(cycloalkyle en C₃ à C₈), CH₂-COOH, CH₂-CONH₂, CH₂-CONHCH₃, CH₂-CON(CH₃)₂, (CH₂)n-phényle, (CH₂)ₙ-thiényle, (CH₂)ₙ- pyridyle, où n peut être égal à 0 - 3 et où phényle, thiényle, pyridyle peuvent être à chaque fois jusqu'à disubstitués par Cl, F, CN, CF₃, alkyle en C₁ à C₃, OH, O-(alkyle en C₁ à C₆), alcynyle en C₂ à C₆, alcényle en C₂ à C₆, C(O)OCH₃, C(O)OCH₂CH₃, C(O)OH, C(O)NH₂, C(O)NHCH₃, C(O)N(CH₃)₂, OC(O)CH₃ ;
R4 signifie alkyle en C₁ à C₆, cycloalkyle en C₃ à C₆, (CH₂)ₙ-phényle, (CH₂)ₙ-thiényle, (CH₂)ₙ-pyridyle, où n peut être égal à 0 - 3 et où phényle, thiényle, pyridyle peuvent être à chaque fois jusqu'à disubstitués par Cl, F, CN, CF₃, alkyle en C₁ à C₃, OH, O-( alkyle en C₁ à C₆) ;
R5 signifie alkyle en C₁ à C₆, cycloalkyle en C₃ à C₆, (CH₂)ₙ-phényle, (CH₂)ₙ-thiényle, (CH₂)ₙ-pyridyle, où n peut être égal à 0 - 3 et où phényle, thiényle, pyridyle peuvent être à chaque fois jusqu'à disubstitués par Cl, F, CN, CF₃, alkyle en C₁ à C₃, OH, O-(alkyle en C₁ à C₆) ;
ou
R4 et R5 forment ensemble un groupe -CH₂-CH₂-, - CH₂-C(CH₃)₂- ou -CH₂-CH₂-CH₂- ;
ou
C)
Y signifie une liaison directe ou -CH₂- ;
X signifie CH₂, CH(CH₃), CH(C₂H₅), CH(C₃H₇) ;
R1 et R1' signifient, indépendamment l'un de l'autre,
H, F, Cl, Br, I, CN, COOH, COO(alkyle en C₁ à C₆), CONH₂, CONH(alkyle en C₁ à C₆), CON(alkyle en C₁ à C₆)₂, alkyle en C₁ à C₆, alcényle en C₂ à C₆, alcynyle en C₂ à C₆, O-(alkyle en C₁ à C₆), O-CH₂-CF₃, O-CH₂-CF₂-CF₃, O-(alkyle en C₄ à C₆), où dans les radicaux alkyle un, plusieurs ou tous les hydrogènes peuvent être remplacés par fluor ou un hydrogène peut être remplacé par OH, OC(O)CH₃, O-CH₂-Ph, NH₂ ou N(COOCH₂Ph)₂ ;
S-(alkyle en C₁ à C₆), S-(CH₂)ₙ-phényle, SO₂-(alkyle en C₁ à C₆), SO₂-(CH₂)ₙ-phényle, où n peut être égal à 0 - 6 et le radical phényle peut être jusqu'à disubstitué par F, Cl, Br, OH, CF₃, NO₂, CN, OCF₃, O-(alkyle en C₁ à C₆), alkyle en C₁ à C₆ ;
SO₂-NH₂, SO₂NH(alkyle en C₁ à C₆), SO₂N(alkyle en C₁ à C₆)₂, NH-(alkyle en C₁ à C₆), N(alkyle en C₁ à C₆)₂, NH(acyle en C₁ à C₇), phényle, O-(CH₂)ₙ-phényle, où n peut être égal à 0 - 4, 1-naphtyle ou 2-naphtyle, 2-pyridyle, 3-pyridyle ou 4-pyridyle, 2-furannyle ou 3-furannyle ou 2-thiényle ou 3-thiényle, où les cycles phényle, naphtyle, pyridyle, furannyle ou thiényle peuvent être à chaque fois monosubstitués à trisubstitués par F, Cl, I, OH, CF₃, CN, OCF₃, O-(alkyle en C₁ à C₆), alkyle en C₁ à C₆, NH₂, NH(alkyle en C₁ à C₆), N(alkyle en C₁ à C₆)₂, COOH, COO-(alkyle en C₁ à C₆), CONH₂ ;
1,2,3-triazol-5-yle, où le cycle triazole peut être substitué en 1ère, 2ème ou 3ème position par méthyle ou benzyle ;
tétrazol-5-yle, où le cycle tétrazole peut être substitué en 1ère ou 2ème position par méthyle ou benzyle ;
R2 signifie alkyle en C₁ à C₆, alcényle en C₂ à C₈, alcynyle en C₂ à C₈, (CH₂)ₙ-phényle, (CH₂)ₙ-thiényle, (CH₂)ₙ-pyridyle, C(O)-(CH₂)ₙ-phényle, C(O)-(CH₂)ₙ-thiényle, C(O)-(CH₂)ₙ-pyridyle, où n peut être égal à 0 - 3 et où phényle, thiényle, pyridyle, furyle peuvent être à chaque fois jusqu'à disubstitués par Cl, F, CN, CF₃, alkyle en C₁ à C₃, OH, O-(alkyle en C₁ à C₆) ;
R3 signifie H, alkyle en C₁ à C₆, F, CN, O-(alkyle en C₁ à C₆), CH₂-COO(alkyle en C₁ à C₆), CH₂-COO(cycloalkyle en C₃ à C₈), CH₂COOH, CH₂-CONH₂, CH₂-CONHCH₃, CH₂-CON(CH₃)₂, (CH₂)ₙ-phényle, (CH₂)ₙ-thiényle, (CH₂)ₙ-pyridyle, où n peut être égal à 0 - 3 et où phényle, thiényle, pyridyle peuvent être à chaque fois jusqu'à disubstitués par Cl, F, CN, CF₃, alkyle en C₁ à C₃, OH, O-(alkyle en C₁ à C₆), alcynyle en C₂ à C₆, alcényle en C₂ à C₆, C(O)OCH₃, C(O)OCH₂CH₃, C(O)OH, C(O)NH₂, C(O)N(CH₃)₂, OC(O)CH₃ ;
R4 signifie alkyle en C₁ à C₆, cycloalkyle en C₃ à C₆, (CH₂)ₙ-phényle, (CH₂)ₙ-thiényle, (CH₂)ₙ-pyridyle, où n peut être égal à 0 - 3 et où phényle, thiényle, pyridyle peuvent être à chaque fois jusqu'à disubstitués par Cl, F, CN, CF₃, alkyle en C₁ à C₃, OH, O-(alkyle en C₁ à C₆) ;
R5 signifie alkyle en C₁ à C₆, cycloalkyle en C₃ à C₆, (CH₂)ₙ-phényle, (CH₂)ₙ-thiényle, (CH₂)ₙ-pyridyle, où n peut être égal à 0 - 3 et où phényle, thiényle, pyridyle peuvent être à chaque fois jusqu'à disubstitués par Cl, F, CN, CF₃, alkyle en C₁ à C₃, OH, 0- (alkyle en C₁ à C₆) ;
ou
R4 et R5 forment ensemble un groupe -CH₂-CH₂-, -CH₂-C(CH₃)₂- ou -CH₂-CH₂-CH₂- ;
ou
D)
Y signifie une liaison directe ou -CH₂- ;
X signifie CH(phényle), où le radical phényle peut être substitué par F, Cl ou Br ; O, S, SO, SO₂ ou N-R6 ;
R1 et R1' signifient, indépendamment l'un de l'autre,
H, F, Cl, Br, I, CN, COOH, COO(alkyle en C₁ à C₆), CONH₂, CONH(alkyle en C₁ à C₆), CON(alkyle en C₁ à C₆)₂, alkyle en C₁ à C₆, alcényle en C₂ à C₆, alcynyle en C₂ à C₆, O-(alkyle en C₁ à C₆), O-CH₂-CF₃, O-CH₂-CF₂-CF₃, O-(alkyle en C₄ à C₆), où dans les radicaux alkyle un, plusieurs ou tous les hydrogènes peuvent être remplacés par fluor ou un hydrogène peut être remplacé par OH, OC(O)CH₃, O-CH₂-Ph, NH₂ ou N(COOCH₂Ph)₂ ; S-(alkyle en C₁ à C₆), S-(CH₂)ₙ-phényle, SO₂-(alkyle en C₁ à C₆), SO₂-(CH₂)ₙ-phényle, où n peut être égal à 0 - 6 et le radical phényle peut être jusqu'à disubstitué par F, Cl, Br, OH, CF₃, NO₂, CN, OCF₃, O-(alkyle en C₁ à C₆), alkyle en C₁ à C₆ ; SO₂-NH₂, SO₂NH(alkyle en C₁ à C₆), SO₂N(alkyle en C₁ à C₆)₂, NH₂, NH-(alkyle en C₁ à C₆), N(alkyle en C₁ à C₆)₂, NH (acyle en C₁ à C₇), phényle, O-(CH₂)ₙ-phényle, où n peut être égal à 0 - 6, 1-naphtyle ou 2-naphtyle, 2-pyridyle, 3-pyridyle ou 4-pyridyle, 2-furannyle ou 3-furannyle ou 2-thiényle ou 3-thiényle, où les cycles phényle, naphtyle, pyridyle, furannyle ou thiényle peuvent être à chaque fois monosubstitués à trisubstitués par F, Cl, I, OH, CF₃, CN, OCF₃, 0-(alkyle en C₁ à C₆), alkyle en C₁ à C₆, NH₂, NH(alkyle en C₁ à C₆), N(alkyle en C₁ à C₆)₂, COOH, COO- (alkyle en C₁ à C₆), CONH₂ ;
1,2,3-triazol-5-yle, où le cycle triazole peut être substitué en 1ère, 2ème ou 3ème position par méthyle ou benzyle ;
tétrazol-5-yle, où le cycle tétrazole peut être substitué en 1ère ou 2ème position par méthyle ou benzyle ;
R2 signifie H, alkyle en C₁ à C₆, (CH₂)ₙ-phényle, (CH₂)ₙ-thiényle, (CH₂)ₙ-pyridyle, C(O)-(CH₂)ₙ-phényle, C(O)-(CH₂)ₙ-thiényle, C(O)-(CH₂)ₙ-pyridyle, où n peut être égal à 0 - 3 et où phényle, thiényle, pyridyle, furyle peuvent être à chaque fois jusqu'à disubstitués par Cl, F, CN, CF₃, alkyle en C₁ à C₃, OH, O-(alkyle en C₁ à C₆) ;
R3 signifie H, alkyle en C₁ à C₆, F, CN, O-(alkyle en C₁ à C₆), CH₂-COO(alkyle en C₁ à C₆), CH₂-COO (cycloalkyle en C₃ à C₈), CH₂-COOH, CH₂-CONH₂, CH₂-CONHCH₃, CH₂- CON (CH₃)₂, (CH₂)ₙ-phényle, (CH₂)ₙ-thiényle, (CH₂)ₙ-pyridyle, où n peut être égal à 0 - 3 et où phényle, thiényle, pyridyle peuvent être à chaque fois jusqu'à disubstitués par Cl, F, CN, CF₃, alkyle en C₁ à C₃, OH, O-(alkyle en C₁ à C₆), alcynyle en C₂ à C₆, alcényle en C₂ à C₆, C(O)OCH₃, C(O)OCH₂CH₃, C(O)OH, C(O)NH₂, C(O)NHCH₃, C(O)N(CH₃)₂, OC(O)CH₃,
R4 signifie alkyle en C₁ à C₆, cycloalkyle en C₃ à C₆, (CH₂)ₙ-phényle, (CH₂)ₙ-thiényle, (CH₂)ₙ-pyridyle, où n peut être égal à 0 - 3 et où phényle, thiényle, pyridyle peuvent être à chaque fois jusqu'à disubstitués par Cl, F, CN, CF₃, alkyle en C₁ à C₃, OH, O-(alkyle en C₁ à C₆) ;
R5 signifie alkyle en C₁ à C₆, cycloalkyle en C₃ à C₆, (CH₂)ₙ-phényle, (CH₂)ₙ-thiényle, (CH₂)ₙ-pyridyle, où n peut être égal à 0 - 3 et où phényle, thiényle, pyridyle peuvent être à chaque fois jusqu'à disubstitués par Cl, F, CN, CF₃, alkyle en C₁ à C₃, OH, O-(alkyle en C₁ à C₆) ;
ou
R4 et R5 forment ensemble un groupe -CH₂-CH₂-, -CH₂-C(CH₃)₂- ou -CH₂-CH₂-CH₂- ;
R6 signifie SO₂-(C₆H₄-4-CH₃)
ou
E)
Y signifie une liaison directe ou -CH₂- ;
X signifie CH₂, CH(CH₃), CH(C₂H₅), CH(C₃H₇) ;
R1 signifie H, F, Cl, Br, I, CH₃, CF₃, O-(alkyle en C₁ à C₃) ;
R1' signifie H, F, Cl, Br, I ;
R2 signifie H ;
R3 signifie H, alkyle en C₁ à C₃ ;
R4 signifie phényle, où le radical phényle peut être jusqu'à disubstitué par F, Cl, Br, I, alkyle en C₁ à C₆, cycloalkyle en C₃ à C₆, O-alkyle en C₁ à C₃, CF₃, OCF₃, O-CH₂-phényle, COOH, COO(alkyle en C₁ à C₆), CONH₂ ;
R5 signifie phényle, où le radical phényle peut être jusqu'à disubstitué par F, Cl, Br, I, alkyle en C₁ à C₆, cycloalkyle en C₃ à C₆, O-(alkyle en C₁ à C₃), CF₃, OCF₃, O-CH₂-phényle, COOH, COO(alkyle en C₁ à C₆), CONH₂ ;
ainsi que leurs sels physiologiquement acceptables.

3. Composés de formule I, selon la revendication 1 ou 2, **caractérisés en ce que**
A)
Y signifie une liaison directe ;
X CH₂, CH(CH₃), CH(C₂H₅), CH(C₃H₇) ;
R1 signifie CN, COOH, COO(alkyle en C₁ à C₆), CONH₂, CON(alkyle en C₁ à C₆)₂, alkyle en C₂ à C₆ ; alcényle en C₂ à C₆ ; alcynyle en C₂ à C₆ ; O-CH₂-CF₃, O-CH₂-CF₂-CF₃, O-(alkyle en C₄ à C₆), où dans les radicaux alkyle un, plusieurs ou tous les hydrogènes peuvent être remplacés par fluor ou un hydrogène peut être remplacé par OH, OC(O)CH₃, O-CH₂-Ph, NH₂ ou N(COOCH₂Ph)₂ ;
S-(alkyle en C₁ à C₆), S-(CH₂)ₙ-phényle, SO-(alkyle en C₁ à C₆), SO-(CH₂)ₙ-phényle, où le radical phényle peut être jusqu'à disubstitué par F, Cl, Br, OH, CF₃, NO₂, CN, OCF₃, O-(alkyle en C₁ à C₆), alkyle en C₁ à C₆ ; NH- (alkyle en C₁ à C₆), N(alkyle en C₁ à C₆)₂, NH(acyle en C₁ à C₇), phényle, O-(CH₂)ₙ-phényle, où n peut être égal à 0 - 6, 1-naphtyle ou 2-naphtyle, 2-pyridyle, 3-pyridyle ou 4-pyridyle, 2-furannyle ou 3-furannyle ou 2-thiényle ou 3-thiényle, où les cycles phényle, naphtyle, pyridyle, furannyle ou thiényle peuvent à chaque fois être monosubstitués à disubstitués par F, Cl, OH, CF₃, CN, OCF₃, O-(alkyle en C₁ à C₆), alkyle en C₁ à C₆, COOH, COO- (alkyle en C₁ à C₆), CONH₂ ;
1,2,3-triazol-5-yle, où le cycle triazole peut être substitué en 1ère, 2ème ou 3ème position par méthyle ou benzyle ;
tétrazol-5-yle, où le cycle tétrazole peut être substitué en 1ère ou 2ème position par méthyle ou benzyle ;
R1' signifie H, F, Cl, CH₃, CF₃, O-(alkyle en C₁ à C₃), SO₂-NH₂, SO₂NH(alkyle en C₁ à C₆), SO₂N(alkyle en C₁ à C₆)₂ ou R1 ;
R2 signifie H, alkyle en C₁ à C₆, (CH₂)ₙ-phényle, (CH₂)ₙ-pyridyle, C(O)-(CH₂)ₙ-phényle, C(O)-(CH₂)ₙ-pyridyle, où n peut être égal à 0 - 3 et où phényle, pyridyle peuvent être à chaque fois jusqu'à disubstitués par Cl, F, CN, CF₃, alkyle en C₁ à C₃, O-(alkyle en C₁ à C₆), C(O)-(alkyle en C₁ à C₆) ;
R3 signifie H, alkyle en C₁ à C₆, F, CN, O- (alkyle en C₁ à C₆), CH₂-COO(alkyle en C₁ à C₆), CH₂-COO(cycloalkyle en C₃ à C₈), CH₂-COOH, CH₂-CONH₂, CH₂-CONHCH₃, CH₂-CON(CH₃)₂, (CH₂)ₙ-phényle, (CH₂)ₙ-pyridyle, où n peut être égal à 0 - 3 et où phényle, pyridyle peuvent être à chaque fois jusqu'à disubstitués par Cl, F, CN, CF₃, alkyle en C₁ à C₃, O-(alkyle en C₁ à C₆) ; alcynyle en C₂ à C₆, C(O)OCH₃, C(O)OCH₂CH₃, C(O)OH, C(O)NH₂, C(O)N(CH₃)₂, OC(O)CH₃ ;
R4 signifie alkyle en C₁ à C₆, cycloalkyle en C₃ à C₆, (CH₂)ₙ-phényle, (CH₂)ₙ-pyridyle, où n peut être égal à 0 - 3 et où phényle, pyridyle peuvent être à chaque fois jusqu'à disubstitués par Cl, F, CN, CF₃, alkyle en C₁ à C₃, O-(alkyle en C₁ à C₆) ;
R5 signifie alkyle en C₁ à C₆, cycloalkyle en C₃ à C₆, (CH₂)ₙ-phényle, (CH₂)ₙ-pyridyle, où n peut être égal à 0 - 3 et où phényle, pyridyle peuvent être à chaque fois jusqu'à disubstitués par Cl, F, CN, CF₃, alkyle en C₁ à C₃, O-(alkyle en C₁ à C₆) ;
ou
R4 et R5 forment ensemble un groupe -CH₂-CH₂-, -CH₂-C(CH₃)₂- ou -CH₂-CH₂-CH₂- ;
ou
B)
Y signifie une liaison directe, -CH₂- ;
X signifie CH₂, CH(CH₃), CH(C₂H₅), CH(C₃H₇) ;
R1 et R1' signifient, indépendamment l'un de l'autre,
H, F, Cl, Br, I, CN, COOH, COO(alkyle en C₁ à C₆), CONH₂, CON(alkyle en C₁ à C₆)₂, alkyle en C₁ à C₆, alcényle en C₂ à C₆, alcynyle en C₂ à C₆, O-(alkyle en C₁ à C₆), O-CH₂-CF₃, O-CH₂-CF₂-CF₃, O-(alkyle en C₄ à C₆), où dans les radicaux alkyle un, plusieurs ou tous les hydrogènes peuvent être remplacés par fluor ou un hydrogène peut être remplacé par OH, OC(O)CH₃, O-CH₂-Ph, NH₂ ou N(COOCH₂Ph)₂ ;
S-(alkyle en C₁ à C₆), S-(CH₂)ₙ-phényle, SO-(alkyle en C₁ à C₆), SO-(CH₂)ₙ-phényle, le radical phényle peut être jusqu'à disubstitué par F, Cl, OH, CF₃, OCF₃, O-(alkyle en C₁ à C₆), alkyle en C₁ à C₆; SO₂-NH₂, SO₂NH(alkyle en C₁ à C₆), SO₂N(alkyle en C₁ à C₆)₂, N(alkyle en C₁ à C₆)₂, NH (acyle en C₁ à C₇), phényle, O-(CH₂)ₙ-phényle, où n peut être égal à 0 - 6, 1-naphtyle ou 2-naphtyle, 2-pyridyle, 3-pyridyle ou 4-pyridyle, 2-furannyle ou 3-furannyle ou 2-thiényle ou 3-thiényle, où les cycles phényle, naphtyle, pyridyle, furannyle ou thiényle peuvent être à chaque fois monosubstitués à trisubstitués par F, Cl, I, OH, CF₃, CN, OCF₃, O-(alkyle en C₁ à C₆), alkyle en C₁ à C₆, NH₂, N(alkyle en C₁ à C₆)₂, COOH, COO- (alkyle en C₁ à C₆), CONH₂ ;
1,2,3-triazol-5-yle, où le cycle triazole peut être substitué en 1ère, 2ème ou 3ème position par méthyle ou benzyle ;
tétrazol-5-yle, où le cycle tétrazole peut être substitué en 1ère ou 2ème position par méthyle ou benzyle ;
R2 signifie H, alkyle en C₁ à C₆, (CH₂)ₙ-phényle, (CH₂)ₙ-pyridyle, C(O)-(CH₂)ₙ-phényle, C(O)-(CH₂)ₙ-pyridyle, où n peut être égal à 0 - 3 et où phényle, pyridyle peuvent être à chaque fois jusqu'à disubstitués par Cl, F, CN, CF₃, alkyle en C₁ à C₃, OH, O-(alkyle en C₁ à C₆), C(O)-alkyle en C₁ à C₆ ;
R3 signifie alkyle en C₄ à C₆, F, CN, N₃, 0-(alkyle en C₁ à C₆), CH₂-COO(alkyle en C₁ à C₆), CH₂-COO(cycloalkyle en C₃ à C₈), CH₂-COOH, CH₂-CONH₂, CH₂-CONHCH₃, CH₂-CON(CH₃)₂, (CH₂)ₙ-phényle, (CH₂)ₙ-pyridyle, où n peut être égal à 0 - 3 et où phényle, pyridyle peuvent être à chaque fois jusqu'à disubstitués par Cl, F, CN, CF₃, alkyle en C₁ à C₃, O-(alkyle en C₁ à C₆), alcynyle en C₂ à C₆, C(O)OCH₃, C(O)OCH₂CH₃, C(O)OH, C(O)NH₂, C(O)NHCH₃, C(O)N(CH₃)₂, OC(O)CH₃ ;
R4 signifie alkyle en C₁ à C₆, cycloalkyle en C₃ à C₆, (CH₂)ₙ-phényle, (CH₂)ₙ-pyridyle, où n peut être égal à 0 - 3 et où phényle, pyridyle peuvent être à chaque fois jusqu'à disubstitués par Cl, F, CN, CF₃, alkyle en C₁ à C₃, O-(alkyle en C₁ à C₆) ;
R5 signifie alkyle en C₁ à C₆, cycloalkyle en C₃ à C₆, (CH₂)ₙ-phényle, (CH₂)ₙ-pyridyle, où n peut être égal à 0 - 3 et où phényle, pyridyle peuvent être à chaque fois jusqu'à disubstitués par Cl, F, CN, CF₃, alkyle en C₁ à C₃, O-(alkyle en C₁ à C₆) ;
ou
R4 et R5 forment ensemble un groupe -CH₂-CH₂-, -CH₂-C(CH₃)₂- ou -CH₂-CH₂-CH₂- ;
ou
C)
Y signifie une liaison directe ou -CH₂- ;
X signifie CH₂, CH(CH₃), CH(C₂H₅), CH(C₃H₇) ;
R1 et R1' signifient, indépendamment l'un de l'autre,
H, F, Cl, Br, I, CN, COOH, COO(alkyle en C₁ à C₆), CONH₂, CON(alkyle en C₁ à C₆)₂, alkyle en C₁ à C₆, alcényle en C₂ à C₆, alcynyle en C₂ à C₆, O-(alkyle en C₁ à C₆), O-CH₂-CF₃, O-CH₂-CF₂-CF₃, O-(alkyle en C₄ à C₆), où dans les radicaux alkyle un, plusieurs ou tous les hydrogènes peuvent être remplacés par fluor ou un hydrogène peut être remplacé par OH, OC(O)CH₃, O-CH₂-Ph, NH₂ ou N(COOCH₂Ph)₂ ;
S-(alkyle en C₁ à C₆), S-(CH₂)ₙ-phényle, SO₂-(alkyle en C₁ à C₆), SO₂-(CH₂)ₙ-phényle, où n peut être égal à 0 - 6 et le radical phényle peut être jusqu'à disubstitué par F, Cl, Br, OH, CF₃, NO₂, OCF₃, O-(alkyle en C₁ à C₆), alkyle en C₁ à C₆ ; SO₂-NH₂, SO₂NH(alkyle en C₁ à C₆), SO₂N(alkyle en C₁ à C₆)₂, N(alkyle en C₁ à C₆)₂, NH(acyle en C₁ à C₇), phényle, O-(CH₂)ₙ-phényle, où n peut être égal à 0 - 4, 1-naphtyle ou 2-naphtyle, 2-pyridyle, 3-pyridyle ou 4-pyridyle, 2-furannyle ou 3-furannyle ou 2-thiényle ou 3-thiényle, où les cycles phényle, naphtyle, pyridyle, furannyle ou thiényle peuvent être à chaque fois monosubstitués à trisubstitués par F, Cl, I, OH, CF₃, CN, OCF₃, O-(alkyle en C₁ à C₆), alkyle en C₁ à C₆, NH₂, NH(alkyle en C₁ à C₆), N(alkyle en C₁ à C₆)₂, COOH, COO- (alkyle en C₁ à C₆), CONH₂ ;
1,2,3-triazol-5-yle, où le cycle triazole peut être substitué en 1ère, 2ème ou 3ème position par méthyle ou benzyle ;
tétrazol-5-yle, où le cycle tétrazole peut être substitué en 1ère ou 2ème position par méthyle ou benzyle ;
R2 signifie alkyle en C₁ à C₆, alcényle en C₂ à C₈, alcynyle en C₂ à C₈, (CH₂)ₙ-phényle, (CH₂)ₙ-thiényle, (CH₂)ₙ-pyridyle, C(O)-(CH₂)ₙ-phényle, C(O)-(CH₂)ₙ-thiényle, C(O)-(CH₂)ₙ-pyridyle, où n peut être égal à 0 - 3 et où phényle, thiényle, pyridyle, furyle peuvent être à chaque fois jusqu'à disubstitués par Cl, F, CN, CF₃, alkyle en C₁ à C₃, OH, O-(alkyle en C₁ à C₆), C(O)-(alkyle en C₁ à C₆) ;
R3 signifie H, alkyle en C₁ à C₆, F, CN, O-(alkyle en C₁ à C₆), CH₂-COO(alkyle en C₁ à C₆), CH₂-COO(cycloalkyle en C₃ à C₈), CH₂-COOH, CH₂-CONH₂, CH₂-CONHCH₃, CH₂-CON(CH₃)₂, (CH₂)ₙ-phényle, (CH₂)ₙ-pyridyle, où n peut être égal à 0 - 3 et où phényle, pyridyle peuvent être à chaque fois jusqu'à disubstitués par Cl, F, CN, CF₃, alkyle en C₁ à C₃, O-(alkyle en C₁ à C₆), alcynyle en C₂ à C₆, C(O)OCH₃, C(O)OCH₂CH₃, C(O)OH, C(O)NH₂, C(O)N(CH₃)₂, OC(O)CH₃ ;
R4 signifie alkyle en C₁ à C₆, cycloalkyle en C₃ à C₆, (CH₂)ₙ-phényle, (CH₂)ₙ-pyridyle, où n peut être égal à 0 - 3 et où phényle, pyridyle peuvent être à chaque fois jusqu'à disubstitués par Cl, F, CN, CF₃, alkyle en C₁ à C₃, O-(alkyle en C₁ à C₆) ;
R5 signifie alkyle en C₁ à C₆, cycloalkyle en C₃ à C₆, (CH₂)ₙ-phényle, (CH₂)ₙ-pyridyle, où n peut être égal à 0 - 3 et où phényle, pyridyle peuvent être à chaque fois jusqu'à disubstitués par Cl, F, CN, CF₃, alkyle en C₁ à C₃, O-(alkyle en C₁ à C₆) ;
ou
R4 et R5 forment ensemble un groupe -CH₂-CH₂-, -CH₂-C(CH₃)₂- ou -CH₂-CH₂-CH₂- ;
ou
D)
Y signifie une liaison directe ou -CH₂- ;
X signifie CH(phényle), où le radical phényle peut être substitué par F ou Cl; O, S, SO₂ ou N-R6 ;
R1 et R1' signifient, indépendamment l'un de l'autre,
H, F, Cl, Br, I, CN, COOH, COO(alkyle en C₁ à C₆), CONH₂, CON (alkyle en C₁ à C₆)₂, alkyle en C₁ à C₆, alcényle en C₂ à C₆, alcynyle en C₂ à C₆, O-(alkyle en C₁ à C₆), O-CH₂-CF₃, O-CH₂-CF₂-CF₃, O-(alkyle en C₄ à C₆), où dans les radicaux alkyle un, plusieurs ou tous les hydrogènes peuvent être remplacés par fluor ou un hydrogène peut être remplacé par OH, OC(O)CH₃, O-CH₂-Ph, NH₂ ou N(COOCH₂Ph)₂ ;
S-(alkyle en C₁ à C₆), S-(CH₂)ₙ-phényle, SO₂-(alkyle en C₁ à C₆), SO₂- (CH₂)ₙ-phényle, où n peut être égal à 0 - 6 et le radical phényle peut être jusqu'à disubstitué par F, Cl, Br, OH, CF₃, NO₂, CN, OCF₃, O-(alkyle en C₁ à C₆), alkyle en C₁ à C₆ ; SO₂-NH₂, SO₂NH(alkyle en C₁ à C₆), SO₂N(alkyle en C₁ à C₆)₂, NH₂, N(alkyle en C₁ à C₆)₂, NH(acyle en C₁ à C₇), phényle, O-(CH₂)ₙ-phényle, où n peut être égal à 0 - 6, 1-naphtyle ou 2-naphtyle, 2-pyridyle, 3-pyridyle ou 4-pyridyle, 2-furannyle ou 3-furannyle ou 2-thiényle ou 3-thiényle, où les cycles phényle, naphtyle, pyridyle, furannyle ou thiényle peuvent être à chaque fois monosubstitués à trisubstitués par F, Cl, I, OH, CF₃, CN, OCF₃, 0-(alkyle en C₁ à C₆), alkyle en C₁ à C₆, NH₂, N(alkyle en C₁ à C₆)₂, COOH, COO-(alkyle en C₁ à C₆), CONH₂ ;
1,2,3-triazol-5-yle, où le cycle triazole peut être substitué en 1ère, 2ème ou 3ème position par méthyle ou benzyle ;
tétrazol-5-yle, où le cycle tétrazole peut être substitué en 1ère ou 2ème position par méthyle ou benzyle ;
R2 signifie H, alkyle en C₁ à C₆, (CH₂)ₙ-phényle, (CH₂)ₙ-thiényle, (CH₂)ₙ-pyridyle, C(O)-(CH₂)ₙ-phényle, C(O)-(CH₂)ₙ-thiényle, C(O)-(CH₂)ₙ-pyridyle, où n peut être égal à 0 - 3 et où phényle, thiényle, pyridyle, furyle peuvent être à chaque fois jusqu'à disubstitués par Cl, F, CN, CF₃, alkyle en C₁ à C₃, OH, O-(alkyle en C₁ à C₆), C(O)-alkyle en C₁ à C₆ ;
R3 signifie H, alkyle en C₁ à C₆, F, CN, O-(alkyle en C₁ à C₆), CH₂-COO (alkyle en C₁ à C₆), CH₂-COO(cycloalkyle en C₃ à C₈), CH₂-COOH, CH₂-CONH₂, CH₂-CONHCH₃, CH₂-CON(CH₃)₂, (CH₂)ₙ-phényle, (CH₂)ₙ-pyridyle, où n peut être égal à 0 - 3 et où phényle, pyridyle peuvent être à chaque fois jusqu'à disubstitués par Cl, F, CN, CF₃, alkyle en C₁ à C₃, O-(alkyle en C₁ à C₆) ; alcynyle en C₂ à C₆, C(O)OCH₃, C(O)OCH₂CH₃, C(O)OH, C(O)NH₂, C(O)NHCH₃, C(O)N(CH₃)₂, OC(O)CH₃ ;
R4 signifie alkyle en C₁ à C₆, cycloalkyle en C₃ à C₆, (CH₂)ₙ-phényle, (CH₂)ₙ-pyridyle, où n peut être égal à 0 - 3 et où phényle, pyridyle peuvent être à chaque fois jusqu'à disubstitués par Cl, F, CN, CF₃, alkyle en C₁ à C₃, O-(alkyle en C₁ à C₆) ;
R5 signifie alkyle en C₁ à C₆, cycloalkyle en C₃ à C₆, (CH₂)ₙ-phényle, (CH₂)ₙ-pyridyle, où n peut être égal à 0 - 3 et où phényle, pyridyle peuvent être à chaque fois jusqu'à disubstitués par Cl, F, CN, CF₃, alkyle en C₁ à C₃, O-(alkyle en C₁ à C₆) ;
ou
R4 et R5 forment ensemble un groupe -CH₂-CH₂-, -CH₂-C(CH₃)₂- ou -CH₂-CH₂-CH₂- ;
R6 signifie SO₂-(C₆H₄-4-CH₃)
ou
E)
Y signifie une liaison directe ou -CH₂- ;
X signifie CH₂, CH(CH₃), CH(C₂H₅), CH(C₃H₇) ;
R1 signifie H, F, Cl, CH₃, CF₃, O-(alkyle en C₁ à C₃) ;
R1' signifie H, F, Cl;
R2 signifie H ;
R3 signifie H, alkyle en C₁ à C₃ ;
R4 signifie phényle, où le radical phényle peut être jusqu'à disubstitué par F, Cl, alkyle en C₁ à C₆, cycloalkyle en C₃ à C₆, O-alkyle en C₁ à C₃, CF₃, O-CH₂-phényle, COOH, COO(alkyle en C₁ à C₆), CONH₂ ;
R5 signifie phényle, où le radical phényle peut être jusqu'à disubstitué par F, Cl, alkyle en C₁ à C₆, cycloalkyle en C₃ à C₈, O-(alkyle en C₁ à C₃), CF₃, O-CH₂-phényle, COOH, COO(alkyle en C₁ à C₆), CONH₂ ;
ainsi que leurs sels physiologiquement acceptables.

4. Composés de formule I, selon la revendication 1, 2 ou 3, **caractérisés en ce que**
Y signifie une liaison directe ;
X signifie CH₂
R1 et R1' signifient, indépendamment l'un de l'autre, H, F, Cl, CN, COOH, CONH₂, COO(alkyle en C₁ à C₃), alkyle en C₁ à C₆, alcényle en C₂ à C₆, alcynyle en C₂ à C₆, où dans les radicaux alkyle, alcényle et alcynyle un hydrogène peut être remplacé par OH, OC(O)CH₃, O-CH₂-Ph, NH₂ ou N(COOCH₂Ph)₂ ; OCF₃, OCH₂CF₃ ; O-(alkyle en C₁ à C₄), où dans les radicaux alkyle un, plusieurs ou tous les hydrogènes peuvent être remplacés par fluor ou un hydrogène peut être remplacé par OH, OC(O)CH₃, O-CH₂-Ph, NH₂ ou N(COOCH₂Ph)₂ ; SO₂-(alkyle en C₁ à C₆), SO₂-(CH₂)ₙ-phényle, où n peut être égal à 0 - 3 et le radical phényle peut être substitué par F, Cl, OH, CF₃, O-(alkyle en C₁ à C₄) ;
NH-(CO)-(alkyle en C₁ à C₃) ; (CH₂)ₙ-phényle, S-(CH₂)ₙ-phényle, O-(CH₂)ₙ-phényle, où n peut être égal à 0 - 3, 1-naphtyle ou 2-naphtyle, 2-pyridyle, 3-pyridyle ou 4-pyridyle, 2-furannyle ou 3-furannyle ou 2-thiényle ou 3-thiényle, où les cycles phényle, naphtyle, pyridyle, furannyle ou thiényle peuvent à chaque fois être substitués par F, Cl, CF₃, alkyle en C₁ à C₆, O-(alkyle en C₁ à C₆) et où dans les radicaux alkyle un hydrogène peut être remplacé par OH, OC(O)CH₃, O-CH₂-Ph, NH₂ ou N(COOCH₂Ph)₂ ;
1,2,3-triazol-5-yle, où le cycle triazole peut être substitué en 1ère, 2ème ou 3ème position par méthyle ou benzyle ;
tétrazol-5-yle, où le cycle tétrazole peut être substitué en 1ère ou 2ème position par méthyle ou benzyle ;
R2 signifie H, alkyle en C₁ à C₄, cycloalkyle en C₅ à C₆ ; (CH₂)ₙ-phényle, où n peut être égal à 0 - 3, C(O)-(alkyle en C₁ à C₄) ou C(O)-phényle ;
R3 signifie F, alkyle en C₄ à C₆, CH₂-phényle, où phényle peut être jusqu'à disubstitué par F, Cl, CF₃, O-(alkyle en C₁ à C₃), alkyle en C₁ à C₃, COOH, CO-O-(alkyle en C₁ à C₃) ou CONH₂ ;
R4 signifie alkyle en C₁ à C₆, cycloalkyle en C₃ à C₆, (CH₂)ₙ-phényle, où n peut être égal à 0 - 3 et le radical phényle peut être jusqu'à disubstitué par F, Cl, O-(alkyle en C₁ à C₄) ou OH ;
R5 signifie alkyle en C₁ à C₆, cycloalkyle en C₃ à C₆, (CH₂)ₙ-phényle, où n peut être égal à 0 - 3 et le radical phényle peut être jusqu'à disubstitué par F, Cl, O-(alkyle en C₁ à C₄) ou OH ;
ainsi que leurs sels physiologiquement acceptables.

5. Composés de formule I, selon l'une ou plusieurs des revendications 1 à 4, **caractérisés en ce que**
R1 signifie 6-Cl
R1' signifie H
R2 signifie H
R3 signifie F
R4 signifie CH₃
R5 signifie CH₃
X signifie CH₂
Y -
ainsi que leurs sels physiologiquement acceptables.

6. Chlorhydrates des composés de formule I selon l'une ou plusieurs des revendications 1 à 5.

7. Médicaments contenant un ou plusieurs des composés selon l'une ou plusieurs des revendications 1 à 6.

8. Médicament contenant un ou plusieurs des composés selon l'une ou plusieurs des revendications 1 à 6 et un ou plusieurs agents amaigrissants.

9. Composés selon l'une ou plusieurs des revendications 1 à 6 destinés à une utilisation comme médicament pour le traitement de l'obésité.

10. Composés selon l'une ou plusieurs des revendications 1 à 6 destinés à une utilisation comme médicament pour la prophylaxie ou le traitement du diabète de type II.

11. Procédé pour la préparation d'un médicament contenant un ou plusieurs des composés selon l'une ou plusieurs des revendications 1 à 6, **caractérisé en ce que** la substance active est mélangée avec un support pharmaceutiquement approprié et ce mélange est amené dans une forme appropriée pour l'administration.

12. Utilisation des composés selon l'une ou plusieurs des revendications 1 à 6 pour la préparation d'un médicament pour le traitement de l'obésité.

13. Utilisation des composés selon l'une ou plusieurs des revendications 1 à 6 pour la préparation d'un médicament pour la prophylaxie ou le traitement du diabète de type II.
